# EUROPEAN PATENT SPECIFICATION

(11) **EP 4 444 712 B1**
(45) Date of publication and mention of the grant of the patent: **01.07.2026**
(21) Application number: 22830470.5
(22) Date of filing: 05.12.2022
(51) Int. Cl.: C07D 403/14, A01N 43/60, A01N 43/653, A01N 43/713, A01P 7/02, A01P 7/04

(54) **PESTICIDALLY ACTIVE PYRIDAZINONE COMPOUNDS**
PYRIDAZINONVERBINDUNGEN MIT PESTIZIDER WIRKUNG
COMPOSÉS DE PYRIDAZINONE À ACTION PESTICIDE

(30) Priority: 10.12.2021 IN 202111057521
(43) Date of publication of application: 16.10.2024
(73) Proprietor: Syngenta Crop Protection AG, 4058 Basel (CH)
(72) Inventor: KILARU, Jagadeesh Prathap, Corlim, Ilhas Goa 403 110 (IN); PHADTE, Mangala, Corlim, Ilhas Goa 403 110 (IN); BERARDOZZI, Simone, 4332 Stein (CH); HALL, Roger Graham, 4332 Stein (CH); JEANGUENAT, André, 4332 Stein (CH); PITTERNA, Thomas, 4332 Stein (CH); WEISS, Matthias, 4332 Stein (CH); MUEHLEBACH, Michel, 4332 Stein (CH)
(74) Representative: SYNGENTA IP
(86) International application number: PCT/EP2022/084409
(87) International publication number: WO 2023/104714

(56) References cited:
- WO-A1-2021/083936
- WO-A1-2021/148639
- WO-A1-2021/177160
- WO-A1-2023/072849
- HERBERICH BRAD ET AL: "Identification of triazolopyridazinones as potent p38[alpha] inhibitors", BIOORGANIC & MEDICINAL CHEMISTRY LETTERS, ELSEVIER, AMSTERDAM NL, vol. 22, no. 2, 23 November 2011 (2011-11-23), pages 1226 - 1229, XP029121627, ISSN: 0960-894X, DOI: 10.1016/J.BMCL.2011.11.067

## Description

The present invention relates to pesticidally active pyridazinone compounds, e.g. as active ingredients, which have pesticidal activity. The invention also relates to preparation of these pyridazinone compounds, to intermediates useful in the preparation of these pyridazinone compounds, to the preparation of these intermediates, to agrochemical compositions which comprise at least one of these pyridazinone compounds, to preparation of these compositions and to the use of these pyridazinone compounds or compositions in agriculture or horticulture for controlling animal pests, including arthropods and in particular insects or representatives of the order *Acarina.* In particular, pesticidally active pyridazin-3-one compounds are disclosed.

The scope of the invention and thus of protection is defined by the appended claims. Subject-matter described in the following, not encompassed by the scope as defined by the claims, is not intended to form part of the invention. In particular, subject-matter relating to methods of treatment of the human or animal body by surgery, therapy of for diagnostic purposes are explicitly excluded from the scope of protection.

WO 2021/083936, WO 2021/148639 and WO 2021/177160 describe certain quinazoline, quinazolinone and quinoline compounds.

It has now surprisingly been found that certain novel pyridazinone compounds, in particular pyridazin-3-one compounds, have pesticidal activity.

The present invention therefore provides, in a first aspect, compounds of formula (I), as well as agrochemically acceptable salts, stereoisomers, enantiomers, tautomers and N-oxides thereof: 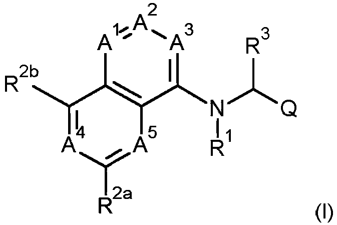 wherein:
A¹, A² and A³ are, independently from each other, N or CR^{Y}; or
A¹=A²-A³, taken together, are NR-C(=X⁰)-N; wherein X⁰ is O or S;
A⁴ and A⁵ are, independently from each other, N or CR^{Y};
Q is 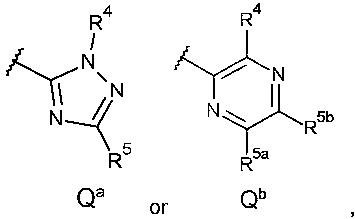 where the staggered line represents the connection of Q to the rest of compound of the formula (I);
R is hydrogen, C₁-C₃alkyl, C₁-C₃haloalkyl, C₁-C₃alkoxy, or C₁-C₃haloalkoxy;
R¹ is hydrogen, C₁-C₆alkyl, C₁-C₆cyanoalkyl, aminocarbonylC₁-C₆alkyl, hydroxycarbonylC₁-C₆alkyl, C₁-C₆nitroalkyl, trimethylsilaneC₁-C₆alkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, or C₃-C₄cycloalkylC₁-C₂alkyl- wherein the C₃-C₄cycloalkyl group is substituted with 1 or 2 halogen atoms, oxetan-3-yl-CH₂-, C₁-C₆alkylcarbonyl, C₁-C₆alkoxycarbonyl, phenyloxycarbonyl, benzyloxycarbonyl, benzyl or benzyl substituted with 1 to 3 substituents independently selected from halogen, C₁-C₆alkoxy and C₁-C₆haloalkyl;
R^{2a} and R^{2b} are each independently selected from hydrogen, C₁-C₃alkyl, C₁-C₃haloalkyl, C₁-C₃haloalkylsulfanyl, C₁-C₃alkoxy, C₁-C₃haloalkoxy, halogen, NO₂, SF₅, CN, C(O)NH₂, C(O)OH, C(S)NH₂, C₃-C₆cycloalkyl, C₃-C₆cycloalkyl substituted with one to three substituents independently selected from R^{x}, C₃-C₆cycloalkylcarbonyl, phenyl, phenyl substituted with one to three substituents independently selected from R^{x}, heteroaryl, heteroaryl substituted with one to three substituents independently selected from R^{x}, OR⁶, piperidin-2-one-1-yl, piperidin-2-one-1-yl substituted with one to two substituents independently selected from R^{x}, pyridin-2-one-1-yl, pyridin-2-one-1-yl substituted with one to two substituents independently selected from R^{x}, azetidin-1-yl, azetidin-1-yl substituted with one to two substituents independently selected from R^{x}, pyrrolidin-1-yl, pyrrolidin-1-yl substituted with one to two substituents independently selected from R^{x}, C₃-C₆cycloalkylC₁-C₄alkyl, C₃-C₆cycloalkylC₁-C₄alkyl substituted with one to two substituents independently selected from R^{z}, C₃-C₆cycloalkylC₁-C₃alkoxy, C₃-C₆cycloalkylC₁-C₃alkoxy substituted with one to two substituents independently selected from R^{x}, C₁-C₅cyanoalkyl, C₁-C₅cyanoalkoxy, C₁-C₄alkylsulfanyl, C₁-C₄alkylsulfanyl substituted with one to three substituents independently selected from R^{x}, C₁-C₄alkylsulfonyl, C₁-C₄alkylsulfonyl substituted with one to three substituents independently selected from R^{x}, C₁-C₄alkylsulfinyl, and C₁-C₄alkylsulfinyl substituted with one to three substituents independently selected from R^{x};
R³ is C₁-C₃alkyl or C₁-C₃haloalkyl;
R⁴ is 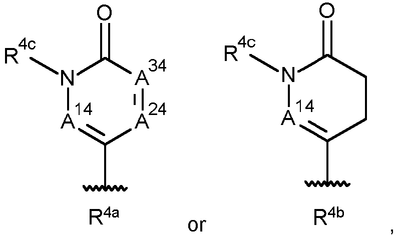 where the staggered line represents the connection of R⁴ to Q^{a} or Q^{b};
A¹⁴, A²⁴, and A³⁴ are, independently of each other, N or CH;
R^{4c} is C₁-C₃alkyl, C₁-C₃haloalkyl, allyl, propargyl, or C₃-C₆cycloalkylC₁-C₄alkyl;
R⁵ is hydrogen, C₁-C₃alkyl, C₁-C₃haloalkyl, C₃-C₄cycloalkyl, C₁-C₃alkoxy, C₃-C₄alkoxyC(O)-, (C₁-C₃alkoxy)₂CH-, halogen, CN, NH₂C(O), amino (i.e. NH₂), (C₁-C₃alkyl)amino, di(C₁-C₃alkyl)amino, hydroxy, C₃-C₄halocycloalkyl, C₃-C₄cyanocycloalkyl, C₂-C₆alkenyl, C₂-C₆haloalkenyl, C₂-C₆alkynyl, C₂-C₆haloalkynyl, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl, C₁-C₄alkylsulfonyl, C₁-C₃alkoxy-C₁-C₃alkyl, C₁-C₃alkoxy-C₁-C₃alkoxy-C₁-C₃alkyl, (C₁-C₃alkyl)sulfonylamino, (C₁-C₃alkyl)sulfonyl(C₁-C₃alkyl)amino, (C₁-C₃alkyl)NHC(O), (C₁-C₃alkyl)₂NC(O), (C₁-C₃cycloalkyl)NHC(O), (C₁-C₃cycloalkyl)(C₁-C₃alkyl)NC(O), (C₁-C₃alkyl)C(O)(C₁-C₃alkyl)N, (C₁-C₃alkyl)C(O)NH, (C₁-C₃alkyl)C(O), (C₁-C₃alkoxy)C(O), HC(O), diphenylmethanimine, C₁-C₃haloalkoxy, phenyl, or a 5-membered heteroaromatic ring; or
R⁵ is phenyl substituted with one to three substituents selected from C₁-C₃alkyl, C₁-C₃haloalkyl, C₁-C₃alkoxy, C₃-C₄cycloalkyl, halogen, CN and hydroxyl; or
R⁵ is a 5-membered heteroaromatic ring substituted with one to three substituents selected from C₁-C₃alkyl, C₁-C₃haloalkyl, C₁-C₃alkoxy, C₃-C₄cycloalkyl, halogen, CN and hydroxyl;
R^{5a} and R^{5b} are, independently of each other, selected from hydrogen, halogen, CN, C₁-C₃alkyl, C₁-C₃haloalkyl, C₃-C₄cycloalkyl, C₁-C₃alkoxy, and C₁-C₃haloalkoxy;
R⁶ is phenyl, benzyl, heteroaryl, or C₃-C₆ cycloalkyl; or
R⁶ is phenyl, benzyl, heteroaryl, or C₃-C₆ cycloalkyl, each of which, independent of each other, is substituted with one to three substituents independently selected from R^{x};
R^{x} is independently selected from halogen, C₁-C₃alkyl, C₁-C₃haloalkyl, C₁-C₃alkoxy, C₁-C₃haloalkoxy, NO₂, SF₅, CN, C(O)NH₂, C(S)NH₂, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl and C₁-C₄alkylsulfonyl;
R^{Y} is selected from hydrogen, C₁-C₃ alkyl, C₁-C₃ haloalkyl, hydroxy, C₁-C₃ alkoxy, C₁-C₃ haloalkoxy, halogen, CN and cyclopropyl; and
R^{Z} is selected from oxo, halogen, C₁-C₃ alkyl, C₁-C₃haloalkyl, C₁-C₃alkoxy, C₁-C₃haloalkoxy and CN.

The present invention also provides a method of preparation of compounds of formula (I) as well as intermediate compounds useful in the preparation of compounds of formula (I).

In a second aspect, the present invention makes available a composition comprising a compound of formula (I), one or more auxiliaries and diluent, and optionally one or more other active ingredient.

In a third aspect, the present invention makes available a method of combating and controlling insects, acarines, nematodes or molluscs, which method comprises applying to a pest, to a locus of a pest, or to a plant susceptible to attack by a pest an insecticidally, acaricidally, nematicidally or molluscicidally effective amount of a compound of formula (I) or a composition comprising such a compound.

In a fourth aspect, the present invention makes available a method for the protection of plant propagation material from the attack by insects, acarines, nematodes or molluscs, which method comprises treating the propagation material, or the site where the propagation material is planted, with an effective amount of a compound of formula (I) or a composition comprising such a compound.

In a fifth aspect, the present invention makes available a plant propagation material, such as a seed, comprising, or treated with or adhered thereto, a compound of formula (I) or a composition comprising such a compound.

The present disclosure in a further aspect provides a method of controlling parasites in or on an animal in need thereof comprising administering an effective amount of a compound of the first aspect. The present disclosure further provides a method of controlling ectoparasites on an animal in need thereof comprising administering an effective amount of a compound of formula (I) as defined in the first aspect. The present disclosure further provides a method for preventing and/or treating diseases transmitted by ectoparasites comprising administering an effective amount of a compound of formula (I) as defined in the first aspect, to an animal in need thereof.

Compounds of formula (I) which have at least one basic centre can form, for example, acid addition salts, for example with strong inorganic acids such as mineral acids, for example perchloric acid, sulfuric acid, nitric acid, nitrous acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula (I) which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

In each case, the compounds of formula (I) according to the invention are in free form, in oxidized form as a N-oxide, or in salt form, e.g. an agronomically usable salt form.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The compounds of formula (I) according to the invention also include hydrates which may be formed during the salt formation.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, 1-methylbutyl, 2-methylbutyl, 3-methylbutyl, 2, 2-dimethylpropyl, 1-ethylpropyl, n-hexyl, n-pentyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl.

The term "C₁-Cₙhaloalkyl" as used herein refers to a straight-chain or branched saturated alkyl radical attached via any of the carbon atoms having 1 to n carbon atoms (as mentioned above), where some or all of the hydrogen atoms in these radicals may be replaced by fluorine, chlorine, bromine and/or iodine, i.e., for example, any one of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. Accordingly, a term "C₁-C₂fluoroalkyl" would refer to a C₁-C₂alkyl radical which carries 1, 2, 3, 4 or 5 fluorine atoms, for example, any one of difluoromethyl, trifluoromethyl, 1-fluoroethyl, 2-fluoroethyl, 2,2-difluoroethyl, 2,2,2-trifluoroethyl, 1,1,2,2-tetrafluoroethyl or pentafluoroethyl.

The term "C₁-Cₙalkoxy" as used herein refers to a straight-chain or branched saturated alkyl radical having 1 to n carbon atoms (as mentioned above) which is attached via an oxygen atom, i.e., for example, any one of the radicals methoxy, ethoxy, n-propoxy, 1-methylethoxy, n-butoxy, 1-methylpropoxy, 2-methylpropoxy or 1,1-dimethylethoxy. The term "C₁-Cₙhaloalkoxy" as used herein refers to a C₁-Cₙalkoxy radical where one or more hydrogen atoms on the alkyl radical is replaced by the same or different halo atom(s) - examples include trifluoromethoxy, 2-fluoroethoxy, 3-fluoropropoxy, 3,3,3-trifluoropropoxy, 4-chlorobutoxy.

The term "C₁-CₙalkoxyC₁-Cₘalkyl" as used herein refers to an alkoxy radical having 1 to n carbon atoms (as mentioned above) which is attached via the oxygen atom to an alkyl radical having 1 to m carbon atoms (as mentioned above), which alkyl radical is connected to the rest of the molecule.

The term "C1-Cncyanoalkyl" as used herein refers to a straight chain or branched saturated C₁-Cₙalkyl radical having 1 to n carbon atoms (as mentioned above), where one of the hydrogen atoms in these radicals is replaced by a cyano group -CN: for example, cyanomethyl, 2-cyanoethyl, 2-cyanopropyl, 3-cyanopropyl, 1-(cyanomethyl)-2-ethyl, 1-(methyl)-2-cyanoethyl, 4-cyanobutyl, and the like.

The term "C₁-Cₙnitroalkyl" as used herein refers to a straight chain or branched saturated C₁-Cₙalkyl radical having 1 to n carbon atoms (as mentioned above), where one of the hydrogen atoms in these radicals is replaced by a nitro group -NO₂: for example, nitromethyl, 2-nitroethyl, 2-nitropropyl, 3-nitropropyl, 1-(nitromethyl)-2-ethyl, 1-(methyl)-2-nitroethyl, 4-nitrobutyl, and the like.

The term "C₃-Cₙcycloalkyl" as used herein refers to 3-n membered cycloalkyl groups such as cyclopropane, cyclobutane, cyclopentane and cyclohexane.

The term "C₃-Cₙcycloalkylcarbonyl" as used herein refers to a 3-n membered cycloalkyl group attached to a carbonyl (C=O) group, which carbonyl group is connected to the rest of the molecule. Similarly the terms "C₁-Cₙalkylcarbonyl", "C₁-Cₙalkoxycarbonyl", "phenyloxycarbonyl" and "benzyloxycarbonyl" as used herein refers to an alkyl, alkoxy, phenyloxy and benzyloxy group attached to a carbonyl (C=O) group, which carbonyl group is connected to the rest of the molecule.

The term "C₃-C₄cycloalkylC₁-C₂alkyl"" as used herein refers to 3 or 4 membered cycloalkyl group with either a methylene or ethylene group, which methylene or ethylene group is connected to the rest of the molecule. In the instance the C₃-C₄cycloalkyl-C₁-C₂alkyl group is substituted, the substituent(s) can be on the cycloalkyl group and/or on the alkyl group.

The term "C₃-C₆cycloalkylC₁-C₄haloalkoxy" as used herein refers to a 3 to 6 membered cycloalkyl group connected to a 1 to 4 membered haloalkoxy group, which haloalkoxy group is connected to the rest of the molecule.

The term "aminocarbonylC₁-Cₙalkyl" as used herein refers to an alkyl radical where one of the hydrogen atoms in the radical is replaced by CONH2 group.

The term "hydroxycarbonylC₁-Cₙalkyl" as used herein refers to an alkyl radical where one of the hydrogen atoms in the radical is replaced by COOH group.

The term "C₁-Cₙalkylsulfanyl" as used herein refers to a C₁-Cₙalkyl moiety linked through a sulfur atom. Similarly, the term "C₁-Cₙhaloalkylthio" or "C₁-Cₙhaloalkylsulfanyl" as used herein refers to a C₁-Cₙhaloalkyl moiety linked through a sulfur atom. Similarly, the term "C₃-Cₙcycloalkylsulfanyl" refers to 3-n membered cycloalkyl moiety linked through a sulfur atom.

The term "C₁-Cₙalkylsulfinyl" as used herein refers to a C₁-Cₙalkyl moiety linked through the sulfur atom of the S(=O) group. Similarly, the term "C₁-Cₙhaloalkylsulfinyl" as used herein refers to a C₁-Cₙhaloalkyl moiety linked through the sulfur atom of the S(=O) group. Similarly, the term "C₃-Cₙcycloalkylsulfinyl" refers to 3-n membered cycloalkyl moiety linked through the sulfur atom of the S(=O) group.

The term "C₁-Cₙalkylsulfonyl" as used herein refers to a C₁-Cₙalkyl moiety linked through the sulfur atom of the S(=O)₂ group. Similarly, the term "C₁-Cₙhaloalkylsulfonyl" as used herein refers to a C₁-Cₙhaloalkyl moiety linked through the sulfur atom of the S(=O)₂ group. Similarly, the term "C₃-Cₙcycloalkylsulfonyl" refers to 3-n membered cycloalkyl moiety linked through the sulfur atom of the S(=O)₂ group

The term "trimethylsilaneC₁-Cₙalkyl" as used herein refers to an alkyl radical where one of the hydrogen atoms in the radical is replaced by a -Si(CH₃)₃ group.

The term "C₂-Cₙalkenyl" as used herein refers to a straight or branched alkenyl chain having from two to n carbon atoms and one or two double bonds, for example, ethenyl, prop-1-enyl, but-2-enyl.

The term "C₂-Cₙhaloalkenyl" as used herein refers to a C₂-Cₙalkenyl moiety substituted with one or more halo atoms which may be the same or different.

The term "C₂-Cₙalkynyl" as used herein refers to a straight or branched alkynyl chain having from two to n carbon atoms and one triple bond, for example, ethynyl, prop-2-ynyl, but-3-ynyl.

The term "C₂-Cₙhaloalkynyl" as used herein refers to a C₂-Cₙalkynyl moiety substituted with one or more halo atoms which may be the same or different.

Halogen or "halo" is generally fluorine, chlorine, bromine or iodine. This also applies, correspondingly, to halogen in combination with other meanings, such as haloalkyl.

The term "heteroaryl" as used herein refers to a 5- or 6-membered aromatic monocyclic ring having 1 to 3 heteroatoms independently selected from N, O and S. Examples are heteroaryls J-1 to J-39 shown in Scheme J below. Preferred heteroaryl is pyridyl, pyrimidyl, and pyrazolyl.

### Table J: Heteroaryl J-1 to J-39:

The arrow in heteroaryls J-1 to J-39 represents the point of attachment to the rest of the compound.

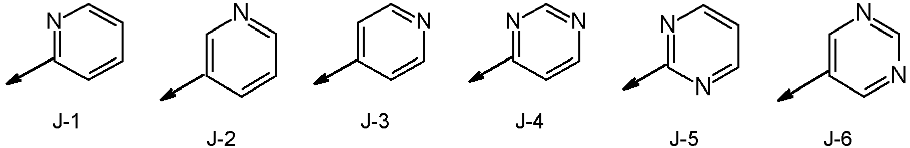

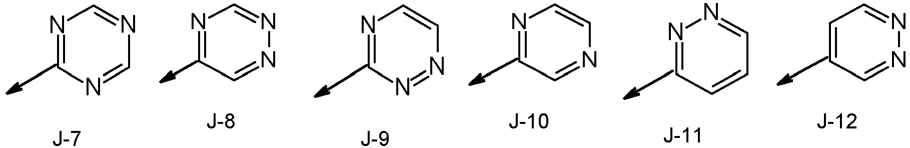

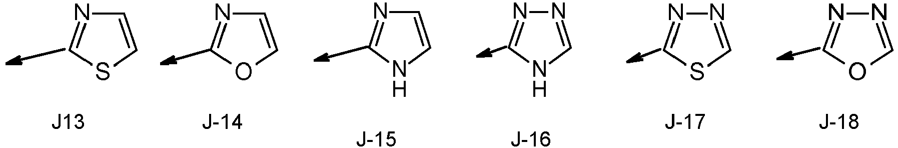

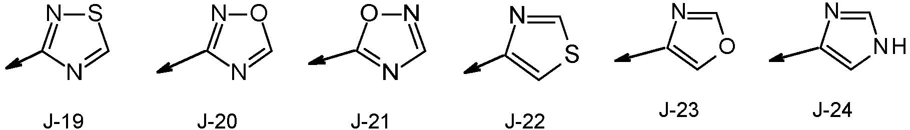

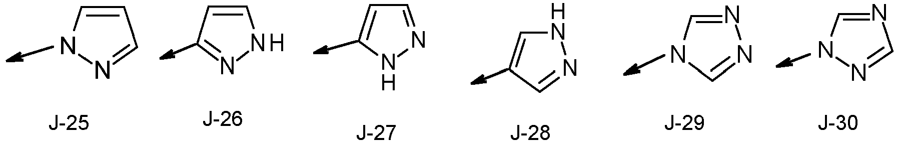

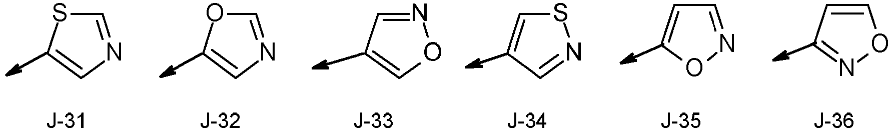

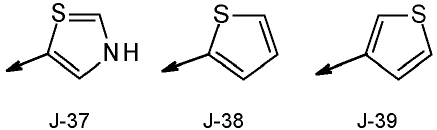

The term "optionally substituted" as used herein means that the group referenced is either unsubstituted or is substituted by a designated substituent, for example, "C₃-C₄cycloalkyl is optionally substituted with 1 or 2 halo atoms" means C₃-C₄cycloalkyl, C₃-C₄cycloalkyl substituted with 1 halo atom and C₃-C₄cycloalkyl substituted with 2 halo atoms.

The staggered line as used herein, for example, in Q^{a}, Q^{b}, R^{4a}, R^{4b}, and T, represent the point of connection / attachment to the rest of the compound.

As used herein, the term "controlling" refers to reducing the number of pests, eliminating pests and/or preventing further pest damage such that damage to a plant or to a plant derived product is reduced.

As used herein, the term "pest" refers to insects, and molluscs that are found in agriculture, horticulture, forestry, the storage of products of vegetable origin (such as fruit, grain and timber); and those pests associated with the damage of man-made structures. The term pest encompasses all stages in the life cycle of the pest.

As used herein, the term "effective amount" refers to the amount of the compound, or a salt thereof, which, upon single or multiple applications provides the desired effect.

An effective amount is readily determined by the skilled person in the art, by the use of known techniques and by observing results obtained under analogous circumstances. In determining the effective amount a number of factors are considered including, but not limited to: the type of plant or derived product to be applied; the pest to be controlled & its lifecycle; the particular compound applied; the type of application; and other relevant circumstances.

As one of ordinary skill in the art will appreciate, compounds of formula (I) contain a stereogenic centre which is indicated with an asterisk in the formula (I*) below: 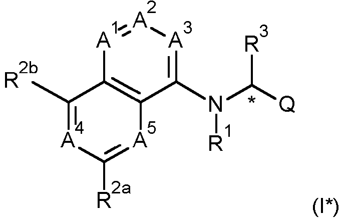 where A¹, A², A³, A⁴, A⁵, Q, R¹, R^{2a}, R^{2b}, and R³ are as defined in the first aspect.

The present invention contemplates both racemates and individual enantiomers. Compounds having preferred stereochemistry are set out below. Particularly preferred compounds of the present invention are compounds of formula (I'): 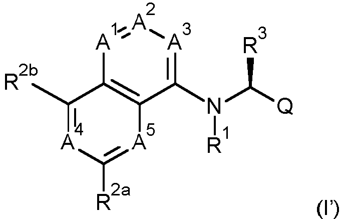 where A¹, A², A³, A⁴, A⁵, Q, R¹, R^{2a}, R^{2b}, and R³ are as defined in the first aspect, as well as stereoisomers, enantiomers, and tautomers of the compounds of formula (I'), as well as N-oxides and agrochemically acceptable salts thereof.

Embodiments according to the invention are provided as set out below.
In an embodiment of each aspect of the invention, A¹, A² and A³ are defined as follows:
A. A¹, A² and A³ are, independently from each other, N or CR^{Y}; or A¹=A²-A³, taken together, are NR-C(=X⁰)-N; wherein X⁰ is O or S;
B. A¹, A² and A³ are, independently from each other, N or CR^{Y}, with the proviso that no more than two out of the three are N; or A¹=A²-A³, taken together, are NR-C(=X⁰)-N; or
C. A¹ and A³ are N, and A² is CR^{Y}; or A¹=A²-A³, taken together, are NR-C(=O)-N; or
D. A¹, A² and A³ are, independently from each other, N or CH; or A¹=A²-A³, taken together, are NR-C(=O)-N; or
E. A¹, A² and A³ are, independently from each other, N or CH, with the proviso that no more than two out of the three are N; or A¹=A²-A³, taken together, are NH-C(=O)-N; or
F. A¹ is N, and A² and A³ are CH; or A¹=A²-A³, taken together, are NH-C(=O)-N; or
G. A¹ and A² are CH, and A³ is N; or A¹=A²-A³, taken together, are NR-C(=O)-N; or
H. A¹ and A³ are N, and A² is CH; or A¹=A²-A³, taken together, are NH-C(=O)-N; or
I. A¹, A² and A³ are, independently from each other, N or CR^{Y}, with the proviso that no more than two out of the three are N; or
J. A¹ and A³ are N, and A² is CR^{Y}; or
K. A¹, A² and A³ are, independently from each other, N or CH; or
L. A¹, A² and A³ are, independently from each other, N or CH, with the proviso that no more than two out of the three are N; or
M. A¹ is N, and A² and A³ are CH; or
N. A¹ and A² are CH, and A³ is N; or
O. A¹ and A³ are N, and A² is CH; or
P. A¹=A²-A³, taken together, are NR-C(=X⁰)-N; or
Q. A¹=A²-A³, taken together, are N(CH₃)-C(=X⁰)-N; or
R. A¹=A²-A³, taken together, are NH-C(=X⁰)-N; or
S. A¹=A²-A³, taken together, are NR-C(=O)-N; or
T. A¹=A²-A³, taken together, are N(CH₃)-C(=O)-N; or
U. A¹=A²-A³, taken together, are NH-C(=O)-N; or
V. A¹=A²-A³, taken together, are NR-C(=S)-N; or
W. A¹=A²-A³, taken together, are NH-C(=S)-N; or
X. A¹=A²-A³, taken together, are N(CH₃)-C(=S)-N.

In an embodiment of each aspect of the invention,
A. X⁰ is O or S; or
B. X⁰ is O; or
C. X⁰ is S.

In an embodiment of each aspect of the invention,
A. A⁴ is CR^{Y}, and A⁵ is N; or
B. A⁴ is CR^{Y}, and A⁵ is CH; or
C. A⁴ is CH, and A⁵ is N; or
D. A⁴ is N, and A⁵ is CH; or
E. A⁴ and A⁵ are both CH.

In an embodiment of each aspect of the invention,
A. A¹ is N, A² and A³ are CH, and A⁴ and A⁵ are both CH; or
B. A¹ and A² are CH, A³ is N, and A⁴ and A⁵ are both CH; or
C. A¹ and A³ are N, A² is CH, and A⁴ is CR^{Y} and A⁵ is CH; or
D. A¹ and A³ are N, A² is CH, and A⁴ is CH and A⁵ is N; or
E. A¹ and A³ are N, A² is CH, and A⁴ is N and A⁵ is CH; or
F. A¹ and A³ are N, A² is CH, and A⁴ is N or CH and A⁵ is CH; or
G. A¹ and A³ are N, A² is CH, and A⁴ is CH and A⁵ is N or CH; or
H. A¹ and A³ are N, A² is CH, and A⁴ and A⁵ are both N; or
I. A¹ and A³ are N, A² is CH, and A⁴ and A⁵ are both CH; or
J. A¹=A²-A³, taken together, are NR-C(=O)-N and A⁴ and A⁵ are both CH; or
K. A¹=A²-A³, taken together, are N(CH₃)-C(=O)-N and A⁴ and A⁵ are both CH; or
L. A¹=A²-A³, taken together, are NH-C(=O)-N and A⁴ and A⁵ are both CH.

In an embodiment of each aspect of the invention, R is
A. hydrogen, C₁-C₃alkyl, C₁-C₃haloalkyl, C₁-C₃alkoxy, or C₁-C₃haloalkoxy; or
B. hydrogen, methyl, ethyl, difluoroethyl or trifluoroethyl; or
C. hydrogen; methyl, ethyl, 2,2-difluoroethyl or 2,2,2,-trifluoroethyl or
D. hydrogen; or
E. methyl, or 2,2,2,-trifluoroethyl; or
F. methyl; or
G. methyl or hydrogen.

In an embodiment of each aspect of the invention, R¹ is
A. hydrogen, C₁-C₆alkyl, C₁-C₆cyanoalkyl, aminocarbonylC₁-C₆alkyl, hydroxycarbonylC₁-C₆alkyl, C₁-C₆nitroalkyl, trimethylsilaneC₁-C₆alkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, or C₃-C₄cycloalkylC₁-C₂alkyl- wherein the C₃-C₄cycloalkyl group is substituted with 1 or 2 halogen atoms, oxetan-3-yl-CH₂-, C₁-C₆alkylcarbonyl, C₁-C₆alkoxycarbonyl, phenyloxycarbonyl, benzyloxycarbonyl, benzyl or benzyl substituted with 1 to 3 substituents independently selected from halogen, C₁-C₆alkoxy and C₁-C₆haloalkyl; or
B. hydrogen, C₁-C₆alkyl, C₁-C₆cyanoalkyl, aminocarbonylC₁-C₆alkyl, hydroxycarbonylC₁-C₆alkyl, C₁-C₆nitroalkyl, trimethylsilaneC₁-C₆alkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, C₃-C₄cycloalkylC₁-C₂alkyl- wherein the C₃-C₄cycloalkyl group is substituted with 1 or 2 halogen atoms, oxetan-3-yl-CH₂-, phenyloxycarbonyl, benzyloxycarbonyl, or benzyl; or
C. hydrogen, C₁-C₆alkyl, C₁-C₆cyanoalkyl, aminocarbonylC₁-C₆alkyl, hydroxycarbonylC₁-C₆alkyl, C₁-C₆nitroalkyl, trimethylsilaneC₁-C₆alkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, benzyloxycarbonyl, or benzyl; or
D. hydrogen, C₁-C₆alkyl, C₁-C₆cyanoalkyl, aminocarbonylC₁-C₆alkyl, hydroxycarbonylC₁-C₆alkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, benzyloxycarbonyl, or benzyl; or
E. hydrogen, C₁-C₆alkyl, C₁-C₆cyanoalkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, benzyloxycarbonyl, or benzyl; or
F. hydrogen, C₁-C₃alkyl, C₁-C₃cyanoalkyl, C₁-C₃alkoxy-C₁-C₃alkyl, C₁-C₃haloalkyl, C₂-C₄alkenyl, C₂-C₄haloalkenyl, C₂-C₄alkynyl, C₂-C₄haloalkynyl, C₃-C₄cycloalkylC₁-C₂alkyl-, benzyloxycarbonyl, or benzyl; or
G. hydrogen, C₁-C₃alkyl, C₁-C₃cyanoalkyl, C₁-C₃alkoxy-C₁-C₃alkyl, C₁-C₃haloalkyl, C₂-C₄alkenyl, C₂-C₄haloalkenyl, C₂-C₄alkynyl, C₂-C₄haloalkynyl, C₃-C₄cycloalkylC₁-C₂alkyl-, benzyloxycarbonyl, or benzyl; or
H. hydrogen, methyl, ethyl, cyanomethyl, methoxymethyl, cyclopropyl-methyl, allyl, propargyl, benzyloxycarbonyl, or benzyl; or
I. hydrogen, methyl, or cyclopropyl-methyl; or
J. hydrogen, or methyl; or
K. hydrogen.

In an embodiment of each aspect of the invention, R^{2a} is
A. hydrogen, C₁-C₃alkyl, C₁-C₃haloalkyl, C₁-C₃haloalkylsulfanyl, C₁-C₃alkoxy, C₁-C₃haloalkoxy, halogen, NO₂, SF₅, CN, C(O)NH₂, C(O)OH, C(S)NH₂, C₃-C₆cycloalkyl, C₃-C₆cycloalkyl substituted with one to three substituents independently selected from R^{x}, C₃-C₆cycloalkylcarbonyl, phenyl, phenyl substituted with one to three substituents independently selected from R^{x}, heteroaryl, heteroaryl substituted with one to three substituents independently selected from R^{x}, OR⁶, piperidin-2-one-1-yl, piperidin-2-one-1-yl substituted with one to two substituents independently selected from R^{x}, pyridin-2-one-1-yl, pyridin-2-one-1-yl substituted with one to two substituents independently selected from R^{x}, azetidin-1-yl, azetidin-1-yl substituted with one to two substituents independently selected from R^{x}, pyrrolidin-1-yl, pyrrolidin-1-yl substituted with one to two substituents independently selected from R^{x}, C₃-C₆cycloalkylC₁-C₄alkyl, C₃-C₆cycloalkylC₁-C₄alkyl substituted with one to two substituents independently selected from R^{z}, C₃-C₆cycloalkylC₁-C₃alkoxy, C₃-C₆cycloalkylC₁-C₃alkoxy substituted with one to two substituents independently selected from R^{x}, C₁-C₅cyanoalkyl, C₁-C₅cyanoalkoxy, C₁-C₄alkylsulfanyl, C₁-C₄alkylsulfanyl substituted with one to three substituents independently selected from R^{x}, C₁-C₄alkylsulfonyl, C₁-C₄alkylsulfonyl substituted with one to three substituents independently selected from R^{x}, C₁-C₄alkylsulfinyl, and C₁-C₄alkylsulfinyl substituted with one to three substituents independently selected from R^{x}; or
B. halogen, C₁-C₃alkyl, C₁-C₃haloalkyl, C₃-C₄cycloalkyl, phenyl, heteroaryl selected from J-1 to J-39, each of C₃-C₄cycloalkyl, phenyl or heteroaryl, independent of each other, is substituted with one to three substituents R^{X}; OR⁶, piperidin-2-one-1-yl, pyridin-2-one-1-yl, azetidin-1-yl optionally substituted with R^{X}, pyrrolidin-1-yl, C₃-C₆cycloalkylC₁-C₄alkyl optionally substituted with R^{X}, C₃-C₆cycloalkylC₁-C₃alkoxy optionally substituted with R^{X}, C₁-C₅cyanoalkyl, C₁-C₅cyanoalkoxy, C₁-C₄alkylsulfonyl optionally substituted with R^{X}, or C₁-C₄alkylsulfinyl optionally substituted with R^{X}; or
C. halogen, C₁-C₃alkyl, C₁-C₃haloalkyl, C₃-C₄cycloalkyl, phenyl, heteroaryl selected from J-1 and J-25, each of C₃-C₄cycloalkyl, phenyl or heteroaryl, independent of each other, is substituted with one to three substituents R^{X}; OR⁶, piperidin-2-one-1-yl, pyridin-2-one-1-yl, azetidin-1-yl optionally substituted with R^{X}, pyrrolidin-1-yl, C₃-C₆cycloalkylC₁-C₄alkyl optionally substituted with R^{X}, C₃-C₆cycloalkylC₁-C₃alkoxy optionally substituted with R^{X}, C₁-C₅cyanoalkyl, C₁-C₅cyanoalkoxy, C₁-C₄alkylsulfonyl optionally substituted with R^{X}, or C₁-C₄alkylsulfinyl optionally substituted with R^{X}; or
D. halogen, C₁-C₃alkyl, C₁-C₃haloalkyl, C₃-C₄cycloalkyl, phenyl, pyrazolyl, each of C₃-C₄cycloalkyl, phenyl, pyrazolyl, independent of each other, is substituted with one to three substituents R^{X}; OR⁶, piperidin-2-one-1-yl, pyridin-2-one-1-yl, azetidin-1-yl optionally substituted with R^{X}, pyrrolidin-1-yl, C₃-C₆cycloalkylC₁-C₄alkyl optionally substituted with R^{X}, C₃-C₆cycloalkylC₁-C₃alkoxy optionally substituted with R^{X}, C₁-C₅cyanoalkyl, C₁-C₅cyanoalkoxy, C₁-C₄alkylsulfonyl optionally substituted with R^{X}, or C₁-C₄alkylsulfinyl optionally substituted with R^{X}; or
E. halogen, C₁-C₃alkyl, C₁-C₃haloalkyl, C₃-C₄cycloalkyl, phenyl or pyrazolyl, each of C₃-C₄cycloalkyl, phenyl, pyrazolyl, independent of each other, is substituted with one to two substituents R^{X}, OR⁶, azetidin-1-yl optionally substituted with R^{X}, C₃-C₆cycloalkylC₁-C₄alkyl optionally substituted with R^{X}, C₃-C₆cycloalkylC₁-C₃alkoxy optionally substituted with R^{X}, C₁-C₄alkylsulfonyl optionally substituted with R^{X}, or C₁-C₄alkylsulfinyl optionally substituted with R^{X}; or
F. halogen, C₁-C₃alkyl, C₁-C₃haloalkyl, C₃-C₄cycloalkyl, C₃-C₄cycloalkyl substituted with one to two substituents R^{X}; OR⁶, C₃-C₆cycloalkylC₁-C₄alkyl, C₃-C₆cycloalkylC₁-C₄alkyl substituted with R^{X}, C₁-C₄alkylsulfonyl, C₁-C₄alkylsulfonyl substituted with R^{X}, C₁-C₄alkylsulfinyl, or C₁-C₄alkylsulfinyl substituted with R^{X}; or
G. halogen, C₁-C₃alkyl, C₁-C₃haloalkyl, C₃-C₄cycloalkyl, C₃-C₄cycloalkyl substituted with one to two halogen, C₁-C₃alkyl, or C₁-C₃haloalkyl; C₃-C₄cycloalkylmethyl, C₃-C₄cycloalkylmethyl substituted with one to two halogen, C₁-C₃alkyl, or C₁-C₃haloalkyl; or C₁-C₂alkylsulfonyl substituted with one to three halogen; or
H. halogen, C₁-C₃alkyl, C₁-C₃haloalkyl, cyclopropyl, cyclopropyl substituted with one to two halogen, methyl, or trifluoromethyl, cyclopropylmethyl substituted with one to two halogen, or trifluoromethyl; or C₁-C₂alkylsulfonyl substituted with one to three halogen; or
I. halogen, C₁-C₃haloalkyl, cyclopropyl substituted with one to two fluorine, methyl, or trifluoromethyl, cyano, cyclopropylmethyl substituted with one to two fluorine, or trifluoromethylsulfonyl; or
J. halogen, C₁-C₃alkyl, C₁-C₃haloalkyl, C₁-C₃haloalkylthio, C₁-C₃alkoxy, C₁-C₃haloalkoxy, CN, C₃-C₆cycloalkyl, C₃-C₆cycloalkyl substituted with one to three substituents independently selected from C₁-C₃alkyl, C₁-C₃haloalkyl, cyano, and halogen; C₃-C₆cycloalkylC₁-C₄alkyl, C₃-C₆cycloalkylC₁-C₄alkyl substituted with one to five substituents independently selected from C₁-C₃alkyl, C₁-C₃haloalkyl, cyano, and halogen; C₁-C₅cyanoalkyl, C₁-C₄alkylsulfonyl, C₁-C₄haloalkylsulfonyl, C₁-C₄alkylsulfinyl, C₁-C₄haloalkylsulfinyl, C₃-C₆cycloalkylsulfanyl, C₃-C₆cycloalkylsulfinyl, or C₃-C₆cycloalkylsulfonyl; or
K. halogen, C₁-C₃alkyl, C₁-C₃haloalkyl, C₁-C₃haloalkylthio, C₁-C₃alkoxy, C₁-C₃haloalkoxy, CN, C₃-C₆cycloalkyl, C₃-C₆cycloalkyl substituted with one or two substituents independently selected from C₁-C₃haloalkyl, cyano, and halogen; C₃-C₆cycloalkylC₁-C₄alkyl, C₃-C₆cycloalkylC₁-C₄alkyl substituted with one to three substituents independently selected from C₁-C₃haloalkyl, cyano, and halogen; C₁-C₅cyanoalkyl, C₁-C₄alkylsulfonyl, C₁-C₄haloalkylsulfonyl, C₁-C₄alkylsulfinyl, C₁-C₄haloalkylsulfinyl, C₃-C₆cycloalkylsulfanyl, C₃-C₆cycloalkylsulfinyl, or C₃-C₆cycloalkylsulfonyl; or
L. halogen, C₁-C₃haloalkyl, C₁-C₃haloalkylthio, C₁-C₃haloalkoxy, C₃-C₆cycloalkyl, C₃-C₆cycloalkyl substituted with one or two substituents independently selected from C₁-C₃haloalkyl, cyano, and halogen; C₃-C₆cycloalkylC₁-C₄alkyl, C₃-C₆cycloalkylC₁-C₄alkyl substituted with one to three substituents independently selected from C₁-C₃haloalkyl, cyano, and halogen; C₁-C₅cyanoalkyl, C₁-C₄alkylsulfonyl, C₁-C₄haloalkylsulfonyl, C₁-C₄alkylsulfinyl, C₁-C₄haloalkylsulfinyl, C₃-C₆cycloalkylsulfanyl, C₃-C₆cycloalkylsulfinyl, or C₃-C₆cycloalkylsulfonyl; or
M. halogen or C₁-C₃haloalkyl; or
N. chlorine, fluorine, bromine, difluoromethyl, or trifluoromethyl; or
O. fluorine, chlorine, bromine, or trifluoromethyl; or
P. trifluoromethyl; or
Q. chlorine or trifluoromethyl;
R. chlorine, bromine, iodine, or trifluoromethyl.

In an embodiment of each aspect of the invention, R^{2b} is
A. hydrogen, C₁-C₃alkyl, C₁-C₃haloalkyl, C₁-C₃haloalkylsulfanyl, C₁-C₃alkoxy, C₁-C₃haloalkoxy, halogen, NO₂, SF₅, CN, C(O)NH₂, C(O)OH, C(S)NH₂, C₃-C₆cycloalkyl, C₃-C₆cycloalkyl substituted with one to three substituents independently selected from R^{x}, C₃-C₆cycloalkylcarbonyl, phenyl, phenyl substituted with one to three substituents independently selected from R^{x}, heteroaryl, heteroaryl substituted with one to three substituents independently selected from R^{x}, OR⁶, piperidin-2-one-1-yl, piperidin-2-one-1-yl substituted with one to two substituents independently selected from R^{x}, pyridin-2-one-1-yl, pyridin-2-one-1-yl substituted with one to two substituents independently selected from R^{x}, azetidin-1-yl, azetidin-1-yl substituted with one to two substituents independently selected from R^{x}, pyrrolidin-1-yl, pyrrolidin-1-yl substituted with one to two substituents independently selected from R^{x}, C₃-C₆cycloalkylC₁-C₄alkyl, C₃-C₆cycloalkylC₁-C₄alkyl substituted with one to two substituents independently selected from R^{z}, C₃-C₆cycloalkylC₁-C₃alkoxy, C₃-C₆cycloalkylC₁-C₃alkoxy substituted with one to two substituents independently selected from R^{x}, C₁-C₅cyanoalkyl, C₁-C₅cyanoalkoxy, C₁-C₄alkylsulfanyl, C₁-C₄alkylsulfanyl substituted with one to three substituents independently selected from R^{x}, C₁-C₄alkylsulfonyl, C₁-C₄alkylsulfonyl substituted with one to three substituents independently selected from R^{x}, C₁-C₄alkylsulfinyl, and C₁-C₄alkylsulfinyl substituted with one to three substituents independently selected from R^{x}; or
B. halogen, C₁-C₃haloalkyl, C₁-C₃haloalkylthio, C₁-C₃alkoxy, C₁-C₃haloalkoxy, or CN; or
C. halogen, C₁-C₃haloalkyl, or C₁-C₃haloalkoxy; or
D. halogen or C₁-C₃haloalkyl; or
E. chlorine, fluorine, bromine, difluoromethyl, or trifluoromethyl; or
F. fluorine, chlorine, bromine, or trifluoromethyl; or
G. trifluoromethyl; or
H. chlorine or trifluoromethyl; or
I. chlorine, bromine, iodine, or trifluoromethyl.

In an embodiment of each aspect of the invention, R³ is
A. C₁-C₃alkyl or C₁-C₃haloalkyl; or
B. methyl or trifluoromethyl; or
C. methyl.

In an embodiment of each aspect of the invention, Q is
A. 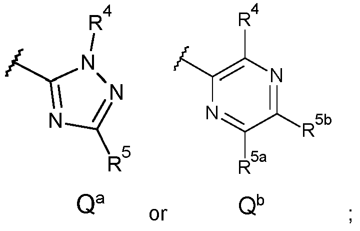 or
B. Q^{a} wherein R⁴ is R^{4a} or R^{4b}; or
C. Q^{a} wherein R⁴ is R^{4a}; or
D. Q^{a} wherein R⁴ is R^{4b}; or
E. Q^{b} wherein R⁴ is R^{4a} or R^{4b} ; or
F. Q^{b} wherein R⁴ is R^{4a}; or
G. Q^{b} wherein R⁴ is R^{4b}.

In an embodiment of each aspect of the invention, Q^{a} is
A. selected from Q^{A}-1 to Q^{A}-16; or
B. selected from Q^{A}-1, Q^{a}-6, Q^{a}-7, Q^{A}-10, and Q^{A}-15; or
C. Q^{A}-1 or Q^{A}-15; or
D. selected from Q^{A}-1 to Q^{A}-16, wherein R⁴ is R^{4a}; or
E. selected from Q^{A}-1 to Q^{A}-16, wherein R⁴ is R^{4b}; or
F. selected from Q^{A}-1, Q^{a}-6, Q^{a}-7, Q^{A}-10, and Q^{A}-15; wherein R⁴ is R^{4a}; or
G. selected from Q^{A}-1, Q^{a}-6, Q^{a}-7, Q^{A}-10, and Q^{A}-15; wherein R⁴ is R^{4b}; or
H. Q^{A}-1 or Q^{A}-15, wherein R⁴ is R^{4a}; or
I. Q^{A}-1 or Q^{A}-15, wherein R⁴ is R^{4b}; or
J. Q^{A}-1 and R⁴ is R^{4a} or R^{4b},

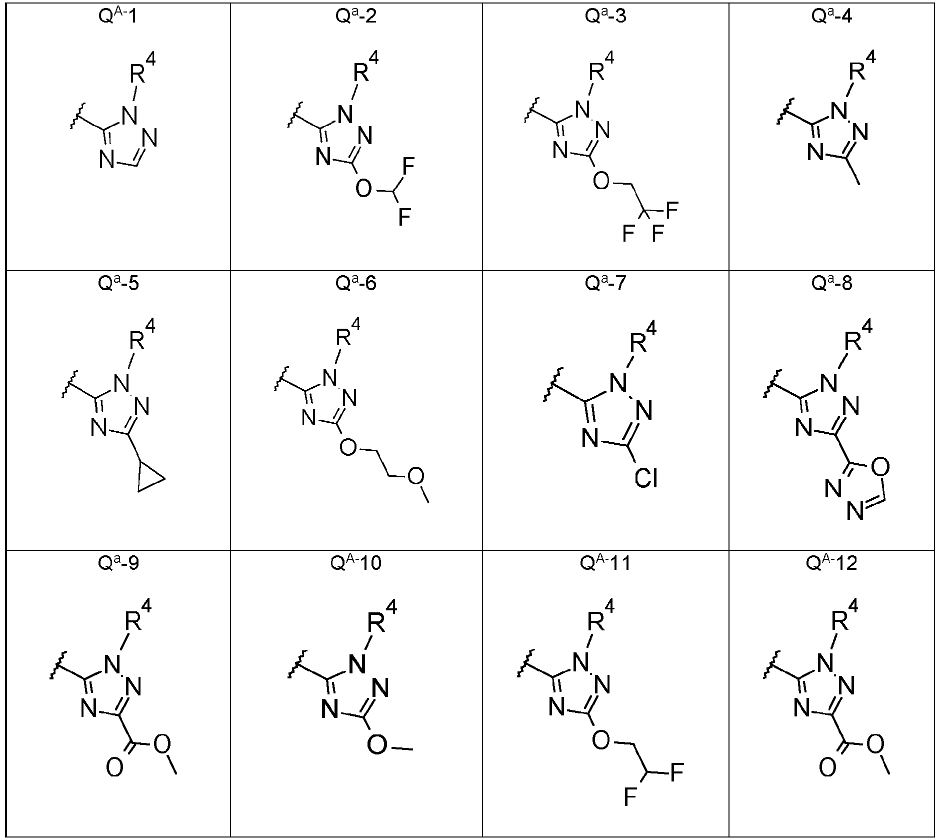

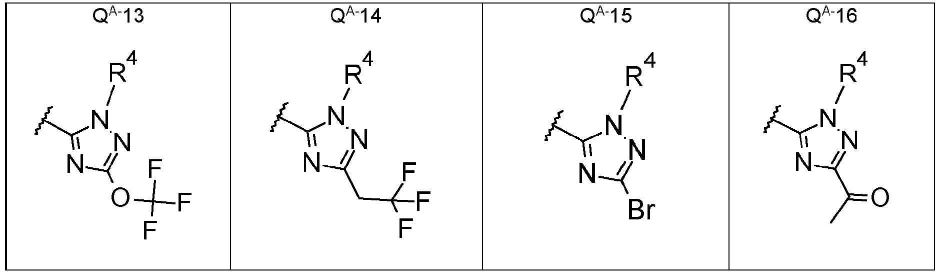

In an embodiment of each aspect of the invention, Q^{b} is
A. selected from Q^{B}-1 to Q^{B}-13; or
B. Q^{B}-1; or
C. selected from Q^{B}-1 to Q^{B}-13, wherein R⁴ is R^{4a}; or
D. selected from Q^{B}-1 to Q^{B}-13, wherein R⁴ is R^{4b}; or
E. Q^{B}-1, and R⁴ is R^{4a}; or
F. Q^{B}-1, and R⁴ is R^{4a}.

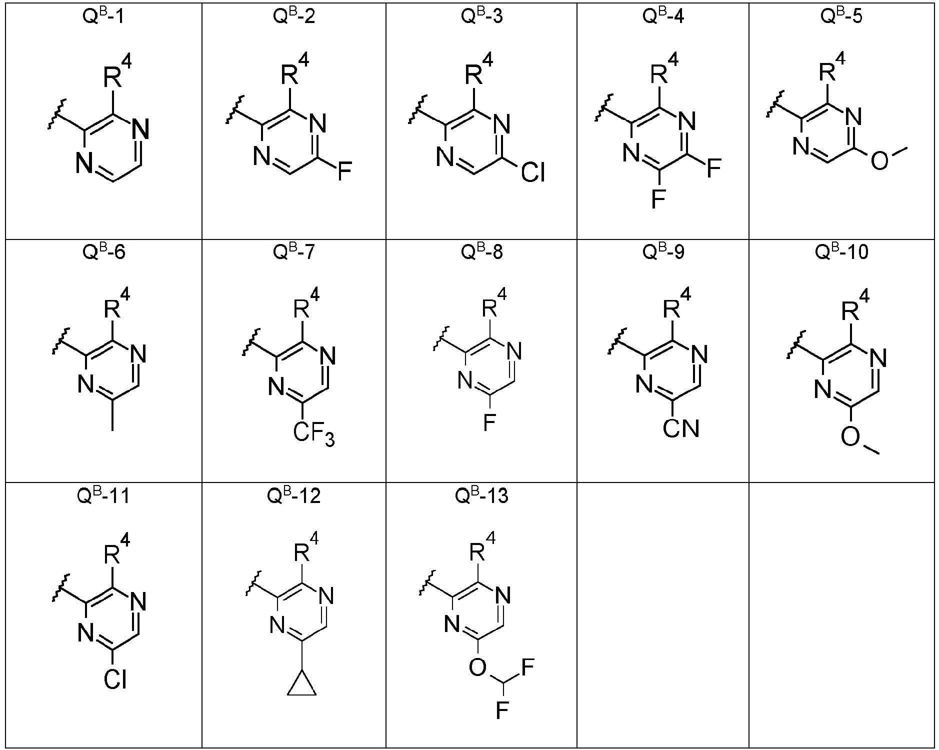

In an embodiment of each aspect of the invention, R⁴ is
A. 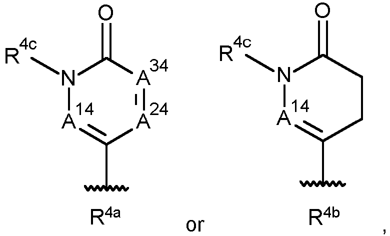 where the staggered line represents the connection of R⁴ to Q^{a} or Q^{b}; and where A¹⁴, A²⁴, and A³⁴ are, independently of each other, N or CH; or
B. 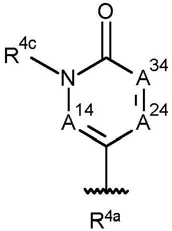 where the staggered line represents the connection of R⁴ to Q^{a} or Q^{b}; and where A¹⁴, A²⁴, and A³⁴ are, independently of each other, N or CH; or
C. 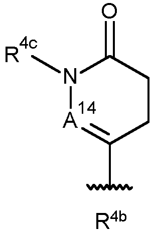 where the staggered line represents the connection of R⁴ to Q^{a} or Q^{b}; and where A¹⁴ is N or CH.

In an embodiment of each aspect of the invention, where R⁴ is R^{4a} and
A. A¹⁴, A²⁴, and A³⁴ are, independently of each other, N or CH, with the proviso that at least one is N; or
B. A¹⁴, A²⁴, and A³⁴ are, independently of each other, N or CH, with the proviso that one is N and the other two are CH; or
C. A¹⁴, A²⁴, and A³⁴ are, independently of each other, N or CH, with the proviso that at least one is CH; or
D. A¹⁴, A²⁴, and A³⁴ are, independently of each other, N or CH, with the proviso that one is CH and the other two are N;
E. A¹⁴ is N, A²⁴ is N or CH, and A³⁴ is N or CH; or
F. A¹⁴ is N or CH, A²⁴ is N, and A³⁴ is N or CH; or
G. A¹⁴ is N or CH, A²⁴ is N or CH, and A³⁴ is N; or
H. A¹⁴ is CH, A²⁴ is N or CH, and A³⁴ is N or CH; or
I. A¹⁴ is N or CH, A²⁴ is CH, and A³⁴ is N or CH; or
J. A¹⁴ is N or CH, A²⁴ is N or CH, and A³⁴ is CH; or
K. A¹⁴ is N, A²⁴ is N, and A³⁴ is N or CH; or
L. A¹⁴ is N, A²⁴ is CH, and A³⁴ is N or CH; or
M. A¹⁴ is CH, A²⁴ is N, and A³⁴ is N or CH; or
N. A¹⁴ is CH, A²⁴ is CH, and A³⁴ is N or CH; or
O. A¹⁴ is N, A²⁴ is N or CH, and A³⁴ is N; or
P. A¹⁴ is N, A²⁴ is N or CH, and A³⁴ is CH; or
Q. A¹⁴ is CH, A²⁴ is N or CH, and A³⁴ is N; or
R. A¹⁴ is CH, A²⁴ is N or CH, and A³⁴ is CH; or
S. A¹⁴ is N or CH, A²⁴ is N, and A³⁴ is N; or
T. A¹⁴ is N or CH, A²⁴ is N, and A³⁴ is CH; or
U. A¹⁴ is N or CH, A²⁴ is CH, and A³⁴ is N; or
V. A¹⁴ is N or CH, A²⁴ is CH, and A³⁴ is CH; or
W. A¹⁴ is N, A²⁴ is N, and A³⁴ is N; or
X. A¹⁴ is N, A²⁴ is N, and A³⁴ is CH; or
Y. A¹⁴ is N, A²⁴ is CH, and A³⁴ is N; or
Z. A¹⁴ is CH, A²⁴ is N, and A³⁴ is N; or
AA. A¹⁴ is N, A²⁴ is CH, and A³⁴ is CH; or
BB. A¹⁴ is CH, A²⁴ is N, and A³⁴ is CH; or
CC.A¹⁴ is CH, A²⁴ is CH, and A³⁴ is N; or
DD.A¹⁴ is CH, A²⁴ is CH, and A³⁴ is CH.

In an embodiment of each aspect of the invention, where R⁴ is R^{4b}
A. A¹⁴ is CH; or
B. A¹⁴ is N.

In an embodiment of each aspect of the invention, R^{4a} is an oxo-triazinyl moiety, or an oxo-diazinyl moiety, or an oxo-pyridyl moiety. Preferably, R^{4a} is an oxo-diazinyl moiety. In an embodiment of each aspect of the invention, R^{4a} is an oxopyridyl, oxopyrimidyl, oxopyrazinyl or oxopyridazinyl moiety.In an embodiment of each aspect of the invention, R^{4b} is an oxo-dihydro-pyridyl or oxo-dihydro-pyridazinyl moiety. R^{4a} and R^{4b} are each connected via a carbon atom on the respective ring to the rest of the compound ("the connecting carbon atom"). The connecting carbon atom and the carbonyl group C=O are in a para position with respect to each other on the oxopyridyl, oxopyrimidyl, oxopyrazinyl or oxopyridazinyl moiety. R^{4a} and R^{4b} have a substituted nitrogen atom in an ortho position with respect to the carbonyl group C=O. Said substituted nitrogen atom is substituted with R^{4c}. In an embodiment of each aspect of the invention, R^{4a} is an oxopyridyl moiety (A¹⁴, A²⁴ and A³⁴ are CH, i.e. R^{4a} is of embodiment DD). In an embodiment of each aspect of the invention, R^{4a} has a non-substituted nitrogen atom in the second ortho position with respect to the carbonyl group C=O. In this case, R^{4a} is an oxopyrimidyl moiety (A¹⁴ and A²⁴ are CH, and A³⁴ is N, i.e. R^{4a} is of embodiment CC), wherein the carbonyl group C=O is between the two nitrogen atoms of the ring. In an embodiment of each aspect of the invention, R^{4a} has one non-substituted nitrogen atom in the meta position with respect to the carbonyl group C=O. In this case, R^{4a} is an oxopyrazinyl moiety (A¹⁴ and A³⁴ are CH, A²⁴ is N, i.e. R^{4a} is of embodiment BB) or an oxopyridazinyl moiety (A¹⁴ is N, A²⁴ and A³⁴ are CH, i.e. R^{4a} is of embodiment AA). In an embodiment of each aspect of the invention, R^{4a} is an oxopyridazinyl moiety (A¹⁴ is N, A²⁴ and A³⁴ are CH, i.e. R^{4a} is of embodiment AA). In an embodiment of each aspect of the invention, R^{4b} is an oxo-dihydro-pyridazinyl moiety (A¹⁴ is N, i.e. R^{4b} is of embodiment A). In an embodiment of each aspect of the invention, R^{4b} is an oxo-dihydro-pyridyl moiety (A¹⁴ is CH, i.e. R^{4b} is of embodiment A).

In an embodiment of each aspect of the invention, R^{4c} is
A. C₁-C₃alkyl, C₁-C₃haloalkyl, allyl, propargyl, or C₃-C₆cycloalkylC₁-C₄alkyl; or
B. C₁-C₂alkyl, C₁-C₂haloalkyl, allyl, propargyl, or C₃-C₄cycloalkylC₁-C₂alkyl; or
C. methyl, ethyl, difluoromethyl, 2,2-difluoroethyl, 2,2,2-trifluoromethyl, allyl, propargyl, or cyclopropylmethyl; or
D. methyl, ethyl, difluoromethyl, 2,2-difluoroethyl, allyl, propargyl, or cyclopropylmethyl; or
E. methyl, ethyl, allyl, propargyl, or cyclopropylmethyl; or
F. methyl or ethyl; or
G. methyl or allyl; or
H. methyl or propargyl; or
I. methyl or cyclopropylmethyl; or
J. methyl.

In an embodiment of each aspect of the invention where Q is Q^{a}, R⁵ is
A. hydrogen, C₁-C₃alkyl, C₁-C₃haloalkyl, C₃-C₄cycloalkyl, C₁-C₃alkoxy, C₃-C₄alkoxyC(O)-, (C₁-C₃alkoxy)₂CH-, halogen, CN, NH₂C(O), amino (i.e. NH₂), (C₁-C₃alkyl)amino, di(C₁-C₃alkyl)amino, hydroxy, C₃-C₄halocycloalkyl, C₃-C₄cyanocycloalkyl, C₂-C₆alkenyl, C₂-C₆haloalkenyl, C₂-C₆alkynyl, C₂-C₆haloalkynyl, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl, C₁-C₄alkylsulfonyl, C₁-C₃alkoxy-C₁-C₃alkyl, C₁-C₃alkoxy-C₁-C₃alkoxy-C₁-C₃alkyl, (C₁-C₃alkyl)sulfonylamino, (C₁-C₃alkyl)sulfonyl(C₁-C₃alkyl)amino, (C₁-C₃alkyl)NHC(0), (C₁-C₃alkyl)₂NC(O), (C₁-C₃cycloalkyl)NHC(O), (C₁-C₃cycloalkyl)(C₁-C₃alkyl)NC(O), (C₁-C₃alkyl)C(O)(C₁-C₃alkyl)N, (C₁-C₃alkyl)C(O)NH, (C₁-C₃alkyl)C(O), (C₁-C₃alkoxy)C(O), HC(O), diphenylmethanimine, C₁-C₃haloalkoxy, phenyl, or a 5-membered heteroaromatic ring; or R⁵ is phenyl substituted with one to three substituents selected from C₁-C₃alkyl, C₁-C₃haloalkyl, C₁-C₃alkoxy, C₃-C₄cycloalkyl, halogen, CN and hydroxyl; or R⁵ is a 5-membered heteroaromatic ring substituted with one to three substituents selected from C₁-C₃alkyl, C₁-C₃haloalkyl, C₁-C₃alkoxy, C₃-C₄cycloalkyl, halogen, CN and hydroxyl; or
B. hydrogen, C₁-C₃alkyl, C₁-C₃haloalkyl, C₃-C₄cycloalkyl, C₁-C₃alkoxy, halogen, C₁-C₃alkoxy-C₁-C₃alkyl, C₁-C₃alkoxy-C₁-C₃alkoxy-C₁-C₃alkyl, (C₁-C₃alkyl)C(O), (C₁-C₃alkoxy)C(O), HC(O), C₁-C₃haloalkoxy or a 5-membered heteroaromatic ring wherein the 5-membered heteroaromatic ring can be optionally substituted with one to three substituents selected from C₁-C₃alkyl, C₁-C₃haloalkyl, C₁-C₃alkoxy, C₃-C₄cycloalkyl, halogen, CN or hydroxy; or
C. hydrogen, C₁-C₃alkyl, C₁-C₃haloalkyl, C₃-C₄cycloalkyl, C₁-C₃alkoxy, halogen, C₁-C₃alkoxy-C₁-C₃alkyl, C₁-C₃alkoxy-C₁-C₃alkoxy-C₁-C₃alkyl, (C₁-C₃alkyl)C(O), (C₁-C₃alkoxy)C(O), HC(O) or C₁-C₃haloalkoxy; or
D. hydrogen, C₁-C₃alkyl, C₁-C₃haloalkyl, C₃-C₄cycloalkyl, C₁-C₃alkoxy, halogen, Cl, Br, C₁-C₃alkoxy-C₁-C₃alkyl, C₁-C₃alkoxy-C₁-C₃alkoxy-C₁-C₃alkyl, (C₁-C₃alkyl)C(O), (C₁-C₃alkoxy)C(O), or C₁-C₂haloalkoxy; or
E. hydrogen, C₁-C₃alkyl, C₁-C₃alkoxy, C₃-C₄cycloalkyl, C₁-C₃haloalkoxy, halogen, C₁-C₃alkoxy-C₁-C₃alkyl, C₁-C₃alkoxy-C₁-C₃alkoxy-C₁-C₃alkyl, (C₁-C₃alkyl)C(O), HC(O), or (C₁-C₃alkoxy)C(O); or
F. hydrogen, C₁-C₂alkyl, C₁-C₂alkoxy, C₃-C₄cycloalkyl, C₁-C₂haloalkoxy, halogen, C₁-C₂alkoxy-C₁-C₂alkyl, C₁-C₂alkoxy-C₁-C₂alkoxy-C₁-C₂alkyl, (C₁-C₂alkyl)C(O), HC(O), or (C₁-C₂alkoxy)C(O); or
G. hydrogen, methyl, trifluoromethoxy, methoxy, cyclopropyl, 2,2-difluroroethoxy, 2,2,2-trifluroroethoxy, difluoromethoxy, 2,2,2-trifluroroethyl, chloro, bromo, methoxyethoxy, methylcarbonyl, or methoxycarbonyl; or
H. hydrogen, methyl, trifluoromethoxy, methoxy, cyclopropyl, difluoromethoxy, chloro, bromo, methoxyethoxy, methylcarbonyl, or methoxycarbonyl; or
I. hydrogen, methyl, methoxy, cyclopropyl, chloro, or methoxyethoxy; or
J. hydrogen, or methyl; or
K. hydrogen.

In an embodiment of each aspect of the invention where Q is Q^{b}, R^{5a} is
A. hydrogen, halogen, CN, C₁-C₃alkyl, C₁-C₃haloalkyl, C₃-C₄cycloalkyl, C₁-C₃alkoxy or C₁-C₃haloalkoxy; or
B. hydrogen, halogen, CN, C₁-C₃alkyl, C₁-C₃haloalkyl, C₃-C₄cycloalkyl or C₁-C₃alkoxy; or
C. hydrogen, halogen, CN, C₁-C₃alkyl, C₁-C₃haloalkyl or C₁-C₃alkoxy; or
D. hydrogen, halogen, CN, C₁-C₃alkyl or C₁-C₃alkoxy; or
E. hydrogen or halogen; or
F. hydrogen.

In an embodiment of each aspect of the invention where Q is Q^{b}, R^{5b} is
A. hydrogen, halogen, CN, C₁-C₃haloalkyl, C₃-C₄cycloalkyl, C₁-C₃alkoxy, or C₁-C₃haloalkoxy; or
B. hydrogen, halogen or C₁-C₃alkoxy; or
C. hydrogen.

In an embodiment of each aspect of the invention, R⁶ is
A. phenyl, benzyl, heteroaryl, or C₃-C₆ cycloalkyl, each of which, independently of each other, is optionally substituted with one substituent selected from R^{X}; or
B. phenyl, benzyl, cyclopropyl or cyclopropyl substituted with one substituent selected from R^{X}.

In an embodiment of each aspect of the invention, R^{X} is independently selected from:
A. halogen, C₁-C₃alkyl, C₁-C₃haloalkyl, C₁-C₃alkoxy, C₁-C₃haloalkoxy, NO₂, SF₅, CN, C(O)NH₂, C(S)NH₂, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl and C₁-C₄alkylsulfonyl; or
B. halogen, C₁-C₃haloalkyl, C₁-C₃alkoxy, C₁-C₃haloalkoxy or CN; or
C. F, Cl, Br, OCF₂H, OCH₃ or CN.

In an embodiment of each aspect of the invention, R^{Y} is independently selected from
A. hydrogen, C₁-C₃ alkyl, C₁-C₃ haloalkyl, hydroxy, C₁-C₃ alkoxy, C₁-C₃ haloalkoxy, halogen, CN and cyclopropyl; or
B. hydrogen, C₁-C₃ alkyl, C₁-C₃ haloalkyl, C₁-C₃ alkoxy, C₁-C₃ haloalkoxy, halogen, CN and cyclopropyl; or
C. hydrogen, C₁-C₃ alkyl, C₁-C₃ haloalkyl, C₁-C₃ alkoxy, C₁-C₃ haloalkoxy, and cyclopropyl; or
D. hydrogen, C₁-C₃ alkyl, chlorine, C₁-C₃ haloalkyl, and C₁-C₃ alkoxy; or
E. hydrogen, chlorine, methyl, trifluoromethyl, and methoxy; or
F. hydrogen, chlorine, methyl; or
G. chlorine, hydrogen.

In an embodiment of each aspect of the invention, R^{Z} is independently selected from:
A. oxo, halogen, C₁-C₃ alkyl, C₁-C₃haloalkyl, C₁-C₃alkoxy, C₁-C₃haloalkoxy and CN; or
B. oxo, halogen, C₁-C₃haloalkyl, C₁-C₃alkoxy, C₁-C₃haloalkoxy or CN; or
C. oxo, F, Cl, Br, OCF₂H, OCH₃ or CN.

The present invention, accordingly, makes available a compound of formula (I) having the substituents A¹, A², A³, A⁴, A⁵, x⁰, Q^{a}, Q^{b}, R, R¹, R^{2a}, R^{2b}, R³, R^{4c}, R⁵, R^{5a}, R^{5b}, R⁶, R^{X}, R^{Y}, and R^{Z} as defined above in all combinations / each permutation.

Accordingly, a compound of formula (I) is made available, for example, with A¹, A² and A³ of embodiment S (i.e. A¹=A²-A³, taken together, are NR-C(=O)-N), A⁴ and A⁵ of embodiment E (i.e. A⁴ and A⁵ are both CH), R of embodiment B (i.e. R is hydrogen, methyl, ethyl, difluoroethyl or trifluoroethyl), R¹ of embodiment I (i.e. R¹ is hydrogen, methyl, or cyclopropyl-methyl), R^{2a} of embodiment M (i.e. R^{2a} is halogen or C₁-C₃haloalkyl), such as R^{2a} of embodiment O (i.e. R^{2a} is fluorine, chlorine, bromine, or trifluoromethyl), R^{2b} of embodiment E (i.e. R^{2b} is chlorine, fluorine, bromine, difluoromethyl, or trifluoromethyl), R³ of embodiment B (i.e. R³ is methyl or trifluoromethyl), Q of embodiment C (i.e. Q is Q^{a} wherein R⁴ is R^{4a}), where Q^{a} is of embodiment H (i.e. Q^{a} is Q^{A}-1 or Q^{A}-15), R^{4a} is of embodiment I (i.e. A¹⁴ is N or CH, A²⁴ is CH, and A³⁴ is N or CH), R^{4c} of embodiment G (i.e. R^{4c} is methyl or allyl).

Also, a compound of formula (I) is made available, for example, with A¹, A² and A³ of embodiment J (i.e. A¹ and A³ are N, and A² is CR^{Y}), with R^{Y} of embodiment F (i.e. R^{Y} is hydrogen, chlorine, methyl), A⁴ and A⁵ of embodiment E (i.e. A⁴ and A⁵ are both CH), R¹ of embodiment F (i.e. R¹ is hydrogen, C₁-C₃alkyl, C₁-C₃cyanoalkyl, C₁-C₃alkoxy-C₁-C₃alkyl, C₁-C₃haloalkyl, C₂-C₄alkenyl, C₂-C₄haloalkenyl, C₂-C₄alkynyl, C₂-C₄haloalkynyl, C₃-C₄cycloalkylC₁-C₂alkyl-, benzyloxycarbonyl, or benzyl), such as R^{2a} of embodiment M (i.e. R^{2a} is halogen or C₁-C₃haloalkyl), R^{2b} of embodiment C (i.e. R^{2b} is halogen, C₁-C₃haloalkyl, or C₁-C₃haloalkoxy), R³ of embodiment C (i.e. R³ is methyl), Q of embodiment C (i.e. Q is Q^{a} wherein R⁴ is R^{4a}), where Q^{a} is Q^{A}-1, R^{4a} is of embodiment AA (i.e. A¹⁴ is N, A²⁴ is CH, and A³⁴ is CH), R^{4c} of embodiment I (i.e. R^{4c} is methyl or cyclopropylmethyl).

Also, a compound of formula (I) is made available, for example, with A¹, A² and A³ of embodiment C (i.e. A¹ and A³ are N, and A² is CR^{Y}; or A¹=A²-A³, taken together, are NR-C(=O)-N), with R^{Y} of embodiment G (i.e. R^{Y} is hydrogen, or chlorine), and R of embodiment G (i.e. R is methyl or hydrogen ), A⁴ and A⁵ of embodiment E (i.e. A⁴ and A⁵ are both CH), R¹ of embodiment J (i.e. R¹ is hydrogen, or methyl); R^{2a} of embodiment R (i.e. R^{2a} is chlorine, bromine, iodine, or trifluoromethyl), R^{2b} of embodiment I (i.e. R^{2b} is chlorine, bromine, iodine, or trifluoromethyl), R³ of embodiment C (i.e. R³ is methyl), Q of embodiment B (i.e. Q is Q^{a} wherein R⁴ is R^{4a} or R^{4b}), where Q^{a} is Q^{A}-1, R^{4a} is of embodiment AA (i.e. A¹⁴ is N, A²⁴ is CH, and A³⁴ is CH), R^{4b} is of embodiment B (i.e. A¹⁴ is N), and R^{4c} of embodiment F (i.e. R^{4c} is methyl or ethyl).

In an embodiment, the compound of the formula (I) is formula (I*^{a}), (I*^{b}), (I*^{c}), (I*^{d}), (I*^{e}) or (I *^{f}) (with the asterisk indicating a stereogenic centre), wherein A¹, A², A³, A⁴, A⁵, R, R¹, R^{2a}, R^{2b}, R³, R^{4c}, R⁵, R^{5a}, R^{5b},and R^{Y} are as defined in the first aspect, each with the corresponding embodiments as described above.

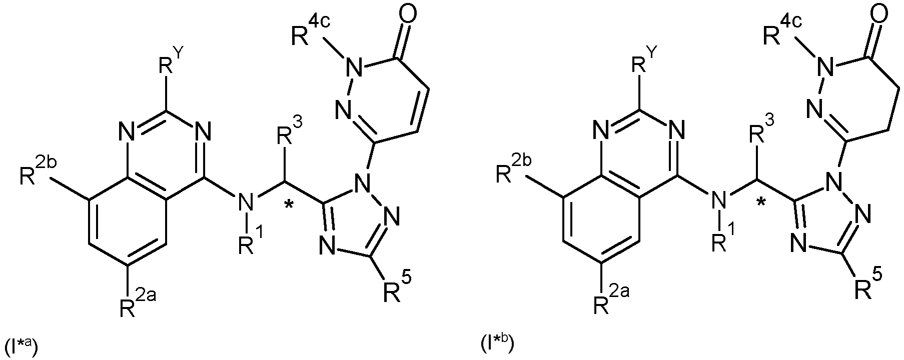

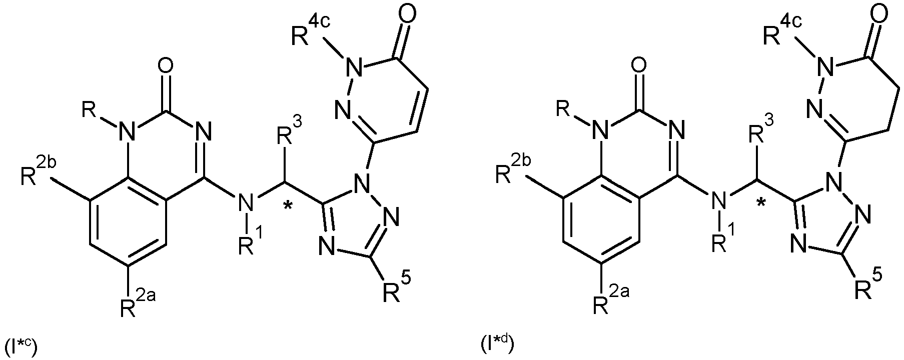

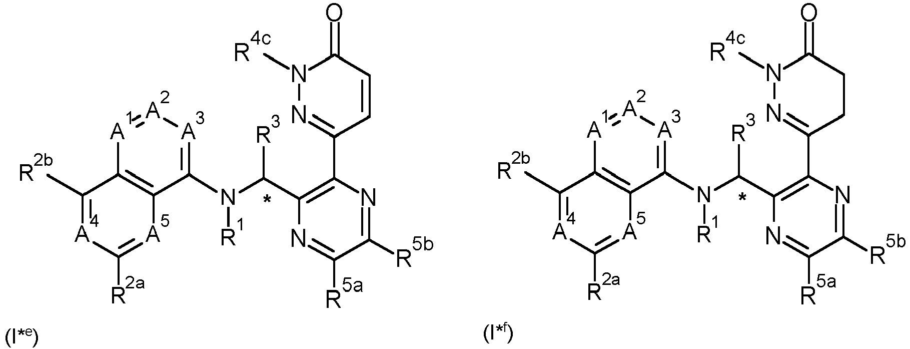

In an embodiment, compounds having preferred stereochemistry depicted in formula (I') would also be preferred for compounds of formulae (I*^{a}), (I*b), (I*c), (I*d), (I*e) or (I*f)]. In a preferred embodiment, a compound of formula (I'^{a}), (I'^{b}), (I'^{c}), (I'^{d}), (I'^{e}) or (I'^{f})] with the following stereochemistry is preferred: 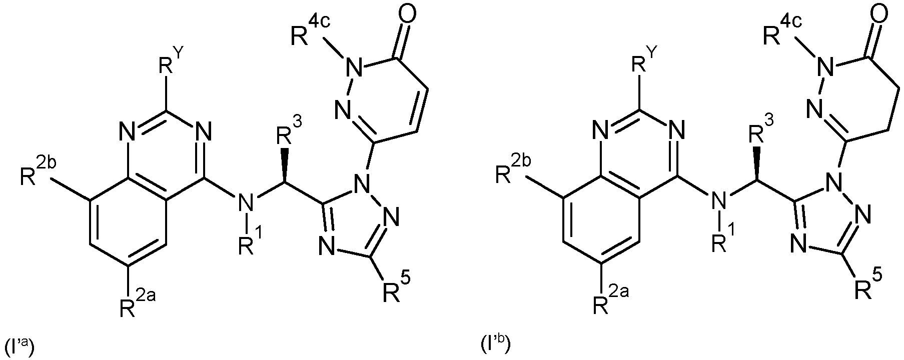 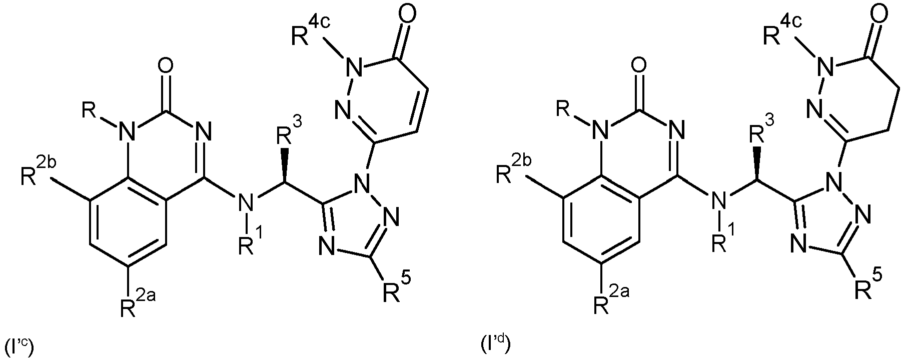 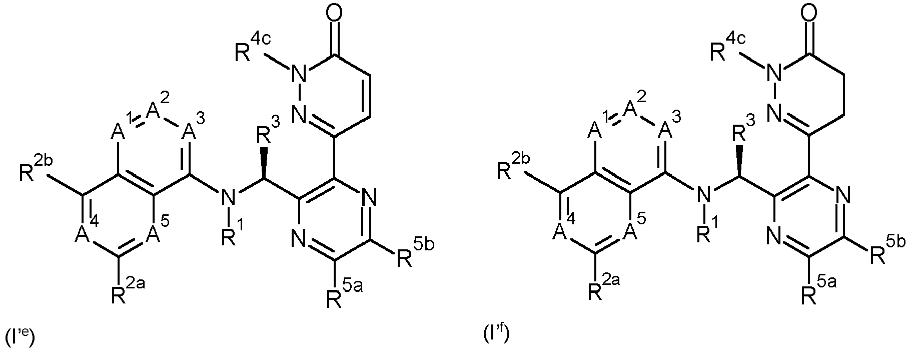 wherein A¹, A², A³, A⁴, A⁵, R, R¹, R^{2a}, R^{2b}, R³, R^{4c}, R⁵, R^{5a}, R^{5b},and R^{Y} are as defined in the first aspect, and stereoisomers, enantiomers, tautomers, and N-oxides, of the compounds of formula (I'^{a}), (I'^{b}), (I'^{c}), (I'^{d}), (I'^{e}) or (I'^{f})], and agrochemically acceptable salts thereof.

Compounds of formula (I) can be prepared by those skilled in the art following known methods. More specifically compounds of formula (I), compounds of formula (I'), and intermediates therefor, can be prepared as described below in the schemes and examples. Certain stereogenic centers have been left unspecified for the clarity and are not intended to limit the teaching of the schemes in any way. The process according to the invention for preparing compounds of formula (I) is carried out by methods known to those skilled in the art.

Compounds of formula (I) 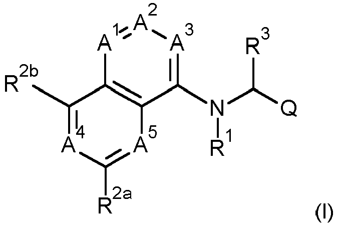
wherein A¹, A², A³, A⁴, A⁵, R¹, R³, Q, R^{2a} and R^{2b} have the same meaning as given above for compounds of the formula (I), can be prepared by reaction of an amine of formula (III) 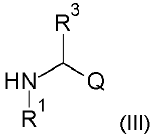
or a salt thereof (such as a hydrohalide salt, preferably a hydrochloride or a hydrobromide salt, or a trifluoroacetic acid salt, or any other equivalent salt), wherein R¹, R³ and Q have the same meaning as given above for compounds of the formula (I), with a compound of formula (II) 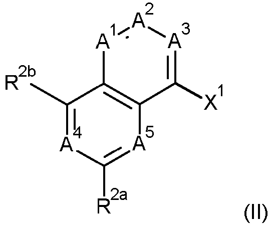
wherein A¹, A², A³, A⁴, A⁵, R^{2a} and R^{2b} have the same meaning as given above for compounds of the formula (I), and wherein X¹ is a leaving group, such as a halogen, hydroxy or sulfonate.

Throughout the description, for instance in Schemes 1 to 11 below, or in Tables A-1 to A-78, Tables B-1 to B-78, Tables C-1 to C-21, and Tables D-1 to D-21, T represents 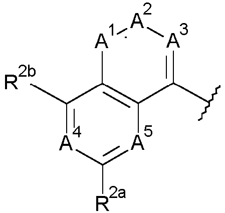 where A¹, A², A³, A⁴, A⁵, R^{2a} and R^{2b} have the same meaning as given above for compounds of the formula (I), and where the staggered line represents the connection to the remainder of the compounds, such as compounds of the formula (I), (Ia), (Ib), (Ic), (Id), (le), (If), (Ig), (Ih), (II), (IV), (IVa), (X), (XI), (XVII) in Schemes 1 to 11.

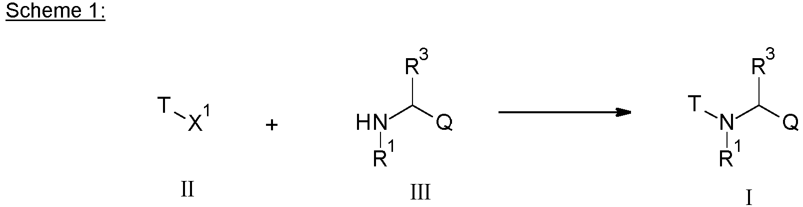

Compounds of the formula (I) can be made, for example, by reaction of a compound of the formula (II), wherein X¹ is a leaving group, such as a halogen, hydroxy or sulfonate, for instance chloride, and wherein T has the meaning given above, with a compound of formula (III), or a salt thereof, wherein R¹, R³ and Q have the same meaning as given above for compounds of the formula (I). In the case that X¹ is hydroxy, it may be advantageous to carry out the reaction in the presence of a dehydration reagent, for instance a peptide coupling reagent, such as, for example, a carbodiimide or propanephosphonic acid cyclic anhydride (T3P^{®}). Such reactions can be conducted neat or in a solvent, preferably in a solvent, such as an organic solvent, for instance acetonitrile, tetrahydrofuran, 2-methyltetrahydrofuran, ethyl acetate, N,N-dimethylacetamide or N,N-dimethylformamide, in a temperature range of -100 to +300 °C, preferably between ambient temperature and 200 °C, with or without the presence of a catalyst, for instance a metal catalyst, such as a palladium complex, and with or without the addition of a base, such as an inorganic base, for instance sodium, potassium or cesium carbonate, or an organic base, such as, for example, triethylamine, diisopropylethylamine or pyridine. Compounds of the formula (II) are either known, or can be prepared in analogy to descriptions found for example in WO 2021/083936 and WO 2021/177160, or they can be prepared by methods known to a person skilled in the art.

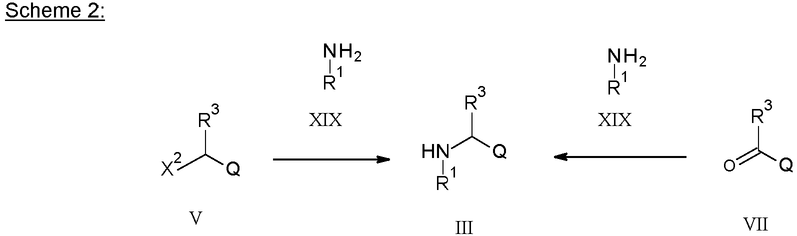

Compounds of formula (III), or a salt thereof, can be made, for example, as shown in scheme 2. Treatment of a compound of the formula (V), wherein R³ and Q have the same meaning as given above for compounds of the formula (I) and wherein X² is a leaving group, such as a halogen or sulfonate, for instance bromide, with an amine of the formula (XIX), or a salt thereof, wherein R¹ has the same meaning as given above for compounds of the formula I, gives compounds of the formula (III), wherein Q, R¹ and R³ have the same meaning as given above for compounds of the formula I. The reaction can be conducted neat or in a solvent, preferably in a solvent, such as an organic solvent, for instance acetonitrile, in a temperature range of -100 to +300 °C, preferably between ambient temperature and 200 °C, with or without the addition of a base, such as an inorganic base, for instance potassium carbonate, or an organic base, such as, for example, triethylamine. Alternatively, treatment of a compound of the formula (VII), wherein R³ and Q have the same meaning as given above for compounds of the formula (I), with an amine of the formula (XIX), or a salt thereof, wherein R¹ has the same meaning as given above for compounds of the formula I, gives compounds of the formula (III), wherein Q, R¹ and R³ have the same meaning as given above for compounds of the formula I. This reaction is done in the presence of a reducing agent, such as for example hydrogen, or a hydride, such as sodium borohydride, with or without a catalyst, such as a hydrogenation catalyst, for example palladium on carbon, with or without the presence of an acid, such as acetic acid, or a Lewis acid, such as zinc bromide or titanium(IV) isopropoxide, in a solvent or without a solvent, such as, for instance, methanol. The reaction can be conducted in a temperature range of -100 to +300 °C, preferably between ambient temperature and 200 °C. Such methods, and the range of conditions to perform them, for the alkylation of amines and for the reductive alkylation of amines (e.g. in the presence of NaBH(OAc)₃ or NaBH₃CN, in a suitable solvent, preferably in acetic acid, at room temperature, analogous to WO2002/088073; or alternatively, by the use of a combination of Ti(i-OiPr)₄ and NaBH₄ as described in Synthesis 2003 (14), 2206) are well known to a person skilled in the art. The amines of formula (XIX), or a salt thereof, wherein R¹ has the same meaning as given above for compounds of the formula (I), are either known, or they can be prepared by methods known to a person skilled in the art.

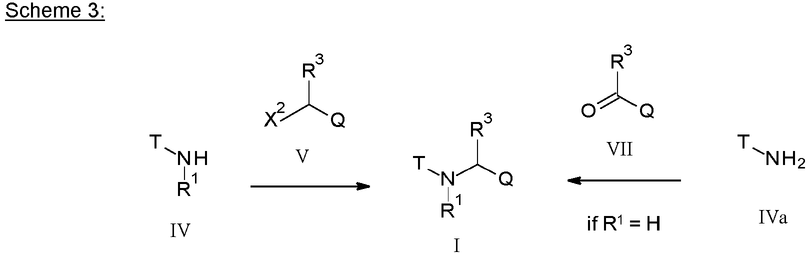

Alternatively, compounds of formula (I) can be made, for example, by reaction of compound of the formula (IV), wherein T has the same meaning as given above in Scheme 1, and R¹ has the same meaning as given above for compounds of the formula (I), with a compound of the formula (V), wherein R³ and Q have the same meaning as given above for compounds of the formula (I), and X² is a leaving group, such as a halogen or sulfonate, for instance chloride or bromide. The reaction can be conducted neat or in a solvent, preferably in a solvent, such as an organic solvent, for instance acetonitrile, in a temperature range of -100 to +300 °C, preferably between ambient temperature and 200 °C, with or without the addition of a base, such as an inorganic base, for instance potassium carbonate, or an organic base, such as, for example, triethylamine. Such methods for the alkylation of amines, and the range of conditions to perform them, are well known to a person skilled in the art. Alternatively, a compound of the formula (I) can be made by reaction of a compound of the formula (IVa), wherein T has the same meaning as given above in Scheme 1, with a compound of the formula (VII), wherein R³ and Q have the same meaning as given above for compounds of the formula (I). This reaction is done in the presence of a reducing agent, such as for example hydrogen, or a hydride, such as sodium borohydride, with or without a catalyst, such as a hydrogenation catalyst, for example palladium on carbon, with or without the presence of an acid, such as acetic acid, or a Lewis acid, such as zinc bromide, in a solvent or without a solvent, such as, for instance, methanol. The reaction can be conducted in a temperature range of -100 to +300 °C, preferably between ambient temperature and 200 °C. Such methods for the reductive alkylation of amines, and the range of conditions to perform them, are well known to a person skilled in the art.

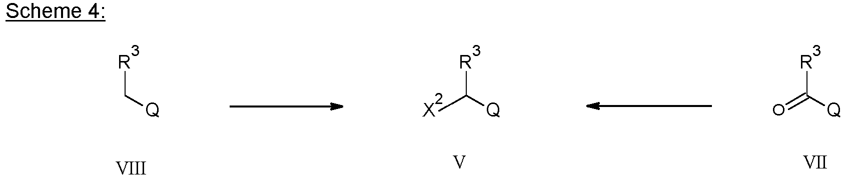

Compounds of formula (V) can be made, for example, as shown in scheme 4. Treatment of a compound of the formula (VIII) with a halogenating agent, such as chlorine or bromine or N-bromosuccinimide, for example, gives compound of the formula (V), wherein the leaving group X² is a halogen, for instance chloride or bromide. This reaction is done with or without a solvent, preferably in a solvent, with or without an additive, such as a radical starter, such as, for example, benzoyl peroxide or azoisobutyronitrile. The reaction can be done with or without exposure to visible light, or to UV light, and it can be conducted in a temperature range of -100 to +300 °C, preferably between ambient temperature and 200 °C.

Alternatively, a compound of the formula (VII) can be treated with a reducing agent, followed by reaction with a sulfonyl chloride, for instance methanesulfonyl chloride, to give a compound of the formula (V), wherein the leaving group X² is a sulfonate, for instance a mesylate. This reaction can be done in a solvent, or without a solvent, in the presence of a base, such as an inorganic base, for instance potassium carbonate, or an organic base, such as an amine base, for instance trimethylamine, or without a base, and it can be conducted in a temperature range of -100 to +300 °C, preferably between ambient temperature and 200 °C. A suitable reducing agent could be, for example, hydrogen, or a hydride, such as sodium borohydride, with or without a catalyst, such as a hydrogenation catalyst, for example palladium on carbon, with or without the presence of an acid, such as acetic acid, or a Lewis acid, such as zinc bromide, in a solvent or without a solvent, such as, for instance, methanol. The reaction can be conducted in a temperature range of -100 to +300 °C, preferably between ambient temperature and 200 °C. Such methods for the halogenation, the reduction of carbonyl compounds and the sulfonylation of alcohols, and the range of conditions to perform them, are well known to a person skilled in the art. The compounds of the formula (VII) and the compounds of formula (VIII) are either known, or they can be prepared by methods known to a person skilled in the art.

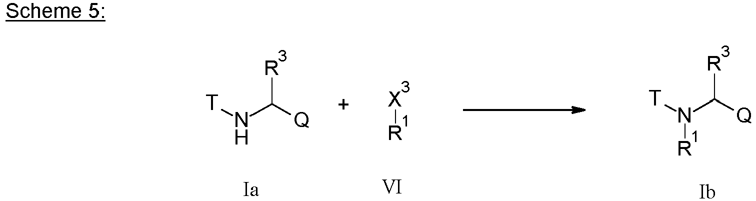

Alternatively, compounds of formula (Ib) wherein T has the same meaning as given above in Scheme 1, and R¹, R³ and Q have the same meaning as given above for compounds of the formula (I), except that R¹ is different from hydrogen, can be made, for example, as shown in scheme 5. A compound of the formula (la), wherein T has the same meaning as given above in Scheme 1, and R³ and Q have the same meaning as given above for compounds of the formula (I), can be reacted with a compound of the formula (VI), wherein R¹ has the same meaning as given above for compounds of the formula (I), except that R¹ is different from hydrogen, and wherein X³ is a leaving group, such as a halogen or sulfonate, for instance a chloride, bromide, iodide or mesylate, to give a compound of formula (Ib). This reaction can be conducted neat or in a solvent, preferably in a solvent, such as an organic solvent, for instance acetonitrile, N,N-dimethylformamide (DMF) or N,N-dimethylacetamide (DMA), or mixtures thereof, in a temperature range of -100 to +300 °C, preferably between ambient temperature and 200 °C, with or without the addition of a base, such as an inorganic base, for instance sodium, potassium or cesium carbonate, or an organic base, such as, for example, triethylamine, diisopropylethylamine or pyridine. Such methods for the alkylation of amines, and the range of conditions to perform them, are well known to a person skilled in the art.

Compounds of the formula (VI), wherein R¹ has the same meaning as given above for compounds of the formula (I), except that R¹ is different from hydrogen, and wherein X³ is a leaving group, such as a halogen or sulfonate, for instance a chloride, bromide, iodide or mesylate, are either known, or they can be prepared by methods known to a person skilled in the art.

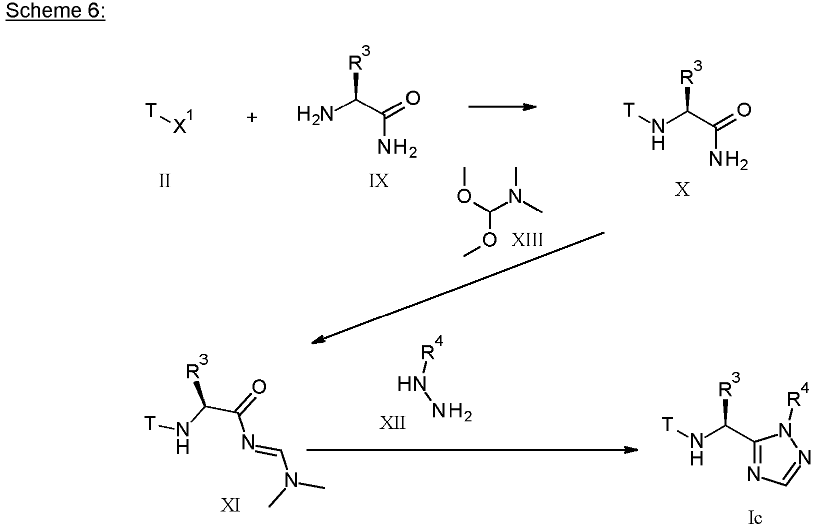

Compounds of formula (Ic), wherein T has the same meaning as given above in Scheme 1, and R³ and R⁴ have the same meaning as given above for compounds of the formula (I), can be made, for example, as shown in scheme 6. Reaction of a compound of the formula (II), wherein T has the same meaning as given above in Scheme 1 and wherein X¹ is a leaving group, such as a halogen or sulfonate, for instance chloride, with a compound of the formula (IX), or a salt thereof, wherein R³ has the same meaning as given above for compounds of the formula (I), gives a compound of the formula (X), wherein T has the same meaning as given above in Scheme 1, and wherein R³ has the same meaning as given above for compounds of the formula (I). The reaction can be conducted neat or in a solvent, preferably in a solvent, such as an organic solvent, for instance acetonitrile, tetrahydrofuran, 2-methyltetrahydrofuran, ethyl acetate, N,N-dimethylacetamide or N,N-dimethylformamide, in a temperature range of -100 to +300 °C, preferably between ambient temperature and 200 °C, with or without the presence of a catalyst, for instance a metal catalyst, such as a palladium complex, and with or without the addition of a base, such as an inorganic base, for instance sodium, potassium or cesium carbonate, or an organic base, such as, for example, triethylamine, diisopropylethylamine or pyridine.

Subsequent treatment of compound of the formula (X), wherein T has the same meaning as given above in Scheme 1, and wherein R³ has the same meaning as given above for compounds of the formula (I), with the known compound (XIII) (N,N-dimethylformamide dimethyl acetal, DMF-DMA) gives a compound of the formula (XI), wherein T has the same meaning as given above in Scheme 1, and wherein R³ has the same meaning as given above for compounds of the formula (I). This reaction can be conducted neat or in a solvent, preferably in a solvent, such as an organic solvent, for instance dichloromethane, tetrahydrofuran, 2-methyltetrahydrofuran or dioxane, in a temperature range of -100 to +300 °C, preferably between ambient temperature and 100 °C, or between ambient temperature and 50 °C, without a base or in the presence of a base, such as an inorganic base, for instance sodium, potassium or cesium carbonate, or an organic base, such as, for example, triethylamine, diisopropylethylamine or pyridine.

Further reaction of compound of the formula (XI), wherein T has the same meaning as given above in Scheme 1, and wherein R³ has the same meaning as given above for compounds of the formula (I), with a hydrazine compound of formula (XII) or a tautomer thereof, or a salt thereof, wherein R⁴ has the same meaning as given above for compounds of the formula (I), preferably R⁴ is R^{4a} as defined above for compounds of the formula (I), gives the compound of the formula (Ic), wherein T has the same meaning as given above in Scheme 1, and wherein R³ and R⁴ have the same meaning as given above for compounds of the formula (I), preferably R⁴ is R^{4a} as defined above for compounds of the formula (I). This reaction can be conducted neat or in a solvent, preferably in a solvent, such as an organic solvent, for instance 1,4-dioxane, or acetic acid, or a mixture of 1,4-dioxane and acetic acid, in a temperature range of -100 to +300 °C, preferably between ambient temperature and 200 °C, or between ambient temperature and 80 °C. Within this sequence of transformations, the intermediate compounds of formula (X) and of formula (XI) can be used as crude products for the subsequent step, or they can be purified, for instance by chromatography, and used in purified form for the next transformation.

Compounds of the formula (IX), or a salt thereof, wherein R³ has the same meaning as given above for compounds of the formula (I), are either known, or they can be prepared by methods known to a person skilled in the art.

Compounds of the formula (li) 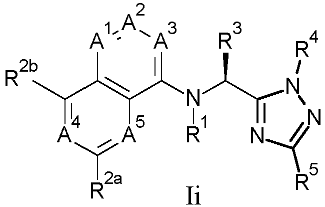
wherein A¹, A², A³, A⁴, A⁵, R¹, R³, R⁴, R⁵, R^{2a} and R^{2b} have the same meaning as given above for compounds of the formula (I), can be prepared by the reaction of an amine of the formula (Illa), or a salt thereof (such as a hydrohalide salt, preferably a hydrochloride or a hydrobromide salt, or a trifluoroacetic acid salt, or any other equivalent salt), 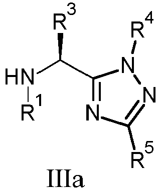
wherein R¹, R³, R⁴ and R⁵ are as described in formula (I), with a compound of the formula (II) 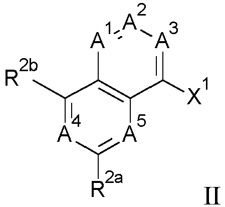
wherein A¹, A², A³, A⁴, A⁵, R^{2a} and R^{2b} are as described in formula (I) and X¹ is a leaving group, such as a halogen or a sulfonate, for instance chloride, under conditions already described in Scheme 1.

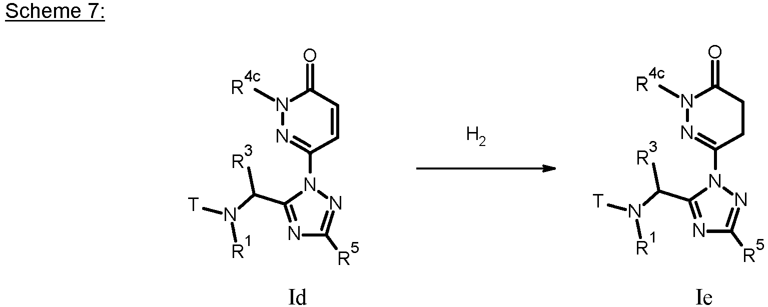

Compound of the formula (le), wherein T has the same meaning as given above in Scheme 1, and R¹, R³, R^{4c} and R⁵ have the same meaning as given above for compounds of the formula (I), can be made (Scheme 7) from compounds of the formula (Id), wherein T has the same meaning as given above in Scheme 1, and R¹, R³, R^{4c} and R⁵ have the same meaning as given above for compounds of the formula (I), by a hydrogenation reaction. This reaction can be done in the presence of hydrogen, or by transfer hydrogenation, such as for instance in the presence of formic acid or a formic acid salt. The reaction can be done in the presence of a catalyst, for instance a homogenous or a heterogenous catalyst. For example, the reaction is done in the presence of a palladium catalyst, such as palladium on carbon. It may be of advantage to perform the reaction in a solvent, or neat, preferably in a solvent, such as, for example, in methanol or ethanol as a solvent. The reaction can be performed in a temperature range of -100 to +300 °C, preferably between ambient temperature and 200 °C, or between ambient temperature and 80 °C, optionally in a pressure vessel.

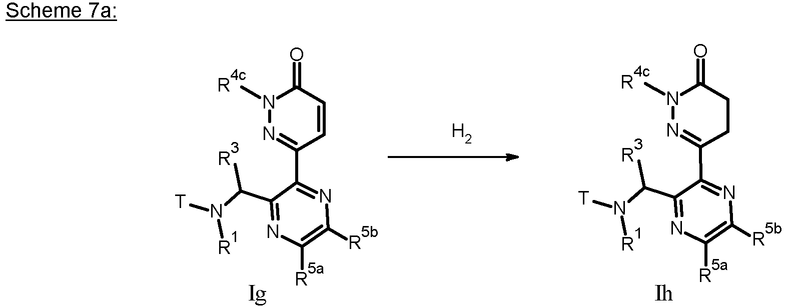

Similarly, compound of the formula (Ih), wherein T has the same meaning as given above in Scheme 1, and R¹, R³, R^{4c}, R^{5a} and R^{5b} have the same meaning as given above for compounds of the formula (I), can be made (Scheme 7a) from compounds of the formula (lg), wherein T has the same meaning as given above in Scheme 1, and R¹, R³, R^{4c}, R^{5a} and R^{5b} have the same meaning as given above for compounds of the formula (I), by a hydrogenation reaction under analogous conditions described in Scheme 7.

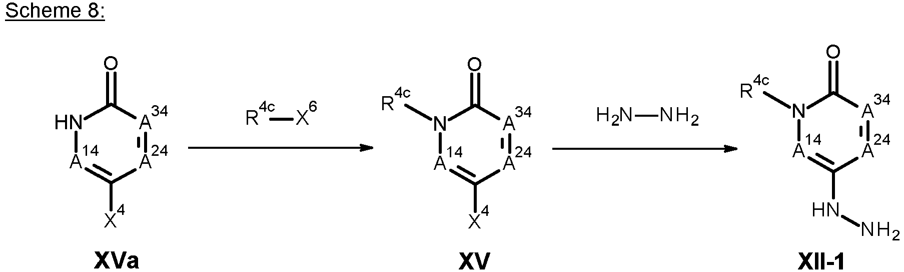

Compounds of the formula (XII-1), the subset of compounds of formula (XII) wherein R⁴ is specifically R^{4a}, or a salt thereof (such as a hydrohalide salt, preferably a hydrochloride or a hydrobromide salt, or a trifluoroacetic acid salt, or any other equivalent salt), in which A¹⁴, A²⁴, A³⁴ and R^{4c} have the same meaning as defined above for compounds of the formula (I), can be made (Scheme 8) by treatment of a compound of the formula (XV), in which A¹⁴, A²⁴, A³⁴ and R^{4c} have the same meaning as defined above for compounds of the formula (I), and X⁴ is a leaving group, such as for example a halogen, a sulfonate, C₁-C₄-sulfanyl, C₁-C₄-sulfinyl or C₁-C₄-sulfonyl, with hydrazine or with hydrazine hydrate, or salts thereof. The reaction can be carried out neat or in a solvent, such as for instance water, or alcohols such as methanol or ethanol, or methoxy cyclopentane, at a temperature between -100 °C and 200 °C, more commonly between 0 °C and 150 °C, such as, for example, at 100 °C. Such reactions are known from the literature, for example from Bioorganic & Medicinal Chemistry Letters (2012), 22(2), 1226-1229. Many compounds of the formula (XV) are known or even commercially available, or they can be made by known methods. In particular, compound of the formula (XV), in which A¹⁴, A²⁴, A³⁴ and R^{4c} have the same meaning as defined above for compounds of the formula (I), and X⁴ is a leaving group, such as for example a halogen, a sulfonate, C₁-C₄-sulfanyl, C₁-C₄-sulfinyl or C₁-C₄-sulfonyl, can be made by treatment of compounds of the formula (XVa), or a tautomer thereof, in which A¹⁴, A²⁴ and A³⁴ and have the same meaning as defined above for compounds of the formula (I), and X⁴ is a leaving group, such as for example a halogen, a sulfonate, C₁-C₄-sulfanyl, C₁-C₄-sulfinyl or C₁-C₄-sulfonyl, with a reagent of the formula R^{4c}-X⁶, wherein R^{4c} has the same meaning as defined above for compounds of the formula (I), and X⁶ is a leaving group, such as a halogen or sulfonate, for instance a chloride, bromide, iodide or mesylate. This reaction can be conducted neat or in a solvent, preferably in a solvent, such as an organic solvent, for instance acetonitrile, N,N-dimethylformamide (DMF) or N,N-dimethylacetamide (DMA), or mixtures thereof, in a temperature range of -100 to +300 °C, preferably between ambient temperature and 200 °C, with or without the addition of a base, such as an inorganic base, for instance sodium, potassium or cesium carbonate, or an organic base, such as, for example, triethylamine, diisopropylethylamine or pyridine. Such alkylation methods, and the range of conditions to perform them, are well known to a person skilled in the art, and described for example in Bioorganic & Medicinal Chemistry Letters (2012), 22(2), 1226-1229. Reagents of the formula R^{4c}-X⁶ and compounds of the formula (XVa), or a tautomer thereof, are known or even commercially available, or they can be made by known methods.

Compounds of the formula (Xlla), a subset of compounds of formula (XII-1) wherein A¹⁴ is N, or a salt thereof, in which A²⁴, A³⁴ and R^{4c} have the same meaning as defined above for compounds of the formula (I), may exist in different tautomeric forms, such as (Xllb) and/or (Xllc), or a salt thereof, in which A²⁴, A³⁴ and R^{4c} have the same meaning as defined above for compounds of the formula (I):

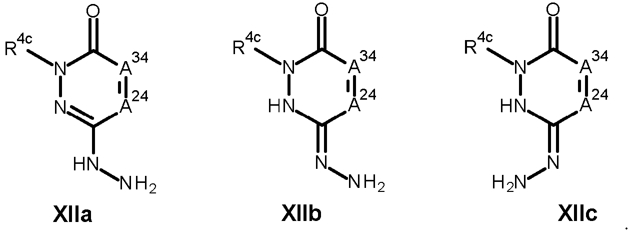

This invention covers all such tautomers and mixtures thereof in all proportions.

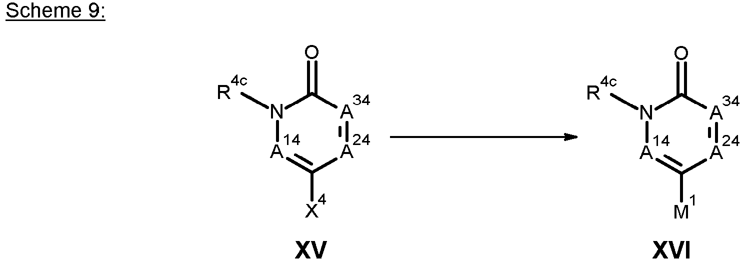

Compounds of the formula (XVI), in which A¹⁴, A²⁴, A³⁴ and R^{4c} have the same meaning as defined above for compounds of the formula (I), and M¹ is a metal-containing substituent, for instance a boron substituent or a tin substituent, such as, for example, tributylstannyl (M¹ is -SnBu₃), borono (M¹ is - B(OH)₂) or a boronate such as 4,4,5,5-tetramethyl-1,3,2-dioxaborolan-2-yl, can be made (Scheme 9) by treatment of a compounds of the formula (XV), in which A¹⁴, A²⁴, A³⁴ and R^{4c} have the same meaning as defined above for compounds of the formula (I), and X⁴ is a leaving group, such as for example a halogen, a sulfonate, C₁-C₄-sulfanyl, C₁-C₄-sulfinyl or C₁-C₄-sulfonyl, with tributyl(tributylstannyl)stannane, or with hypoboric acid, or with 4,4,5,5-tetramethyl-2-(4,4,5,5-tetramethyl-1,3,2-dioxaborolan-2-yl)-1,3,2-dioxaborolane (also known as bis(pinacolato)diboron). The reaction can be done in the presence of a catalyst, such as a metal catalyst, for instance a palladium catalyst, for example palladium acetate, and in the presence of a ligand, such as a phosphine ligand, for example 2-dicyclohexylphosphino-2',4',6'-triisopropylbiphenyl (XPhos). The reaction can be done in the presence of a base, such as an alkoxide or a carboxylate base, for instance potassium acetate. The reaction can be done neat or in a solvent, for instance in dioxane or toluene as a solvent, at a temperature between -100 °C and 200 °C, more commonly between 0 °C and 150 °C, such as, for example, at 100 °C. Some compounds of the formula (XVI) are known or even commercially available, or they can be made by known methods.

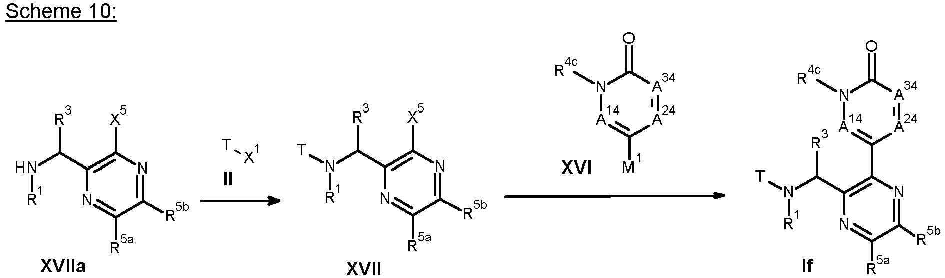

Compounds of the formula (If), wherein T has the same meaning as given above in Scheme 1, and wherein A¹⁴, A²⁴, A³⁴, R¹, R³, R^{4c}, R^{5a} and R^{5b} have the same meaning as given above for compounds of the formula (I), can be made (Scheme 10) from compounds of the formula (XVII), wherein T has the same meaning as given above in Scheme 1, and wherein R¹, R³, R^{5a} and R^{5b} have the same meaning as given above for compounds of the formula (I), and in which X⁵ is a leaving group such as for example chlorine, bromine or iodine, by reaction with compounds of the formula (XVI), in which A¹⁴, A²⁴, A³⁴ and R^{4c} have the same meaning as defined above for compounds of the formula (I), and M¹ is a metal-containing substituent which has the same meaning as given above in Scheme 9. The reaction can be done in the presence of a catalyst, such as a palladium catalyst, for instance 1,1'-bis(diphenylphosphino)-ferrocene]palladium(II) dichloride (PdCl₂dppf), in the presence of a base, such as a carbonate base, for example Cs₂CO₃, or such a carboxylate base, for instance potassium acetate. The reaction can be done neat or in a solvent, for instance in dioxane as a solvent, at a temperature between -100 °C and 200 °C, more commonly between 0 °C and 150 °C, such as, for example, at 85 °C. Such reactions known as Suzuki-Miyaura or Stille cross-coupling reactions are familiar to a person skilled in the art.

Compounds of the formula (XVII), wherein T has the same meaning as given above in Scheme 1, and wherein R¹, R³, R^{5a} and R^{5b} have the same meaning as given above for compounds of the formula (I), and in which X⁵ is a leaving group such as for example chlorine, bromine or iodine, can be made by reacting compounds of the formula (XVlla), or a salt thereof, wherein R¹, R³, R^{5a} and R^{5b} have the same meaning as given above for compounds of the formula (I), and in which X⁵ is a leaving group such as for example chlorine, bromine or iodine, with compounds of the formula (II), wherein T has the same meaning as given above in Scheme 1, and wherein X¹ is a leaving group, such as a halogen or sulfonate, for instance chloride, under analogous conditions already described above in Scheme 1. Compounds of the formula (XVlla), or a salt thereof, wherein T has the same meaning as given above in Scheme 1, and wherein R¹, R³, R^{5a} and R^{5b} have the same meaning as given above for compounds of the formula (I), and in which X⁵ is a leaving group such as for example chlorine, bromine or iodine, are known from the literature, for instance from WO2021/170881, WO2021/037614, WO2020/208036, WO2020/201398 or WO2020/070049, or they can be made in analogy to descriptions found therein.

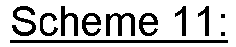

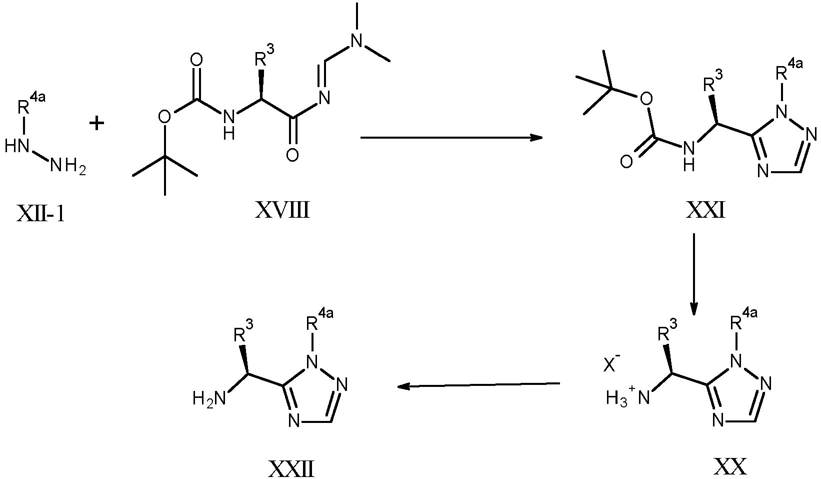

Compounds of the formula (XXII), wherein R³ and R^{4a} are as defined for compounds of the formula (I), can be made (Scheme 11) from compounds of the formula (XX), wherein R³ and R^{4a} are as defined for compounds of the formula (I) and X⁻ is an anion, by treatment with a base, such as for example a hydroxide base or a carbonate base, for example sodium hydroxide or potassium carbonate, or an ion exchange resin. Such procedures are well known to a person skilled in the art, and known from the literature and text books.

The anion X⁻ is the conjugate base of an acid, such as an inorganic acid, for instance hydrochloric acid, hydrobromic acid, hydrogen fluoride, hydrogen iodide, sulfuric acid, or the like, or of an organic acid, such as a carboxylic acid or a sulfonic acid, for instance trifluoroacetic acid, or methane sulfonic acid, or para-toluene sulfonic acid. A great number of such acids are known to a person skilled in the art.

Compounds of the formula (XX), wherein R³ and R^{4a} are as defined for compounds of the formula (I) and X⁻ is an anion, can be made from compounds of the formula (XXI), wherein R³ and R^{4a} are as defined for compounds of the formula (I), by treatment with an acid, such as the acids listed above. The reaction can be done neat or in a solvent, for instance an organic solvent, such as in methanol, CPME, tetrahydrofuran, 2-methyltetrahydrofuran, dichloromethane or in dioxane, or in an inorganic solvent, such as in water, or in a mixture of such solvents. The reaction can be done in a temperature range between -100 °C and 200 °C, more commonly between 0 °C and 150 °C, such as, for example, at ambient temperature.

Compounds of the formula (XXI), wherein R³ and R^{4a} are as defined for compounds of the formula (I), can be made, for example, from compounds of the formula (XII-1) or a tautomer thereof, or a salt thereof, in which R^{4a} is as defined for compounds of the formula (I), by treatment with compounds of the formula (XVIII), wherein R³ is as defined for compounds of the formula (I). The reaction can be done neat, or in a solvent, for instance an organic solvent, or in a mixture of solvents, such as in dioxane and acetic acid as a solvent. The reaction can be performed in the presence or in the absence of a drying agent, such as for example in the presence of molecular sieves, at a temperature between -100 °C and 200 °C, more commonly between 0 °C and 150 °C, such as, for example, at 80 °C. Compounds of the formula (XVIII), wherein R³ is as defined for compounds of the formula (I), are known, for instance from WO2021/083936 or WO2021/165195, or they can be made in analogy to descriptions found therein.

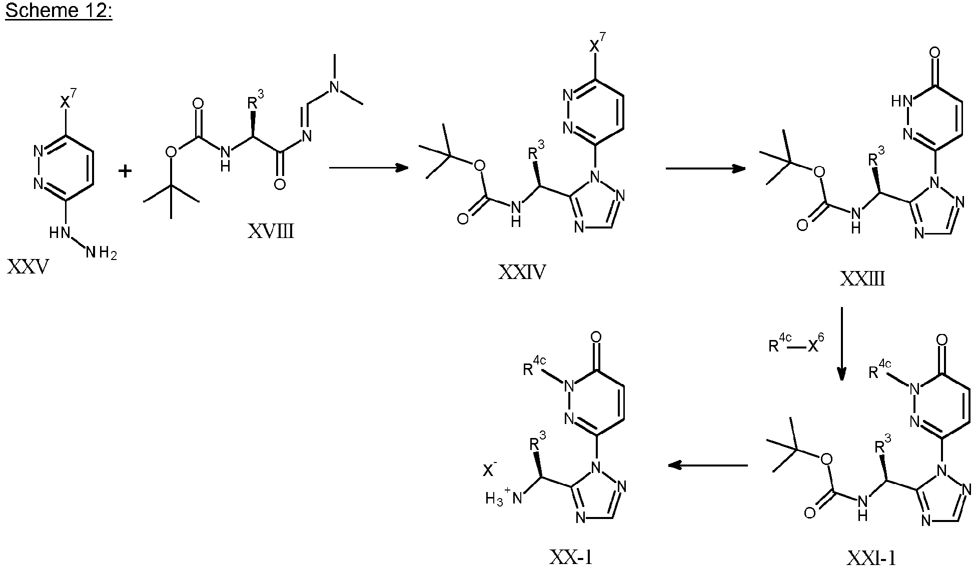

Compounds of the formula (XX-1), a subset of compounds of formula (XX), wherein R³ and R^{4c} have the same meaning as defined above for compounds of the formula (I) and X⁻ is an anion which has the same meaning as given above in Scheme 11, can be made (Scheme 12) from compounds of the formula (XXI-1), a subset of compounds of formula (XXI), wherein R³ and R^{4c} have the same meaning as defined above for compounds of the formula (I), by treatment with an acid under conditions already described above in Scheme 11 (transformation XXI into XX).

Compounds of the formula (XXI-1), a subset of compounds of formula (XXI), wherein R³ and R^{4c} have the same meaning as defined above for compounds of the formula (I), can be made by treatment of a compound of the formula (XXIII), or a tautomer thereof, wherein R³ has the same meaning as defined above for compounds of the formula (I), with a reagent of the formula R^{4c}-X⁶, wherein R^{4c} has the same meaning as defined above for compounds of the formula (I), and X⁶ is a leaving group, such as a halogen or sulfonate, for instance a chloride, bromide, iodide or mesylate, under similar conditions already described above in Scheme 8 (transformation XVa into XV).

A compound of the formula (XXIII), or a tautomer thereof, wherein R³ has the same meaning as defined above for compounds of the formula (I), can be made by treatment of compounds of the formula (XXIV), wherein R³ has the same meaning as defined above for compounds of the formula (I) and wherein X⁷ is a leaving group, such as a halogen, for instance a chloride, bromide or iodide, with for example benzaldoxime PhC=NOH, preferably (E)-benzaldehyde oxime, in the presence of a base, such as potassium or cesium carbonate, optionally in the presence of a palladium catalyst such as RockPhos-G3-palladacycle ( [(2-Di-tert-butylphosphino-3-methoxy-6-methyl-2',4',6'-triisopropyl-1,1'-biphenyl)-2-(2-aminobiphenyl)]palladium(II) methanesulfonate), in an aprotic solvent such as acetonitrile or N,N-dimethylformamide DMF, at temperatures between 0 and 100 °C, preferably between room temperature and 80 °C, as described, for example, in Angew. Chem. Int. Ed. 56 (16), 4478-4482, 2017.

Compound of the formula (XXIV), wherein R³ has the same meaning as defined above for compounds of the formula (I) and wherein X⁷ is a leaving group, such as a halogen, for instance a chloride, bromide or iodide, can be made by treatment of compounds of the formula (XXV), or a tautomer thereof, or a salt thereof, wherein X⁷ is a leaving group, such as a halogen, for instance a chloride, bromide or iodide, with a compound of the formula (XVIII), wherein R³ is as defined for compounds of the formula (I), under similar conditions already described above in Scheme 11 (transformation XII-1 + XVIII into XXI). Compounds of the formula (XXV), or a tautomer thereof, or a salt thereof, wherein X⁷ is a leaving group, such as a halogen, for instance a chloride, bromide or iodide, are known or even commercially available, or they can be made by known methods.

Compounds of the formula (XXIII) (all substituents as defined above under Scheme 12) may exist in a different tautomeric form 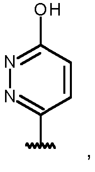 where the staggered line represents the connection to the remainder of said compounds of the formula (XXIII). This invention covers all such tautomers and mixtures thereof in all proportions.

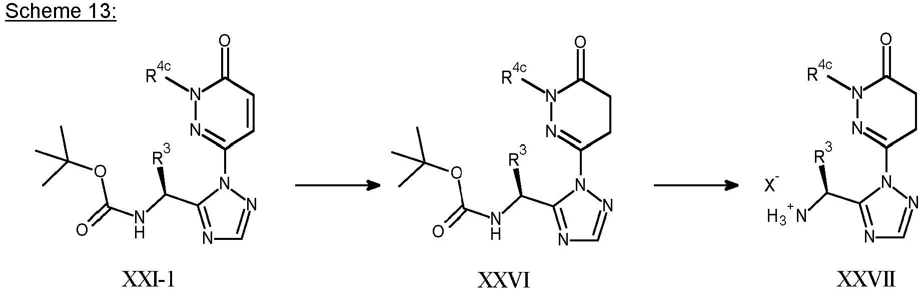

Compounds of the formula (XXVII), wherein R³ and R^{4c} have the same meaning as defined above for compounds of the formula (I) and X⁻ is an anion which has the same meaning as given above in Scheme 11, can be made (Scheme 13) from compounds of the formula (XXVI), wherein R³ and R^{4c} hav the same meaning as defined above for compounds of the formula (I), by treatment with an acid under conditions already described above in Scheme 11 (transformation XXI into XX).

Compounds of the formula (XXVI), wherein R³ and R^{4c} have the same meaning as defined above for compounds of the formula (I), can be made from compounds of the formula (XXI-I) described above, wherein R³ and R^{4c} have the same meaning as defined above for compounds of the formula (I), by a hydrogenation reaction under analogous conditions already described above in Scheme 7.

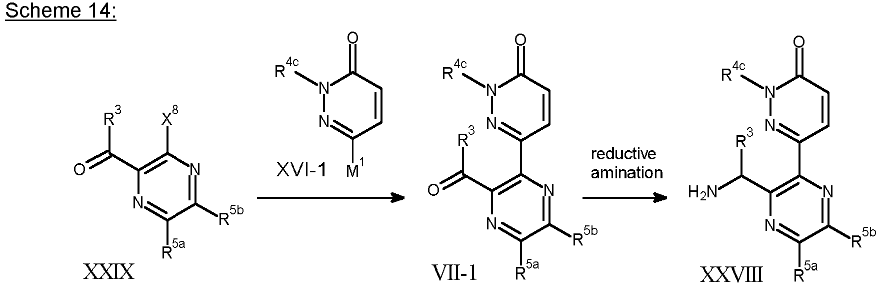

Compounds of the formula (XXVIII), or a salt thereof (such as a hydrohalide salt, preferably a hydrochloride or a hydrobromide salt, or a trifluoroacetic acid salt, or any other equivalent salt), wherein R³, R^{4c}, R^{5a} and R^{5b} have the same meaning as defined above for compounds of the formula (I), can be made (Scheme 14) from compounds of the formula (VII-1), a subset of compounds of formula (VII), wherein R³, R^{4c}, R^{5a} and R^{5b} have the same meaning as defined above for compounds of the formula (I), by a reductive amination reaction under analogous conditions already described above in Scheme 2 (transformation VII into III).

Compounds of the formula (VII-1), a subset of compounds of formula (VII), wherein R³, R^{4c}, R^{5a} and R^{5b} have the same meaning as defined above for compounds of the formula (I), can be made by treatment of compounds of the formula (XXIX), wherein R³, R^{5a} and R^{5b} have the same meaning as defined above for compounds of the formula (I), and in which X⁸ is a leaving group, such as a halogen, for instance a chloride, bromide or iodide, with a compound of the formula (XVI-1), a subset of compounds of formula (XVI), wherein R^{4c} has the same meaning as defined above for compounds of the formula (I) and M¹ is a metal-containing substituent which has the same meaning as given above in Scheme 9, under similar conditions already described above in Scheme 10 (transformation XVII + XVI into If). Compounds of the formula (XXIX), wherein R³, R^{5a} and R^{5b} have the same meaning as defined above for compounds of the formula (I), and in which X⁸ is a leaving group, such as a halogen, for instance a chloride, bromide or iodide, are known or even commercially available, or they can be made by known methods.

Alternatively, compounds of the formula (VII-1), a subset of compounds of formula (VII), wherein R³, R^{4c}, R^{5a} and R^{5b} have the same meaning as defined above for compounds of the formula (I), can be made by oxidation of compounds of the formula (XXXI) described below (Scheme 15), wherein R³, R^{4c}, R^{5a} and R^{5b} have the same meaning as defined above for compounds of the formula (I), for example using Dess-Martin periodinane (or similar hypervalent iodine reagents), commonly conducted in chlorinated solvents, such as dichloromethane or chloroform, at temperatures between 0 and 50 °C, preferably around room temperature.

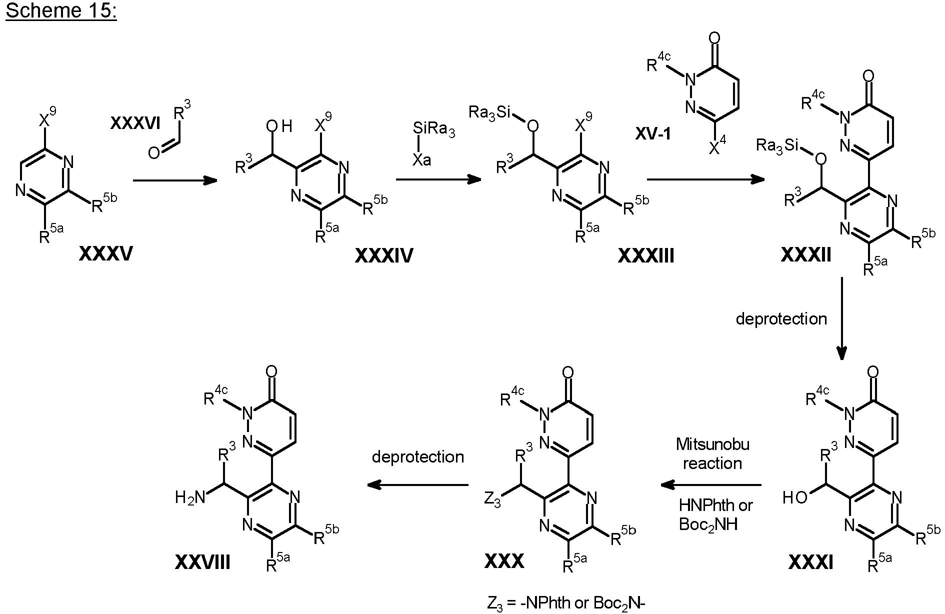

Alternatively, compounds of the formula (XXVIII), or a salt thereof (such as a hydrohalide salt, preferably a hydrochloride or a hydrobromide salt, or a trifluoroacetic acid salt, or any other equivalent salt), wherein R³, R^{4c}, R^{5a} and R^{5b} have the same meaning as defined above for compounds of the formula (I), can be made (Scheme 15) from compounds of the formula (XXX), wherein R³, R^{4c}, R^{5a} and R^{5b} have the same meaning as defined above for compounds of the formula (I) and Z₃ is -NPhth (N-phthalimide group) or -NBoc₂ (N-bis(tert-butyloxycarbonyl) group), typically by treatment with either hydrazine (preferably hydrazine hydrate or hydrazine monohydrate) in an alcohol solvent such as ethanol or isopropanol (Z₃ is -NPhth), or with an acid such as trifluoroacetic acid or hydrochloric acid in the presence of a suitable solvent such as dichloromethane, tetrahydrofuran, 2-methyltetrahydrofuran or dioxane (Z₃ is -NBoc₂), under deprotection conditions known to a person skilled in the art, and described in the literature, such as for example in: Protective Groups in Organic Synthesis, 3rd Edition Theodora W. Green (The Rowland Institute for Science) and Peter G. M. Wuts (Pharmacia and Upjohn Company), John Wiley & Sons, Inc., New York, NY. 1999, ISBN 0-471-16019-9.

Compounds of the formula (XXX), wherein R³, R^{4c}, R^{5a} and R^{5b} have the same meaning as defined above for compounds of the formula (I) and Z₃ is -NPhth (N-phthalimide group) or -NBoc₂ (N-bis(tert-butyloxycarbonyl) group), can be made from compounds of the formula (XXXI), wherein R³, R^{4c}, R^{5a} and R^{5b} have the same meaning as defined above for compounds of the formula (I), by a Mitsunobu reaction. Such a reaction involves treating compounds of the formula (XXXI) with an azodicarboxylate, such as diethyl azodicarboxylate or diisopropyl azodicarboxylate, in the presence of a phosphine, such as triphenylphosphine or tributylphosphine, and of an amine such as phthalimide (HNPhth) or bis(tert-butoxycarbonyl)amine (HNBoc₂). Mitsunobu reactions (and conditions to perform them) are known by those skilled in the art and described for instance in Chem. Rev. 2009, 109, 2551-2651.

Compounds of the formula (XXXI), wherein R³, R^{4c}, R^{5a} and R^{5b} have the same meaning as defined above for compounds of the formula (I), can be made from silyl ether compounds of the formula (XXXII), wherein R³, R^{4c}, R^{5a} and R^{5b} have the same meaning as defined above for compounds of the formula (I), and in which each of the group Ra is independently C₁-C₄alkyl, by deprotection, for instance by treatment with fluoride, for example with tetrabutylammonium fluoride, in an inert solvent, such as for example tetrahydrofuran or 2-methyltetrahydrofuran. The reaction can be done in a temperature range of -10°C to 80°C, for instance between 0°C and 30°C. Such deprotection reactions are known to a person skilled in the art, and described in the literature, for instance in: Protective Groups in Organic Synthesis, 3rd Edition Theodora W. Green (The Rowland Institute for Science) and Peter G. M. Wuts (Pharmacia and Upjohn Company). John Wiley & Sons, Inc., New York, NY. 1999, ISBN 0-471-16019-9.

Alternatively, compounds of the formula (XXXI), wherein R³, R^{4c}, R^{5a} and R^{5b} have the same meaning as defined above for compounds of the formula (I), may be made by reduction of compounds of the formula (VII-1) described above (Scheme 14), a subset of compounds of formula (VII), wherein R³, R^{4c}, R^{5a} and R^{5b} have the same meaning as defined above for compounds of the formula (I), for example with sodium borohydride NaBH₄, under conditions known known to a person skilled in the art (see for example WO2012/082997, p. 141), preferably in MeOH as solvent.

Compounds of the formula (XXXII), wherein R³, R^{4c}, R^{5a} and R^{5b} have the same meaning as defined above for compounds of the formula (I), and in which each of the group Ra is independently C₁-C₄alkyl, can be made from silyl ether compounds of the formula (XXXIII), wherein R³, R^{5a} and R^{5b} have the same meaning as defined above for compounds of the formula (I), X⁹ is a leaving group, such as a halogen, for instance a bromide or iodide, and in which each of the group Ra is independently C₁-C₄alkyl, by metalation, such as by treatment with a Turbo Grignard reagent (iPrMgCl·LiCl) or with butyl lithium. Such metalations are known to a person skilled in the art, and described in the literature, for instance in Carey, Francis A. (2007). "Organometallic compounds of Group I and II metals". Advanced Organic Chemistry: Reaction and Synthesis Pt. B (Kindle ed.). Springer. ISBN 978-0-387-44899-2. The lithium- or magnesium species thus generated can be transmetalated, for instance with a zinc halide, for example zinc chloride, and subsequently coupled with compounds of the formula (XV-1), a subset of compounds of the formula (XV), wherein R^{4c} has the same meaning as defined above for compounds of the formula (I), and X⁴ is a leaving group, such as a halogen, for example a bromide or iodide, in the presence of a catalyst, for instance a palladium catalyst, for example tris(dibenzylideneacetone)dipalladium(0), and of a ligand, for instance a phosphine ligand, such as for example tri(2-furyl)phosphine, in an inert solvent, such as for example tetrahydrofuran or 2-methyltetrahydrofuran. The reaction can be done in a temperature range of -100°C to 100°C, for instance between -78°C and 80°C. This transformation is known to a person skilled in the art, for instance as Negishi cross-coupling reaction, and described in the literature, for example in: Jie Jack Li, Name Reactions, A Collection of Detailed Mechanisms and Synthetic Applications, Springer, ISBN: 978-3-030-50865-4.

Compounds of the formula (XXXIII), wherein R³, R^{5a} and R^{5b} have the same meaning as defined above for compounds of the formula (I), X⁹ is a leaving group, such as a halogen, for instance a bromide or iodide, and in which each of the group Ra is independently C₁-C₄alkyl, can be made from compounds of the formula (XXXIV), wherein R³, R^{5a} and R^{5b} have the same meaning as defined above for compounds of the formula (I), and X⁹ is a leaving group, such as a halogen, for instance a bromide or iodide, by treatment with a silylating agent of the formula Ra₃Si-Xa, wherein Xa is a leaving group, such as for example chloride, bromide, iodide or triflate, and in which each of the group Ra is independently C₁-C₄alkyl (Ra₃Si is trialkylsilyl, for instance dimethyl-tert-butylsilyl; in compounds Ra₃Si-Xa, Ra is a straight or branched C₁-C₄alkyl, such as methyl or tert-butyl), in the presence of a base, such as an amine base, for instance imidazole, in an inert solvent, such as for example tetrahydrofuran or 2-methyltetrahydrofuran. The reaction can be done in a temperature range of 0°C to 100°C, for instance between 10°C and 80°C. Such silylation reactions are known to a person skilled in the art, and described in the literature, such as for example in: Protective Groups in Organic Synthesis, 3rd Edition Theodora W. Green (The Rowland Institute for Science) and Peter G. M. Wuts (Pharmacia and Upjohn Company). John Wiley & Sons, Inc., New York, NY. 1999, ISBN 0-471-16019-9.

Compounds of the formula (XXXIV), wherein R³, R^{5a} and R^{5b} have the same meaning as defined above for compounds of the formula (I), and X⁹ is a leaving group, such as a halogen, for instance a bromide or iodide, can be made from compounds of the formula (XXXV), wherein R^{5a} and R^{5b} have the same meaning as defined above for compounds of the formula (I), and X⁹ is a leaving group, such as a halogen, for instance a bromide or iodide, by treatment with a base, such as a lithium amide base, for instance lithium 2,2,6,6-tetramethylpiperidide, followed by reaction of the lithiated species with aldehyde compounds of the formula (XXXVI), wherein R³ has the same meaning as given above in formula (I). This reaction can be done neat or in a solvent, for instance in an organic solvent, such as for example in tetrahydrofuran or 2-methyltetrahydrofuran as a solvent. The reaction can be done in a temperature range of -100°C to 100°C, for instance between -80°C and 0°C, for example at 0°C or at -78°C. Compounds of the formula (XXXV), wherein R^{5a} and R^{5b} have the same meaning as defined above for compounds of the formula (I), and X⁹ is a leaving group, such as a halogen, for instance a bromide or iodide, and compounds of the formula (XXXVI), wherein R³ has the same meaning as given above in formula (I), are known or even commercially available, or they can be made by known methods.

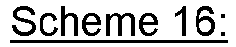

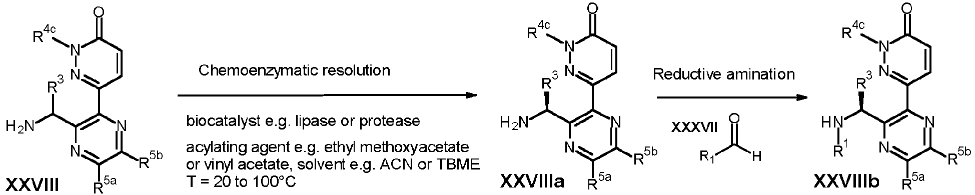

Compounds of formula (XXVlllb), or a salt thereof (such as a hydrohalide salt, preferably a hydrochloride or a hydrobromide salt, or a trifluoroacetic acid salt, or any other equivalent salt), wherein R¹, R³, R^{4c}, R^{5a} and R^{5b} have the same meaning as defined above for compounds of the formula (I), may be made (Scheme 16) by treatment of compounds of formula (XXVllla), or a salt thereof, wherein R³, R^{4c}, R^{5a} and R^{5b} have the same meaning as defined above for compounds of the formula (I), with compounds of formula (XXXVII), wherein R¹ is as defined in formula I, by a reductive amination reaction under analogous conditions already described above in Scheme 2 (transformation VII into III).

Compounds of formula (XXVllla), or a salt thereof, wherein R³, R^{4c}, R^{5a} and R^{5b} have the same meaning as defined above for compounds of the formula (I), may be obtained by biocatalyzed deracemization of compounds of formula (XXVIII), or a salt thereof, wherein R³, R^{4c}, R^{5a} and R^{5b} have the same meaning as defined above for compounds of the formula (I). This may be done for instance using a lipase, e.g. *Candida Antarctica* lipase B or *Pseudomonas fluorescens* lipase, eventually in immobilized form (e.g. Novozym^{®} 435) in presence of an acyl donor, e.g. ethyl methoxyacetate or vinyl acetate, in a suitable solvent such as acetonitrile or methyl tert-butyl ether at temperatures between 20 °C to 100 °C. Such processes are described for instance in J. Org. Chem. 2007, 72, 6918-6923 or Adv. Synth. Catal. 2007, 349, 1481-1488. The expected stereochemical outcome of such enzymatic deracemization are known of those skilled in the art and are documented in the literature, for instance in J. Org. Chem. 1991, 56, 2656-2665 or J. Am. Chem. Soc. 2015, 137, 3996-4009.

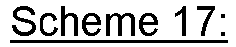

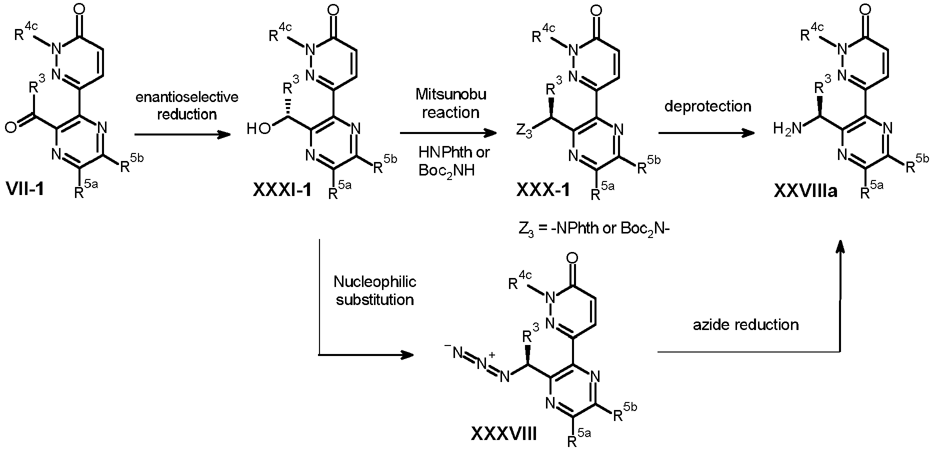

In an alternative process (Scheme 17), compounds of formula (XXVIII), or a salt thereof, wherein R³, R^{4c}, R^{5a} and R^{5b} have the same meaning as defined above for compounds of the formula (I), may be obtained from compounds of the formula (XXX-1), wherein R³, R^{4c}, R^{5a} and R^{5b} have the same meaning as defined above for compounds of the formula (I) and Z₃ is -NPhth (N-phthalimide group) or -NBoc₂ (N-bis(*tert*-butyloxycarbonyl) group), under deprotection conditions already described above in Scheme 15 (transformation XXX into XXVIII).

Compounds of the formula (XXX-1), wherein R³, R^{4c}, R^{5a} and R^{5b} have the same meaning as defined above for compounds of the formula (I) and Z₃ is -NPhth (N-phthalimide group) or -NBoc₂ (N-bis(*tert-*butyloxycarbonyl) group), may be obtained from compounds of the formula (XXXI-1), wherein R³, R^{4c}, R^{5a} and R^{5b} have the same meaning as defined above for compounds of the formula (I), by a Mitsunobu reaction with phthalimide (HNPhth) or bis(*tert*-butoxycarbonyl)amine(HNBoc₂) under conditions already described above in Scheme 15 (transformation XXXI into XXX). Such processes are known by those skilled in the art to proceed with inversion of the stereocenter.

Compounds of the formula (XXXI-1), wherein R³, R^{4c}, R^{5a} and R^{5b} have the same meaning as defined above for compounds of the formula (I), may be obtained by enantioselective reduction of ketones of formula (VII-1), wherein R³, R^{4c}, R^{5a} and R^{5b} have the same meaning as defined above for compounds of the formula (I). Such reductions can be done using a catalyst, for instance a ruthenium or a rhodium catalyst with a chiral ligand such as RuCl[(*R,R*)-TsDPEN](mesitylene) or RuBF₄[(*R*,*R*)-TsDPEN](*p-*cymene) in the presence of a hydrogen donor system such as for example HCOOH/Et₃N or HCO₂NH₄. Such processes are described in the literature for instance in J. Org. Chem. 2017, 82, 5607. Alternatively, compounds of formula (XXVllla), or a salt thereof, wherein R³, R^{4c}, R^{5a} and R^{5b} have the same meaning as defined above for compounds of the formula (I), may be obtained by reduction of azide compounds of formula (XXXVIII), wherein R³, R^{4c}, R^{5a} and R^{5b} have the same meaning as defined above for compounds of the formula (I), by treatment with for instance triphenylphosphine (or tributylphosphine) and water (2 steps Staudinger reduction), or by hydrogenation using for example a palladium catalyst in the presence of hydrogen. Procedures and conditions for such azide reductions are well known to a person skilled in the art, and known from the literature and text books. Compounds of formula (XXXVIII), wherein R³, R^{4c}, R^{5a} and R^{5b} have the same meaning as defined above for compounds of the formula (I), may be obtained by treatment of alcohol compounds of the formula (XXXI-1), wherein R³, R^{4c}, R^{5a} and R^{5b} have the same meaning as defined above for compounds of the formula (I), with an azidation reagent such as diphenyl phosphoryl azide (amongst others like sodium azide, trimethylsilyl azide or tetrabutylammonium azide), in a solvent such as toluene, tetrahydrofuran or 2-methyltetrahydrofuran, in the presence of a base such as for example 1,8-diazabicyclo(5.4.0)undec-7-ene DBU, and at temperatures preferably around room temperature. Such processes are known by those skilled in the art to proceed with inversion of the stereocenter and are described in the literature for instance in Adv. Synth. Catal. 2018, 360, 2157-2165.

Any of the compounds of the formula (XX), (XXII) (substituents as defined in Scheme 11), formula (XX-1) (substituents as defined in Scheme 12), formula (XXVII) (substituents as defined in Scheme 13), formula (XXVIII) (substituents as defined in Schemes 14/15), formula (XXVIIIa) (substituents as defined in Schemes 16/17), and formula (XXVIIIb) (substituents as defined in Schemes 16), or (where applicable) a salt thereof, or (where applicable) a free base thereof, represent a particular subset of compounds of the formula (III) defined above in Scheme 1, hence can be used according to descriptions outlined in said Scheme 1 for the preparation of compounds of the formula (I).

Compounds of the formula (XXVIII), (XXVIIIa) or (XXVIIIb) (substituent definitions as in Schemes 14, 15, 16 and 17) wherein the fragment 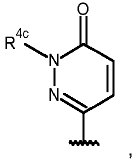 in which R^{4c} is as defined above under formula (I) and where the staggered line represents the connection to the remainder of said compounds of the formula (XXVIII), (XXVIIIa) or (XXVIIIb), is replaced by the fragment 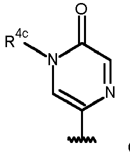 can be accessed in analogy to chemistry descriptions found in Schemes 14, 15, 16 and 17, but by replacing the starting materials of the formula XVI-1 and XV-1 with respectively compounds of the formula XVI-2 and XV-2, wherein R^{4c}, X⁴ and M¹ retain their definitions:

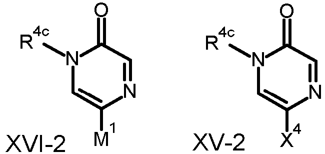

Such compounds of the formula XVI-2 and XV-2 are known or even commercially available, or they can be made by known methods.

Depending on the procedure or the reaction conditions, the reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reactions are advantageously carried out in a temperature range from approximately -80°C to approximately +140°C, preferably from approximately -30°C to approximately +100°C, in many cases in the range between ambient temperature and approximately +80°C.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula (I) can be prepared in a manner known *per se.* Thus, for example, acid addition salts of compounds of formula (I) are obtained by treatment with a suitable acid or a suitable ion exchanger reagent and salts with bases are obtained by treatment with a suitable base or with a suitable ion exchanger reagent.

Salts of compounds of formula (I) can be converted in the customary manner into the free compounds of formula (I), acid addition salts, for example, by treatment with a suitable basic compound or with a suitable ion exchanger reagent and salts with bases, for example, by treatment with a suitable acid or with a suitable ion exchanger reagent.

Salts of compounds of formula (I) can be converted in a manner known per se into other salts of compounds of formula (I), acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula (I), which have salt-forming properties can be obtained in free form or in the form of salts.

The compounds of formula (I) and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula (I), in free form or in salt form, which can be obtained depending on which starting materials and procedures have been chosen can be separated in a known manner into the pure diastereomers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl cellulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

N-oxides can be prepared by reacting a compound of the formula (I) with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 2000/15615.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula (I) and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of formula (I) according to the following Tables A-1 to A-78, B-1 to B-78, C-1 to C-21 and D-1 to D-21 can be prepared according to the methods described above. The examples which follow are intended to illustrate the invention and show preferred compounds of formula (I), in the form of a compound of formula (I-A), (I-B), (I-C) and (I-D).

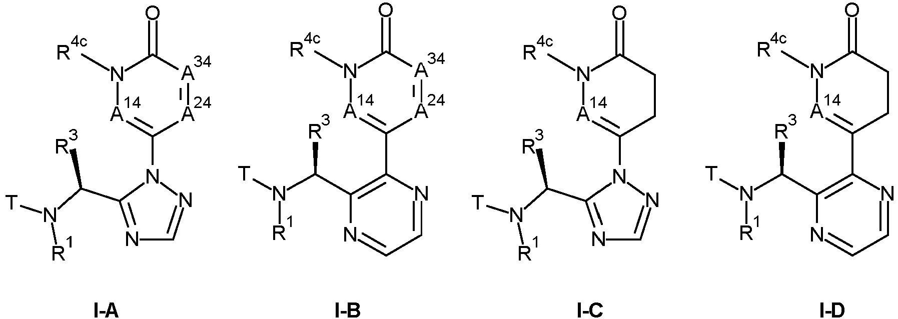

### Tables A-1 to A-78 (Formula I-A)

Table A-1 provides 32 compounds A-1.001 to A-1.032 of formula I-A wherein A¹⁴ is N, A²⁴ is N, A³⁴ is N, R¹ is H, R³ is CH₃, R^{4c} is CH₃ and T are as defined in table Z. For example, A-1.002 is

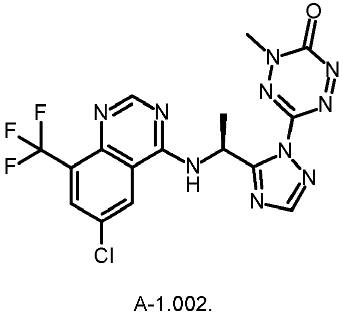

Table A-2 provides 32 compounds A-2.001 to A-2.032 of formula I-A wherein A¹⁴ is N, A¹⁴ is N, A³⁴ is N, R¹ is H, R³ is CH₃, R^{4c} is CH₂-CF₂H and T are as defined in table Z.

Table A-3 provides 32 compounds A-3.001 to A-3.032 of formula I-A wherein A¹⁴ is N, A²⁴ is N, A³⁴ is N, R¹ is H, R³ is CH₃, R^{4c} is CH₂-cyclopropyl and T are as defined in table Z.

Table A-4 provides 32 compounds A-4.001 to A-4.032 of formula I-A wherein A¹⁴ is N, A²⁴ is N, A³⁴ is N, R¹ is CH₃, R³ is CH₃, R^{4c} is CH₃ and T are as defined in table Z.

Table A-5 provides 32 compounds A-5.001 to A-5.032 of formula I-A wherein A¹⁴ is N, A²⁴ is N, A³⁴ is N, R¹ is CH₃, R³ is CH₃, R^{4c} is CH₂-CF₂H and T are as defined in table Z.

Table A-6 provides 32 compounds A-6.001 to A-6.032 of formula I-A wherein A¹⁴ is N, A²⁴ is N, A³⁴ is N, R¹ is CH₃, R³ is CH₃, R^{4c} is CH₂-cyclopropyl and T are as defined in table Z.

Table A-7 provides 32 compounds A-7.001 to A-7.032 of formula I-A wherein A¹⁴ is N, A²⁴ is N, A³⁴ is **N,** R¹ is CH₂-cyclopropyl, R³ is CH₃, R^{4c} is CH₃ and T are as defined in table Z.

Table A-8 provides 32 compounds A-8.001 to A-8.032 of formula I-A wherein A¹⁴ is N, A²⁴ is N, A³⁴ is N, R¹ is CH₂-cyclopropyl, R³ is CH₃, R^{4c} is CH₂-CF₂H and T are as defined in table Z.

Table A-9 provides 32 compounds A-9.001 to A-9.032 of formula I-A wherein A¹⁴ is N, A²⁴ is N, A³⁴ is N, R¹ is CH₂-cyclopropyl, R³ is CH₃, R^{4c} is CH₂-cyclopropyl and T are as defined in table Z.

Table A-10 provides 32 compounds A-10.001 to A-10.032 of formula I-A wherein A¹⁴ is N, A²⁴ is N, A³⁴ is CH, R¹ is H, R³ is CH₃, R^{4c} is CH₃ and T are as defined in table Z.

Table A-11 provides 32 compounds A-11.001 to A-11.032 of formula I-A wherein A¹⁴ is N, A²⁴ is N, A³⁴ is CH, R¹ is H, R³ is CH₃, R^{4c} is CH₂-CF₂H and T are as defined in table Z.

Table A-12 provides 32 compounds A-12.001 to A-12.032 of formula I-A wherein A¹⁴ is N, A²⁴ is N, A³⁴ is CH, R¹ is H, R³ is CH₃, R^{4c} is CH₂-cyclopropyl and T are as defined in table Z.

Table A-13 provides 32 compounds A-13.001 to A-13.032 of formula I-A wherein A¹⁴ is N, A²⁴ is N, A³⁴ is CH, R¹ is CH₃, R³ is CH₃, R^{4c} is CH₃ and T are as defined in table Z.

Table A-14 provides 32 compounds A-14.001 to A-14.032 of formula I-A wherein A¹⁴ is N, A²⁴ is N, A³⁴ is CH, R¹ is CH₃, R³ is CH₃, R^{4c} is CH₂-CF₂H and T are as defined in table Z.

Table A-15 provides 32 compounds A-15.001 to A-15.032 of formula I-A wherein A¹⁴ is N, A²⁴ is N, A³⁴ is CH, R¹ is CH₃, R³ is CH₃, R^{4c} is CH₂-cyclopropyl and T are as defined in table Z.

Table A-16 provides 32 compounds A-16.001 to A-16.032 of formula I-A wherein A¹⁴ is N, A²⁴ is N, A³⁴ is CH, R¹ is CH₂-cyclopropyl, R³ is CH₃, R^{4c} is CH₃ and T are as defined in table Z.

Table A-17 provides 32 compounds A-17.001 to A-17.032 of formula I-A wherein A¹⁴ is N, A²⁴ is N, A³⁴ is CH, R¹ is CH₂-cyclopropyl, R³ is CH₃, R^{4c} is CH₂-CF₂H and T are as defined in table Z.

Table A-18 provides 32 compounds A-18.001 to A-18.032 of formula I-A wherein A¹⁴ is N, A²⁴ is N, A³⁴ is CH, R¹ is CH₂-cyclopropyl, R³ is CH₃, R^{4c} is CH₂-cyclopropyl and T are as defined in table Z.

Table A-19 provides 32 compounds A-19.001 to A-19.032 of formula I-A wherein A¹⁴ is N, A²⁴ is CH, A³⁴ is N, R¹ is H, R³ is CH₃, R^{4c} is CH₃ and T are as defined in table Z.

Table A-20 provides 32 compounds A-20.001 to A-20.032 of formula I-A wherein A¹⁴ is N, A²⁴ is CH, A³⁴ is N, R¹ is H, R³ is CH₃, R^{4c} is CH₂-CF₂H and T are as defined in table Z.

Table A-21 provides 32 compounds A-21.001 to A-21.032 of formula I-A wherein A¹⁴ is N, A²⁴ is CH, A³⁴ is N, R¹ is H, R³ is CH₃, R^{4c} is CH₂-cyclopropyl and T are as defined in table Z.

Table A-22 provides 32 compounds A-22.001 to A-22.032 of formula I-A wherein A¹⁴ is N, A²⁴ is CH, A³⁴ is N, R¹ is CH₃, R³ is CH₃, R^{4c} is CH₃ and T are as defined in table Z.

Table A-23 provides 32 compounds A-23.001 to A-23.032 of formula I-A wherein A¹⁴ is N, A²⁴ is CH, A³⁴ is N, R¹ is CH₃, R³ is CH₃, R^{4c} is CH₂-CF₂H and T are as defined in table Z.

Table A-24 provides 32 compounds A-24.001 to A-24.032 of formula I-A wherein A¹⁴ is N, A²⁴ is CH, A³⁴ is N, R¹ is CH₃, R³ is CH₃, R^{4c} is CH₂-cyclopropyl and T are as defined in table Z.

Table A-25 provides 32 compounds A-25.001 to A-25.032 of formula I-A wherein A¹⁴ is N, A²⁴ is CH, A³⁴ is N, R¹ is CH₂-cyclopropyl, R³ is CH₃, R^{4c} is CH₃ and T are as defined in table Z.

Table A-26 provides 32 compounds A-26.001 to A-26.032 of formula I-A wherein A¹⁴ is N, A²⁴ is CH, A³⁴ is N, R¹ is CH₂-cyclopropyl, R³ is CH₃, R^{4c} is CH₂-CF₂H and T are as defined in table Z.

Table A-27 provides 32 compounds A-27.001 to A-27.032 of formula I-A wherein A¹⁴ is N, A²⁴ is CH, A³⁴ is N, R¹ is CH₂-cyclopropyl, R³ is CH₃, R^{4c} is CH₂-cyclopropyl and T are as defined in table Z.

Table A-28 provides 32 compounds A-28.001 to A-28.032 of formula I-A wherein A¹⁴ is N, A²⁴ is CH, A³⁴ is CH, R¹ is H, R³ is CH₃, R^{4c} is CH₃ and T are as defined in table Z.

Table A-29 provides 32 compounds A-29.001 to A-29.032 of formula I-A wherein A¹⁴ is N, A²⁴ is CH, A³⁴ is CH, R¹ is H, R³ is CH₃, R^{4c} is CH₂-CF₂H and T are as defined in table Z.

Table A-30 provides 32 compounds A-30.001 to A-30.032 of formula I-A wherein A¹⁴ is N, A²⁴ is CH, A³⁴ is CH, R¹ is H, R³ is CH₃, R^{4c} is CH₂-cyclopropyl and T are as defined in table Z.

Table A-31 provides 32 compounds A-31.001 to A-31.032 of formula I-A wherein A¹⁴ is N, A²⁴ is CH, A³⁴ is CH, R¹ is CH₃, R³ is CH₃, R^{4c} is CH₃ and T are as defined in table Z.

Table A-32 provides 32 compounds A-32.001 to A-32.032 of formula I-A wherein A¹⁴ is N, A²⁴ is CH, A³⁴ is CH, R¹ is CH₃, R³ is CH₃, R^{4c} is CH₂-CF₂H and T are as defined in table Z.

Table A-33 provides 32 compounds A-33.001 to A-33.032 of formula I-A wherein A¹⁴ is N, A²⁴ is CH, A³⁴ is CH, R¹ is CH₃, R³ is CH₃, R^{4c} is CH₂-cyclopropyl and T are as defined in table Z.

Table A-34 provides 32 compounds A-34.001 to A-34.032 of formula I-A wherein A¹⁴ is N, A²⁴ is CH, A³⁴ is CH, R¹ is CH₂-cyclopropyl, R³ is CH₃, R^{4c} is CH₃ and T are as defined in table Z.

Table A-35 provides 32 compounds A-35.001 to A-35.032 of formula I-A wherein A¹⁴ is N, A²⁴ is CH, A³⁴ is CH, R¹ is CH₂-cyclopropyl, R³ is CH₃, R^{4c} is CH₂-CF₂H and T are as defined in table Z.

Table A-36 provides 32 compounds A-36.001 to A-36.032 of formula I-A wherein A¹⁴ is N, A²⁴ is CH, A³⁴ is CH, R¹ is CH₂-cyclopropyl, R³ is CH₃, R^{4c} is CH₂-cyclopropyl and T are as defined in table Z.

Table A-37 provides 32 compounds A-37.001 to A-37.032 of formula I-A wherein A¹⁴ is CH, A²⁴ is N, A³⁴ is N, R¹ is H, R³ is CH₃, R^{4c} is CH₃ and T are as defined in table Z.

Table A-38 provides 32 compounds A-38.001 to A-38.032 of formula I-A wherein A¹⁴ is CH, A²⁴ is N, A³⁴ is N, R¹ is H, R³ is CH₃, R^{4c} is CH₂-CF₂H and T are as defined in table Z.

Table A-39 provides 32 compounds A-39.001 to A-39.032 of formula I-A wherein A¹⁴ is CH, A²⁴ is N, A³⁴ is N, R¹ is H, R³ is CH₃, R^{4c} is CH₂-cyclopropyl and T are as defined in table Z.

Table A-40 provides 32 compounds A-40.001 to A-40.032 of formula I-A wherein A¹⁴ is CH, A²⁴ is N, A³⁴ is N, R¹ is CH₃, R³ is CH₃, R^{4c} is CH₃ and T are as defined in table Z.

Table A-41 provides 32 compounds A-41.001 to A-41.032 of formula I-A wherein A¹⁴ is CH, A²⁴ is N, A³⁴ is N, R¹ is CH₃, R³ is CH₃, R^{4c} is CH₂-CF₂H and T are as defined in table Z.

Table A-42 provides 32 compounds A-42.001 to A-42.032 of formula I-A wherein A¹⁴ is CH, A²⁴ is N, A³⁴ is N, R¹ is CH₃, R³ is CH₃, R^{4c} is CH₂-cyclopropyl and T are as defined in table Z.

Table A-43 provides 32 compounds A-43.001 to A-43.032 of formula I-A wherein A¹⁴ is CH, A²⁴ is N, A³⁴ is N, R¹ is CH₂-cyclopropyl, R³ is CH₃, R^{4c} is CH₃ and T are as defined in table Z.

Table A-44 provides 32 compounds A-44.001 to A-44.032 of formula I-A wherein A¹⁴ is CH, A²⁴ is N, A³⁴ is N, R¹ is CH₂-cyclopropyl, R³ is CH₃, R^{4c} is CH₂-CF₂H and T are as defined in table Z.

Table A-45 provides 32 compounds A-45.001 to A-45.032 of formula I-A wherein A¹⁴ is CH, A²⁴ is N, A³⁴ is N, R¹ is CH₂-cyclopropyl, R³ is CH₃, R^{4c} is CH₂-cyclopropyl and T are as defined in table Z.

Table A-46 provides 32 compounds A-46.001 to A-46.032 of formula I-A wherein A¹⁴ is CH, A²⁴ is N, A³⁴ is CH, R¹ is H, R³ is CH₃, R^{4c} is CH₃ and T are as defined in table Z.

Table A-47 provides 32 compounds A-47.001 to A-47.032 of formula I-A wherein A¹⁴ is CH, A²⁴ is N, A³⁴ is CH, R¹ is H, R³ is CH₃, R^{4c} is CH₂-CF₂H and T are as defined in table Z.

Table A-48 provides 32 compounds A-48.001 to A-48.032 of formula I-A wherein A¹⁴ is CH, A²⁴ is N, A³⁴ is CH, R¹ is H, R³ is CH₃, R^{4c} is CH₂-cyclopropyl and T are as defined in table Z.

Table A-49 provides 32 compounds A-49.001 to A-49.032 of formula I-A wherein A¹⁴ is CH, A²⁴ is N, A³⁴ is CH, R¹ is CH₃, R³ is CH₃, R^{4c} is CH₃ and T are as defined in table Z.

Table A-50 provides 32 compounds A-50.001 to A-50.032 of formula I-A wherein A¹⁴ is CH, A²⁴ is N, A³⁴ is CH, R¹ is CH₃, R³ is CH₃, R^{4c} is CH₂-CF₂H and T are as defined in table Z.

Table A-51 provides 32 compounds A-51.001 to A-51.032 of formula I-A wherein A¹⁴ is CH, A²⁴ is N, A³⁴ is CH, R¹ is CH₃, R³ is CH₃, R^{4c} is CH₂-cyclopropyl and T are as defined in table Z.

Table A-52 provides 32 compounds A-52.001 to A-52.032 of formula I-A wherein A¹⁴ is CH, A²⁴ is N, A³⁴ is CH, R¹ is CH₂-cyclopropyl, R³ is CH₃, R^{4c} is CH₃ and T are as defined in table Z.

Table A-53 provides 32 compounds A-53.001 to A-53.032 of formula I-A wherein A¹⁴ is CH, A²⁴ is N, A³⁴ is CH, R¹ is CH₂-cyclopropyl, R³ is CH₃, R^{4c} is CH₂-CF₂H and T are as defined in table Z.

Table A-54 provides 32 compounds A-54.001 to A-54.032 of formula I-A wherein A¹⁴ is CH, A²⁴ is N, A³⁴ is CH, R¹ is CH₂-cyclopropyl, R³ is CH₃, R^{4c} is CH₂-cyclopropyl and T are as defined in table Z.

Table A-55 provides 32 compounds A-55.001 to A-55.032 of formula I-A wherein A¹⁴ is CH, A²⁴ is CH, A³⁴ is N, R¹ is H, R³ is CH₃, R^{4c} is CH₃ and T are as defined in table Z.

Table A-56 provides 32 compounds A-56.001 to A-56.032 of formula I-A wherein A¹⁴ is CH, A²⁴ is CH, A³⁴ is N, R¹ is H, R³ is CH₃, R^{4c} is CH₂-CF₂H and T are as defined in table Z.

Table A-57 provides 32 compounds A-57.001 to A-57.032 of formula I-A wherein A¹⁴ is CH, A²⁴ is CH, A³⁴ is N, R¹ is H, R³ is CH₃, R^{4c} is CH₂-cyclopropyl and T are as defined in table Z.

Table A-58 provides 32 compounds A-58.001 to A-58.032 of formula I-A wherein A¹⁴ is CH, A²⁴ is CH, A³⁴ is N, R¹ is CH₃, R³ is CH₃, R^{4c} is CH₃ and T are as defined in table Z.

Table A-59 provides 32 compounds A-59.001 to A-59.032 of formula I-A wherein A¹⁴ is CH, A²⁴ is CH, A³⁴ is N, R¹ is CH₃, R³ is CH₃, R^{4c} is CH₂-CF₂H and T are as defined in table Z.

Table A-60 provides 32 compounds A-60.001 to A-60.032 of formula I-A wherein A¹⁴ is CH, A²⁴ is CH, A³⁴ is N, R¹ is CH₃, R³ is CH₃, R^{4c} is CH₂-cyclopropyl and T are as defined in table Z.

Table A-61 provides 32 compounds A-61.001 to A-61.032 of formula I-A wherein A¹⁴ is CH, A²⁴ is CH, A³⁴ is N, R¹ is CH₂-cyclopropyl, R³ is CH₃, R^{4c} is CH₃ and T are as defined in table Z.

Table A-62 provides 32 compounds A-62.001 to A-62.032 of formula I-A wherein A¹⁴ is CH, A²⁴ is CH, A³⁴ is N, R¹ is CH₂-cyclopropyl, R³ is CH₃, R^{4c} is CH₂-CF₂H and T are as defined in table Z.

Table A-63 provides 32 compounds A-63.001 to A-63.032 of formula I-A wherein A¹⁴ is CH, A²⁴ is CH, A³⁴ is N, R¹ is CH₂-cyclopropyl, R³ is CH₃, R^{4c} is CH₂-cyclopropyl and T are as defined in table Z.

Table A-64 provides 32 compounds A-64.001 to A-64.032 of formula I-A wherein A¹⁴ is CH, A²⁴ is CH, A³⁴ is CH, R¹ is H, R³ is CH₃, R^{4c} is CH₃ and T are as defined in table Z.

Table A-65 provides 32 compounds A-65.001 to A-65.032 of formula I-A wherein A¹⁴ is CH, A²⁴ is CH, A³⁴ is CH, R¹ is H, R³ is CH₃, R^{4c} is CH₂-CF₂H and T are as defined in table Z.

Table A-66 provides 32 compounds A-66.001 to A-66.032 of formula I-A wherein A¹⁴ is CH, A²⁴ is CH, A³⁴ is CH, R¹ is H, R³ is CH₃, R^{4c} is CH₂-cyclopropyl and T are as defined in table Z.

Table A-67 provides 32 compounds A-67.001 to A-67.032 of formula I-A wherein A¹⁴ is CH, A²⁴ is CH, A³⁴ is CH, R¹ is CH₃, R³ is CH₃, R^{4c} is CH₃ and T are as defined in table Z.

Table A-68 provides 32 compounds A-68.001 to A-68.032 of formula I-A wherein A¹⁴ is CH, A²⁴ is CH, A³⁴ is CH, R¹ is CH₃, R³ is CH₃, R^{4c} is CH₂-CF₂H and T are as defined in table Z.

Table A-69 provides 32 compounds A-69.001 to A-69.032 of formula I-A wherein A¹⁴ is CH, A²⁴ is CH, A³⁴ is CH, R¹ is CH₃, R³ is CH₃, R^{4c} is CH₂-cyclopropyl and T are as defined in table Z.

Table A-70 provides 32 compounds A-70.001 to A-70.032 of formula I-A wherein A¹⁴ is CH, A²⁴ is CH, A³⁴ is CH, R¹ is CH₂-cyclopropyl, R³ is CH₃, R^{4c} is CH₃ and T are as defined in table Z.

Table A-71 provides 32 compounds A-71.001 to A-71.032 of formula I-A wherein A¹⁴ is CH, A²⁴ is CH, A³⁴ is CH, R¹ is CH₂-cyclopropyl, R³ is CH₃, R^{4c} is CH₂-CF₂H and T are as defined in table Z.

Table A-72 provides 32 compounds A-72.001 to A-72.032 of formula I-A wherein A¹⁴ is CH, A²⁴ is CH, A³⁴ is CH, R¹ is CH₂-cyclopropyl, R³ is CH₃, R^{4c} is CH₂-cyclopropyl and T are as defined in table Z.

Table A-73 provides 32 compounds A-73.001 to A-73.032 of formula I-A wherein A¹⁴ is N, A²⁴ is CH, A³⁴ is CH, R¹ is H, R³ is CH₃, R^{4c} is CH₂CH₃ and T are as defined in table Z.

Table A-74 provides 32 compounds A-74.001 to A-74.032 of formula I-A wherein A¹⁴ is N, A²⁴ is CH, A³⁴ is CH, R¹ is CH₃, R³ is CH₃, R^{4c} is CH₂CH₃ and T are as defined in table Z.

Table A-75 provides 32 compounds A-75.001 to A-75.032 of formula I-A wherein A¹⁴ is N, A²⁴ is CH, A³⁴ is CH, R¹ is CH₂-cyclopropyl, R³ is CH₃, R^{4c} is CH₂CH₃ and T are as defined in table Z.

Table A-76 provides 32 compounds A-76.001 to A-76.032 of formula I-A wherein A¹⁴ is CH, A²⁴ is N, A³⁴ is CH, R¹ is H, R³ is CH₃, R^{4c} is CH₂CH₃ and T are as defined in table Z.

Table A-77 provides 32 compounds A-77.001 to A-77.032 of formula I-A wherein A¹⁴ is CH, A²⁴ is N, A³⁴ is CH, R¹ is CH₃, R³ is CH₃, R^{4c} is CH₂CH₃ and T are as defined in table Z.

Table A-78 provides 32 compounds A-78.001 to A-78.032 of formula I-A wherein A¹⁴ is CH, A²⁴ is N, A³⁴ is CH, R¹ is CH₂-cyclopropyl, R³ is CH₃, R^{4c} is CH₂CH₃ and T are as defined in table Z.

### Tables B-1 to B-78 (Formula I-B)

Table B-1 provides 32 compounds B-1.001 to B-1.032 of formula I-B wherein A¹⁴ is N, A²⁴ is N, A³⁴ is N, R¹ is H, R³ is CH₃, R^{4c} is CH₃ and T are as defined in table Z.

Table B-2 provides 32 compounds B-2.001 to B-2.032 of formula I-B wherein A¹⁴ is N, A²⁴ is N, A³⁴ is N, R¹ is H, R³ is CH₃, R^{4c} is CH₂-CF₂H and T are as defined in table Z.

Table B-3 provides 32 compounds B-3.001 to B-3.032 of formula I-B wherein A¹⁴ is N, A²⁴ is N, A³⁴ is N, R¹ is H, R³ is CH₃, R^{4c} is CH₂-cyclopropyl and T are as defined in table Z.

Table B-4 provides 32 compounds B-4.001 to B-4.032 of formula I-B wherein A¹⁴ is N, A²⁴ is N, A³⁴ is N, R¹ is CH₃, R³ is CH₃, R^{4c} is CH₃ and T are as defined in table Z.

Table B-5 provides 32 compounds B-5.001 to B-5.032 of formula I-B wherein A¹⁴ is N, A²⁴ is N, A³⁴ is N, R¹ is CH₃, R³ is CH₃, R^{4c} is CH₂-CF₂H and T are as defined in table Z.

Table B-6 provides 32 compounds B-6.001 to B-6.032 of formula I-B wherein A¹⁴ is N, A²⁴ is N, A³⁴ is N, R¹ is CH₃, R³ is CH₃, R^{4c} is CH₂-cyclopropyl and T are as defined in table Z.

Table B-7 provides 32 compounds B-7.001 to B-7.032 of formula I-B wherein A¹⁴ is N, A²⁴ is N, A³⁴ is N, R¹ is CH₂-cyclopropyl, R³ is CH₃, R^{4c} is CH₃ and T are as defined in table Z.

Table B-8 provides 32 compounds B-8.001 to B-8.032 of formula I-B wherein A¹⁴ is N, A²⁴ is N, A³⁴ is N, R¹ is CH₂-cyclopropyl, R³ is CH₃, R^{4c} is CH₂-CF₂H and T are as defined in table Z.

Table B-9 provides 32 compounds B-9.001 to B-9.032 of formula I-B wherein A¹⁴ is N, A²⁴ is N, A³⁴ is N, R¹ is CH₂-cyclopropyl, R³ is CH₃, R^{4c} is CH₂-cyclopropyl and T are as defined in table Z.

Table B-10 provides 32 compounds B-10.001 to B-10.032 of formula I-B wherein A¹⁴ is N, A²⁴ is N, A³⁴ is CH, R¹ is H, R³ is CH₃, R^{4c} is CH₃ and T are as defined in table Z.

Table B-11 provides 32 compounds B-11.001 to B-11.032 of formula I-B wherein A¹⁴ is N, A²⁴ is N, A³⁴ is CH, R¹ is H, R³ is CH₃, R^{4c} is CH₂-CF₂H and T are as defined in table Z.

Table B-12 provides 32 compounds B-12.001 to B-12.032 of formula I-B wherein A¹⁴ is N, A²⁴ is N, A³⁴ is CH, R¹ is H, R³ is CH₃, R^{4c} is CH₂-cyclopropyl and T are as defined in table Z.

Table B-13 provides 32 compounds B-13.001 to B-13.032 of formula I-B wherein A¹⁴ is N, A²⁴ is N, A³⁴ is CH, R¹ is CH₃, R³ is CH₃, R^{4c} is CH₃ and T are as defined in table Z.

Table B-14 provides 32 compounds B-14.001 to B-14.032 of formula I-B wherein A¹⁴ is N, A²⁴ is N, A³⁴ is CH, R¹ is CH₃, R³ is CH₃, R^{4c} is CH₂-CF₂H and T are as defined in table Z.

Table B-15 provides 32 compounds B-15.001 to B-15.032 of formula I-B wherein A¹⁴ is N, A²⁴ is N, A³⁴ is CH, R¹ is CH₃, R³ is CH₃, R^{4c} is CH₂-cyclopropyl and T are as defined in table Z.

Table B-16 provides 32 compounds B-16.001 to B-16.032 of formula I-B wherein A¹⁴ is N, A²⁴ is N, A³⁴ is CH, R¹ is CH₂-cyclopropyl, R³ is CH₃, R^{4c} is CH₃ and T are as defined in table Z.

Table B-17 provides 32 compounds B-17.001 to B-17.032 of formula I-B wherein A¹⁴ is N, A²⁴ is N, A³⁴ is CH, R¹ is CH₂-cyclopropyl, R³ is CH₃, R^{4c} is CH₂-CF₂H and T are as defined in table Z.

Table B-18 provides 32 compounds B-18.001 to B-18.032 of formula I-B wherein A¹⁴ is N, A²⁴ is N, A³⁴ is CH, R¹ is CH₂-cyclopropyl, R³ is CH₃, R^{4c} is CH₂-cyclopropyl and T are as defined in table Z.

Table B-19 provides 32 compounds B-19.001 to B-19.032 of formula I-B wherein A¹⁴ is N, A²⁴ is CH, A³⁴ is N, R¹ is H, R³ is CH₃, R^{4c} is CH₃ and T are as defined in table Z.

Table B-20 provides 32 compounds B-20.001 to B-20.032 of formula I-B wherein A¹⁴ is N, A²⁴ is CH, A³⁴ is N, R¹ is H, R³ is CH₃, R^{4c} is CH₂-CF₂H and T are as defined in table Z.

Table B-21 provides 32 compounds B-21.001 to B-21.032 of formula I-B wherein A¹⁴ is N, A²⁴ is CH, A³⁴ is N, R¹ is H, R³ is CH₃, R^{4c} is CH₂-cyclopropyl and T are as defined in table Z.

Table B-22 provides 32 compounds B-22.001 to B-22.032 of formula I-B wherein A¹⁴ is N, A²⁴ is CH, A³⁴ is N, R¹ is CH₃, R³ is CH₃, R^{4c} is CH₃ and T are as defined in table Z.

Table B-23 provides 32 compounds B-23.001 to B-23.032 of formula I-B wherein A¹⁴ is N, A²⁴ is CH, A³⁴ is N, R¹ is CH₃, R³ is CH₃, R^{4c} is CH₂-CF₂H and T are as defined in table Z.

Table B-24 provides 32 compounds B-24.001 to B-24.032 of formula I-B wherein A¹⁴ is N, A²⁴ is CH, A³⁴ is N, R¹ is CH₃, R³ is CH₃, R^{4c} is CH₂-cyclopropyl and T are as defined in table Z.

Table B-25 provides 32 compounds B-25.001 to B-25.032 of formula I-B wherein A¹⁴ is N, A²⁴ is CH, A³⁴ is N, R¹ is CH₂-cyclopropyl, R³ is CH₃, R^{4c} is CH₃ and T are as defined in table Z.

Table B-26 provides 32 compounds B-26.001 to B-26.032 of formula I-B wherein A¹⁴ is N, A²⁴ is CH, A³⁴ is N, R¹ is CH₂-cyclopropyl, R³ is CH₃, R^{4c} is CH₂-CF₂H and T are as defined in table Z.

Table B-27 provides 32 compounds B-27.001 to B-27.032 of formula I-B wherein A¹⁴ is N, A²⁴ is CH, A³⁴ is N, R¹ is CH₂-cyclopropyl, R³ is CH₃, R^{4c} is CH₂-cyclopropyl and T are as defined in table Z.

Table B-28 provides 32 compounds B-28.001 to B-28.032 of formula I-B wherein A¹⁴ is N, A²⁴ is CH, A³⁴ is CH, R¹ is H, R³ is CH₃, R^{4c} is CH₃ and T are as defined in table Z.

Table B-29 provides 32 compounds B-29.001 to B-29.032 of formula I-B wherein A¹⁴ is N, A²⁴ is CH, A³⁴ is CH, R¹ is H, R³ is CH₃, R^{4c} is CH₂-CF₂H and T are as defined in table Z.

Table B-30 provides 32 compounds B-30.001 to B-30.032 of formula I-B wherein A¹⁴ is N, A²⁴ is CH, A³⁴ is CH, R¹ is H, R³ is CH₃, R^{4c} is CH₂-cyclopropyl and T are as defined in table Z.

Table B-31 provides 32 compounds B-31.001 to B-31.032 of formula I-B wherein A¹⁴ is N, A²⁴ is CH, A³⁴ is CH, R¹ is CH₃, R³ is CH₃, R^{4c} is CH₃ and T are as defined in table Z.

Table B-32 provides 32 compounds B-32.001 to B-32.032 of formula I-B wherein A¹⁴ is N, A²⁴ is CH, A³⁴ is CH, R¹ is CH₃, R³ is CH₃, R^{4c} is CH₂-CF₂H and T are as defined in table Z.

Table B-33 provides 32 compounds B-33.001 to B-33.032 of formula I-B wherein A¹⁴ is N, A²⁴ is CH, A³⁴ is CH, R¹ is CH₃, R³ is CH₃, R^{4c} is CH₂-cyclopropyl and T are as defined in table Z.

Table B-34 provides 32 compounds B-34.001 to B-34.032 of formula I-B wherein A¹⁴ is N, A²⁴ is CH, A³⁴ is CH, R¹ is CH₂-cyclopropyl, R³ is CH₃, R^{4c} is CH₃ and T are as defined in table Z.

Table B-35 provides 32 compounds B-35.001 to B-35.032 of formula I-B wherein A¹⁴ is N, A²⁴ is CH, A³⁴ is CH, R¹ is CH₂-cyclopropyl, R³ is CH₃, R^{4c} is CH₂-CF₂H and T are as defined in table Z.

Table B-36 provides 32 compounds B-36.001 to B-36.032 of formula I-B wherein A¹⁴ is N, A²⁴ is CH, A³⁴ is CH, R¹ is CH₂-cyclopropyl, R³ is CH₃, R^{4c} is CH₂-cyclopropyl and T are as defined in table Z.

Table B-37 provides 32 compounds B-37.001 to B-37.032 of formula I-B wherein A¹⁴ is CH, A²⁴ is N, A³⁴ is N, R¹ is H, R³ is CH₃, R^{4c} is CH₃ and T are as defined in table Z.

Table B-38 provides 32 compounds B-38.001 to B-38.032 of formula I-B wherein A¹⁴ is CH, A²⁴ is N, A³⁴ is N, R¹ is H, R³ is CH₃, R^{4c} is CH₂-CF₂H and T are as defined in table Z.

Table B-39 provides 32 compounds B-39.001 to B-39.032 of formula I-B wherein A¹⁴ is CH, A²⁴ is N, A³⁴ is N, R¹ is H, R³ is CH₃, R^{4c} is CH₂-cyclopropyl and T are as defined in table Z.

Table B-40 provides 32 compounds B-40.001 to B-40.032 of formula I-B wherein A¹⁴ is CH, A²⁴ is N, A³⁴ is N, R¹ is CH₃, R³ is CH₃, R^{4c} is CH₃ and T are as defined in table Z.

Table B-41 provides 32 compounds B-41.001 to B-41.032 of formula I-B wherein A¹⁴ is CH, A²⁴ is N, A³⁴ is N, R¹ is CH₃, R³ is CH₃, R^{4c} is CH₂-CF₂H and T are as defined in table Z.

Table B-42 provides 32 compounds B-42.001 to B-42.032 of formula I-B wherein A¹⁴ is CH, A²⁴ is N, A³⁴ is N, R¹ is CH₃, R³ is CH₃, R^{4c} is CH₂-cyclopropyl and T are as defined in table Z.

Table B-43 provides 32 compounds B-43.001 to B-43.032 of formula I-B wherein A¹⁴ is CH, A²⁴ is N, A³⁴ is N, R¹ is CH₂-cyclopropyl, R³ is CH₃, R^{4c} is CH₃ and T are as defined in table Z.

Table B-44 provides 32 compounds B-44.001 to B-44.032 of formula I-B wherein A¹⁴ is CH, A²⁴ is N, A³⁴ is N, R¹ is CH₂-cyclopropyl, R³ is CH₃, R^{4c} is CH₂-CF₂H and T are as defined in table Z.

Table B-45 provides 32 compounds B-45.001 to B-45.032 of formula I-B wherein A¹⁴ is CH, A²⁴ is N, A³⁴ is N, R¹ is CH₂-cyclopropyl, R³ is CH₃, R^{4c} is CH₂-cyclopropyl and T are as defined in table Z.

Table B-46 provides 32 compounds B-46.001 to B-46.032 of formula I-B wherein A¹⁴ is CH, A²⁴ is N, A³⁴ is CH, R¹ is H, R³ is CH₃, R^{4c} is CH₃ and T are as defined in table Z.

Table B-47 provides 32 compounds B-47.001 to B-47.032 of formula I-B wherein A¹⁴ is CH, A²⁴ is N, A³⁴ is CH, R¹ is H, R³ is CH₃, R^{4c} is CH₂-CF₂H and T are as defined in table Z.

Table B-48 provides 32 compounds B-48.001 to B-48.032 of formula I-B wherein A¹⁴ is CH, A²⁴ is N, A³⁴ is CH, R¹ is H, R³ is CH₃, R^{4c} is CH₂-cyclopropyl and T are as defined in table Z.

Table B-49 provides 32 compounds B-49.001 to B-49.032 of formula I-B wherein A¹⁴ is CH, A²⁴ is N, A³⁴ is CH, R¹ is CH₃, R³ is CH₃, R^{4c} is CH₃ and T are as defined in table Z.

Table B-50 provides 32 compounds B-50.001 to B-50.032 of formula I-B wherein A¹⁴ is CH, A²⁴ is N, A³⁴ is CH, R¹ is CH₃, R³ is CH₃, R^{4c} is CH₂-CF₂H and T are as defined in table Z.

Table B-51 provides 32 compounds B-51.001 to B-51.032 of formula I-B wherein A¹⁴ is CH, A²⁴ is N, A³⁴ is CH, R¹ is CH₃, R³ is CH₃, R^{4c} is CH₂-cyclopropyl and T are as defined in table Z.

Table B-52 provides 32 compounds B-52.001 to B-52.032 of formula I-B wherein A¹⁴ is CH, A²⁴ is N, A³⁴ is CH, R¹ is CH₂-cyclopropyl, R³ is CH₃, R^{4c} is CH₃ and T are as defined in table Z.

Table B-53 provides 32 compounds B-53.001 to B-53.032 of formula I-B wherein A¹⁴ is CH, A²⁴ is N, A³⁴ is CH, R¹ is CH₂-cyclopropyl, R³ is CH₃, R^{4c} is CH₂-CF₂H and T are as defined in table Z.

Table B-54 provides 32 compounds B-54.001 to B-54.032 of formula I-B wherein A¹⁴ is CH, A²⁴ is N, A³⁴ is CH, R¹ is CH₂-cyclopropyl, R³ is CH₃, R^{4c} is CH₂-cyclopropyl and T are as defined in table Z.

Table B-55 provides 32 compounds B-55.001 to B-55.032 of formula I-B wherein A¹⁴ is CH, A²⁴ is CH, A³⁴ is N, R¹ is H, R³ is CH₃, R^{4c} is CH₃ and T are as defined in table Z.

Table B-56 provides 32 compounds B-56.001 to B-56.032 of formula I-B wherein A¹⁴ is CH, A²⁴ is CH, A³⁴ is N, R¹ is H, R³ is CH₃, R^{4c} is CH₂-CF₂H and T are as defined in table Z.

Table B-57 provides 32 compounds B-57.001 to B-57.032 of formula I-B wherein A¹⁴ is CH, A²⁴ is CH, A³⁴ is N, R¹ is H, R³ is CH₃, R^{4c} is CH₂-cyclopropyl and T are as defined in table Z.

Table B-58 provides 32 compounds B-58.001 to B-58.032 of formula I-B wherein A¹⁴ is CH, A²⁴ is CH, A³⁴ is N, R¹ is CH₃, R³ is CH₃, R^{4c} is CH₃ and T are as defined in table Z.

Table B-59 provides 32 compounds B-59.001 to B-59.032 of formula I-B wherein A¹⁴ is CH, A²⁴ is CH, A³⁴ is N, R¹ is CH₃, R³ is CH₃, R^{4c} is CH₂-CF₂H and T are as defined in table Z.

Table B-60 provides 32 compounds B-60.001 to B-60.032 of formula I-B wherein A¹⁴ is CH, A²⁴ is CH, A³⁴ is N, R¹ is CH₃, R³ is CH₃, R^{4c} is CH₂-cyclopropyl and T are as defined in table Z.

Table B-61 provides 32 compounds B-61.001 to B-61.032 of formula I-B wherein A¹⁴ is CH, A²⁴ is CH, A³⁴ is N, R¹ is CH₂-cyclopropyl, R³ is CH₃, R^{4c} is CH₃ and T are as defined in table Z.

Table B-62 provides 32 compounds B-62.001 to B-62.032 of formula I-B wherein A¹⁴ is CH, A²⁴ is CH, A³⁴ is N, R¹ is CH₂-cyclopropyl, R³ is CH₃, R^{4c} is CH₂-CF₂H and T are as defined in table Z.

Table B-63 provides 32 compounds B-63.001 to B-63.032 of formula I-B wherein A¹⁴ is CH, A²⁴ is CH, A³⁴ is N, R¹ is CH₂-cyclopropyl, R³ is CH₃, R^{4c} is CH₂-cyclopropyl and T are as defined in table Z.

Table B-64 provides 32 compounds B-64.001 to B-64.032 of formula I-B wherein A¹⁴ is CH, A²⁴ is CH, A³⁴ is CH, R¹ is H, R³ is CH₃, R^{4c} is CH₃ and T are as defined in table Z.

Table B-65 provides 32 compounds B-65.001 to B-65.032 of formula I-B wherein A¹⁴ is CH, A²⁴ is CH, A³⁴ is CH, R¹ is H, R³ is CH₃, R^{4c} is CH₂-CF₂H and T are as defined in table Z.

Table B-66 provides 32 compounds B-66.001 to B-66.032 of formula I-B wherein A¹⁴ is CH, A²⁴ is CH, A³⁴ is CH, R¹ is H, R³ is CH₃, R^{4c} is CH₂-cyclopropyl and T are as defined in table Z.

Table B-67 provides 32 compounds B-67.001 to B-67.032 of formula I-B wherein A¹⁴ is CH, A²⁴ is CH, A³⁴ is CH, R¹ is CH₃, R³ is CH₃, R^{4c} is CH₃ and T are as defined in table Z.

Table B-68 provides 32 compounds B-68.001 to B-68.032 of formula I-B wherein A¹⁴ is CH, A²⁴ is CH, A³⁴ is CH, R¹ is CH₃, R³ is CH₃, R^{4c} is CH₂-CF₂H and T are as defined in table Z.

Table B-69 provides 32 compounds B-69.001 to B-69.032 of formula I-B wherein A¹⁴ is CH, A²⁴ is CH, A³⁴ is CH, R¹ is CH₃, R³ is CH₃, R^{4c} is CH₂-cyclopropyl and T are as defined in table Z.

Table B-70 provides 32 compounds B-70.001 to B-70.032 of formula I-B wherein A¹⁴ is CH, A²⁴ is CH, A³⁴ is CH, R¹ is CH₂-cyclopropyl, R³ is CH₃, R^{4c} is CH₃ and T are as defined in table Z.

Table B-71 provides 32 compounds B-71.001 to B-71.032 of formula I-B wherein A¹⁴ is CH, A²⁴ is CH, A³⁴ is CH, R¹ is CH₂-cyclopropyl, R³ is CH₃, R^{4c} is CH₂-CF₂H and T are as defined in table Z.

Table B-72 provides 32 compounds B-72.001 to B-72.032 of formula I-B wherein A¹⁴ is CH, A²⁴ is CH, A³⁴ is CH, R¹ is CH₂-cyclopropyl, R³ is CH₃, R^{4c} is CH₂-cyclopropyl and T are as defined in table Z.

Table B-73 provides 32 compounds B-73.001 to B-73.032 of formula I-B wherein A¹⁴ is N, A²⁴ is CH, A³⁴ is CH, R¹ is H, R³ is CH₃, R^{4c} is CH₂CH₃ and T are as defined in table Z.

Table B-74 provides 32 compounds B-74.001 to B-74.032 of formula I-B wherein A¹⁴ is N, A²⁴ is CH, A³⁴ is CH, R¹ is CH₃, R³ is CH₃, R^{4c} is CH₂CH₃ and T are as defined in table Z.

Table B-75 provides 32 compounds B-75.001 to B-75.032 of formula I-B wherein A¹⁴ is N, A²⁴ is CH, A³⁴ is CH, R¹ is CH₂-cyclopropyl, R³ is CH₃, R^{4c} is CH₂CH₃ and T are as defined in table Z.

Table B-76 provides 32 compounds B-76.001 to B-76.032 of formula I-B wherein A¹⁴ is CH, A²⁴ is N, A³⁴ is CH, R¹ is H, R³ is CH₃, R^{4c} is CH₂CH₃ and T are as defined in table Z.

Table B-77 provides 32 compounds B-77.001 to B-77.032 of formula I-B wherein A¹⁴ is CH, A²⁴ is N, A³⁴ is CH, R¹ is CH₃, R³ is CH₃, R^{4c} is CH₂CH₃ and T are as defined in table Z.

Table B-78 provides 32 compounds B-78.001 to B-78.032 of formula I-B wherein A¹⁴ is CH, A²⁴ is N, A³⁴ is CH, R¹ is CH₂-cyclopropyl, R³ is CH₃, R^{4c} is CH₂CH₃ and T are as defined in table Z.

### Tables C-1 to C-21 (Formula I-C)

Table C-1 provides 32 compounds C-1.001 to C-1.032 of formula I-C wherein A¹⁴ is N, R' is H, R³ is CH₃, R^{4c} is CH₃ and T are as defined in table Z.

Table C-2 provides 32 compounds C-2.001 to C-2.032 of formula I-C wherein A¹⁴ is N, R¹ is H, R³ is CH₃, R^{4c} is CH₂-CF₂H and T are as defined in table Z.

Table C-3 provides 32 compounds C-3.001 to C-3.032 of formula I-C wherein A¹⁴ is N, R¹ is H, R³ is CH₃, R^{4c} is CH₂-cyclopropyl and T are as defined in table Z.

Table C-4 provides 32 compounds C-4.001 to C-4.032 of formula I-C wherein A¹⁴ is N, R¹ is CH₃, R³ is CH₃, R^{4c} is CH₃ and T are as defined in table Z.

Table C-5 provides 32 compounds C-5.001 to C-5.032 of formula I-C wherein A¹⁴ is N, R¹ is CH₃, R³ is CH₃, R^{4c} is CH₂-CF₂H and T are as defined in table Z.

Table C-6 provides 32 compounds C-6.001 to C-6.032 of formula I-C wherein A¹⁴ is N, R¹ is CH₃, R³ is CH₃, R^{4c} is CH₂-cyclopropyl and T are as defined in table Z.

Table C-7 provides 32 compounds C-7.001 to C-7.032 of formula I-C wherein A¹⁴ is N, R¹ is CH₂-cyclopropyl, R³ is CH₃, R^{4c} is CH₃ and T are as defined in table Z.

Table C-8 provides 32 compounds C-8.001 to C-8.032 of formula I-C wherein A¹⁴ is N, R¹ is CH₂-cyclopropyl, R³ is CH₃, R^{4c} is CH₂-CF₂H and T are as defined in table Z.

Table C-9 provides 32 compounds C-9.001 to C-9.032 of formula I-C wherein A¹⁴ is N, R¹ is CH₂-cyclopropyl, R³ is CH₃, R^{4c} is CH₂-cyclopropyl and T are as defined in table Z.

Table C-10 provides 32 compounds C-10.001 to C-10.032 of formula I-C wherein A¹⁴ is CH, R¹ is H, R³ is CH₃, R^{4c} is CH₃ and T are as defined in table Z.

Table C-11 provides 32 compounds C-11.001 to C-11.032 of formula I-C wherein A¹⁴ is CH, R¹ is H, R³ is CH₃, R^{4c} is CH₂-CF₂H and T are as defined in table Z.

Table C-12 provides 32 compounds C-12.001 to C-12.032 of formula I-C wherein A¹⁴ is CH, R¹ is H, R³ is CH₃, R^{4c} is CH₂-cyclopropyl and T are as defined in table Z.

Table C-13 provides 32 compounds C-13.001 to C-13.032 of formula I-C wherein A¹⁴ is CH, R¹ is CH₃, R³ is CH₃, R^{4c} is CH₃ and T are as defined in table Z.

Table C-14 provides 32 compounds C-14.001 to C-14.032 of formula I-C wherein A¹⁴ is CH, R¹ is CH₃, R³ is CH₃, R^{4c} is CH₂-CF₂H and T are as defined in table Z.

Table C-15 provides 32 compounds C-15.001 to C-15.032 of formula I-C wherein A¹⁴ is CH, R¹ is CH₃, R³ is CH₃, R^{4c} is CH₂-cyclopropyl and T are as defined in table Z.

Table C-16 provides 32 compounds C-16.001 to C-16.032 of formula I-C wherein A¹⁴ is CH, R¹ is CH₂-cyclopropyl, R³ is CH₃, R^{4c} is CH₃ and T are as defined in table Z.

Table C-17 provides 32 compounds C-17.001 to C-17.032 of formula I-C wherein A¹⁴ is CH, R¹ is CH₂-cyclopropyl, R³ is CH₃, R^{4c} is CH₂-CF₂H and T are as defined in table Z.

Table C-18 provides 32 compounds C-18.001 to C-18.032 of formula I-C wherein A¹⁴ is CH, R¹ is CH₂-cyclopropyl, R³ is CH₃, R^{4c} is CH₂-cyclopropyl and T are as defined in table Z.

Table C-19 provides 32 compounds C-19.001 to C-19.032 of formula I-C wherein A¹⁴ is N, R¹ is H, R³ is CH₃, R^{4c} is CH₂CH₃ and T are as defined in table Z.

Table C-20 provides 32 compounds C-20.001 to C-20.032 of formula I-C wherein A¹⁴ is N, R¹ is CH₃, R³ is CH₃, R^{4c} is CH₂CH₃ and T are as defined in table Z.

Table C-21 provides 32 compounds C-21.001 to C-21.032 of formula I-C wherein A¹⁴ is N, R¹ is CH₂-cyclopropyl, R³ is CH₃, R^{4c} is CH₂CH₃ and T are as defined in table Z.

### Tables D-1 to D-21 (Formula I-D)

Table D-1 provides 32 compounds D-1.001 to D-1.032 of formula I-D wherein A¹⁴ is N, R¹ is H, R³ is CH₃, R^{4c} is CH₃ and T are as defined in table Z.

Table D-2 provides 32 compounds D-2.001 to D-2.032 of formula I-D wherein A¹⁴ is N, R¹ is H, R³ is CH₃, R^{4c} is CH₂-CF₂H and T are as defined in table Z.

Table D-3 provides 32 compounds D-3.001 to D-3.032 of formula I-D wherein A¹⁴ is N, R¹ is H, R³ is CH₃, R^{4c} is CH₂-cyclopropyl and T are as defined in table Z.

Table D-4 provides 32 compounds D-4.001 to D-4.032 of formula I-D wherein A¹⁴ is N, R¹ is CH₃, R³ is CH₃, R^{4c} is CH₃ and T are as defined in table Z.

Table D-5 provides 32 compounds D-5.001 to D-5.032 of formula I-D wherein A¹⁴ is N, R¹ is CH₃, R³ is CH₃, R^{4c} is CH₂-CF₂H and T are as defined in table Z.

Table D-6 provides 32 compounds D-6.001 to D-6.032 of formula I-D wherein A¹⁴ is N, R¹ is CH₃, R³ is CH₃, R^{4c} is CH₂-cyclopropyl and T are as defined in table Z.

Table D-7 provides 32 compounds D-7.001 to D-7.032 of formula I-D wherein A¹⁴ is N, R¹ is CH₂-cyclopropyl, R³ is CH₃, R^{4c} is CH₃ and T are as defined in table Z.

Table D-8 provides 32 compounds D-8.001 to D-8.032 of formula I-D wherein A¹⁴ is N, R¹ is CH₂-cyclopropyl, R³ is CH₃, R^{4c} is CH₂-CF₂H and T are as defined in table Z.

Table D-9 provides 32 compounds D-9.001 to D-9.032 of formula I-D wherein A¹⁴ is N, R¹ is CH₂-cyclopropyl, R³ is CH₃, R^{4c} is CH₂-cyclopropyl and T are as defined in table Z.

Table D-10 provides 32 compounds D-10.001 to D-10.032 of formula I-D wherein A¹⁴ is CH, R¹ is H, R³ is CH₃, R^{4c} is CH₃ and T are as defined in table Z.

Table D-11 provides 32 compounds D-11.001 to D-11.032 of formula I-D wherein A¹⁴ is CH, R¹ is H, R³ is CH₃, R^{4c} is CH₂-CF₂H and T are as defined in table Z.

Table D-12 provides 32 compounds D-12.001 to D-12.032 of formula I-D wherein A¹⁴ is CH, R¹ is H, R³ is CH₃, R^{4c} is CH₂-cyclopropyl and T are as defined in table Z.

Table D-13 provides 32 compounds D-13.001 to D-13.032 of formula I-D wherein A¹⁴ is CH, R¹ is CH₃, R³ is CH₃, R^{4c} is CH₃ and T are as defined in table Z.

Table D-14 provides 32 compounds D-14.001 to D-14.032 of formula I-D wherein A¹⁴ is CH, R¹ is CH₃, R³ is CH₃, R^{4c} is CH₂-CF₂H and T are as defined in table Z.

Table D-15 provides 32 compounds D-15.001 to D-15.032 of formula I-D wherein A¹⁴ is CH, R¹ is CH₃, R³ is CH₃, R^{4c} is CH₂-cyclopropyl and T are as defined in table Z.

Table D-16 provides 32 compounds D-16.001 to D-16.032 of formula I-D wherein A¹⁴ is CH, R¹ is CH₂-cyclopropyl, R³ is CH₃, R^{4c} is CH₃ and T are as defined in table Z.

Table D-17 provides 32 compounds D-17.001 to D-17.032 of formula I-D wherein A¹⁴ is CH, R¹ is CH₂-cyclopropyl, R³ is CH₃, R^{4c} is CH₂-CF₂H and T are as defined in table Z.

Table D-18 provides 32 compounds D-18.001 to D-18.032 of formula I-D wherein A¹⁴ is CH, R¹ is CH₂-cyclopropyl, R³ is CH₃, R^{4c} is CH₂-cyclopropyl and T are as defined in table Z.

Table D-19 provides 32 compounds D-19.001 to D-19.032 of formula I-D wherein A¹⁴ is N, R¹ is H, R³ is CH₃, R^{4c} is CH₂CH₃ and T are as defined in table Z.

Table D-20 provides 32 compounds D-20.001 to D-20.032 of formula I-D wherein A¹⁴ is N, R¹ is CH₃, R³ is CH₃, R^{4c} is CH₂CH₃ and T are as defined in table Z.

Table D-21 provides 32 compounds D-21.001 to D-21.032 of formula I-D wherein A¹⁴ is N, R¹ is CH₂-cyclopropyl, R³ is CH₃, R^{4c} is CH₂CH₃ and T are as defined in table Z.

**Table Z: Substituent definitions of T**

| Index | T | Index | T | Index | T |
|---|---|---|---|---|---|
| 1 | 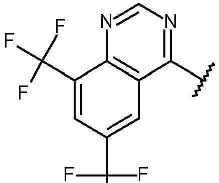 | 12 | 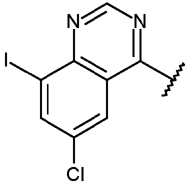 | 23 | 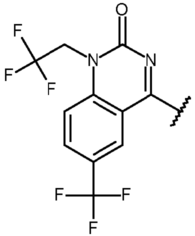 |
| 2 | 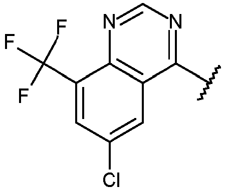 | 13 | 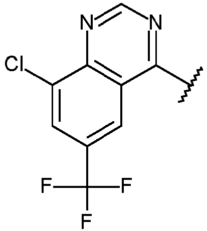 | 24 | 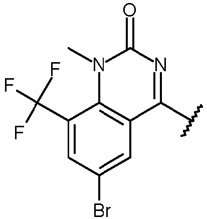 |
| 3 | 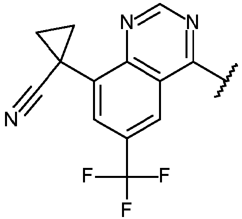 | 14 | 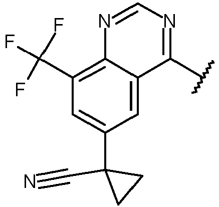 | 25 | 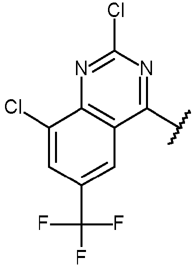 |
| 4 | 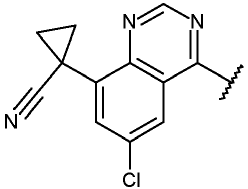 | 15 | 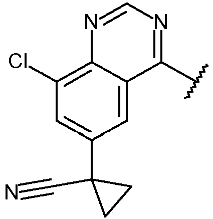 | 26 | 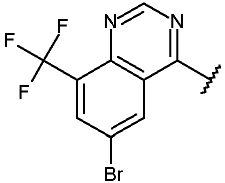 |
| 5 | 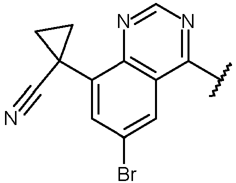 | 16 | 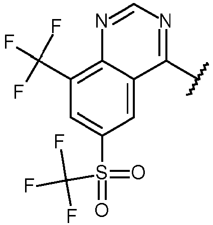 | 27 | 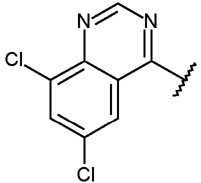 |
| 6 | 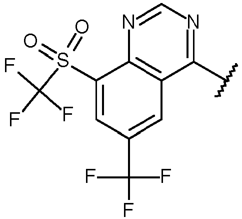 | 17 | 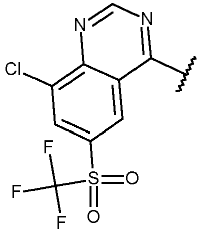 | 28 | 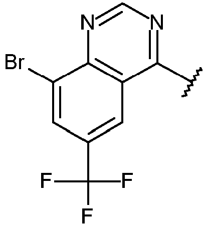 |
| 7 | 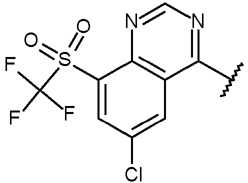 | 18 | 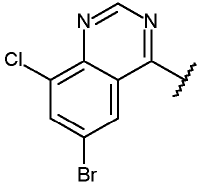 | 29 | 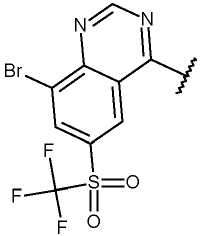 |
| 8 | 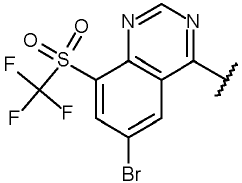 | 19 | 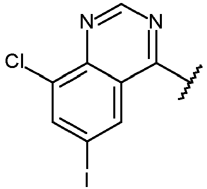 | 30 | 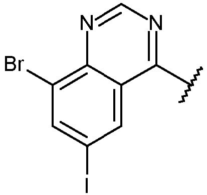 |
| 9 | 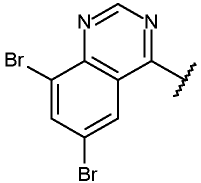 | 20 | 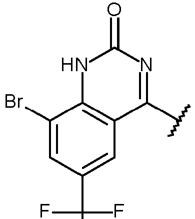 | 31 | 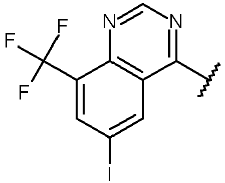 |
| 10 | 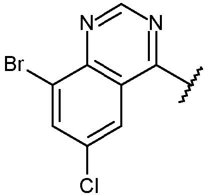 | 21 | 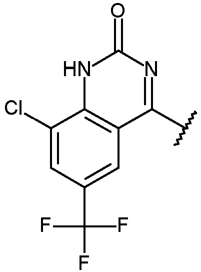 | 32 | 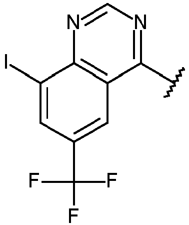 |
| 11 | 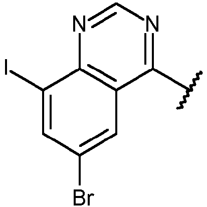 | 22 | 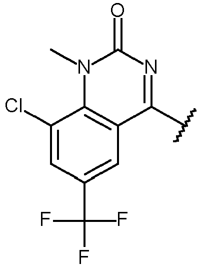 | 33 | |

Also made available are certain intermediate compounds of formulae II(i), III(i), IV(i), IVa(i), V(i), VI(i), VII(i), VIII(i), IX(i), X(i), XI(i), XII(i), XIV(i), XV(i), XVI(i), XX(i), XXI(i), and XXII(i), some of which are novel. For example:
- a compound of the formula XII(i), or a tautomer thereof, or a salt thereof: 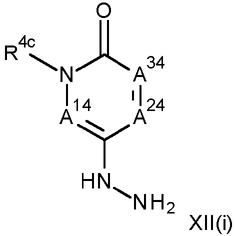 wherein A¹⁴, A²⁴, A³⁴ and R^{4c} are as defined in any one of the Tables A-1 to A-78, and Tables B-1 to B-78 provided that said compound is not 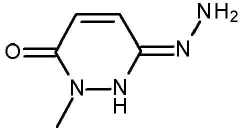 6-hydrazinyl-2-methyl-3(2H)-pyridazinone, nor a tautomer thereof; nor 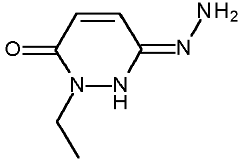 6-hydrazinyl-2-ethyl-3(2H)-pyridazinone, nor a tautomer thereof, both of which may be identified from Herberich et al, 2011, Bioorganic & Medicinal Chemistry Letters, vol.22, no. 2, pages 1226-1229, "Identification of triazolopyridazinones as potent p38[alpha] inhibitors".
- compounds of the formula XVI(i): 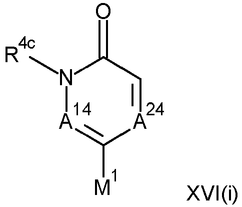 wherein one of A¹⁴ and A²⁴ is N, the other one is CH, R^{4c} are as defined in any one of the Tables A-1 to A-78 and Tables B-1 to B-78, and M¹ is a metal-containing substituent, for instance a boron substituent or a tin substituent, such as, for example, tributylstannyl, borono or 4,4,5,5-tetramethyl-1,3,2-dioxaborolan-2-yl, provided said compounds of the formula XVI(i) is not:
   - 2-methyl-6-trimethylstannyl-pyridazin-3-one (CAS 2254337-53-4),
   - [5-oxo-4-(2,2,2-trifluoroethyl)pyrazin-2-yl]boronic acid (CAS 2509138-33-2),
   - 5-(4,4,5,5-tetramethyl-1,3,2-dioxaborolan-2-yl)-1-(2,2,2-trifluoroethyl)pyrazin-2-one (CAS 2509138-04-7),
   - (2-methyl-6-(4,4,5,5-tetramethyl-1,3,2-dioxaborolan-2-yl)pyridazin-3-one (CAS 1610374-18-9),
   - 2-isopropyl-6-(4,4,5,5-tetramethyl-1,3,2-dioxaborolan-2-yl)pyridazin-3-one (CAS 2768712-84-9),
   - (1-methyl-6-oxo-pyridazin-3-yl)boronic acid (CAS 1872185-45-9), nor
   - (1-isopropyl-6-oxo-pyridazin-3-yl)boronic acid (CAS 2334476-21-8).
- compounds of the formulae XX(i), XXI(i) and XXII(i), 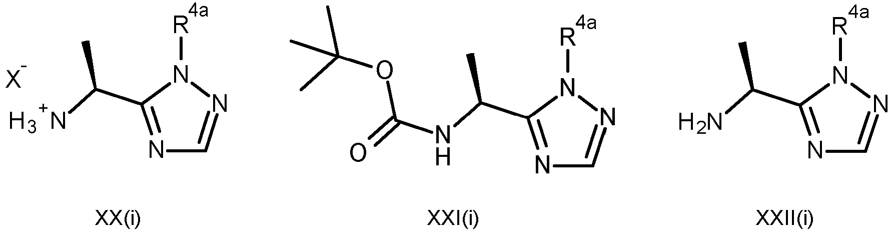 wherein R^{4a} , including A¹⁴, A²⁴, A³⁴ and R^{4c}, is as defined in any one Tables A-1 to A-78, and Tables B-1 to B-78, and wherein X⁻ is an anion, i.e. the conjugate base of an acid, such as an inorganic acid, for instance hydrochloric acid, hydrobromic acid, hydrogen fluoride, hydrogen iodide, sulfuric acid, or the like, or of an organic acid, such as a carboxylic acid or a sulfonic acid, for instance trifluoroacetic acid, or methane sulfonic acid, or para-toluene sulfonic acid. Preferably A¹⁴ is N, A²⁴ is CH, A³⁴ is CH and R^{4c} is selected from methyl or ethyl,
- compounds of the formulae XXVI(i), XXVII(i) and XXVII-a(i), 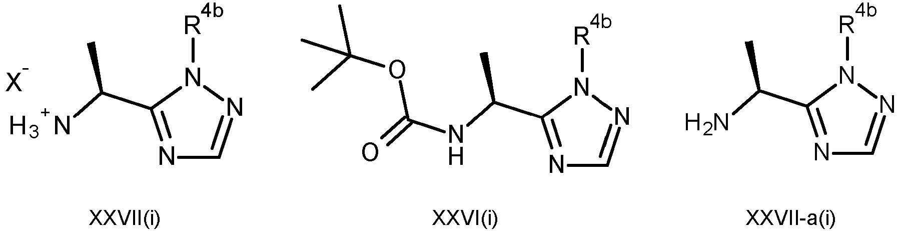 wherein R^{4b} , including A¹⁴ and R^{4c}, is as defined in any one Tables C-1 to C-21, and Tables D-1 to D-21, and wherein X⁻ is an anion, i.e. the conjugate base of an acid, such as an inorganic acid, for instance hydrochloric acid, hydrobromic acid, hydrogen fluoride, hydrogen iodide, sulfuric acid, or the like, or of an organic acid, such as a carboxylic acid or a sulfonic acid, for instance trifluoroacetic acid, or methane sulfonic acid, or para-toluene sulfonic acid. Preferably A¹⁴ is N, and R^{4c} is selected from methyl or ethyl.

In a further aspect, the present invention accordingly makes available compounds of formulae II(i), III(i), IV(i), IVa(i), V(i), VI(i), VII(i), VIII(i), IX(i), X(i), XI(i), XII(i), XIV(i), XV(i), XVI(i), XX(i), XXI(i), and XXII(i), wherein in each case, as applicable, A¹, A², A³, A⁴, A⁵, X⁰, R¹, R^{2a}, R^{2b}, R³, Q^{a} (including R⁵, R^{4c} and R⁴ as R^{4a} or R^{4b}), Q^{b} (including R^{5a}, R^{5b}, R^{4c} and R⁴ as R^{4a} or R^{4b}), R⁴, R^{4a} (including A¹⁴, A²⁴, A³⁴ and R^{4c}), R^{4b} (including A¹⁴ and R^{4c}), R^{X}, R^{Y}, and R^{Z} are as defined for formula (I) in the first aspect, and
- T is 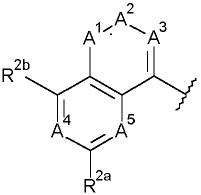 where the staggered line represents the connection to the remainder of the compounds of the formulae (II), (IV), (IVa), (X), (XI), (XVII)
- in respect of compounds of formula II(i), X¹ is a leaving group, such as a halogen, hydroxy or sulfonate, for instance chloride;
- in respect of compounds of formula V(i), X² is a leaving group, such as a halogen, for instance chloride or bromide, or a sulfonate, for instance a mesylate;
- in respect of compounds of formula VI(i), X² is a leaving group, such as a halogen, for instance a chloride, bromide, iodide, or a sulfonate, for instance a mesylate;
- in respect of compounds of formula XV(i), X⁴ is a leaving group, such as for example a halogen, for instance chloro, a sulfonate, C₁-C₄-sulfanyl, C₁-C₄-sulfinyl or C₁-C₄-sulfonyl;
- in respect of compounds of formula XVI(i), M¹ is a metal-containing substituent, for instance a boron substituent or a tin substituent, such as, for example, tributylstannyl, borono or 4,4,5,5-tetramethyl-1,3,2-dioxaborolan-2-yl;
- in respect of compounds of formula XX(i), X⁻ is an anion, namely the conjugate base of an acid, such as an inorganic acid, for instance hydrochloric acid, hydrobromic acid, hydrogen fluoride, hydrogen iodide, sulfuric acid, or the like, or of an organic acid, such as a carboxylic acid or a sulfonic acid, for instance trifluoroacetic acid, or methane sulfonic acid, or para-toluene sulfonic acid.

Furthermore, the corresponding embodiments illustrated for formula (I) also apply to the compounds of formulae II(i), III(i), IV(i), IVa(i), V(i), VI(i), VII(i), VIII(i), IX(i), X(i), XI(i), XII(i), XIV(i), XV(i), XVI(i), XX(i), XXI(i), and XXII(i), as applicable.

The compounds of formula (I) according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i.e. in destruction of the pests, which takes place either immediately or only after some time has elapsed, for example during ecdysis, or indirectly, for example in a reduced oviposition and/or hatching rate.

Examples of the above mentioned animal pests are:
from the order *Acarina,* for example,
Acalitus spp., Aculus spp., Acaricalus spp., Aceria spp., Acarus siro, Amblyomma spp., Argas spp., Boophilus spp., Brevipalpus spp., Bryobia spp., Calipitrimerus spp., Chorioptes spp., Dermanyssus gallinae, Dermatophagoides spp., Eotetranychus spp., Eriophyes spp., Hemitarsonemus spp., Hyalomma spp., Ixodes spp., Olygonychus spp., Ornithodoros spp., Polyphagotarsone latus, Panonychus spp., Phyllocoptruta oleivora, Phytonemus spp., Polyphagotarsonemus spp., Psoroptes spp., Rhipicephalus spp., Rhizoglyphus spp., Sarcoptes spp., Steneotarsonemus spp., Tarsonemus spp. and Tetranychus spp.;
from the order *Anoplura,* for example,
Haematopinus spp., Linognathus spp., Pediculus spp., Pemphigus spp. and Phylloxera spp.; from the order *Coleoptera,* for example,
Agriotes spp., Amphimallon majale, Anomala orientalis, Anthonomus spp., Aphodius spp., Astylus atromaculatus, Ataenius spp., Atomaria linearis, Chaetocnema tibialis, Cerotoma spp., Conoderus spp., Cosmopolites spp., Cotinis nitida, Curculio spp., Cyclocephala spp., Dermestes spp., Diabrotica spp., Diloboderus abderus, Epilachna spp., Eremnus spp., Heteronychus arator, Hypothenemus hampei, Lagria vilosa, Leptinotarsa decemlineata, Lissorhoptrus spp., Liogenys spp., Maecolaspis spp., Maladera castanea, Megascelis spp., Melighetes aeneus, Melolontha spp., Myochrous armatus, Orycaephilus spp., Otiorhynchus spp., Phyllophaga spp., Phlyctinus spp., Popillia spp., Psylliodes spp., Rhyssomatus aubtilis, Rhizopertha spp., Scarabeidae, Sitophilus spp., Sitotroga spp., Somaticus spp., Sphenophorus spp., Sternechus subsignatus, Tenebrio spp., Tribolium spp. and Trogoderma spp.;
from the order *Diptera,* for example,
Aedes spp., Anopheles spp., Antherigona soccata,Bactrocea oleae, Bibio hortulanus, Bradysia spp., Calliphora erythrocephala, Ceratitis spp., Chrysomyia spp., Culex spp., Cuterebra spp., Dacus spp., Delia spp., Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp., Rivelia quadrifasciata, Scatella spp., Sciara spp., Stomoxys spp., Tabanus spp., Tannia spp. and Tipula spp.;
from the order *Hemiptera,* for example,
Acanthocoris scabrator, Acrosternum spp., Adelphocoris lineolatus, Aleurodes spp., Amblypelta nitida, Bathycoelia thalassina, Blissus spp., Cimex spp., Clavigralla tomentosicollis, Creontiades spp., Distantiella theobroma, Dichelops furcatus, Dysdercus spp., Edessa spp., Euchistus spp., Eurydema pulchrum, Eurygaster spp., Halyomorpha halys, Horcias nobilellus, Leptocorisa spp., Lygus spp., Margarodes spp., Murgantia histrionic, Neomegalotomus spp., Nesidiocoris tenuis, Nezara spp., Nysius simulans, Oebalus insularis, Piesma spp., Piezodorus spp., Rhodnius spp., Sahlbergella singularis, Scaptocoris castanea, Scotinophara spp. , Thyanta spp , Triatoma spp., Vatiga illudens;
from the order *Homoptera,* for example,
Acyrthosium pisum, Adalges spp., Agalliana ensigera, Agonoscena targionii, Aleurodicus spp., Aleurocanthus spp., Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp., Brachycaudus spp., Brevicoryne brassicae, Cacopsylla spp., Cavariella aegopodii Scop., Ceroplaster spp., Chrysomphalus aonidium, Chrysomphalus dictyospermi, Cicadella spp., Cofana spectra, Cryptomyzus spp., Cicadulina spp., Coccus hesperidum, Dalbulus maidis, Dialeurodes spp., Diaphorina citri, Diuraphis noxia, Dysaphis spp., Empoasca spp., Eriosoma larigerum, Erythroneura spp., Gascardia spp., Glycaspis brimblecombei, Hyadaphis pseudobrassicae, Hyalopterus spp., Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp., Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp., Phorodon humuli, Phylloxera spp., Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp., Trialeurodes spp., Tridiscus sporoboli, Trionymus spp., Trioza erytreae , Unaspis citri, Zygina flammigera, Zyginidia scutellaris, ;
from the order *Hymenoptera,* for example,
Acromyrmex, Arge spp., Atta spp., Cephus spp., Diprion spp., Diprionidae, Gilpinia polytoma, Hoplocampa spp., Lasius spp., Monomorium pharaonis, Neodiprion spp., Pogonomyrmex spp., Slenopsis invicta, Solenopsis spp. and Vespa spp.;
from the order *Isoptera,* for example,
Coptotermes spp., Corniternes cumulans, Incisitermes spp., Macrotermes spp., Mastotermes spp., Microtermes spp., Reticulitermes spp.; Solenopsis geminate
from the order *Lepidoptera,* for example,
Acleris spp., Adoxophyes spp., Aegeria spp., Agrotis spp., Alabama argillaceae, Amylois spp., Anticarsia gemmatalis, Archips spp., Argyresthia spp., Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp., Crocidolomia binotalis, Cryptophlebia leucotreta, Cydalima perspectalis, Cydia spp., Diaphania perspectalis, Diatraea spp., Diparopsis castanea, Earias spp., Elasmopalpus lignosellus, Eldana saccharina, Ephestia spp., Epinotia spp., Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp., Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp., Noctua spp., Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp., Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
Damalinea spp. and Trichodectes spp.;
from the order *Orthoptera,* for example,
Blatta spp., Blattella spp., Gryllotalpa spp., Leucophaea maderae, Locusta spp., Neocurtilla hexadactyla, Periplaneta spp. , Scapteriscus spp., and Schistocerca spp.;
from the order *Psocoptera,* for example,
Liposcelis spp.;
from the order *Siphonaptera,* for example,
Ceratophyllus spp., Ctenocephalides spp. and Xenopsylla cheopis;
from the order *Thysanoptera,* for example,
Calliothrips phaseoli, Frankliniella spp., Heliothrips spp., Hercinothrips spp., Parthenothrips spp., Scirtothrips aurantii, Sericothrips variabilis, Taeniothrips spp., Thrips spp;
from the order *Thysanura,* for example, Lepisma saccharina.

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The active ingredients according to the invention can be used for controlling, i.e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broaD-1 eaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. B. *elatior, B. semperflorens, B. tubéreux), Bougainvillea* spp., *Brachycome* spp., *Brassica* spp. (ornamental), *Calceolaria* spp., *Capsicum annuum, Catharanthus roseus, Canna* spp., *Centaurea* spp., *Chrysanthemum* spp., *Cineraria* spp. *(C. maritime), Coreopsis* spp., *Crassula coccinea, Cuphea ignea, Dahlia* spp., *Delphinium* spp., *Dicentra spectabilis, Dorotheantus* spp., *Eustoma grandiflorum, Forsythia* spp., *Fuchsia* spp., *Geranium gnaphalium, Gerbera* spp., *Gomphrena globosa, Heliotropium* spp., *Helianthus* spp., *Hibiscus* spp., *Hortensia* spp., *Hydrangea* spp., *Hypoestes phyllostachya, Impatiens* spp. (*I. Walleriana), Iresines* spp., *Kalanchoe* spp., *Lantana camara, Lavatera trimestris, Leonotis leonurus, Lilium* spp., *Mesembryanthemum* spp., *Mimulus* spp., *Monarda* spp., *Nemesia* spp., *Tagetes* spp., *Dianthus* spp. (carnation), *Canna* spp., *Oxalis* spp., *Bellis* spp., *Pelargonium* spp. *(P. peltatum, P. Zonale), Viola* spp. (pansy), *Petunia* spp., *Phlox* spp., *Plecthranthus* spp., *Poinsettia* spp., *Parthenocissus* spp. *(P. quinquefolia, P. tricuspidata), Primula* spp., *Ranunculus* spp., *Rhododendron* spp., *Rosa* spp. (rose), *Rudbeckia* spp., *Saintpaulia* spp., *Salvia* spp., *Scaevola aemola, Schizanthus wisetonensis, Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: Allium spp. (A. sativum, A.. cepa, A. oschaninii, A. Porrum, A. ascalonicum, A. fistulosum), Anthriscus cerefolium, Apium graveolus, Asparagus officinalis, Beta vulgarus, Brassica spp. (B. Oleracea, B. Pekinensis, B. rapa), Capsicum annuum, Cicer arietinum, Cichorium endivia, Cichorum spp. (C. intybus, C. endivia), Citrillus lanatus, Cucumis spp. (C. sativus, C. melo), Cucurbita spp. (C. pepo, C. maxima), Cyanara spp. (C. scolymus, C. cardunculus), Daucus carota, Foeniculum vulgare, Hypericum spp., Lactuca sativa, Lycopersicon spp. (L. esculentum, L. lycopersicum), Mentha spp., Ocimum basilicum, Petroselinum crispum, Phaseolus spp. (P. vulgaris, P. coccineus), Pisum sativum, Raphanus sativus, Rheum rhaponticum, Rosemarinus spp., Salvia spp., Scorzonera hispanica, Solanum melongena, Spinacea oleracea, Valerianella spp. (V. locusta, V. eriocarpa) and Vicia faba.

Preferred ornamental species include African violet, Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia, rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca (preferably in vegetables, vineyards), Leptinotarsa (preferably in potatos) and Chilo supressalis (preferably in rice).

The compounds of formula (I) are particularly suitable for control of
- a pest of the order Hemiptera, for example, one or more of the species *Bemisia tabaci, Aphis craccivora, Myzus persicae, Rhopalosiphum padi, Nilaparvata lugens,* and *Euschistus heros* (preferably in vegetables, soybeans, and sugarcane);
- a pest of the order Lepidoptera, for example, one or more of the species Spodoptera littoralis, Spodoptera frugiperda, Plutella xylostella, Cnaphalocrocis medinalis, Cydia pomonella, Chrysodeixis includes, Chilo suppressalis, Elasmopalpus lignosellus, Pseudoplusia includens, and Tuta absoluta (preferably in vegetables and corn);
- a pest of the order Thysanoptera, such as the family Thripidae, for example, one or more of *Thrips tabaci* and *Frankliniella occidentalis* (preferably in vegetables); and
- soil pests (such as of the order Coleoptera), for example, the species *Diabrotica balteata, Agriotes* spp. and *Leptinotarsa decemlineata* (preferably in vegetables and corn).

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from *Bacillus thuringiensis,* such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example *Photorhabdus* spp. or *Xenorhabdus* spp., such as *Photorhabdus luminescens, Xenorhabdus nematophilus;* toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer *(Ostrinia nubilalis* and *Sesamia nonagrioides)* by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer *(Ostrinia nubilalis* and *Sesamia nonagrioides)* by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de I'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
**4. MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603** × **MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch).

The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerance to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention provides a compound of the first aspect for use in therapy. The present invention provides a compound of the first aspect, for use in controlling parasites in or on an animal. The present invention further provides a compound of the first aspect, for use in controlling ectoparasites on an animal. The present invention further provides a compound of the first aspect, for use in preventing and/or treating diseases transmitted by ectoparasites.

The present invention provides the use of a compound of the first aspect, for the manufacture of a medicament for controlling parasites in or on an animal. The present invention further provides the use of a compound of the first aspect, for the manufacture of a medicament for controlling ectoparasites on an animal. The present invention further provides the use of a compound of the first aspect, for the manufacture of a medicament for preventing and/or treating diseases transmitted by ectoparasites.

The present invention provides the use of a compound of the first aspect, in controlling parasites in or on an animal. The present invention further provides the use of a compound of the first aspect , in controlling ectoparasites on an animal.

The term "controlling" when used in context of parasites in or on an animal refers to reducing the number of pests or parasites, eliminating pests or parasites and/or preventing further pest or parasite infestation.

The term "treating" when used used in context of parasites in or on an animal refers to restraining, slowing, stopping or reversing the progression or severity of an existing symptom or disease.

The term "preventing" when used used in context of parasites in or on an animal refers to the avoidance of a symptom or disease developing in the animal.

The term "animal" when used used in context of parasites in or on an animal may refer to a mammal and a non-mammal, such as a bird or fish. In the case of a mammal, it may be a human or non-human mammal. Non-human mammals include, but are not limited to, livestock animals and companion animals. Livestock animals include, but are not limited to, cattle, camellids, pigs, sheep, goats and horses. Companion animals include, but are not limited to, dogs, cats and rabbits.

A "parasite" is a pest which lives in or on the host animal and benefits by deriving nutrients at the host animal's expense. An "endoparasite" is a parasite which lives in the host animal. An "ectoparasite" is a parasite which lives on the host animal. Ectoparasites include, but are not limited to, acari, insects and crustaceans (e.g. sea lice). The Acari (or Acarina) sub-class comprises ticks and mites. Ticks include, but are not limited to, members of the following genera: *Rhipicaphalus,* for example, *Rhipicaphalus (Boophilus) microplus* and *Rhipicephalus sanguineus; Amblyomrna; Dermacentor, Haemaphysalis; Hyalomma; Ixodes; Rhipicentor; Margaropus; Argas; Otobius;* and *Omithodoros.* Mites include, but are not limited to, members of the following genera: *Chorioptes,* for example *Chorioptes bovis; Psoroptes,* for example *Psoroptes ovis; Cheyletiella; Dermanyssus;* for example *Dermanyssus gallinae; Ortnithonyssus; Demodex,* for example *Demodex canis; Sarcoptes,* for example *Sarcoptes scabiei;* and *Psorergates.* Insects include, but are not limited to, members of the orders: Siphonaptera, Diptera, Phthiraptera, Lepidoptera, Coleoptera and Homoptera. Members of the Siphonaptera order include, but are not limited to, *Ctenocephalides felis* and *Ctenocephatides canis.* Members of the Diptera order include, but are not limited to, *Musca spp.;* bot fly, for example *Gasterophilus intestinalis* and *Oestrus ovis;* biting flies; horse flies, for example *Haematopota spp.* and *Tabunus spp.; haematobia,* for example *haematobia irritans; Stomoxys; Lucilia;* midges; and mosquitoes. Members of the Phthiraptera class include, but are not limited to, blood sucking lice and chewing lice, for example *Bovicola Ovis* and *Bovicola Bovis.*

The term "effective amount" when used used in context of parasites in or on an animal refers to the amount or dose of the compound of the invention, or a salt thereof, which, upon single or multiple dose administration to the animal, provides the desired effect in or on the animal. The effective amount can be readily determined by the attending diagnostician, as one skilled in the art, by the use of known techniques and by observing results obtained under analogous circumstances. In determining the effective amount a number of factors are considered by the attending diagnostician, including, but not limited to: the species of mammal; its size, age, and general health; the parasite to be controlled and the degree of infestation; the specific disease or disorder involved; the degree of involvement or the severity of the disease or disorder; the response of the individual; the particular compound administered; the mode of administration; the bioavailability characteristics of the preparation administered; the dose regimen selected; the use of concomitant medication; and other relevant circumstances.

The compounds of the invention may be administered to the animal by any route which has the desired effect including, but not limited to topically, orally, parenterally and subcutaneously. Topical administration is preferred. Formulations suitable for topical administration include, for example, solutions, emulsions and suspensions and may take the form of a pour-on, spot-on, spray-on, spray race or dip. In the alternative, the compounds of the invention may be administered by means of an ear tag or collar.

Salt forms of the compounds of the invention include both pharmaceutically acceptable salts and veterinary acceptable salts, which can be different to agrochemically acceptable salts. Pharmaceutically and veterinary acceptable salts and common methodology for preparing them are well known in the art. See, for example, Gould, P.L., "Salt selection for basic drugs", International Journal of Pharmaceutics, 33: 201 -217 (1986); Bastin, R.J., et al. "Salt Selection and Optimization Procedures for Pharmaceutical New Chemical Entities", Organic Process Research and Development, 4: 427-435 (2000); and Berge, S.M., et al., "Pharmaceutical Salts", Journal of Pharmaceutical Sciences, 66: 1-19, (1977). One skilled in the art of synthesis will appreciate that the compounds of the invention are readily converted to and may be isolated as a salt, such as a hydrochloride salt, using techniques and conditions well known to one of ordinary skill in the art. In addition, one skilled in the art of synthesis will appreciate that the compounds of the invention are readily converted to and may be isolated as the corresponding free base from the corresponding salt.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO 2005113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | Agrilus planipennis | Ash |
| Cerambycidae | Anoplura glabripennis | Hardwoods |
| Scolytidae | Xylosandrus crassiusculus | Hardwoods |
| | X. mutilatus | Hardwoods |
| | Tomicus piniperda | Conifers |

**Table B. Examples of native woodborers of economic importance.**

| Family | Species | Host or Crop Infested |
|---|---|---|
| Buprestidae | Agrilus anxius | Birch |
| | Agrilus politus | Willow, Maple |
| | Agrilus sayi | Bayberry, Sweetfern |
| | Agrilus vittaticolllis | Apple, Pear, Cranberry, Serviceberry, Hawthorn |
| | Chrysobothris femorata | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | Texania campestris | Basswood, Beech, Maple, Oak, Sycamore, Willow, Yellow-poplar |
| Cerambycidae | Goes pulverulentus | Beech, Elm, Nuttall, Willow, Black oak, Cherrybark oak, Water oak, Sycamore |
| | Goes tigrinus | Oak |
| | Neoclytus acuminatus | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | Neoptychodes trilineatus | Fig, Alder, Mulberry, Willow, Netleaf hackberry |
| | Oberea ocellata | Sumac, Apple, Peach, Plum, Pear, Currant, Blackberry |
| | Oberea tripunctata | Dogwood, Viburnum, Elm, Sourwood, Blueberry, Rhododendron, Azalea, Laurel, Poplar, Willow, Mulberry |
| | Oncideres cingulata | Hickory, Pecan, Persimmon, Elm, Sourwood, Basswood, Honeylocust, Dogwood, Eucalyptus, Oak, Hackberry, Maple, Fruit trees |
| | Saperda calcarata | Poplar |
| | Strophiona nitens | Chestnut, Oak, Hickory, Walnut, Beech, Maple |
| Scolytidae | Corthylus columbianus | Maple, Oak, Yellow-poplar, Beech, Boxelder, Sycamore, Birch, Basswood, Chestnut, Elm |
| | Dendroctonus frontalis | Pine |
| | Dryocoetes betulae | Birch, Sweetgum, Wild cherry, Beech, Pear |
| | Monarthrum fasciatum | Oak, Maple, Birch, Chestnut, Sweetgum, Blackgum, Poplar, Hickory, Mimosa, Apple, Peach, Pine |
| | Phloeotribus liminaris | Peach, Cherry, Plum, Black cherry, Elm, Mulberry, Mountain-ash |
| | Pseudopityophthorus pruinosus | Oak, American beech, Black cherry, Chickasaw plum, Chestnut, Maple, Hickory, Hornbeam, Hophornbeam |
| Sesiidae | Paranthrene simulans | Oak, American chestnut |
| | Sannina uroceriformis | Persimmon |
| | Synanthedon exitiosa | Peach, Plum, Nectarine, Cherry, Apricot, Almond, Black cherry |
| | Synanthedon pictipes | Peach, Plum, Cherry, Beach, Black Cherry |
| | Synanthedon rubrofascia | Tupelo |
| | Synanthedon scitula | Dogwood, Pecan, Hickory, Oak, Chestnut, Beech, Birch, Black cherry, Elm, Mountain-ash, Viburnum, Willow, Apple, Loquat, Ninebark, Bayberry |
| | Vitacea polistiformis | Grape |

The present invention may be also used to control any insect pests that may be present in turfgrass, including for example beetles, caterpillars, fire ants, ground pearls, millipedes, sow bugs, mites, mole crickets, scales, mealybugs, ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, *C. lurida), Rhizotrogus spp.* (e.g. European chafer, *R. majalis), Cotinus spp.* (e.g. Green June beetle, *C. nitida), Popillia spp.* (e.g. Japanese beetle, *P. japonica), Phyllophaga spp.* (e.g. May/June beetle), *Ataenius spp.* (e.g. Black turfgrass ataenius, A. *spretulus), Maladera spp.* (e.g. Asiatic garden beetle, M. *castanea)* and *Tomarus spp.),* ground pearls *(Margarodes* spp.), mole crickets (tawny, southern, and short-winged; *Scapteriscus* spp., *Gryllotalpa africana)* and leatherjackets (European crane fly, *Tipula spp.).*

The present invention may also be used to control insect pests of turfgrass that are thatch dwelling, including armyworms (such as fall armyworm *Spodoptera frugiperda,* and common armyworm *Pseudaletia unipuncta),* cutworms, billbugs *(Sphenophorus spp.,* such as S. *venatus vestitus* and S. *parvulus),* and sod webworms (such as *Crambus spp.* and the tropical sod webworm, *Herpetogramma phaeopteralis).*

The present invention may also be used to control insect pests of turfgrass that live above the ground and feed on the turfgrass leaves, including chinch bugs (such as southern chinch bugs, *Blissus insularis),* Bermudagrass mite *(Eriophyes cynodoniensis),* rhodesgrass mealybug *(Antonina graminis),* two-lined spittlebug *(Prosapia bicincta),* leafhoppers, cutworms *(Noctuidae* family), and greenbugs.

The present invention may also be used to control other pests of turfgrass such as red imported fire ants *(Solenopsis invicta)* that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..
Of the order Mallophagida: Trimenopon spp., Menopon spp., Trinoton spp., Bovicola spp., Werneckiella spp., Lepikentron spp., Damalina spp., Trichodectes spp. and Felicola spp..
Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp.,
Of the order Siphonapterida, for example Pulex spp., Ctenocephalides spp., Xenopsylla spp., Ceratophyllus spp.,
Of the order Heteropterida, for example Cimex spp., Triatoma spp., Rhodnius spp., Panstrongylus spp.,
Of the order Blattarida, for example Blatta orientalis, Periplaneta americana, Blattelagermanica and Supella spp..
Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..
Of the orders Actinedida (Prostigmata) and Acaridida (Astigmata), for example Acarapis spp., Cheyletiella spp., Ornithocheyletia spp., Myobia spp., Psorergatesspp., Demodexspp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp.

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds of formulae (I), and (I'), or salts thereof, are especially suitable for controlling one or more pests selected from the family: Noctuidae, Plutellidae, Chrysomelidae, Thripidae, Pentatomidae, Tortricidae, Delphacidae, Aphididae, Noctuidae, Crambidae, Meloidogynidae, and Heteroderidae. In a preferred embodiment of each aspect, a compound TX (where the abbreviation "TX" means "one compound selected from the compounds defined in Tables A-1 to A-78, B-1 to B-78, C-1 to C-21 and D-1 to D-21 ") controls one or more of pests selected from the family: Noctuidae, Plutellidae, Chrysomelidae, Thripidae, Pentatomidae, Tortricidae, Delphacidae, Aphididae, Noctuidae, Crambidae, Meloidogynidae, and Heteroderidae.

The compounds of formulae (I), and (I'), or salts thereof, are especially suitable for controlling one or more of pests selected from the genus: *Spodoptera spp., Plutella spp., Frankliniella spp., Thrips spp., Euschistus spp., Cydia spp., Nilaparvata spp., Myzus spp., Aphis spp., Diabrotica spp., Rhopalosiphum spp., Pseudoplusia spp* and *Chilo spp..* In a preferred embodiment of each aspect, a compound TX (where the abbreviation "TX" means "one compound selected from the compounds defined in Tables A-1 to A-78, B-1 to B-78, C-1 to C-21 and D-1 to D-21 ") controls one or more of pests selected from the genus: *Spodoptera spp., Plutella spp., Frankliniella spp., Thrips spp., Euschistus spp., Cydia spp., Nilaparvata spp., Myzus spp., Aphis spp., Diabrotica spp., Rhopalosiphum spp., Pseudoplusia spp* and *Chilo spp.*

The compounds of formulae (I), and (I'), or salts thereof, are especially suitable for controlling one or more of *Spodoptera littoralis, Plutella xylostella, Frankliniella occidentalis, Thrips tabaci, Euschistus heros, Cydia pomonella, Nilaparvata lugens, Myzus persicae, Chrysodeixis includens, Aphis craccivora, Diabrotica balteata, Rhopalosiphum padi,* and *Chilo suppressalis.*

In a preferred embodiment of each aspect, a compound TX (where the abbreviation "TX" means "one compound selected from the compounds defined in Tables A-1 to A-78, B-1 to B-78, C-1 to C-21 and D-1 to D-21") controls one or more of *Spodoptera littoralis, Plutella xylostella, Frankliniella occidentalis, Thrips tabaci, Euschistus heros, Cydia pomonella, Nilaparvata lugens, Myzus persicae, Chrysodeixis includens, Aphis craccivora, Diabrotica balteata, Rhopalosiphum Padia,* and *Chilo Suppressalis,* such as *Spodoptera littoralis +* TX, *Plutella xylostella +* TX; *Frankliniella occidentalis +* TX, *Thrips tabaci* + TX, *Euschistus heros* + TX, *Cydia pomonella* + TX, *Nilaparvata lugens* + TX, *Myzus persicae* + TX, *Chrysodeixis includens* + TX, *Aphis craccivora* + TX, *Diabrotica balteata* + TX, *Rhopalosiphum Padi +* TX, and *Chilo suppressalis +* TX.

In an embodiment, of each aspect, one compound from Tables A-1 to A-78, B-1 to B-78, C-1 to C-21 and D-1 to D-21 is suitable for controlling *Spodoptera littoralis, Plutella xylostella, Frankliniella occidentalis, Thrips tabaci, Euschistus heros, Cydia pomonella, Nilaparvata lugens, Myzus persicae, Chrysodeixis includens, Aphis craccivora, Diabrotica balteata, Rhopalosiphum padi,* and *Chilo suppressalis* in cotton, vegetable, maize, cereal, rice and soya crops.

In an embodiment, one compound from Tables A-1 to A-78, B-1 to B-78, C-1 to C-21 and D-1 to D-21 is suitable for controlling *Mamestra* (preferably in vegetables), *Cydia pomonella* (preferably in apples), *Empoasca* (preferably in vegetables, vineyards), *Leptinotarsa* (preferably in potatos) and *Chilo supressalis* (preferably in rice).

Compounds according to the invention may possess any number of benefits including, inter alia, advantageous levels of biological activity for protecting plants against insects or superior properties for use as agrochemical active ingredients (for example, greater biological activity, an advantageous spectrum of activity, an increased safety profile against non-target organisms above and below ground (such as fish, birds and bees), improved physico-chemical properties, or increased biodegradability). In particular, it has been surprisingly found that certain compounds of formula (I) may show an advantageous safety profile with respect to non-target arthropods, in particular pollinators such as honey bees, solitary bees, and bumble bees. Most particularly, Apis mellifera.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, N,N-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, D-1imonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, N-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 I/ha, especially from 10 to 1000 I/ha.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultrA-1ow volume suspension (SU), an ultrA-1ow volume liquid (UL), a technical concentrate (TK), a dispersible concentrate (DC), a wettable powder (WP), a soluble granule (SG) or any technically feasible formulation in combination with agriculturally acceptable adjuvants.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following mixtures of the compounds of formula (I) with active ingredients are preferred (where the abbreviation "TX" means "one compound selected from the compounds defined in the Tables A-1 to A-78, B-1 to B-78, C-1 to C-21 and D-1 to D-21 and Table P"):
an adjuvant selected from the group of substances consisting of petroleum oils (alternative name) (628) + TX;
abamectin + TX, acequinocyl + TX, acetamiprid + TX, acetoprole + TX, acrinathrin + TX, acynonapyr + TX, afidopyropen + TX, afoxolaner + TX, alanycarb + TX, allethrin + TX, alpha-cypermethrin + TX, alphamethrin + TX, amidoflumet + TX, aminocarb + TX, azocyclotin + TX, bensultap + TX, benzoximate + TX, benzpyrimoxan + TX, betacyfluthrin + TX, beta-cypermethrin + TX, bifenazate + TX, bifenthrin + TX, binapacryl + TX, bioallethrin + TX, S-bioallethrin + TX, bioresmethrin + TX, bistrifluron + TX, broflanilide + TX, brofluthrinate + TX, bromophos-ethyl + TX, buprofezine + TX, butocarboxim + TX, cadusafos + TX, carbaryl + TX, carbosulfan + TX, cartap + TX, CAS number: 1632218-00-8 + TX, CAS number: 1808115-49-2 + TX, CAS number: 2032403-97-5 + TX, CAS number: 2044701-44-0 + TX, CAS number: 2128706-05-6 + TX, CAS number: 2095470-94-1 + TX, CAS number: 2377084-09-6 + TX, CAS number: 1445683-71-5 + TX, CAS number: 2408220-94-8 + TX, CAS number: 2408220-91-5 + TX, CAS number: 1365070-72-9 + TX, CAS number: 2171099-09-3 + TX, CAS number: 2396747-83-2 + TX, CAS number: 2133042-31-4 + TX, CAS number: 2133042-44-9 + TX, CAS number: 1445684-82-1 + TX, CAS number: 1445684-82-1 + TX, CAS number: 1922957-45-6 + TX, CAS number: 1922957-46-7 + TX, CAS number: 1922957-47-8 + TX, CAS number: 1922957-48-9 + TX, CAS number: 2415706-16-8 + TX, CAS number: 1594624-87-9 + TX, CAS number: 1594637-65-6 + TX, CAS number: 1594626-19-3 + TX, CAS number: 1990457-52-7 + TX, CAS number: 1990457-55-0 + TX, CAS number: 1990457-57-2 + TX, CAS number: 1990457-77-6 + TX, CAS number: 1990457-66-3 + TX, CAS number: 1990457-85-6 + TX, CAS number: 2220132-55-6 + TX, CAS number: 1255091-74-7 + TX, CAS number: RNA (Leptinotarsa decemlineata-specific recombinant double-stranded interfering GS2) + TX, CAS number: 2719848-60-7 + TX, CAS number: 1956329-03-5 + TX, chlorantraniliprole + TX, chlordane + TX, chlorfenapyr + TX, chloroprallethrin + TX, chromafenozide + TX, clenpirin + TX, cloethocarb + TX, clothianidin + TX, 2-chlorophenyl N-methylcarbamate (CPMC) + TX, cyanofenphos + TX, cyantraniliprole + TX, cyclaniliprole + TX, cyclobutrifluram + TX, cycloprothrin + TX, cycloxaprid + TX, cyenopyrafen + TX, cyetpyrafen (or etpyrafen) + TX, cyflumetofen + TX, cyfluthrin + TX, cyhalodiamide + TX, cyhalothrin + TX, cypermethrin + TX, cyphenothrin + TX, cyproflanilide + TX, cyromazine + TX, deltamethrin + TX, diafenthiuron + TX, dialifos + TX, dibrom + TX, dicloromezotiaz + TX, diflovidazine + TX, diflubenzuron + TX, dimpropyridaz + TX, dinactin + TX, dinocap + TX, dinotefuran + TX, dioxabenzofos + TX, emamectin (or emamectin benzoate) + TX, empenthrin + TX, epsilon - momfluorothrin + TX, epsilon-metofluthrin + TX, esfenvalerate + TX, ethion + TX, ethiprole + TX, etofenprox + TX, etoxazole + TX, famphur + TX, fenazaquin + TX, fenfluthrin + TX, , fenmezoditiaz + TX, fenitrothion + TX, fenobucarb + TX, fenothiocarb + TX, fenoxycarb + TX, fenpropathrin + TX, fenpyroximate + TX, fensulfothion + TX, fenthion + TX, fentinacetate + TX, fenvalerate + TX, fipronil + TX, flometoquin + TX, flonicamid + TX, fluacrypyrim + TX, fluazaindolizine + TX, fluazuron + TX, flubendiamide + TX, flubenzimine + TX, fluchlordiniliprole + TX, flucitrinate + TX, flucycloxuron + TX, flucythrinate + TX, fluensulfone + TX, flufenerim + TX, flufenprox + TX, flufiprole + TX, fluhexafon + TX, flumethrin + TX, fluopyram + TX, flupentiofenox + TX, flupyradifurone + TX, flupyrimin + TX, fluralaner + TX, fluvalinate + TX, fluxametamide + TX, fosthiazate + TX, gamma-cyhalothrin + TX, guadipyr + TX, halofenozide + TX, halfenprox + TX, heptafluthrin + TX, hexythiazox + TX, hydramethylnon + TX, imicyafos + TX, imidacloprid + TX, imiprothrin + TX, indazapyroxamet + TX, indoxacarb + TX, iodomethane + TX, iprodione + TX, isocycloseram + TX, isothioate + TX, ivermectin + TX, kappa-bifenthrin + TX, kappa-tefluthrin + TX, lambda-Cyhalothrin + TX, lepimectin + TX, lotilaner + TX, lufenuron + TX, metaflumizone + TX, metaldehyde + TX, metam + TX, methomyl + TX, methoxyfenozide + TX, metofluthrin + TX, metolcarb + TX, mexacarbate + TX, milbemectin + TX, momfluorothrin + TX, niclosamide + TX, nicofluprole + TX; nitenpyram + TX, nithiazine + TX, omethoate + TX, oxamyl + TX, oxazosulfyl + TX, parathion-ethyl + TX, permethrin + TX, phenothrin + TX, phosphocarb + TX, piperonylbutoxide + TX, pirimicarb + TX, pirimiphos-ethyl + TX, pirimiphos-methyl + TX, Polyhedrosis virus + TX, prallethrin + TX, profenofos + TX, profluthrin + TX, propargite + TX, propetamphos + TX, propoxur + TX, prothiophos + TX, protrifenbute + TX, pyflubumide + TX, pymetrozine + TX, pyraclofos + TX, pyrafluprole + TX, pyridaben + TX, pyridalyl + TX, pyrifluquinazon + TX, pyrimidifen + TX, pyriminostrobin + TX, pyriprole + TX, pyriproxyfen + TX, resmethrin + TX, sarolaner + TX, selamectin + TX, silafluofen + TX, spinetoram + TX, spinosad + TX, spirobudifen + TX; spirodiclofen + TX, spiromesifen + TX, spiropidion + TX, spirotetramat + TX, spidoxamat + TX, sulfoxaflor + TX, tebufenozide + TX, tebufenpyrad + TX, tebupirimiphos + TX, tefluthrin + TX, temephos + TX, tetrachlorantraniliprole + TX, tetradiphon + TX, tetramethrin + TX, tetramethylfluthrin + TX, tetranactin + TX, tetraniliprole + TX, theta-cypermethrin + TX, thiacloprid + TX, thiamethoxam + TX, thiocyclam + TX, thiodicarb + TX, thiofanox + TX, thiometon + TX, thiosultap + TX, tigolaner + TX, tiorantraniliprole + TX; tioxazafen + TX, tolfenpyrad + TX, toxaphene + TX, tralomethrin + TX, transfluthrin + TX, triazamate + TX, triazophos + TX, trichlorfon + TX, trichloronate + TX, trichlorphon + TX, trifluenfuronate + TX, triflumezopyrim + TX, tyclopyrazoflor + TX, zeta-cypermethrin + TX, Extract of seaweed and fermentation product derived from melasse + TX, Extract of seaweed and fermentation product derived from melasse comprising urea + TX, amino acids + TX, potassium and molybdenum and EDTA-chelated manganese + TX, Extract of seaweed and fermented plant products + TX, Extract of seaweed and fermented plant products comprising phytohormones + TX, vitamins + TX, EDTA-chelated copper + TX, zinc + TX, and iron + TX, azadirachtin + TX, Bacillus aizawai + TX, Bacillus chitinosporus AQ746 (NRRL Accession No B-21 618) + TX, Bacillus firmus + TX, Bacillus kurstaki + TX, Bacillus mycoides AQ726 (NRRL Accession No. B-21664) + TX, Bacillus pumilus (NRRL Accession No B-30087) + TX, Bacillus pumilus AQ717 (NRRL Accession No. B-21662) + TX, Bacillus sp. AQ178 (ATCC Accession No. 53522) + TX, Bacillus sp. AQ175 (ATCC Accession No. 55608) + TX, Bacillus sp. AQ177 (ATCC Accession No. 55609) + TX, Bacillus subtilis unspecified + TX, Bacillus subtilis AQ153 (ATCC Accession No. 55614) + TX, Bacillus subtilis AQ30002 (NRRL Accession No. B-50421) + TX, Bacillus subtilis AQ30004 (NRRL Accession No. B- 50455) + TX, Bacillus subtilis AQ713 (NRRL Accession No. B-21661) + TX, Bacillus subtilis AQ743 (NRRL Accession No. B-21665) + TX, Bacillus thuringiensis AQ52 (NRRL Accession No. B-21619) + TX, Bacillus thuringiensis BD#32 (NRRL Accession No B-21530) + TX, Bacillus thuringiensis subspec. kurstaki BMP 123 + TX, Beauveria bassiana + TX, D-limonene + TX, Granulovirus + TX, Harpin + TX, Helicoverpa armigera Nucleopolyhedrovirus + TX, Helicoverpa zea Nucleopolyhedrovirus + TX, Heliothis virescens Nucleopolyhedrovirus + TX, Heliothis punctigera Nucleopolyhedrovirus + TX, Metarhizium spp. + TX, Muscodor albus 620 (NRRL Accession No. 30547) + TX, Muscodor roseus A3-5 (NRRL Accession No. 30548) + TX, Neem tree based products + TX, Paecilomyces fumosoroseus + TX, Paecilomyces lilacinus + TX, Pasteuria nishizawae + TX, Pasteuria penetrans + TX, Pasteuria ramosa + TX, Pasteuria thornei + TX, Pasteuria usgae + TX, P-cymene + TX, Plutella xylostella Granulosis virus + TX, Plutella xylostella Nucleopolyhedrovirus + TX, Polyhedrosis virus + TX, pyrethrum + TX, QRD 420 (a terpenoid blend) + TX, QRD 452 (a terpenoid blend) + TX, QRD 460 (a terpenoid blend) + TX, Quillaja saponaria + TX, Rhodococcus globerulus AQ719 (NRRL Accession No B-21663) + TX, Spodoptera frugiperda Nucleopolyhedrovirus + TX, Streptomyces galbus (NRRL Accession No. 30232) + TX, Streptomyces sp. (NRRL Accession No. B-30145) + TX, Terpenoid blend + TX, and Verticillium spp. + TX;
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX;
an anthelmintic selected from the group of substances consisting of abamectin (1) + TX, crufomate (1011) + TX, cyclobutrifluram + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX;
an avicide selected from the group of substances consisting of chloralose (127) + TX, endrin (1122) + TX, fenthion (346) + TX, pyridin-4-amine (IUPAC name) (23) and strychnine (745) + TX;
a bactericide selected from the group of substances consisting of 1-hydroxy-1H-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX;
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX;
a soil sterilant selected from the group of substances consisting of iodomethane (IUPAC name) (542) and methyl bromide (537) + TX;
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX;
an insect pheromone selected from the group of substances consisting of (E)-dec-5-en-1-yl acetate with (E)-dec-5-en-1-ol (IUPAC name) (222) + TX, (E)-tridec-4-en-1-yl acetate (IUPAC name) (829) + TX, (E)-6-methylhept-2-en-4-ol (IUPAC name) (541) + TX, (*E*,*Z*)-tetradeca-4,10-dien-1-yl acetate (IUPAC name) (779) + TX, (Z)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (Z)-hexadec-13-en-11-yn-1-yl acetate (IUPAC name) (438) + TX, (*Z*)-icos-13-en-10-one (IUPAC name) (448) + TX, (Z)-tetradec-7-en-1-al (IUPAC name) (782) + TX, (*Z*)-tetradec-9-en-1-ol (IUPAC name) (783) + TX, (Z)-tetradec-9-en-1-yl acetate (IUPAC name) (784) + TX, (7E,9Z)-dodeca-7,9-dien-1-yl acetate (IUPAC name) (283) + TX, (9Z,11E)-tetradeca-9,11-dien-1-yl acetate (IUPAC name) (780) + TX, (9Z,12E)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, Gossyplure^{®} (alternative name; 1:1 mixture of the (Z,E) and (Z,Z) isomers of hexadeca-7,11-dien-1-yl-acetate) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX;
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX;
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX;
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cyclobutrifluram + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX, fluopyram + TX;
a nitrification inhibitor selected from the group of substances consisting of potassium ethylxanthate [CCN] and nitrapyrin (580) + TX;
a plant activator selected from the group of substances consisting of acibenzolar (6) + TX, acibenzolar-S-methyl (6) + TX, probenazole (658) and *Reynoutria* sachalinensis extract (alternative name) (720) + TX;
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (including alpha-bromadiolone) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX;
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX;
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX;
a virucide selected from the group of substances consisting of imanin (alternative name) [CCN] and ribavirin (alternative name) [CCN] + TX;
a wound protectant selected from the group of substances consisting of mercuric oxide (512) + TX, octhilinone (590) and thiophanate-methyl (802) + TX;
a biologically active substance selected from 1,1-bis(4-chlorophenyl)-2-ethoxyethanol + TX, 2,4-dichlorophenyl benzenesulfonate + TX, 2-fluoro-N-methyl-N-1-naphthylacetamide + TX, 4-chlorophenyl phenyl sulfone + TX, acetoprole + TX, aldoxycarb + TX, amidithion + TX, amidothioate + TX, amiton + TX, amiton hydrogen oxalate + TX, amitraz + TX, aramite + TX, arsenous oxide + TX, azobenzene + TX, azothoate + TX, benomyl + TX, benoxafos + TX, benzyl benzoate + TX, bixafen + TX, brofenvalerate + TX, bromocyclen + TX, bromophos + TX, bromopropylate + TX, buprofezin + TX, butocarboxim + TX, butoxycarboxim + TX, butylpyridaben + TX, calcium polysulfide + TX, camphechlor + TX, carbanolate + TX, carbophenothion + TX, cymiazole + TX, chinomethionat + TX, chlorbenside + TX, chlordimeform + TX, chlordimeform hydrochloride + TX, chlorfenethol + TX, chlorfenson + TX, chlorfensulfide + TX, chlorobenzilate + TX, chloromebuform + TX, chloromethiuron + TX, chloropropylate + TX, chlorthiophos + TX, cinerin I + TX, cinerin II + TX, cinerins + TX, closantel + TX, coumaphos + TX, crotamiton + TX, crotoxyphos + TX, cufraneb + TX, cyanthoate + TX, DCPM + TX, DDT + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, dichlofluanid + TX, dichlorvos + TX, dicliphos + TX, dienochlor + TX, dimefox + TX, dinex + TX, dinex-diclexine + TX, dinocap-4 + TX, dinocap-6 + TX, dinocton + TX, dinopenton + TX, dinosulfon + TX, dinoterbon + TX, dioxathion + TX, diphenyl sulfone + TX, disulfiram + TX, DNOC + TX, dofenapyn + TX, doramectin + TX, endothion + TX, eprinomectin + TX, ethoate-methyl + TX, etrimfos + TX, fenazaflor + TX, fenbutatin oxide + TX, fenothiocarb + TX, fenpyrad + TX, fenpyroximate + TX, fenpyrazamine + TX, fenson + TX, fentrifanil + TX, flubenzimine + TX, flucycloxuron + TX, fluenetil + TX, fluorbenside + TX, FMC 1137 + TX, formetanate + TX, formetanate hydrochloride + TX, formparanate + TX, gamma-HCH + TX, glyodin + TX, halfenprox + TX, hexadecyl cyclopropanecarboxylate + TX, isocarbophos + TX, jasmolin I + TX, jasmolin II + TX, jodfenphos + TX, lindane + TX, malonoben + TX, mecarbam + TX, mephosfolan + TX, mesulfen + TX, methacrifos + TX, methyl bromide + TX, metolcarb + TX, mexacarbate + TX, milbemycin oxime + TX, mipafox + TX, monocrotophos + TX, morphothion + TX, moxidectin + TX, naled + TX, 4-chloro-2-(2-chloro-2-methyl-propyl)-5-[(6-iodo-3-pyridyl)methoxy]pyridazin-3-one + TX, nifluridide + TX, nikkomycins + TX, nitrilacarb + TX, nitrilacarb 1:1 zinc chloride complex + TX, omethoate + TX, oxydeprofos + TX, oxydisulfoton + TX, pp'-DDT + TX, parathion + TX, permethrin + TX, phenkapton + TX, phosalone + TX, phosfolan + TX, phosphamidon + TX, polychloroterpenes + TX, polynactins + TX, proclonol + TX, promacyl + TX, propoxur + TX, prothidathion + TX, prothoate + TX, pyrethrin I + TX, pyrethrin II + TX, pyrethrins + TX, pyridaphenthion + TX, pyrimitate + TX, quinalphos + TX, quintiofos + TX, R-1492 + TX, phosglycin + TX, rotenone + TX, schradan + TX, sebufos + TX, selamectin + TX, sophamide + TX, SSI-121 + TX, sulfiram + TX, sulfluramid + TX, sulfotep + TX, sulfur + TX, diflovidazin + TX, tau-fluvalinate + TX, TEPP + TX, terbam + TX, tetradifon + TX, tetrasul + TX, thiafenox + TX, thiocarboxime + TX, thiofanox + TX, thiometon + TX, thioquinox + TX, thuringiensin + TX, triamiphos + TX, triarathene + TX, triazophos + TX, triazuron + TX, trifenofos + TX, trinactin + TX, vamidothion + TX, vaniliprole + TX, bethoxazin + TX, copper dioctanoate + TX, copper sulfate + TX, cybutryne + TX, dichlone + TX, dichlorophen + TX, endothal + TX, fentin + TX, hydrated lime + TX, nabam + TX, quinoclamine + TX, quinonamid + TX, simazine + TX, triphenyltin acetate + TX, triphenyltin hydroxide + TX, crufomate + TX, piperazine + TX, thiophanate + TX, chloralose + TX, fenthion + TX, pyridin-4-amine + TX, strychnine + TX, 1-hydroxy-1H-pyridine-2-thione + TX, 4-(quinoxalin-2-ylamino)benzenesulfonamide + TX, 8-hydroxyquinoline sulfate + TX, bronopol + TX, copper hydroxide + TX, cresol + TX, dipyrithione + TX, dodicin + TX, fenaminosulf + TX, formaldehyde + TX, hydrargaphen + TX, kasugamycin + TX, kasugamycin hydrochloride hydrate + TX, nickel bis(dimethyldithiocarbamate) + TX, nitrapyrin + TX, octhilinone + TX, oxolinic acid + TX, oxytetracycline + TX, potassium hydroxyquinoline sulfate + TX, probenazole + TX, streptomycin + TX, streptomycin sesquisulfate + TX, tecloftalam + TX, thiomersal + TX, Adoxophyes orana GV + TX, Agrobacterium radiobacter + TX, Amblyseius spp. + TX, Anagrapha falcifera NPV + TX, Anagrus atomus + TX, Aphelinus abdominalis + TX, Aphidius colemani + TX, Aphidoletes aphidimyza + TX, Autographa californica NPV + TX, Bacillus sphaericus Neide + TX, Beauveria brongniartii + TX, Chrysoperla carnea + TX, Cryptolaemus montrouzieri + TX, Cydia pomonella GV + TX, Dacnusa sibirica + TX, Diglyphus isaea + TX, Encarsia formosa + TX, Eretmocerus eremicus + TX, Heterorhabditis bacteriophora and H. megidis + TX, Hippodamia convergens + TX, Leptomastix dactylopii + TX, Macrolophus caliginosus + TX, Mamestra brassicae NPV + TX, Metaphycus helvolus + TX, Metarhizium anisopliae var. acridum + TX, Metarhizium anisopliae var. anisopliae + TX, Neodiprion sertifer NPV and N. lecontei NPV + TX, Orius spp. + TX, Paecilomyces fumosoroseus + TX, Phytoseiulus persimilis + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Trichogramma spp. + TX, Typhlodromus occidentalis + TX , Verticillium lecanii + TX, apholate + TX, bisazir + TX, busulfan + TX, dimatif + TX, hemel + TX, hempa + TX, metepa + TX, methiotepa + TX, methyl apholate + TX, morzid + TX, penfluron + TX, tepa + TX, thiohempa + TX, thiotepa + TX, tretamine + TX, uredepa + TX, (E)-dec-5-en-1-yl acetate with (E)-dec-5-en-1-ol + TX, (E)-tridec-4-en-1-yl acetate + TX, (E)-6-methylhept-2-en-4-ol + TX, (E,Z)-tetradeca-4,10-dien-1-yl acetate + TX, (Z)-dodec-7-en-1-yl acetate + TX, (Z)-hexadec-11-enal + TX, (Z)-hexadec-11-en-1-yl acetate + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, 14-methyloctadec-1-ene + TX, 4-methylnonan-5-ol with 4-methylnonan-5-one + TX, alpha-multistriatin + TX, brevicomin + TX, codlelure + TX, codlemone + TX, cuelure + TX, disparlure + TX, dodec-8-en-1-yl acetate + TX, dodec-9-en-1-yl acetate + TX, dodeca-8 + TX, 10-dien-1-yl acetate + TX, dominicalure + TX, ethyl 4-methyloctanoate + TX, eugenol + TX, frontalin + TX, grandlure + TX, grandlure I + TX, grandlure II + TX, grandlure III + TX, grandlure IV + TX, hexalure + TX, ipsdienol + TX, ipsenol + TX, japonilure + TX, lineatin + TX, litlure + TX, looplure + TX, medlure + TX, megatomoic acid + TX, methyl eugenol + TX, muscalure + TX, octadeca-2,13-dien-1-yl acetate + TX, octadeca-3,13-dien-1-yl acetate + TX, orfralure + TX, oryctalure + TX, ostramone + TX, siglure + TX, sordidin + TX, sulcatol + TX, tetradec-11-en-1-yl acetate + TX, trimedlure + TX, trimedlure A + TX, trimedlure B₁ + TX, trimedlure B₂ + TX, trimedlure C + TX, trunc-call + TX, 2-(octylthio)ethanol + TX, butopyronoxyl + TX, butoxy(polypropylene glycol) + TX, dibutyl adipate + TX, dibutyl phthalate + TX, dibutyl succinate + TX, diethyltoluamide + TX, dimethyl carbate + TX, dimethyl phthalate + TX, ethyl hexanediol + TX, hexamide + TX, methoquin-butyl + TX, methylneodecanamide + TX, oxamate + TX, picaridin + TX, 1-dichloro-1-nitroethane + TX, 1,1-dichloro-2,2-bis(4-ethylphenyl)ethane + TX, 1,2-dichloropropane with 1,3-dichloropropene + TX, 1-bromo-2-chloroethane + TX, 2,2,2-trichloro-1-(3,4-dichlorophenyl)ethyl acetate + TX, 2,2-dichlorovinyl 2-ethylsulfinylethyl methyl phosphate + TX, 2-(1,3-dithiolan-2-yl)phenyl dimethylcarbamate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-chlorovinyl diethyl phosphate + TX, 2-imidazolidone + TX, 2-isovalerylindan-1,3-dione + TX, 2-methyl(prop-2-ynyl)aminophenyl methylcarbamate + TX, 2-thiocyanatoethyl laurate + TX, 3-bromo-1-chloroprop-1-ene + TX, 3-methyl-1-phenylpyrazol-5-yl dimethylcarbamate + TX, 4-methyl(prop-2-ynyl)amino-3,5-xylyl methylcarbamate + TX, 5,5-dimethyl-3-oxocyclohex-1-enyl dimethylcarbamate + TX, acethion + TX, acrylonitrile + TX, aldrin + TX, allosamidin + TX, allyxycarb + TX, alpha-ecdysone + TX, aluminium phosphide + TX, aminocarb + TX, anabasine + TX, athidathion + TX, azamethiphos + TX, Bacillus thuringiensis delta endotoxins + TX, barium hexafluorosilicate + TX, barium polysulfide + TX, barthrin + TX, Bayer 22/190 + TX, Bayer 22408 + TX, beta-cyfluthrin + TX, beta-cypermethrin + TX, bioethanomethrin + TX, biopermethrin + TX, bis(2-chloroethyl) ether + TX, borax + TX, bromfenvinfos + TX, bromo-DDT + TX, bufencarb + TX, butacarb + TX, butathiofos + TX, butonate + TX, calcium arsenate + TX, calcium cyanide + TX, carbon disulfide + TX, carbon tetrachloride + TX, cartap hydrochloride + TX, cevadine + TX, chlorbicyclen + TX, chlordane + TX, chlordecone + TX, chloroform + TX, chloropicrin + TX, chlorphoxim + TX, chlorprazophos + TX, cis-resmethrin + TX, cismethrin + TX, clocythrin + TX, copper acetoarsenite + TX, copper arsenate + TX, copper oleate + TX, coumithoate + TX, cryolite + TX, CS 708 + TX, cyanofenphos + TX, cyanophos + TX, cyclethrin + TX, cythioate + TX, d-tetramethrin + TX, DAEP + TX, dazomet + TX, decarbofuran + TX, diamidafos + TX, dicapthon + TX, dichlofenthion + TX, dicresyl + TX, dicyclanil + TX, dieldrin + TX, diethyl 5-methylpyrazol-3-yl phosphate + TX, dilor + TX, dimefluthrin + TX, dimetan + TX, dimethrin + TX, dimethylvinphos + TX, dimetilan + TX, dinoprop + TX, dinosam + TX, dinoseb + TX, diofenolan + TX, dioxabenzofos + TX, dithicrofos + TX, DSP + TX, ecdysterone + TX, El 1642 + TX, EMPC + TX, EPBP + TX, etaphos + TX, ethiofencarb + TX, ethyl formate + TX, ethylene dibromide + TX, ethylene dichloride + TX, ethylene oxide + TX, EXD + TX, fenchlorphos + TX, fenethacarb + TX, fenitrothion + TX, fenoxacrim + TX, fenpirithrin + TX, fensulfothion + TX, fenthion-ethyl + TX, flucofuron + TX, fosmethilan + TX, fospirate + TX, fosthietan + TX, furathiocarb + TX, furethrin + TX, guazatine + TX, guazatine acetates + TX, sodium tetrathiocarbonate + TX, halfenprox + TX, HCH + TX, HEOD + TX, heptachlor + TX, heterophos + TX, HHDN + TX, hydrogen cyanide + TX, hyquincarb + TX, IPSP + TX, isazofos + TX, isobenzan + TX, isodrin + TX, isofenphos + TX, isolane + TX, isoprothiolane + TX, isoxathion + TX, juvenile hormone I + TX, juvenile hormone II + TX, juvenile hormone III + TX, kelevan + TX, kinoprene + TX, lead arsenate + TX, leptophos + TX, lirimfos + TX, lythidathion + TX, m-cumenyl methylcarbamate + TX, magnesium phosphide + TX, mazidox + TX, mecarphon + TX, menazon + TX, mercurous chloride + TX, mesulfenfos + TX, metam + TX, metam-potassium + TX, metam-sodium + TX, methanesulfonyl fluoride + TX, methocrotophos + TX, methoprene + TX, methothrin + TX, methoxychlor + TX, methyl isothiocyanate + TX, methylchloroform + TX, methylene chloride + TX, metoxadiazone + TX, mirex + TX, naftalofos + TX, naphthalene + TX, NC-170 + TX, nicotine + TX, nicotine sulfate + TX, nithiazine + TX, nornicotine + TX, O-5-dichloro-4-iodophenyl O-ethyl ethylphosphonothioate + TX, O,O-diethyl O-4-methyl-2-oxo-2H-chromen-7-yl phosphorothioate + TX, O,O-diethyl O-6-methyl-2-propylpyrimidin-4-yl phosphorothioate + TX, O,O,O',O'-tetrapropyl dithiopyrophosphate + TX, oleic acid + TX, para-dichlorobenzene + TX, parathion-methyl + TX, pentachlorophenol + TX, pentachlorophenyl laurate + TX, PH 60-38 + TX, phenkapton + TX, phosnichlor + TX, phosphine + TX, phoxim-methyl + TX, pirimetaphos + TX, polychlorodicyclopentadiene isomers + TX, potassium arsenite + TX, potassium thiocyanate + TX, precocene I + TX, precocene II + TX, precocene III + TX, primidophos + TX, profluthrin + TX, promecarb + TX, prothiofos + TX, pyrazophos + TX, pyresmethrin + TX, quassia + TX, quinalphos-methyl + TX, quinothion + TX, rafoxanide + TX, resmethrin + TX, rotenone + TX, kadethrin + TX, ryania + TX, ryanodine + TX, sabadilla + TX, schradan + TX, sebufos + TX, SI-0009 + TX, thiapronil + TX, sodium arsenite + TX, sodium cyanide + TX, sodium fluoride + TX, sodium hexafluorosilicate + TX, sodium pentachlorophenoxide + TX, sodium selenate + TX, sodium thiocyanate + TX, sulcofuron + TX, sulcofuron-sodium + TX, sulfuryl fluoride + TX, sulprofos + TX, tar oils + TX, tazimcarb + TX, TDE + TX, tebupirimfos + TX, temephos + TX, terallethrin + TX, tetrachloroethane + TX, thicrofos + TX, thiocyclam + TX, thiocyclam hydrogen oxalate + TX, thionazin + TX, thiosultap + TX, thiosultap-sodium + TX, tralomethrin + TX, transpermethrin + TX, triazamate + TX, trichlormetaphos-3 + TX, trichloronat + TX, trimethacarb + TX, tolprocarb + TX, triclopyricarb + TX, triprene + TX, veratridine + TX, veratrine + TX, XMC + TX, zetamethrin + TX, zinc phosphide + TX, zolaprofos + TX, meperfluthrin + TX, tetramethylfluthrin + TX, bis(tributyltin) oxide + TX, bromoacetamide + TX, ferric phosphate + TX, niclosamide-olamine + TX, tributyltin oxide + TX, pyrimorph + TX, trifenmorph + TX, 1,2-dibromo-3-chloropropane + TX, 1,3-dichloropropene + TX, 3,4-dichlorotetrahydrothiophene 1,1-dioxide + TX, 3-(4-chlorophenyl)-5-methylrhodanine + TX, 5-methyl-6-thioxo-1,3,5-thiadiazinan-3-ylacetic acid + TX, 6-isopentenylaminopurine + TX, anisiflupurin + TX, benclothiaz + TX, cytokinins + TX, DCIP + TX, furfural + TX, isamidofos + TX, kinetin + TX, Myrothecium verrucaria composition + TX, tetrachlorothiophene + TX, xylenols + TX, zeatin + TX, potassium ethylxanthate + TX ,acibenzolar + TX, acibenzolar-S-methyl + TX, Reynoutria sachalinensis extract + TX, alpha-chlorohydrin + TX, antu + TX, barium carbonate + TX, bisthiosemi + TX, brodifacoum + TX, bromadiolone + TX, bromethalin + TX, chlorophacinone + TX, cholecalciferol + TX, coumachlor + TX, coumafuryl + TX, coumatetralyl + TX, crimidine + TX, difenacoum + TX, difethialone + TX, diphacinone + TX, ergocalciferol + TX, flocoumafen + TX, fluoroacetamide + TX, flupropadine + TX, flupropadine hydrochloride + TX, norbormide + TX, phosacetim + TX, phosphorus + TX, pindone + TX, pyrinuron + TX, scilliroside + TX, sodium fluoroacetate + TX, thallium sulfate + TX, warfarin + TX, 2-(2-butoxyethoxy)ethyl piperonylate + TX, 5-(1,3-benzodioxol-5-yl)-3-hexylcyclohex-2-enone + TX, farnesol with nerolidol + TX, verbutin + TX, MGK 264 + TX, piperonyl butoxide + TX, piprotal + TX, propyl isomer + TX, S421 + TX, sesamex + TX, sesasmolin + TX, sulfoxide + TX, anthraquinone + TX, copper naphthenate + TX, copper oxychloride + TX, dicyclopentadiene + TX, thiram + TX, zinc naphthenate + TX, ziram + TX, imanin + TX, ribavirin + TX, chloroinconazide + TX, mercuric oxide + TX, thiophanate-methyl + TX, azaconazole + TX, bitertanol + TX, bromuconazole + TX, cyproconazole + TX, difenoconazole + TX, diniconazole + TX, epoxiconazole + TX, fenbuconazole + TX, fluquinconazole + TX, flusilazole + TX, flutriafol + TX, furametpyr + TX, hexaconazole + TX, imazalil + TX, imibenconazole + TX, ipconazole + TX, metconazole + TX, myclobutanil + TX, paclobutrazole + TX, pefurazoate + TX, penconazole + TX, prothioconazole + TX, pyrifenox + TX, prochloraz + TX, propiconazole + TX, pyrisoxazole + TX, simeconazole + TX, tebuconazole + TX, tetraconazole + TX, triadimefon + TX, triadimenol + TX, triflumizole + TX, triticonazole + TX, ancymidol + TX, fenarimol + TX, nuarimol + TX, bupirimate + TX, dimethirimol + TX, ethirimol + TX, dodemorph + TX, fenpropidin + TX, fenpropimorph + TX, spiroxamine + TX, tridemorph + TX, cyprodinil + TX, mepanipyrim + TX, pyrimethanil + TX, fenpiclonil + TX, fludioxonil + TX, benalaxyl + TX, furalaxyl + TX, metalaxyl + TX, R-metalaxyl + TX, ofurace + TX, oxadixyl + TX, carbendazim + TX, debacarb + TX, fuberidazole + TX, thiabendazole + TX, chlozolinate + TX, dichlozoline + TX, myclozoline + TX, procymidone + TX, vinclozoline + TX, boscalid + TX, carboxin + TX, fenfuram + TX, flutolanil + TX, mepronil + TX, oxycarboxin + TX, penthiopyrad + TX, thifluzamide + TX, dodine + TX, iminoctadine + TX, azoxystrobin + TX, dimoxystrobin + TX, enestroburin + TX, fenaminstrobin + TX, flufenoxystrobin + TX, fluoxastrobin + TX, kresoxim-methyl + TX, metominostrobin + TX, trifloxystrobin + TX, orysastrobin + TX, picoxystrobin + TX, pyraclostrobin + TX, pyrametostrobin + TX, pyraoxystrobin + TX, ferbam + TX, mancozeb + TX, maneb + TX, metiram + TX, propineb + TX, zineb + TX, captafol + TX, captan + TX, fluoroimide + TX, folpet + TX, tolylfluanid + TX, bordeaux mixture + TX, copper oxide + TX, mancopper + TX, oxine-copper + TX, nitrothal-isopropyl + TX, edifenphos + TX, iprobenphos + TX, phosdiphen + TX, tolclofos-methyl + TX, anilazine + TX, benthiavalicarb + TX, blasticidin-S + TX, chloroneb + TX, chlorothalonil + TX, cyflufenamid + TX, cymoxanil + TX, cyclobutrifluram + TX, diclocymet + TX, diclomezine + TX, dicloran + TX, diethofencarb + TX, dimethomorph + TX, flumorph + TX, dithianon + TX, ethaboxam + TX, etridiazole + TX, famoxadone + TX, fenamidone + TX, fenoxanil + TX, ferimzone + TX, fluazinam + TX, flumetylsulforim + TX, fluopicolide + TX, fluoxytioconazole + TX, flusulfamide + TX, fluxapyroxad + TX, fenhexamid + TX, fosetyl-aluminium + TX, hymexazol + TX, iprovalicarb + TX, cyazofamid + TX, methasulfocarb + TX, metrafenone + TX, pencycuron + TX, phthalide + TX, polyoxins + TX, propamocarb + TX, pyribencarb + TX, proquinazid + TX, pyroquilon + TX, pyriofenone + TX, quinoxyfen + TX, quintozene + TX, tiadinil + TX, triazoxide + TX, tricyclazole + TX, triforine + TX, validamycin + TX, valifenalate + TX, zoxamide + TX, mandipropamid + TX, flubeneteram + TX, isopyrazam + TX, sedaxane + TX, benzovindiflupyr + TX, pydiflumetofen + TX, 3-difluoromethyl-1-methyl-1H-pyrazole-4-carboxylic acid (3',4',5'-trifluoro-biphenyl-2-yl)-amide + TX, isoflucypram + TX, isotianil + TX, dipymetitrone + TX, 6-ethyl-5,7-dioxo-pyrrolo[4,5][1,4]dithiino[1,2-c]isothiazole-3-carbonitrile + TX, 2-(difluoromethyl)-N-[3-ethyl-1,1-dimethyl-indan-4-yl]pyridine-3-carboxamide + TX, 4-(2,6-difluorophenyl)-6-methyl-5-phenyl-pyridazine-3-carbonitrile + TX, (R)-3-(difluoromethyl)-1-methyl-N-[1,1,3-trimethylindan-4-yl]pyrazole-4-carboxamide + TX, 4-(2-bromo-4-fluoro-phenyl)-N-(2-chloro-6-fluoro-phenyl)-2,5-dimethyl-pyrazol-3-amine + TX, 4- (2- bromo- 4-fluorophenyl) - N- (2- chloro- 6- fluorophenyl) - 1, 3- dimethyl- 1H- pyrazol- 5- amine + TX, fluindapyr + TX, coumethoxystrobin (jiaxiangjunzhi) + TX, Ivbenmixianan + TX, dichlobentiazox + TX, mandestrobin + TX, 3-(4,4-difluoro-3,4-dihydro-3,3-dimethylisoquinolin-1-yl)quinolone + TX, 2-[2-fluoro-6-[(8-fluoro-2-methyl-3-quinolyl)oxy]phenyl]propan-2-ol + TX, oxathiapiprolin + TX, tert-butyl N-[6-[[[(1-methyltetrazol-5-yl)-phenyl-methylene]amino]oxymethyl]-2-pyridyl]carbamate + TX, pyraziflumid + TX, inpyrfluxam + TX, trolprocarb + TX, mefentrifluconazole + TX, ipfentrifluconazole+ TX, 2-(difluoromethyl)-N-[(3R)-3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine + TX, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate + TX, but-3-ynyl N-[6-[[(Z)-[(1-methyltetrazol-5-yl)-phenyl-methylene]amino]oxymethyl]-2-pyridyl]carbamate + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine + TX, pyridachlometyl + TX, 3-(difluoromethyl)-1-methyl-N-[1,1,3-trimethylindan-4-yl]pyrazole-4-carboxamide + TX, 1-[2-[[1-(4-chlorophenyl)pyrazol-3-yl]oxymethyl]-3-methyl-phenyl]-4-methyl-tetrazol-5-one + TX, 1-methyl-4-[3-methyl-2-[[2-methyl-4-(3,4,5-trimethylpyrazol-1-yl)phenoxy]methyl]phenyl]tetrazol-5-one + TX, aminopyrifen + TX, ametoctradin + TX, amisulbrom + TX, penflufen + TX, (Z,2E)-5-[1-(4-chlorophenyl)pyrazol-3-yl]oxy-2-methoxyimino-N,3-dimethyl-pent-3-enamide + TX, florylpicoxamid + TX, fenpicoxamid + TX, metarylpicoxamid + TX, tebufloquin + TX, ipflufenoquin + TX, quinofumelin + TX, isofetamid + TX, ethyl 1-[[4-[[2-(trifluoromethyl)-1,3-dioxolan-2-yl]methoxy]phenyl]methyl]pyrazole-3-carboxylate + TX (may be prepared from the methods described in WO 2020/056090), ethyl 1-[[4-[(Z)-2-ethoxy-3,3,3-trifluoro-prop-1-enoxy]phenyl]methyl]pyrazole-3-carboxylate + TX (may be prepared from the methods described in WO 2020/056090), methyl N-[[4-[1-(4-cyclopropyl-2,6-difluoro-phenyl)pyrazol-4-yl]-2-methylphenyl]methyl]carbamate + TX (may be prepared from the methods described in WO 2020/097012), methyl N-[[4-[1-(2,6-difluoro-4-isopropyl-phenyl)pyrazol-4-yl]-2-methyl-phenyl]methyl]carbamate + TX (may be prepared from the methods described in WO 2020/097012), 6-chloro-3-(3-cyclopropyl-2-fluoro-phenoxy)-N-[2-(2,4-dimethylphenyl)-2,2-difluoro-ethyl]-5-methyl-pyridazine-4-carboxamide + TX (may be prepared from the methods described in WO 2020/109391), 6-chloro-N-[2-(2-chloro-4-methylphenyl)-2,2-difluoro-ethyl]-3-(3-cyclopropyl-2-fluoro-phenoxy)-5-methyl-pyridazine-4-carboxamide + TX (may be prepared from the methods described in WO 2020/109391), 6-chloro-3-(3-cyclopropyl-2-fluoro-phenoxy)-N-[2-(3,4-dimethylphenyl)-2,2-difluoro-ethyl]-5-methyl-pyridazine-4-carboxamide + TX (may be prepared from the methods described in WO 2020/109391), N-[2-[2,4-dichloro-phenoxy]phenyl]-3-(difluoromethyl)-1-methyl-pyrazole-4-carboxamide + TX, N-[2-[2-chloro-4-(trifluoromethyl)phenoxy]phenyl]-3-(difluoromethyl)-1-methyl-pyrazole-4-carboxamide + TX, benzothiostrobin + TX, phenamacril + TX, 5-amino-1,3,4-thiadiazole-2-thiol zinc salt (2:1) + TX, fluopyram + TX, flufenoxadiazam + TX, flutianil + TX, fluopimomide + TX, pyrapropoyne + TX, picarbutrazox + TX, 2-(difluoromethyl)-N-(3-ethyl-1,1-dimethyl-indan-4-yl)pyridine-3-carboxamide + TX, 2- (difluoromethyl) - N- ((3R) - 1, 1, 3- trimethylindan- 4- yl) pyridine- 3- carboxamide + TX, 4-[[6-[2-(2,4-difluorophenyl)-1,1-difluoro-2-hydroxy-3-(1,2,4-triazol-1-yl)propyl]-3-pyridyl]oxy]benzonitrile + TX, metyltetraprole + TX, 2- (difluoromethyl) - N- ((3R) - 1, 1, 3- trimethylindan- 4- yl) pyridine- 3-carboxamide + TX, α- (1, 1- dimethylethyl) - α- [4'- (trifluoromethoxy) [1, 1'- biphenyl] - 4- yl] -5-pyrimidinemethanol + TX, fluoxapiprolin + TX, enoxastrobin + TX, methyl (Z)-3-methoxy-2-[2-methyl-5-[4-(trifluoromethyl)triazol-2-yl]phenoxy]prop-2-enoate + TX, methyl (Z)-3-methoxy-2-[2-methyl-5-(4-propyltriazol-2-yl)phenoxy]prop-2-enoate + TX, methyl (Z)-2-[5-(3-isopropylpyrazol-1-yl)-2-methyl-phenoxy]-3-methoxy-prop-2-enoate + TX, methyl (Z)-3-methoxy-2-[2-methyl-5-(3-propylpyrazol-1-yl)phenoxy]prop-2-enoate + TX, methyl (Z)-3-methoxy-2-[2-methyl-5-[3-(trifluoromethyl)pyrazol-1-yl]phenoxy]prop-2-enoate + TX (these compounds may be prepared from the methods described in WO2020/079111), methyl (Z)-2-(5-cyclohexyl-2-methyl-phenoxy)-3-methoxy-prop-2-enoate + TX, methyl (Z)-2-(5-cyclopentyl-2-methyl-phenoxy)-3-methoxy-prop-2-enoate + TX (these compounds may be prepared from the methods described in WO2020/193387), 4-[[6-[2-(2,4-difluorophenyl)-1,1-difluoro-2-hydroxy-3-(1,2,4-triazol-1-yl)propyl]-3-pyridyl]oxy] benzonitrile + TX, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile + TX, trinexapac + TX, coumoxystrobin + TX, zhongshengmycin + TX, thiodiazole copper + TX, zinc thiazole + TX, amectotractin + TX, iprodione + TX, seboctylamine + TX; N'-[5-bromo-2-methyl-6-[(1S)-1-methyl-2-propoxy-ethoxy]-3-pyridyl]-N-ethyl-N-methyl-formamidine + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine + TX (these compounds may be prepared from the methods described in WO2015/155075); N'-[5-bromo-2-methyl-6-(2-propoxypropoxy)-3-pyridyl]-N-ethyl-N-methyl-formamidine + TX (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine+ TX, N'-[4-(1-cyclopropyl-2,2,2-trifluoro-1-hydroxy-ethyl)-5-methoxy-2-methyl-phenyl]-N-isopropyl-N-methyl-formamidine + TX (these compounds may be prepared from the methods described in WO2018/228896); N-ethyl-N'-[5-methoxy-2-methyl-4-[(2-trifluoromethyl)oxetan-2-yl]phenyl]-N-methyl-formamidine + TX, N-ethyl-N'-[5-methoxy-2-methyl-4-[(2-trifuoromethyl)tetrahydrofuran-2-yl]phenyl]-N-methyl-formamidine + TX (these compounds may be prepared from the methods described in WO02019/110427); N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-3,3,3-trifluoro-1-methyl-propyl]-8-fluoro-quinoline-3-carboxamide + TX, N-[(1R)-1-benzyl-1,3-dimethyl-butyl]-7,8-difluoro-quinoline-3-carboxamide + TX, N-[(1S)-1-benzyl-1,3-dimethyl-butyl]-7,8-difluoro-quinoline-3-carboxamide + TX, 8-fluoro-N-[(1R)-1-[(3-fluorophenyl)methyl]-1,3-dimethyl-butyl]quinoline-3-carboxamide + TX, 8-fluoro-N-[(1S)-1-[(3-fluorophenyl)methyl]-1 ,3-dimethyl-butyl]quinoline-3-carboxamide + TX, N-[(1R)-1-benzyl-1,3-dimethyl-butyl]-8-fluoro-quinoline-3-carboxamide + TX, N-[(1S)-1-benzyl-1,3-dimethyl-butyl]-8-fluoro-quinoline-3-carboxamide + TX, N-((1R)-1-benzyl-3-chloro-1-methyl-but-3-enyl)-8-fluoro-quinoline-3-carboxamide + TX, N-((1S)-1-benzyl-3-chloro-1-methyl-but-3-enyl)-8-fluoro-quinoline-3-carboxamide + TX (these compounds may be prepared from the methods described in WO2017/153380); 1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 1-(6-chloro-7-methyl-pyrazolo[1,5-a]pyridin-3-yl)-4,4-difluoro-3,3-dimethyl-isoquinoline + TX (these compounds may be prepared from the methods described in WO2017/025510); 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 6-chloro-4,4-difluoro-3,3-dimethyl-1-(4-methylbenzimidazol-1-yl)isoquinoline + TX, 4,4-difluoro-1-(5-fluoro-4-methyl-benzimidazol-1-yl)-3,3-dimethyl-isoquinoline + TX, 3-(4,4-difluoro-3,3-dimethyl-1-isoquinolyl)-7,8-dihydro-6H-cyclopenta[e]benzimidazole + TX (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide + TX, N,2-dimethoxy-N-[[4-[5-(trifluoromethyl)-1,2,4-oxadiazol-3-yl]phenyl]methyl]propanamide + TX, N-ethyl-2-methyl-N-[[4-[5-(trifluoromethyl)-1,2,4-oxadiazol-3-yl]phenyl]methyl]propanamide + TX, 1-methoxy-3-methyl-1-[[4-[5-(trifluoromethyl)-1,2,4-oxadiazol-3-yl]phenyl]methyl]urea + TX, 1,3-dimethoxy-1-[[4-[5-(trifluoromethyl)-1,2,4-oxadiazol-3-yl]phenyl]methyl]urea + TX, 3-ethyl-1-methoxy-1-[[4-[5-(trifluoromethyl)-1,2,4-oxadiazol-3-yl]phenyl]methyl]urea + TX, N-[[4-[5-(trifluoromethyl)-1,2,4-oxadiazol-3-yl]phenyl]methyl]propanamide + TX, 4,4-dimethyl-2-[[4-[5-(trifluoromethyl)-1,2,4-oxadiazol-3-yl]phenyl]methyl]isoxazolidin-3-one + TX, 5,5-dimethyl-2-[[4-[5-(trifluoromethyl)-1,2,4-oxadiazol-3-yl]phenyl]methyl]isoxazolidin-3-one + TX, ethyl 1-[[4-[5-(trifluoromethyl)-1,2,4-oxadiazol-3-yl]phenyl]methyl]pyrazole-4-carboxylate + TX, N,N-dimethyl-1-[[4-[5-(trifluoromethyl)-1,2,4-oxadiazol-3-yl]phenyl]methyl]-1,2,4-triazol-3-amine + TX. The compounds in this paragraph may be prepared from the methods described in WO 2017/055473, WO 2017/055469, WO 2017/093348 and WO 2017/118689; 2-[6-(4-chlorophenoxy)-2-(trifluoromethyl)-3-pyridyl]-1-(1,2,4-triazol-1-yl)propan-2-ol + TX (this compound may be prepared from the methods described in WO 2017/029179); 2-[6-(4-bromophenoxy)-2-(trifluoromethyl)-3-pyridyl]-1-(1,2,4-triazol-1-yl)propan-2-ol + TX (this compound may be prepared from the methods described in WO 2017/029179); 3-[2-(1-chlorocyclopropyl)-3-(2-fluorophenyl)-2-hydroxy-propyl]imidazole-4-carbonitrile + TX (this compound may be prepared from the methods described in WO 2016/156290); 3-[2-(1-chlorocyclopropyl)-3-(3-chloro-2-fluoro-phenyl)-2-hydroxy-propyl]imidazole-4-carbonitrile + TX (this compound may be prepared from the methods described in WO 2016/156290); (4-phenoxyphenyl)methyl 2-amino-6-methyl-pyridine-3-carboxylate + TX (this compound may be prepared from the methods described in WO 2014/006945); 2,6-Dimethyl-1H,5H-[1,4]dithiino[2,3-c:5,6-c']dipyrrole-1,3,5,7(2H,6H)-tetrone + TX (this compound may be prepared from the methods described in WO 2011/138281); N-methyl-4-[5-(trifluoromethyl)-1,2,4-oxadiazol-3-yl]benzenecarbothioamide + TX; N-methyl-4-[5-(trifluoromethyl)-1,2,4-oxadiazol-3-yl]benzamide + TX; (Z,2E)-5-[1-(2,4-dichlorophenyl)pyrazol-3-yl]oxy-2-methoxyimino-N,3-dimethyl-pent-3-enamide + TX (this compound may be prepared from the methods described in WO 2018/153707); N'-(2-chloro-5-methyl-4-phenoxyphenyl)-N-ethyl-N-methyl-formamidine + TX; N'-[2-chloro-4-(2-fluorophenoxy)-5-methyl-phenyl]-N-ethyl-N-methyl-formamidine + TX (this compound may be prepared from the methods described in WO 2016/202742); 2-(difluoromethyl)-N-[(3S)-3-ethyl-1,1-dimethyl-indan-4-yl]pyridine-3-carboxamide + TX (this compound may be prepared from the methods described in WO 2014/095675); (5-methyl-2-pyridyl)-[4-[5-(trifluoromethyl)-1,2,4-oxadiazol-3-yl]phenyl]methanone + TX, (3-methylisoxazol-5-yl)-[4-[5-(trifluoromethyl)-1,2,4-oxadiazol-3-yl]phenyl]methanone + TX (these compounds may be prepared from the methods described in WO 2017/220485); 2-oxo-N-propyl-2-[4-[5-(trifluoromethyl)-1,2,4-oxadiazol-3-yl]phenyl]acetamide + TX (this compound may be prepared from the methods described in WO 2018/065414); ethyl 1-[[5-[5-(trifluoromethyl)-1,2,4-oxadiazol-3-yl]-2-thienyl]methyl]pyrazole-4-carboxylate + TX (this compound may be prepared from the methods described in WO 2018/158365); 2,2-difluoro-N-methyl-2-[4-[5-(trifluoromethyl)-1,2,4-oxadiazol-3-yl]phenyl]acetamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[N-methoxy-C-methyl-carbonimidoyl]-4-[5-(trifluoromethyl)-1,2,4-oxadiazol-3-yl]benzamide + TX (these compounds may be prepared from the methods described in WO 2018/202428);
microbials including: *Acinetobacter Iwoffii* + TX, *Acremonium alternatum* + TX + TX, *Acremonium* cephalosporium + TX + TX, *Acremonium diospyri* + TX, *Acremonium obclavatum* + TX, *Adoxophyes orana granulovirus* (AdoxGV) (Capex^{®}) + TX, *Agrobacterium radiobacter* strain K84 (Galltrol-A^{®}) + TX, *Alternaria alternate* + TX, *Alternaria cassia* + TX, *Alternaria destruens* (Smolder^{®}) + TX, *Ampelomyces quisqualis* (AQ10^{®}) + TX, *Aspergillus flavus* AF36 (AF36^{®}) + TX, *Aspergillus flavus* NRRL 21882 (Aflaguard^{®}) + TX, *Aspergillus* spp. + TX, *Aureobasidium pullulans* + TX, *Azospirillum* + TX, (MicroAZ^{®} + TX, TAZO B^{®}) + TX, *Azotobacter* + TX, *Azotobacter chroocuccum* (Azotomeal^{®}) + TX, *Azotobacter* cysts (Bionatural Blooming Blossoms^{®}) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart^{™} Rhizoboost^{®}) + TX, *Bacillus licheniformis* strain 3086 (EcoGuard^{®} + TX, Green Releaf^{®}) + TX, *Bacillus circulans* + TX, *Bacillus firmus* (BioSafe^{®} + TX, BioNem-WP^{®} + TX, VOTiVO^{®}) + TX, *Bacillus firmus* strain I-1582 + TX, *Bacillus macerans* + TX, *Bacillus marismortui* + TX, *Bacillus megaterium* + TX, *Bacillus mycoides* strain AQ726 + TX, *Bacillus papillae* (Milky Spore Powderm^{®}) + TX, *Bacillus pumilus* spp. + TX, *Bacillus pumilus* strain GB34 (Yield Shield^{®}) + TX, *Bacillus pumilus* strain AQ717 + TX, *Bacillus pumilus* strain QST 2808 (Sonata^{®} + TX, Ballad Plus^{®}) + TX, *Bacillus spahericus* (VectoLex^{®}) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE^{®} + TX, Serenade^{®} + TX, Rhapsody^{®}) + TX, *Bacillus subtilis* strain QST 714 (JAZZ^{®}) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro^{®} + TX, Rhizopro^{®}) + TX, *Bacillus thuringiensis* Cry 2Ae + TX, *Bacillus thuringiensis* Cry1Ab + TX, *Bacillus thuringiensis aizawai* GC 91 (Agree^{®}) + TX, *Bacillus thuringiensis israelensis* (BMP123^{®} + TX, Aquabac^{®} + TX, VectoBac^{®}) + TX, *Bacillus thuringiensis kurstaki* (Javelin^{®} + TX, Deliver^{®} + TX, CryMax^{®} + TX, Bonide^{®} + TX, Scutella WP^{®} + TX, Turilav WP ^{®} + TX, Astuto^{®} + TX, Dipel WP^{®} + TX, Biobit^{®} + TX, Foray^{®}) + TX, *Bacillus thuringiensis kurstaki* BMP 123 (Baritone^{®}) + TX, *Bacillus thuringiensis kurstaki* HD-1 (Bioprotec-CAF / 3P^{®}) + TX, *Bacillus thuringiensis* strain BD#32 + TX, *Bacillus thuringiensis* strain AQ52 + TX, *Bacillus thuringiensis var. aizawai* (XenTari^{®} + TX, DiPel^{®}) + TX, bacteria spp. (GROWMEND^{®} + TX, GROWSWEET^{®} + TX, Shootup^{®}) + TX, bacteriophage of *Clavipacter michiganensis* (AgriPhage^{®}) + TX, Bakflor^{®} + TX, *Beauveria bassiana* (Beaugenic^{®} + TX, Brocaril WP^{®}) + TX, *Beauveria bassiana* GHA (Mycotrol ES^{®} + TX, Mycotrol O^{®} + TX, BotaniGuard^{®}) + TX, *Beauveria brongniartii* (Engerlingspilz^{®} + TX, Schweizer Beauveria^{®} + TX, Melocont^{®}) + TX, *Beauveria* spp. + TX, *Botrytis cineria* + TX, *Bradyrhizobium japonicum* (TerraMax^{®}) + TX, *Brevibacillus brevis* + TX, *Bacillus thuringiensis tenebrionis* (Novodor^{®}) + TX, BtBooster + TX, *Burkholderia cepacia* (Deny^{®} + TX, Intercept^{®} + TX, Blue Circle^{®}) + TX, *Burkholderia gladii* + TX, *Burkholderia gladioli* + TX, *Burkholderia* spp. + TX, Canadian thistle fungus (CBH Canadian Bioherbicide^{®}) + TX, *Candida butyri* + TX, *Candida famata* + TX, *Candida fructus* + TX, *Candida glabrata* + TX, *Candida guilliermondii* + TX, *Candida melibiosica* + TX, *Candida oleophila* strain O + TX, *Candida parapsilosis* + TX, *Candida pelliculosa* + TX, *Candida pulcherrima* + TX, *Candida reukaufii* + TX, *Candida saitoana* (Bio-Coat^{®} + TX, Biocure^{®}) + TX, *Candida sake* + TX, *Candida* spp. + TX, *Candida tenius* + TX, *Cedecea dravisae* + TX, *Cellulomonas flavigena* + TX, *Chaetomium cochliodes* (Nova-Cide^{®}) + TX, *Chaetomium globosum* (Nova-Cide^{®}) + TX, *Chromobacterium subtsugae* strain PRAA4-1T (Grandevo^{®}) + TX, *Cladosporium cladosporioides +* TX, *Cladosporium oxysporum +* TX, *Cladosporium chlorocephalum +* TX, *Cladosporium* spp. + TX, *Cladosporium tenuissimum +* TX, *Clonostachys rosea* (EndoFine^{®}) + TX, *Colletotrichum acutatum +* TX, *Coniothyrium minitans* (Cotans WG^{®}) + TX, *Coniothyrium* spp. + TX, *Cryptococcus albidus* (YIELDPLUS^{®}) + TX, *Cryptococcus humicola +* TX, *Cryptococcus infirmo-miniatus +* TX, *Cryptococcus laurentii +* TX, *Cryptophlebia leucotreta granulovirus* (Cryptex^{®}) + TX, *Cupriavidus campinensis +* TX, *Cydia pomonella granulovirus* (CYD-X^{®}) + TX, *Cydia pomonella granulovirus* (Madex^{®} + TX, Madex Plus^{®} + TX, Madex Maxi Carpovirusine^{®}) + TX, *Cylindrobasidium laeve* (Stu mpout^{®}) + TX, *Cylindrocladium +* TX, *Debaryomyces hansenii +* TX, *Drechslera hawaiinensis* + TX, *Enterobacter cloacae* + TX, *Enterobacteriaceae* + TX, *Entomophtora virulenta* (Vektor^{®}) + TX, *Epicoccum nigrum +* TX, *Epicoccum purpurascens +* TX, *Epicoccum* spp. + TX, *Filobasidium floriforme* + TX, *Fusarium acuminatum +* TX, *Fusarium chlamydosporum +* TX, *Fusarium oxysporum* (Fusaclean^{®} / Biofox C^{®}) + TX, *Fusarium proliferatum +* TX, *Fusarium* spp. + TX, *Galactomyces geotrichum +* TX, *Gliocladium catenulatum* (Primastop^{®} + TX, Prestop^{®}) + TX, *Gliocladium roseum +* TX, *Gliocladium* spp. (SoilGard^{®}) + TX, *Gliocladium virens* (Soilgard^{®}) + TX, *Granulovirus* (Granupom^{®}) + TX, *Halobacillus halophilus +* TX, *Halobacillus litoralis* + TX, *Halobacillus trueperi* + TX, *Halomonas* spp. + TX, *Halomonas subglaciescola +* TX, *Halovibrio variabilis +* TX, *Hanseniaspora uvarum +* TX, *Helicoverpa armigera nucleopolyhedrovirus* (Helicovex^{®}) + TX, *Helicoverpa zea nuclear polyhedrosis virus* (Gemstar^{®}) + TX, Isoflavone - formononetin (Myconate^{®}) + TX, *Kloeckera apiculata* + TX, *Kloeckera* spp. + TX, *Lagenidium giganteum* (Laginex^{®}) + TX, *Lecanicillium longisporum* (Vertiblast^{®}) + TX, *Lecanicillium muscarium* (Vertikil^{®}) + TX, *Lymantria Dispar nucleopolyhedrosis virus* (Disparvirus^{®}) + TX, *Marinococcus halophilus* + TX, *Meira geulakonigii* + TX, *Metarhizium anisopliae* (Met52^{®}) + TX, *Metarhizium anisopliae* (Destruxin WP^{®}) + TX, *Metschnikowia fruticola* (Shemer^{®}) + TX, *Metschnikowia pulcherrima* + TX, *Microdochium dimerum* (Antibot^{®}) + TX, *Micromonospora coerulea +* TX, *Microsphaeropsis ochracea* + TX, *Muscodor albus* 620 (Muscudor^{®}) + TX, *Muscodor roseus* strain A3-5 + TX, *Mycorrhizae* spp. (AMykor^{®} + TX, Root Maximizers^{®}) + TX, *Myrothecium verrucaria* strain AARC-0255 (DiTera^{®}) + TX, BROS PLUS^{®} + TX, *Ophiostoma piliferum* strain D97 (Sylvanex^{®}) + TX, *Paecilomyces farinosus +* TX, *Paecilomyces fumosoroseus* (PFR-97^{®} + TX, PreFeRal^{®}) + TX, *Paecilomyces linacinus* (Biostat WP^{®}) + TX, *Paecilomyces lilacinus* strain 251 (MeloCon WG^{®}) + TX, *Paenibacillus polymyxa* + TX, *Pantoea agglomerans* (BlightBan C9-1^{®}) + TX, *Pantoea* spp. + TX, *Pasteuria* spp. (Econem^{®}) + TX, *Pasteuria nishizawae* + TX, *Penicillium aurantiogriseum +* TX, *Penicillium billai* (Jumpstart^{®} + TX, TagTeam^{®}) + TX, *Penicillium brevicompactum +* TX, *Penicillium frequentans +* TX, *Penicillium griseofulvum +* TX, *Penicillium purpurogenum +* TX, *Penicillium* spp. + TX, *Penicillium viridicatum +* TX, *Phlebiopsis gigantean* (Rotstop^{®}) + TX, phosphate solubilizing bacteria (Phosphomeal^{®}) + TX, *Phytophthora cryptogea +* TX, *Phytophthora palmivora* (Devine^{®}) + TX, *Pichia anomala* + TX, *Pichia guilermondii +* TX, *Pichia membranaefaciens +* TX, *Pichia onychis* + TX, *Pichia stipites* + TX, *Pseudomonas aeruginosa +* TX, *Pseudomonas aureofasciens* (Spot-Less Biofungicide^{®}) + TX, *Pseudomonas cepacia* + TX, *Pseudomonas chlororaphis* (AtEze^{®}) + TX, *Pseudomonas corrugate* + TX, *Pseudomonas fluorescens* strain A506 (BlightBan A506^{®}) + TX, *Pseudomonas putida +* TX, *Pseudomonas reactans +* TX, *Pseudomonas* spp. + TX, *Pseudomonas syringae* (Bio-Save^{®}) + TX, *Pseudomonas viridiflava +* TX, *Pseudomons fluorescens* (Zequanox^{®}) + TX, *Pseudozyma flocculosa* strain PF-A22 UL (Sporodex L^{®}) + TX, *Puccinia canaliculata* + TX, *Puccinia thlaspeos* (Wood Warrior^{®}) + TX, *Pythium paroecandrum +* TX, *Pythium oligandrum* (Polygandron^{®} + TX, Polyversum^{®}) + TX, *Pythium periplocum +* TX, *Rhanella aquatilis +* TX, *Rhanella* spp. + TX, *Rhizobia* (Dormal^{®} + TX, Vault^{®}) + TX, *Rhizoctonia* + TX, *Rhodococcus globerulus* strain AQ719 + TX, *Rhodosporidium diobovatum +* TX, *Rhodosporidium toruloides +* TX, *Rhodotorula* spp. + TX, *Rhodotorula glutinis +* TX, *Rhodotorula graminis +* TX, *Rhodotorula mucilagnosa +* TX, *Rhodotorula rubra +* TX, *Saccharomyces cerevisiae* + TX, *Salinococcus roseus* + TX, *Sclerotinia minor +* TX, *Sclerotinia minor* (SARRITOR^{®}) + TX, *Scytalidium* spp. + TX, *Scytalidium uredinicola* + TX, *Spodoptera exigua nuclear polyhedrosis virus* (Spod-X^{®} + TX, Spexit^{®}) + TX, *Serratia marcescens +* TX, *Serratia plymuthica* + TX, *Serratia* spp. + TX, *Sordaria fimicola* + TX, *Spodoptera littoralis nucleopolyhedrovirus* (Littovir^{®}) + TX, *Sporobolomyces roseus* + TX, *Stenotrophomonas maltophilia* + TX, *Streptomyces* ahygroscopicus + TX, *Streptomyces albaduncus +* TX, *Streptomyces exfoliates +* TX, *Streptomyces galbus +* TX, *Streptomyces griseoplanus +* TX, *Streptomyces griseoviridis* (Mycostop^{®}) + TX, *Streptomyces lydicus* (Actinovate^{®}) + TX, *Streptomyces lydicus* WYEC-108 (ActinoGrow^{®}) + TX, *Streptomyces violaceus +* TX, *Tilletiopsis minor +* TX, *Tilletiopsis* spp. + TX, *Trichoderma asperellum* (T34 Biocontrol^{®}) + TX, *Trichoderma gamsii* (Tenet^{®}) + TX, *Trichoderma atroviride* (Plantmate^{®}) + TX, *Trichoderma hamatum* TH 382 + TX, *Trichoderma harzianum rifai* (Mycostar^{®}) + TX, *Trichoderma harzianum* T-22 (Trianum-P^{®} + TX, PlantShield HC^{®} + TX, RootShield^{®} + TX, Trianum-G^{®}) + TX, *Trichoderma harzianum* T-39 (Trichodex^{®}) + TX, *Trichoderma inhamatum +* TX, *Trichoderma koningii +* TX, *Trichoderma* spp. LC 52 (Sentinel^{®}) + TX, *Trichoderma lignorum* + TX, *Trichoderma longibrachiatum* + TX, *Trichoderma polysporum* (Binab T^{®}) + TX, *Trichoderma taxi* + TX, *Trichoderma virens +* TX, *Trichoderma virens* (formerly Gliocladium virens GL-21) (SoilGuard^{®}) + TX, *Trichoderma viride +* TX, *Trichoderma viride* strain ICC 080 (Remedier^{®}) + TX, *Trichosporon pullulans +* TX, *Trichosporon* spp. + TX, *Trichothecium* spp. + TX, *Trichothecium roseum +* TX, *Typhula phacorrhiza* strain 94670 + TX, *Typhula phacorrhiza* strain 94671 + TX, *Ulocladium atrum +* TX, *Ulocladium oudemansii* (Botry-Zen^{®}) + TX, *Ustilago maydis +* TX, various bacteria and supplementary micronutrients (Natural II^{®}) + TX, various fungi (Millennium Microbes^{®}) + TX, *Verticillium chlamydosporium +* TX, *Verticillium lecanii* (Mycotal^{®} + TX, Vertalec^{®}) + TX, Vip3Aa20 (VIPtera^{®}) + TX, *Virgibaclillus marismortui +* TX, *Xanthomonas campestris pv. Poae* (Camperico^{®}) + TX, *Xenorhabdus bovienii +* TX, *Xenorhabdus nematophilus;*
Plant extracts including: pine oil (Retenol^{®}) + TX, azadirachtin (Plasma Neem Oil@ + TX, AzaGuard^{®} + TX, MeemAzal^{®} + TX, Molt-X^{®} + TX, Botanical IGR (Neemazad^{®} + TX, Neemix^{®}) + TX, canola oil (Lilly Miller Vegol^{®}) + TX, *Chenopodium ambrosioides near ambrosioides* (Requiem^{®}) + TX, *Chrysanthemum* extract (Crisant^{®}) + TX, extract of neem oil (Trilogy^{®}) + TX, essentials oils of *Labiatae* (Botania^{®}) + TX, extracts of clove rosemary peppermint and thyme oil (Garden insect killer^{®}) + TX, Glycinebetaine (Greenstim^{®}) + TX, garlic + TX, lemongrass oil (GreenMatch^{®}) + TX, neem oil + TX, *Nepeta cataria* (Catnip oil) + TX, *Nepeta catarina +* TX, nicotine + TX, oregano oil (MossBuster^{®}) + TX, *Pedaliaceae* oil (Nematon^{®}) + TX, pyrethrum + TX, *Quillaja saponaria* (NemaQ^{®}) + TX, *Reynoutria sachalinensis* (Regalia^{®} + TX, Sakalia^{®}) + TX, rotenone (Eco Roten^{®}) + TX, *Rutaceae* plant extract (Soleo^{®}) + TX, soybean oil (Ortho ecosense^{®}) + TX, tea tree oil (Timorex Gold^{®}) + TX, thymus oil + TX, AGNIQUE^{®} MMF + TX, BugOil^{®} + TX, mixture of rosemary sesame pepermint thyme and cinnamon extracts (EF 300^{®}) + TX, mixture of clove rosemary and peppermint extract (EF 400^{®}) + TX, mixture of clove pepermint garlic oil and mint (Soil Shot^{®}) + TX, kaolin (Screen^{®}) + TX, storage glucam of brown algae (Laminarin^{®});
pheromones including: blackheaded fireworm pheromone (3M Sprayable Blackheaded Fireworm Pheromone^{®}) + TX, Codling Moth Pheromone (Paramount dispenser-(CM)/ Isomate C-Plus^{®}) + TX, Grape Berry Moth Pheromone (3M MEC-GBM Sprayable Pheromone^{®}) + TX, Leafroller pheromone (3M MEC - LR Sprayable Pheromone^{®}) + TX, Muscamone (Snip7 Fly Bait^{®} + TX, Starbar Premium Fly Bait^{®}) + TX, Oriental Fruit Moth Pheromone (3M oriental fruit moth sprayable pheromone^{®}) + TX, Peachtree Borer Pheromone (lsomate-P^{®}) + TX, Tomato Pinworm Pheromone (3M Sprayable pheromone^{®}) + TX, Entostat powder (extract from palm tree) (Exosex CM^{®}) + TX, (3E,8Z,11Z)-3,8,11-Tetradecatrienyl acetate + TX, (7Z,11Z,13E)-7,11,13-Hexadecatrienal + TX, (E,Z)-7,9-Dodecadien-1-yl acetate + TX, 2-Methyl-1-butanol + TX, Calcium acetate + TX, Scenturion^{®} + TX, Biolure^{®} + TX, Check-Mate^{®} + TX, Lavandulyl senecioate + TX;
Macrobials including: *Aphelinus abdominalis +* TX, *Aphidius ervi* (Aphelinus-System^{®}) + TX, *Acerophagus papaya* + TX, *Adalia bipunctata* (Adalia-System^{®}) + TX, *Adalia bipunctata* (Adaline^{®}) + TX, *Adalia bipunctata* (Aphidalia^{®}) + TX, *Ageniaspis citricola +* TX, *Ageniaspis fuscicollis +* TX, *Amblyseius andersoni* (Anderline^{®} + TX, Andersoni-System^{®}) + TX, *Amblyseius californicus* (Amblyline^{®} + TX, Spical^{®}) + TX, *Amblyseius cucumeris* (Thripex^{®} + TX, Bugline cucumeris^{®}) + TX, *Amblyseius fallacis* (Fallacis^{®}) + TX, *Amblyseius swirskii* (Bugline swirskii^{®} + TX, Swirskii-Mite^{®}) + TX, *Amblyseius womersleyi* (WomerMite^{®}) + TX, *Amitus hesperidum +* TX, *Anagrus atomus +* TX, *Anagyrus fusciventris* + TX, *Anagyrus kamali +* TX, *Anagyrus loecki +* TX, *Anagyrus pseudococci* (Citripar^{®}) + TX, *Anicetus benefices* + TX, *Anisopteromalus calandrae* + TX, *Anthocoris nemoralis* (Anthocoris-System^{®}) + TX, *Aphelinus abdominalis* (Apheline^{®} + TX, Aphiline^{®}) + TX, *Aphelinus asychis +* TX, *Aphidius colemani* (Aphipar^{®}) + TX, *Aphidius ervi* (Ervipar^{®}) + TX, *Aphidius gifuensis +* TX, *Aphidius matricariae* (Aphipar-M^{®}) + TX, *Aphidoletes aphidimyza* (Aphidend^{®}) + TX, *Aphidoletes aphidimyza* (Aphidoline^{®}) + TX, *Aphytis lingnanensis* + TX, *Aphytis melinus* + TX, *Aprostocetus hagenowii +* TX, *Atheta coriaria* (Staphyline^{®}) + TX, *Bombus* spp. + TX, *Bombus terrestris* (Natupol Beehive^{®}) + TX, *Bombus terrestris* (Beeline^{®} + TX, Tripol^{®}) + TX, *Cephalonomia stephanoderis +* TX, *Chilocorus nigritus* + TX, *Chrysoperla carnea* (Chrysoline^{®}) + TX, *Chrysoperla carnea* (Chrysopa^{®}) + TX, *Chrysoperla rufilabris +* TX, *Cirrospilus ingenuus +* TX, *Cirrospilus quadristriatus* + TX, *Citrostichus phyllocnistoides* + TX, *Closterocerus chamaeleon* + TX, *Closterocerus* spp. + TX, *Coccidoxenoides perminutus* (Planopar^{®}) + TX, *Coccophagus cowperi +* TX, *Coccophagus lycimnia +* TX, *Cotesia flavipes +* TX, *Cotesia plutellae +* TX, *Cryptolaemus montrouzieri* (Cryptobug^{®} + TX, Cryptoline^{®}) + TX, *Cybocephalus nipponicus +* TX, *Dacnusa sibirica +* TX, *Dacnusa sibirica* (Minusa^{®}) + TX, *Diglyphus isaea* (Diminex^{®}) + TX, *Delphastus catalinae* (Delphastus^{®}) + TX, *Delphastus pusillus +* TX, *Diachasmimorpha krausii +* TX, *Diachasmimorpha longicaudata +* TX, *Diaparsis jucunda* + TX, *Diaphorencyrtus aligarhensis +* TX, *Diglyphus isaea +* TX, *Diglyphus isaea* (Miglyphus^{®} + TX, Digline^{®}) + TX, *Dacnusa sibirica* (DacDigline^{®} + TX, Minex^{®}) + TX, *Diversinervus* spp. + TX, *Encarsia citrina* + TX, *Encarsia formosa* (Encarsia max^{®} + TX, Encarline^{®} + TX, En-Strip^{®}) + TX, *Eretmocerus eremicus* (Enermix^{®}) + TX, *Encarsia guadeloupae +* TX, *Encarsia haitiensis +* TX, *Episyrphus balteatus* (Syrphidend^{®}) + TX, *Eretmoceris siphonini +* TX, *Eretmocerus californicus +* TX, *Eretmocerus eremicus* (Ercal^{®} + TX, Eretline e^{®}) + TX, *Eretmocerus eremicus* (Bemimix^{®}) + TX, *Eretmocerus hayati +* TX, *Eretmocerus mundus* (Bemipar^{®} + TX, Eretline m^{®}) + TX, *Eretmocerus siphonini +* TX, *Exochomus quadripustulatus* + TX, *Feltiella acarisuga* (Spidend^{®}) + TX, *Feltiella acarisuga* (Feltiline^{®}) + TX, *Fopius arisanus +* TX, *Fopius ceratitivorus +* TX, Formononetin (Wirless Beehome^{®}) + TX, *Franklinothrips vespiformis* (Vespop^{®}) + TX, *Galendromus occidentalis +* TX, *Goniozus legneri* + TX, Habrobracon *hebetor +* TX, *Harmonia axyridis* (HarmoBeetle^{®}) + TX, *Heterorhabditis* spp. (Lawn Patrol^{®}) + TX, *Heterorhabditis bacteriophora* (NemaShield HB^{®} + TX, Nemaseek^{®} + TX, Terranem-Nam^{®} + TX, Terranem^{®} + TX, Larvanem^{®} + TX, B-Green^{®} + TX, NemAttack ^{®} + TX, Nematop^{®}) + TX, *Heterorhabditis megidis* (Nemasys H^{®} + TX, BioNem H^{®} + TX, Exhibitline hm^{®} + TX, Larvanem-M^{®}) + TX, *Hippodamia convergens +* TX, *Hypoaspis aculeifer* (Aculeifer-System^{®} + TX, Entomite-A^{®}) + TX, *Hypoaspis miles* (Hypoline m^{®} + TX, Entomite-M^{®}) + TX, *Lbalia leucospoides +* TX, *Lecanoideus floccissimus +* TX, *Lemophagus errabundus +* TX, *Leptomastidea abnormis* + TX, *Leptomastix dactylopii* (Leptopar^{®}) + TX, *Leptomastix epona* + TX, *Lindorus lophanthae +* TX, *Lipolexis oregmae +* TX, *Lucilia caesar* (Natufly^{®}) + TX, *Lysiphlebus testaceipes +* TX, *Macrolophus caliginosus* (Mirical-N^{®} + TX, Macroline c^{®} + TX, Mirical^{®}) + TX, *Mesoseiulus longipes* + TX, *Metaphycus flavus +* TX, *Metaphycus lounsburyi* + TX, *Micromus angulatus* (Milacewing^{®}) + TX, *Microterys flavus +* TX, *Muscidifurax raptorellus* and *Spalangia cameroni* (Biopar^{®}) + TX, *Neodryinus typhlocybae +* TX, *Neoseiulus californicus +* TX, *Neoseiulus cucumeris* (THRYPEX^{®}) + TX, *Neoseiulus fallacis +* TX, *Nesideocoris tenuis* (NesidioBug^{®} + TX, Nesibug^{®}) + TX, *Ophyra aenescens* (Biofly^{®}) + TX, *Orius insidiosus* (Thripor-I^{®} + TX, Oriline i^{®}) + TX, *Orius laevigatus* (Thripor-L^{®} + TX, Oriline I^{®}) + TX, *Orius majusculus* (Oriline m^{®}) + TX, *Orius strigicollis* (Thripor-S^{®}) + TX, *Pauesia juniperorum +* TX, *Pediobius foveolatus +* TX, *Phasmarhabditis hermaphrodita* (Nemaslug^{®}) + TX, *Phymastichus coffea* + TX, *Phytoseiulus macropilus +* TX, *Phytoseiulus persimilis* (Spidex^{®} + TX, Phytoline p^{®}) + TX, *Podisus maculiventris* (Podisus^{®}) + TX, *Pseudacteon curvatus +* TX, *Pseudacteon obtusus* + TX, *Pseudacteon tricuspis +* TX, *Pseudaphycus maculipennis +* TX, *Pseudleptomastix mexicana* + TX, *Psyllaephagus pilosus +* TX, *Psyttalia concolor* (complex) + TX, *Quadrastichus* spp. + TX, *Rhyzobius lophanthae* + TX, *Rodolia cardinalis +* TX, *Rumina decollate* + TX, *Semielacher petiolatus +* TX, *Sitobion avenae* (Ervibank^{®}) + TX, *Steinernema carpocapsae* (Nematac C^{®} + TX, Millenium^{®} + TX, BioNem C^{®} + TX, NemAttack^{®} + TX, Nemastar^{®} + TX, Capsanem^{®}) + TX, *Steinernema feltiae* (NemaShield^{®} + TX, Nemasys F^{®} + TX, BioNem F^{®} + TX, Steinernema-System^{®} + TX, NemAttack^{®} + TX, Nemaplus^{®} + TX, Exhibitline sf^{®} + TX, Scia-rid^{®} + TX, Entonem^{®}) + TX, *Steinernema kraussei* (Nemasys L^{®} + TX, BioNem L^{®} + TX, Exhibitline srb^{®}) + TX, *Steinernema riobrave* (BioVector^{®} + TX, BioVektor^{®}) + TX, *Steinernema scapterisci* (Nematac S^{®}) + TX, *Steinernema* spp. + TX, *Steinernematid* spp. (Guardian Nematodes^{®}) + TX, *Stethorus punctillum* (Stethorus^{®}) + TX, *Tamarixia radiate* + TX, *Tetrastichus setifer +* TX, *Thripobius semiluteus* + TX, *Torymus sinensis +* TX, *Trichogramma brassicae* (Tricholine b^{®}) + TX, *Trichogramma brassicae* (Tricho-Strip^{®}) + TX, *Trichogramma evanescens +* TX, *Trichogramma minutum +* TX, *Trichogramma ostriniae* + TX, *Trichogramma platneri +* TX, *Trichogramma pretiosum +* TX, *Xanthopimpla stemmator + TX;*
other biologicals including: abscisic acid + TX, bioSea^{®} + TX, *Chondrostereum purpureum* (Chontrol Paste^{®}) + TX, *Colletotrichum gloeosporioides* (Collego^{®}) + TX, Copper Octanoate (Cueva^{®}) + TX, Delta traps (Trapline d^{®}) + TX, Erwinia amylovora (Harpin) (ProAct^{®} + TX, Ni-HIBIT Gold CST^{®}) + TX, fatty acids derived from a natural by-product of extra virgin olive oil (FLIPPER^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, *Microctonus hyperodae* + TX, *Mycoleptodiscus terrestris* (Des-X^{®}) + TX, BioGain^{®} + TX, Aminomite^{®} + TX, Zenox^{®} + TX, Pheromone trap (Thripline ams^{®}) + TX, potassium bicarbonate (MilStop^{®}) + TX, potassium salts of fatty acids (Sanova^{®}) + TX, potassium silicate solution (Sil-Matrix^{®}) + TX, potassium iodide + potassiumthiocyanate (Enzicur^{®}) + TX, SuffOil-X^{®} + TX, Spider venom + TX, *Nosema locustae* (Semaspore Organic Grasshopper Control^{®}) + TX, Sticky traps (Trapline YF^{®} + TX, Rebell Amarillo^{®}) + TX and Traps (Takitrapline y + b^{®}) + TX;
   (1) **antibacterial agents** selected from the group of:
      (1.1) bacteria, examples of which are *Bacillus mojavensis* strain R3B (Accession No. NCAIM (P) B001389) (WO 2013/034938) from Certis USA LLC, a subsidiary of Mitsui & Co. + TX; *Bacillus pumilus,* in particular strain BU F-33, having NRRL Accession No. 50185 (available as part of the CARTISSA^{®} product from BASF, EPA Reg. No. 71840-19) + TX; *Bacillus subtilis,* in particular strain QST713/AQ713 (available as SERENADE OPTI or SERENADE ASO from Bayer CropScience LP, US, having NRRL Accession No. B21661, U.S. Patent No. 6,060,051) + TX; *Bacillus subtilis* strain BU1814, (available as VELONDIS^{®} PLUS, VELONDIS^{®} FLEX and VELONDIS^{®} EXTRA from BASF SE) + TX; *Bacillus subtilis* var. *amyloliquefaciens* strain FZB24 having Accession No. DSM 10271 (available from Novozymes as TAEGRO^{®} or TAEGRO^{®} ECO (EPA Registration No. 70127-5)) + TX; *Bacillus subtilis* CX-9060 from Certis USA LLC, a subsidiary of Mitsui & Co. + TX; *Bacillus* sp., in particular strain D747 (available as DOUBLE NICKEL^{®} from Kumiai Chemical Industry Co., Ltd.), having Accession No. FERM BP-8234, U.S. Patent No. 7,094,592 + TX; *Paenibacillus* sp. strain having Accession No. NRRL B-50972 or Accession No. NRRL B-67129, WO 2016/154297 + TX; *Paenibacillus polymyxa,* in particular strain AC-1 (e.g. TOPSEED^{®} from Green Biotech Company Ltd.) + TX; *Pantoea agglomerans,* in particular strain E325 (Accession No. NRRL B-21856) (available as BLOOMTIME BIOLOGICAL^{™} FD BIOPESTICIDE from Northwest Agri Products) + TX; *Pseudomonas proradix* (e.g. PRORADIX^{®} from Sourcon Padena) + TX; and
      (1.2) fungi, examples of which are *Aureobasidium pullulans,* in particular blastospores of strain DSM14940, blastospores of strain DSM 14941 or mixtures of blastospores of strains DSM14940 and DSM14941 (e.g., BOTECTOR^{®} and BLOSSOM PROTECT^{®} from bio-ferm, CH) + TX; *Pseudozyma aphidis* (as disclosed in WO2011/151819 by Yissum Research Development Company of the Hebrew University of Jerusalem) + TX; Saccharomyces cerevisiae, in particular strains CNCM No. 1-3936, CNCM No. 1-3937, CNCM No. 1-3938 or CNCM No. 1-3939 (as disclosed in WO 2010/086790 from Lesaffre et Compagnie, FR) + TX;
   (2) **biological fungicides** selected from the group of:
      (2.1) bacteria, examples of which are *Agrobacterium radiobacter* strain K84 (e.g. GALLTROL-A^{®} from AgBioChem, CA) + TX; *Agrobacterium radiobacter* strain K1026 (e.g. NOGALL^{™} from BASF SE) + TX; *Bacillus subtilis* var. *amyloliquefaciens* strain FZB24 having Accession No. DSM 10271 (available from Novozymes as TAEGRO^{®} or TAEGRO^{®} ECO (EPA Registration No. 70127-5)) + TX; *Bacillus amyloliquefaciens,* in particular strain D747 (available as Double Nickel^{™} from Kumiai Chemical Industry Co., Ltd., having accession number FERM BP-8234, US Patent No. 7,094,592) + TX; *Bacillus amyloliquefaciens* strain F727 (also known as strain MBI110) (NRRL Accession No. B-50768, WO 2014/028521) (STARGUS^{®} from Marrone Bio Innovations) + TX; *Bacillus amyloliquefaciens* strain FZB42, Accession No. DSM 23117 (available as RHIZOVITAL^{®} from ABiTEP, DE) + TX; *Bacillus amyloliquefaciens* isolate B246 (e.g. AVOGREEN^{™} from University of Pretoria) + TX; *Bacillus licheniformis,* in particular strain SB3086, having Accession No. ATCC 55406, WO 2003/000051 (available as ECOGUARD^{®} Biofungicide and GREEN RELEAF^{™} from Novozymes) + TX + TX; *Bacillus licheniformis* FMCH001 and *Bacillus subtilis* FMCH002 (QUARTZO^{®} (WG) and PRESENCE^{®} (WP) from FMC Corporation) + TX; *Bacillus methylotrophicus* strain BAC-9912 (from Chinese Academy of Sciences' Institute of Applied Ecology) + TX; *Bacillus mojavensis* strain R3B (Accession No. NCAIM (P) B001389) (WO 2013/034938) from Certis USA LLC, a subsidiary of Mitsui & Co. + TX; *Bacillus mycoides,* isolate, having Accession No. B-30890 (available as BMJ TGAI^{®} or WG and LifeGard^{™} from Certis USA LLC, a subsidiary of Mitsui & Co.) + TX; *Bacillus pumilus,* in particular strain QST2808 (available as SONATA^{®} from Bayer CropScience LP, US, having Accession No. NRRL B-30087 and described in U.S. Patent No. 6,245,551) + TX; *Bacillus pumilus,* in particular strain GB34 (available as Yield Shield^{®} from Bayer AG, DE) + TX; *Bacillus pumilus,* in particular strain BU F-33, having NRRL Accession No. 50185 (available as part of the CARTISSA product from BASF, EPA Reg. No. 71840-19) + TX; *Bacillus subtilis,* in particular strain QST713/AQ713 (available as SERENADE OPTI or SERENADE ASO from Bayer CropScience LP, US, having NRRL Accession No. B21661 and described in U.S. Patent No. 6,060,051) + TX; *Bacillus subtilis* Y1336 (available as BIOBAC^{®} WP from Bion-Tech, Taiwan, registered as a biological fungicide in Taiwan under Registration Nos. 4764, 5454, 5096 and 5277) + TX; *Bacillus subtilis* strain MBI 600 (available as SUBTILEX from BASF SE), having Accession Number NRRL B-50595, U.S. Patent No. 5,061,495 + TX; *Bacillus subtilis* strain GB03 (available as Kodiak^{®} from Bayer AG, DE) + TX; *Bacillus subtilis* strain BU1814, (available as VELONDIS^{®} PLUS, VELONDIS^{®} FLEX and VELONDIS^{®} EXTRA from BASF SE) + TX; *Bacillus subtilis* CX-9060 from Certis USA LLC, a subsidiary of Mitsui & Co. + TX; *Bacillus subtilis* KTSB strain (FOLIACTIVE^{®} from Donaghys) + TX; *Bacillus subtilis* IAB/BS03 (AVIV^{™} from STK Bio-Ag Technologies, PORTENTO^{®} from Idai Nature) + TX; *Bacillus subtilis* strain Y1336 (available as BIOBAC^{®} WP from Bion-Tech, Taiwan, registered as a biological fungicide in Taiwan under Registration Nos. 4764, 5454, 5096 and 5277) + TX; *Paenibacillus epiphyticus* (WO 2016/020371) from BASF SE + TX; *Paenibacillus polymyxa* ssp. plantarum (WO 2016/020371) from BASF SE + TX; *Paenibacillus* sp. strain having Accession No. NRRL B-50972 or Accession No. NRRL B-67129, WO 2016/154297 + TX; *Pseudomonas chlororaphis* strain AFS009, having Accession No. NRRL B-50897, WO 2017/019448 (e.g., HOWLER^{™} and ZIO^{®} from AgBiome Innovations, US) + TX; *Pseudomonas chlororaphis,* in particular strain MA342 (e.g. CEDOMON^{®}, CERALL^{®}, and CEDRESS^{®} by Bioagri and Koppert) + TX; *Pseudomonas fluorescens* strain A506 (e.g. BLIGHTBAN^{®} A506 by NuFarm) + TX; *Pseudomonas proradix* (e.g. PRORADIX^{®} from Sourcon Padena) + TX; *Streptomyces griseoviridis* strain K61 (also known as *Streptomyces galbus* strain K61) (Accession No. DSM 7206) (MYCOSTOP^{®} from Verdera, PREFENCE^{®} from BioWorks, cf. Crop Protection 2006, 25, 468-475) + TX; *Streptomyces lydicus* strain WYEC108 (also known as *Streptomyces lydicus* strain WYCD108US) (ACTINO-IRON^{®} and ACTINOVATE^{®} from Novozymes) + TX; and
      (2.2) fungi, examples of which are *Ampelomyces quisqualis,* in particular strain AQ 10 (e.g. AQ 10^{®} by IntrachemBio Italia) + TX; *Ampelomyces quisqualis* strain AQ10, having Accession No. CNCM 1-807 (e.g., AQ 10^{®} by IntrachemBio Italia) + TX; *Aspergillus flavus* strain NRRL 21882 (products known as AFLA-GUARD^{®} from Syngenta/ChemChina) + TX; *Aureobasidium pullulans,* in particular blastospores of strain DSM14940 + TX; *Aureobasidium pullulans,* in particular blastospores of strain DSM 14941 + TX; *Aureobasidium pullulans,* in particular mixtures of blastospores of strains DSM14940 and DSM 14941 (e.g. Botector^{®} by bio-ferm, CH) + TX; *Chaetomium cupreum* (Accession No. CABI 353812) (e.g. BIOKUPRUM^{™} by AgriLife) + TX; *Chaetomium globosum* (available as RIVADIOM^{®} by Rivale) + TX; *Cladosporium cladosporioides,* strain H39, having Accession No. CBS122244, US 2010/0291039 (by Stichting Dienst Landbouwkundig Onderzoek) + TX; *Coniothyrium minitans,* in particular strain CON/M/91-8 (Accession No. DSM9660, e.g. Contans ^{®} from Bayer CropScience Biologics GmbH) + TX; *Cryptococcus flavescens,* strain 3C (NRRL Y-50378), (B2.2.99) + TX; *Dactylaria candida +* TX; Dilophosphora alopecuri (available as TWIST FUNGUS^{®}) + TX; Fusarium oxysporum, strain Fo47 (available as FUSACLEAN^{®} by Natural Plant Protection) + TX; Gliocladium catenulatum (Synonym: Clonostachys rosea f. catenulate) strain J1446 (e.g. Prestop ^{®} by Lallemand) + TX; Gliocladium roseum (also known as Clonostachys rosea f rosea), in particular strain 321U from Adjuvants Plus, strain ACM941 as disclosed in Xue (Efficacy of Clonostachys rosea strain ACM941 and fungicide seed treatments for controlling the root tot complex of field pea, Can Jour Plant Sci 83(3): 519-524), or strain IK726 (Jensen DF, et al. Development of a biocontrol agent for plant disease control with special emphasis on the near commercial fungal antagonist Clonostachys rosea strain 'IK726', Australas Plant Pathol. 2007,36:95-101) + TX; Lecanicillium lecanii (formerly known as Verticillium lecanii) conidia of strain KV01 (e.g. Vertalec^{®} by Koppert/Arysta) + TX; Metschnikowia fructicola, in particular strain NRRL Y-30752, (B2.2.3) + TX; Microsphaeropsis ochracea + TX; Muscodor roseus, in particular strain A3-5 (Accession No. NRRL 30548) + TX; Penicillium steckii (DSM 27859, WO 2015/067800) from BASF SE + TX; Penicillium vermiculatum + TX; Phlebiopsis gigantea strain VRA 1992 (ROTSTOP^{®} C from Danstar Ferment) + TX; Pichia anomala, strain WRL-076 (NRRL Y-30842), U.S. Patent No. 7,579,183 + TX; Pseudozyma flocculosa, strain PF-A22 UL (available as SPORODEX^{®} L by Plant Products Co., CA) + TX; Saccharomyces cerevisiae, in particular strain LASO2 (from Agro-Levures et Dérivés), strain LAS117 cell walls (CEREVISANE^{®} from Lesaffre, ROMEO^{®} from BASF SE), strains CNCM No. 1-3936, CNCM No. 1-3937, CNCM No. 1-3938, CNCM No. 1-3939 (WO 2010/086790) from Lesaffre et Compagnie, FR + TX; Simplicillium lanosoniveum + TX; Talaromyces flavus, strain V117b + TX; Trichoderma asperelloides JM41R (Accession No. NRRL B-50759) (TRICHO PLUS^{®} from BASF SE) + TX; Trichoderma asperellum, in particular, strain kd (e.g. T-Gro from Andermatt Biocontrol) + TX; Trichoderma asperellum, in particular strain SKT-1, having Accession No. FERM P-16510 (e.g. ECO-HOPE^{®} from Kumiai Chemical Industry), strain T34 (e.g. T34 Biocontrol by Biocontrol Technologies S.L., ES) or strain ICC 012 from Isagro + TX; Trichoderma atroviride, in particular strain SC1 (having Accession No. CBS 122089, WO 2009/116106 and U.S. Patent No. 8,431,120 (from Bi-PA)), strain 77B (T77 from Andermatt Biocontrol) or strain LU132 (e.g. Sentinel from Agrimm Technologies Limited) + TX; Trichoderma atroviride, strain CNCM 1-1237 (e.g. Esquive^{®} WP from Agrauxine, FR) + TX; Trichoderma atroviride, strain no. V08/002387 + TX; Trichoderma atroviride, strain NMI no. V08/002388 + TX; Trichoderma atroviride, strain NMI no. V08/002389 + TX; Trichoderma atroviride, strain NMI no. V08/002390 + TX; Trichoderma atroviride, strain LC52 (e.g. Tenet by Agrimm Technologies Limited) + TX; Trichoderma atroviride, strain ATCC 20476 (IMI 206040) + TX; Trichoderma atroviride, strain T11 (IMI352941/ CECT20498) + TX; Trichoderma atroviride, strain SKT-1 (FERM P-16510), JP Patent Publication (Kokai) 11-253151 A + TX; Trichoderma atroviride, strain SKT-2 (FERM P-16511), JP Patent Publication (Kokai) 11-253151 A + TX; Trichoderma atroviride, strain SKT-3 (FERM P-17021), JP Patent Publication (Kokai) 11-253151 A + TX; Trichoderma fertile (e.g. product TrichoPlus from BASF) + TX; Trichoderma gamsii (formerly T. viride), strain ICC080 (IMI CC 392151 CABI, e.g. BioDerma by AGROBIOSOL DE MEXICO, S.A. DE C.V.) + TX; Trichoderma gamsii (formerly T. viride), strain ICC 080 (IMI CC 392151 CABI) (available as BIODERMA^{®} by AGROBIOSOL DE MEXICO, S.A. DE C.V.) + TX; Trichoderma harmatum + TX; Trichoderma harmatum, having Accession No. ATCC 28012 + TX; Trichoderma harzianum strain T-22 (e.g. Trianum-P from Andermatt Biocontrol or Koppert) or strain Cepa SimbT5 (from Simbiose Agro) + TX; Trichoderma harzianum + TX; Trichoderma harzianum rifai T39 (e.g. Trichodex^{®} from Makhteshim, US) + TX; Trichoderma harzianum, strain ITEM 908 (e.g. Trianum-P from Koppert) + TX; Trichoderma harzianum, strain TH35 (e.g. Root-Pro by Mycontrol) + TX; Trichoderma harzianum, strain DB 103 (available as T-GRO^{®} 7456 by Dagutat Biolab) + TX; Trichoderma polysporum, strain IMI 206039 (e.g. Binab TF WP by BINAB Bio-Innovation AB, Sweden) + TX; Trichoderma stromaticum, having Accession No. Ts3550 (e.g. Tricovab by CEPLAC, Brazil) + TX; Trichoderma virens (also known as Gliocladium virens), in particular strain GL-21 (e.g. SoilGard by Certis, US) + TX; Trichoderma virens strain G-41, formerly known as Gliocladium virens (Accession No. ATCC 20906) (e.g., ROOTSHIELD^{®} PLUS WP and TURFSHIELD^{®} PLUS WP from BioWorks, US) + TX; Trichoderma viride, strain TV1(e.g. Trianum-P by Koppert) + TX; Trichoderma viride, in particular strain B35 (Pietr et al., 1993, Zesz. Nauk. A R w Szczecinie 161: 125-137) + TX; mixtures of Trichoderma asperellum strain ICC 012 (also known as Trichoderma harzianum ICC012), having Accession No. CABI CC IMI 392716 and Trichoderma gamsii (formerly T. viride) strain ICC 080, having Accession No. IMI 392151 (e.g., BIO-TAM^{™} from Isagro USA, Inc. and BIODERMA^{®} by Agrobiosol de Mexico, S.A. de C.V.) + TX; Ulocladium oudemansii strain U3, having Accession No. NM 99/06216 (e.g., BOTRY-ZEN^{®} by Botry-Zen Ltd, New Zealand and BOTRYSTOP^{®} from BioWorks, Inc.) + TX; Verticillium albo-atrum (formerly V. dahliae), strain WCS850 having Accession No. WCS850, deposited at the Central Bureau for Fungi Cultures (e.g., DUTCH TRIG^{®} by Tree Care Innovations) + TX; Verticillium chlamydosporium + TX;
   (3) **biological control agents** having **an effect for improving plant growth and/or plant health** selected from the group of:
      (3.1) bacteria, examples of which are *Azospirillum brasilense* (e.g., VIGOR^{®} from KALO, Inc.) + TX; *Azospirillum lipoferum* (e.g., VERTEX-IF^{™} from TerraMax, Inc.) + TX; *Azorhizobium caulinodans,* in particular strain ZB-SK-5 + TX; *Azotobacter chroococcum,* in particular strain H23 + TX; *Azotobacter vinelandii,* in particular strain ATCC 12837 + TX; a mixture of *Azotobacter vinelandii* and *Clostridium pasteurianum* (available as INVIGORATE^{®} from Agrinos) + TX; *Bacillus amyloliquefaciens* pm414 (LOLI-PEPTA^{®} from Biofilm Crop Protection) + TX; *Bacillus amyloliquefaciens* SB3281 (ATCC # PTA-7542, WO 2017/205258) + TX; *Bacillus amyloliquefaciens* TJ1000 (available as QUIKROOTS^{®} from Novozymes) + TX; *Bacillus amyloliquefaciens,* in particular strain IN937a + TX; *Bacillus amyloliquefaciens,* in particular strain FZB42 (e.g. RHIZOVITAL^{®} from ABiTEP, DE) + TX; *Bacillus amyloliquefaciens* BS27 (Accession No. NRRL B-5015) + TX; *Bacillus cereus* family member EE128 (NRRL No. B-50917) + TX; *Bacillus cereus* family member EE349 (NRRL No. B-50928) + TX; *Bacillus cereus,* in particular strain BP01 (ATCC 55675, e.g. MEPICHLOR^{®} from Arysta Lifescience, US) + TX; *Bacillus firmus,* in particular strain CNMC 1-1582 (e.g. VOTIVO^{®} from BASF SE) + TX; *Bacillus mycoides* BT155 (NRRL No. B-50921) + TX; *Bacillus mycoides* EE118 (NRRL No. B-50918) + TX; *Bacillus mycoides* EE141 (NRRL No. B-50916) + TX; *Bacillus mycoides* BT46-3 (NRRL No. B-50922) + TX; *Bacillus pumilus,* in particular strain QST2808 (having Accession No. NRRL No. B-30087) + TX; *Bacillus pumilus,* in particular strain GB34 (e.g. YIELD SHIELD^{®} from Bayer Crop Science, DE) + TX; *Bacillus siamensis,* in particular strain KCTC 13613T + TX; *Bacillus subtilis,* in particular strain QST713/AQ713 (having NRRL Accession No. B-21661 and described in U.S. Patent No. 6,060,051, available as SERENADE^{®} OPTI or SERENADE^{®} ASO from Bayer CropScience LP, US) + TX; *Bacillus subtilis,* in particular strain AQ30002 (having Accession Nos. NRRL B-50421 and described in U.S. Patent Application No. 13/330,576) + TX; *Bacillus subtilis,* in particular strain AQ30004 (and NRRL B-50455 and described in U.S. Patent Application No. 13/330,576) + TX; *Bacillus subtilis* strain BU1814, (available as TEQUALIS^{®} from BASF SE), *Bacillus* subtilis rm303 (RHIZOMAX^{®} from Biofilm Crop Protection) + TX; *Bacillus thuringiensis* BT013A (NRRL No. B-50924) also known as *Bacillus thuringiensis 4Q7* + TX; a mixture of *Bacillus licheniformis* FMCH001 and *Bacillus subtilis* FMCH002 (available as QUARTZO^{®} (WG), PRESENCE^{®} (WP) from FMC Corporation) + TX; *Bacillus subtilis,* in particular strain MBI 600 (e.g. SUBTILEX^{®} from BASF SE) + TX; *Bacillus tequilensis,* in particular strain NII-0943 + TX; *Bradyrhizobium japonicum* (e.g. OPTIMIZE^{®} from Novozymes) + TX; *Delftia acidovorans,* in particular strain RAY209 (e.g. BIOBOOST^{®} from Brett Young Seeds) + TX; *Mesorhizobium cicer* (e.g., NODULATOR from BASF SE) + TX; *Lactobacillus* sp. (e.g. LACTOPLANT^{®} from LactoPAFI) + TX; *Rhizobium leguminosarium biovar viciae* (e.g., NODULATOR from BASF SE) + TX; Pseudomonas proradix (e.g. PRORADIX^{®} from Sourcon Padena) + TX; Pseudomonas aeruginosa, in particular strain PN1 + TX; Rhizobium leguminosarum, in particular bv. viceae strain Z25 (Accession No. CECT 4585) + TX; Paenibacillus polymyxa, in particular strain AC-1 (e.g. TOPSEED^{®} from Green Biotech Company Ltd.) + TX; Serratia marcescens, in particular strain SRM (Accession No. MTCC 8708) + TX; Sinorhizobium meliloti strain NRG-185-1 (NITRAGIN^{®} GOLD from Bayer CropScience) + TX; Thiobacillus sp. (e.g. CROPAID^{®} from Cropaid Ltd UK) + TX; and
      (3.2) fungi, examples of which are *Purpureocillium lilacinum* (previously known as *Paecilomyces lilacinus)* strain 251 (AGAL 89/030550, e.g. BioAct from Bayer CropScience Biologics GmbH) + TX; *Penicillium bilaii,* strain ATCC 22348 (e.g. JumpStart^{®} from Acceleron BioAg), *Talaromyces flavus,* strain V117b + TX; Trichoderma atroviride strain CNCM 1-1237 (e.g. Esquive^{®} WP from Agrauxine, FR), Trichoderma viride, e.g. strain B35 (Pietr et al., 1993, Zesz. Nauk. A R w Szczecinie 161: 125-137) + TX; Trichoderma atroviride strain LC52 (also known as Trichoderma atroviride strain LU132, e.g. Sentinel from Agrimm Technologies Limited) + TX; Trichoderma atroviride strain SC1 described in International Application No. PCT/IT2008/000196) + TX;Trichoderma asperellum strain kd (e.g. T-Gro from Andermatt Biocontrol) + TX; Trichoderma asperellum strain Eco-T (Plant Health Products, ZA), Trichoderma harzianum strain T-22 (e.g. Trianum-P from Andermatt Biocontrol or Koppert) + TX; Myrothecium verrucaria strain AARC-0255 (e.g. DiTera^{™} from Valent Biosciences) + TX; Penicillium bilaii strain ATCC ATCC20851 + TX; Pythium oligandrum strain M1 (ATCC 38472, e.g. Polyversum from Bioprepraty, CZ) + TX; Trichoderma virens strain GL-21 (e.g. SoilGard^{®} from Certis, USA) + TX; Verticillium albo-atrum (formerly V. dahliae) strain WCS850 (CBS 276.92, e.g. Dutch Trig from Tree Care Innovations) + TX; Trichoderma atroviride, in particular strain no. V08/002387, strain no. NMI No. V08/002388, strain no. NMI No. V08/002389, strain no. NMI No. V08/002390 + TX; Trichoderma harzianum strain ITEM 908, Trichoderma harzianum, strain TSTh20 + TX; Trichoderma harzianum strain 1295-22 + TX; Pythium oligandrum strain DV74 + TX; Rhizopogon amylopogon (e.g. comprised in Myco-Sol from Helena Chemical Company) + TX; Rhizopogon fulvigleba (e.g. comprised in Myco-Sol from Helena Chemical Company) + TX;Trichoderma virens strain GI-3 + TX;
   (4) **insecticidally active biological control agents** selected from
      (4.1) bacteria, examples of which are *Agrobacterium radiobacter* strain K84 (Galltrol from AgBiochem Inc.) + TX; *Bacillus amyloliquefaciens,* in particular strain PTS-4838 (e.g. AVEO from Valent Biosciences, US) + TX; *Bacillus firmus,* in particular strain CNMC 1-1582 (e.g. VOTIVO^{®} from BASF SE) + TX; *Bacillus mycoides,* isolate J. (e.g. BmJ from Certis USA LLC, a subsidiary of Mitsui & Co.) + TX; *Bacillus sphaericus,* in particular Serotype H5a5b strain 2362 (strain ABTS-1743) (e.g. VECTOLEX^{®} from Valent BioSciences, US) + TX; *Bacillus thuringiensis* subsp. aizawai, in particular strain ABTS-1857 (SD-1372, e.g. XENTARI^{®} from Valent BioSciences) + TX; *Bacillus thuringiensis* subsp. aizawai, in particular serotype H-7 (e.g. FLORBAC^{®} WG from Valent BioSciences, US) + TX; *Bacillus thuringiensis israelensis* strain BMP 144 (e.g. AQUABAC^{®} by Becker Microbial Products IL) + TX; *Bacillus thuringiensis* subsp. *israelensis* (serotype H-14) strain AM65-52 (Accession No. ATCC 1276) (e.g. VECTOBAC^{®} by Valent BioSciences, US) + TX; *Bacillus thuringiensis* subsp. *aizawai* strain GC-91 + TX; Bacillus thuringiensis var. Colmeri (e.g. TIANBAOBTC by Changzhou Jianghai Chemical Factory) + TX; Bacillus thuringiensis var. japonensis strain Buibui + TX; Bacillus thuringiensis subsp. kurstaki strain BMP 123 from Becker Microbial Products, IL + TX; Bacillus thuringiensis subsp. kurstaki strain BMP 123 by Becker Microbial Products, IL, e.g. BARITONE from Bayer CropScience + TX; Bacillus thuringiensis subsp. kurstaki strain HD-1 (e.g. DIPEL^{®} ES from Valent BioSciences, US) + TX; Bacillus thuringiensis var. kurstaki strain EVB-113-19 (e.g., BIOPROTEC^{®} from AEF Global) + TX; Bacillus thuringiensis subsp. kurstaki strain ABTS 351 + TX; Bacillus thuringiensis subsp. kurstaki strain PB 54 + TX; Bacillus thuringiensis subsp. kurstaki strain SA 11, (JAVELIN from Certis, US) + TX; Bacillus thuringiensis subsp. kurstaki strain SA 12 (THURICIDE from Certis, US) + TX; Bacillus thuringiensis subsp. kurstaki strain EG 2348 (LEPINOX from Certis, US) + TX; Bacillus thuringiensis subsp. kurstaki strain EG 7841 (CRYMAX from Certis, US) + TX; Bacillus thuringiensis subsp. tenebrionis strain NB 176 (SD-5428, e.g. NOVODOR^{®} FC from BioFa DE) + TX; Brevibacillus laterosporus (LATERAL from Ecolibrium Biologicals) + TX; Burkholderia spp., in particular Burkholderia rinojensis strain A396 (also known as Burkholderia rinojensis strain MBI 305) (Accession No. NRRL B-50319 + TX; WO 2011/106491 and WO 2013/032693 + TX; e.g. MBI206 TGAI and ZELTO^{®} from Marrone Bio Innovations) + TX; Chromobacterium subtsugae, in particular strain PRAA4-1T (MBI-203 + TX; e.g. GRANDEVO^{®} from Marrone Bio Innovations) + TX; Lecanicillium muscarium Ve6 (MYCOTAL from Koppert) + TX; Paenibacillus popilliae (formerly Bacillus popilliae + TX; e.g. MILKY SPORE POWDER^{™} and MILKY SPORE GRANULAR^{™} from St. Gabriel Laboratories) + TX; Pasteuria nishizawae strain Pn1 (CLARIVA from Syngenta/ChemChina) + TX;Serratia entomophila (e.g. INVADE^{®} by Wrightson Seeds) + TX; Serratia marcescens, in particular strain SRM (Accession No. MTCC 8708) + TX;Trichoderma asperellum (TRICHODERMAX from Novozymes) + TX; Wolbachia pipientis ZAP strain (e.g., ZAP MALES^{®} from MosquitoMate) + TX; and
      (4.2) fungi, examples of which are *Beauveria bassiana* strain ATCC 74040 (e.g. NATURALIS^{®} from Intrachem Bio Italia) + TX; *Beauveria bassiana* strain GHA (Accession No. ATCC74250, e.g. BOTANIGUARD^{®} ES and MYCONTROL-O^{®} from Laverlam International Corporation) + TX; *Beauveria bassiana* strain ATP02 (Accession No. DSM 24665) + *TX;lsaria fumosorosea* (previously known as *Paecilomyces fumosoroseus)* strain Apopka 97) PREFERAL from SePRO + TX; *Metarhizium anisopliae* 3213-1 (deposited under NRRL accession number 67074) (WO 2017/066094 + TX; Pioneer Hi-Bred International) + TX; *Metarhizium robertsii* 15013-1 (deposited under NRRL accession number 67073) + TX; *Metarhizium robertsii* 23013-3 (deposited under NRRL accession number 67075) + TX; *Paecilomyces lilacinus* strain 251 (MELOCON from Certis, US) + TX; *Zoophtora radicans +* TX;
   (5) **Viruses** selected from the group consisting of *Adoxophyes orana* (summer fruit tortrix) granulosis virus (GV) + TX; *Cydia pomonella* (codling moth) granulosis virus (GV) + TX; Helicoverpa armigera (cotton bollworm) nuclear polyhedrosis virus (NPV) + TX; *Spodoptera exigua* (beet armyworm) mNPV + TX; *Spodoptera frugiperda* (fall armyworm) mNPV + TX; *Spodoptera littoralis* (African cotton leafworm) NPV + TX;
   (6) **Bacteria and fungi which can be added as 'inoculant' to plants or plant parts or plant organs and which, by virtue of their particular properties, promote plant growth and plant health** selected from *Agrobacterium spp. + TX;* Azorhizobium caulinodans + TX; Azospirillum spp. + TX; Azotobacter spp. + TX; Bradyrhizobium spp. + TX; Burkholderia spp., in particular Burkholderia cepacia (formerly known as Pseudomonas cepacia) + TX; Gigaspora spp., or Gigaspora monosporum + TX; Glomus spp. + TX; Laccaria spp. + TX; LactoBacillus buchneri + TX; Paraglomus spp. + TX; Pisolithus tinctorus + TX; Pseudomonas spp. + TX; Rhizobium spp., in particular Rhizobium trifolii + TX; Rhizopogon spp. + TX; Scleroderma spp. + TX; Suillus spp. + TX; Streptomyces spp. + TX;
   (7) **Plant extracts and products formed by microorganisms including proteins and secondary metabolites which can be used as biological control agents,** selected from *Allium sativum* (NEMGUARD from Eco-Spray + TX; BRALIC from ADAMA) + TX; Armour-Zen + TX; *Artemisia absinthium +* TX; Azadirachtin (e.g. AZATIN XL from Certis, US) + TX; Biokeeper WP + TX; Brassicaceae extract, in particular oilseed rape powder or mustard powder + TX; Cassia nigricans + TX; Celastrus angulatus + TX; Chenopodium anthelminticum + TX; Chitin + TX; Dryopteris filix-mas + TX; Equisetum arvense + TX; Fortune Aza + TX; Fungastop + TX; Heads Up (Chenopodium quinoa saponin extract) + TX; PROBLAD (naturally occurring Blad polypeptide from Lupin seeds), Certis EU + TX; FRACTURE (naturally occurring Blad polypeptide from Lupin seeds), FMC + TX; Pyrethrum/Pyrethrins + TX; Quassia amara + TX; Quercus + TX; Quillaja extract (QL AGRI 35 from BASF) + TX; Reynoutria sachalinensis extract (REGALLIA / REGALIA MAXX from Marrone Bio) + TX; "Requiem ^{™} Insecticide" + TX; Rotenone + TX; ryania/ryanodine + TX; Symphytum officinale + TX; Tanacetum vulgare + TX; Thymol + TX; Thymol mixed with Geraniol (CEDROZ from Eden Research) + TX; Thymol mixed with Geraniol and Eugenol (MEVALONE from Eden Research) + TX; Triact 70 + TX; TriCon + TX; Tropaeulum majus + TX; Melaleuca alternifolia extract (TIMOREX GOLD from STK) + TX; Urtica dioica + TX; Veratrin + TX; and Viscum album + TX; and
      a safener, such as benoxacor + TX, cloquintocet (including cloquintocet-mexyl) + TX, cyprosulfamide + TX, dichlormid + TX, fenchlorazole (including fenchlorazole-ethyl) + TX, fenclorim + TX, fluxofenim + TX, furilazole + TX, isoxadifen (including isoxadifen-ethyl) + TX, mefenpyr (including mefenpyr-diethyl) + TX, metcamifen + TX and oxabetrinil + TX.

The references in brackets behind the active ingredients, e.g. [3878-19-1] refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright ^{©} 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula (I) selected from the compounds defined in the Tables A-1 to A-78, B-1 to B-78, C-1 to C-21 and D-1 to D-21 with active ingredients described above comprises a compound selected from one compound defined in the Tables A-1 to A-78, B-1 to B-78, C-1 to C-21 and D-1 to D-21 and an active ingredient as described above preferably in a mixing ratio of from 100:1 to 1:6000, especially from 50:1 to 1:50, more especially in a ratio of from 20:1 to 1:20, even more especially from 10:1 to 1:10, very especially from 5:1 to 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The compounds and mixtures as described above can be used in a method for controlling pests, which comprises applying a composition comprising a compound or mixture respectively as described above to the pests or their environment, with the exception of a method for treatment of the human or animal body by surgery or therapy and diagnostic methods practised on the human or animal body.

The mixtures comprising a compound of formula (I) selected from the compounds defined in the Tables A-1 to A-78, B-1 to B-78, C-1 to C-21 and D-1 to D-21 and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula (I) and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of formula (I) of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula (I). The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

The compounds of the invention can be distinguished from other similar compounds by virtue of greater efficacy at low application rates and/or different pest control, which can be verified by the person skilled in the art using the experimental procedures, using lower concentrations if necessary, for example 10 ppm, 5 ppm, 2 ppm, 1 ppm or 0.2 ppm; or lower application rates, such as 300, 200 or 100, mg of Al per m². The greater efficacy can be observed by an increased safety profile (against non-target organisms above and below ground (such as fish, birds and bees), improved physico-chemical properties, or increased biodegradability).

In each aspect and embodiment of the invention, "consisting essentially" and inflections thereof are a preferred embodiment of "comprising" and its inflections, and "consisting of" and inflections thereof are a preferred embodiment of "consisting essentially of" and its inflections.

The disclosure in the present application makes available each and every combination of embodiments disclosed herein.

It should be noted that the disclosure herein in respect of a compound of formula (I) applies equally in respect of a compound of each of formulae (I*), (I-A), (I-B), (I-C) and (I-D) and Tables A-1 to A-78, B-1 to B-78, C-1 to C-21 and D-1 to D-21.

### EXAMPLES

### Formulation examples

The following Examples further illustrate, but do not limit, the invention.

| Wettable powders | a) | b) | c) |
|---|---|---|---|
| active ingredients | 25 % | 50 % | 75 % |
| sodium lignosulfonate | 5% | 5 % | - |
| sodium lauryl sulfate | 3% | - | 5 % |
| sodium diisobutylnaphthalenesulfonate | - | 6 % | 10 % |
| phenol polyethylene glycol ether (7-8 mol of ethylene oxide) | - | 2 % | - |
| highly dispersed silicic acid | 5% | 10 % | 10 % |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25 % | 50 % | 75 % |
| light mineral oil | 5 % | 5% | 5 % |
| highly dispersed silicic acid | 5 % | 5% | - |
| Kaolin | 65 % | 40 % | - |
| Talcum | - | | 20 % |

The combination is thoroughly mixed with the adjuvants and the mixture is thoroughly ground in a suitable mill, affording powders that can be used directly for seed treatment.

### Emulsifiable concentrate

| | |
|---|---|
| active ingredients | 10% |
| octylphenol polyethylene glycol ether (4-5 mol of ethylene oxide) | 3% |
| calcium dodecylbenzenesulfonate | 3% |
| castor oil polyglycol ether (35 mol of ethylene oxide) | 4% |
| Cyclohexanone | 30% |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

### Extruder granules

| | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

### Coated granules

| | |
|---|---|
| Active ingredients | 8 % |
| polyethylene glycol (mol. wt. 200) | 3 % |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

### Preparation examples

The following preparation examples further illustrate, but do not limit, the invention. Those skilled in the art will promptly recognize appropriate variations from the procedures both as to reactants and as to reaction conditions and techniques. Throughout this description, temperatures are given in degrees Celsius (°C). "Mp" means melting point in °C. Unless indicated otherwise, ¹H NMR spectra are recorded at 400 MHz and ¹⁹F NMR spectra are recorded at 377 MHz. Chemical shifts are recorded in ppm. The following abbreviations are used: s = singlet; br s = broad singlet; d = doublet; br d = broad doublet; dd = double doublet; dt = double triplet; t = triplet, tt = triple triplet, q = quartet, quin = quintuplet, sept = septet; m = multiplet. Either one of the LCMS methods below was used to characterize the compounds. The characteristic LCMS values obtained for each compound were the retention time ("Rt", recorded in minutes) and the measured molecular ion (M+H)⁺ or (M-H)⁻.

### LCMS Methods:

### Method 1:

Spectra were recorded on a Mass Spectrometer from Agilent Technologies (6410 Triple Quadrupole mass spectrometer) equipped with an equipped with an electrospray source (Polarity: positive or negative ions, MS2 Scan, Capillary: 7.00 kV, Fragmentor: 120 V, Desolvation Temperature: 350°C, Gas Flow: 11 L/min, Nebulizer Gas: 40 psi, Mass range: 110 to 650 Da) and a 1200 Series HPLC from Agilent: quaternary pump, heated column compartment and diode-array detector. Column: KINETEX EVO C18, 2.6 µm, 50 x 4.6 mm, Temp: 40 °C, DAD Wavelength (nm): 254, Solvent Gradient: A = water + 5% Acetonitrile + 0.1 % HCOOH, B= Acetonitrile + 0.1 % HCOOH: gradient: 0 min 10% B, 90%A; 0.9-1.8 min 100% B; 1.8-2.2 min 100-10% B; 2.2-2.5 min 10%B; Flow (mL/min) 1.8.

### Method 2:

Spectra were recorded on a Mass Spectrometer from Waters (SQD, SQDII Single quadrupole mass spectrometer) equipped with an electrospray source (Polarity: positive and negative ions, Capillary: 3.00 kV, Cone range: 41 V, Extractor: 2.00 V, Source Temperature: 150°C, Desolvation Temperature: 5000°C, Cone Gas Flow: 50 I/h, Desolvation Gas Flow: 1000 I/h, Mass range: 110 to 800 Da) and an Acquity UPLC from Waters: Binary pump, heated column compartment, diode-array detector and ELSD detector. Column: Waters UPLC HSS T3 C18, 1.8 µm, 30 x 2.1 mm, Temp: 40 °C, PDA Wavelength range (nm): 200 to 400, Solvent Gradient: A = water + 5% Acetonitrile + 0.1 % HCOOH, B = Acetonitrile + 0.05 % HCOOH, gradient: 10-100% B in 1.3 min; Flow (ml/min) 0.6.

### Method 3:

Spectra were recorded on a Mass Spectrometer from Waters (SQD, SQDII or QDA Single quadrupole mass spectrometer) equipped with an electrospray source (Polarity: positive and negative ions), Capillary: 0.8-3.00 kV, Cone: 5-30 V, Source Temperature: 120-150°C, Desolvation Temperature: 350-600°C, Cone Gas Flow: 50-150 I/h, Desolvation Gas Flow: 650-1000 I/h, Mass range: 50 to 900 Da and an Acquity UPLC from Waters Corporation: Binary pump, heated column compartment , diode-array detector and ELSD. Column: Waters UPLC HSS T3, 1.8 µm, 30 x 2.1 mm, Temp: 60°C, DAD Wavelength range (nm): 210 to 400, Runtime: 1.5 min; Solvents: A = water + 5% MeOH + 0.05 % HCOOH, B= Acetonitrile + 0.05 % HCOOH; Flow (ml/min) 0.85, Gradient: 10% B isocratic for 0.2 min, then 10-100% B in 1.0 min, 100% B isocratic for 0.2min, 100-10% B in 0.05min, 10% B isocratic for 0.05 min.

### Method 4:

Spectra were recorded on a Mass Spectrometer from Agilent Technologies (6410 Triple Quadrupole mass spectrometer) equipped with an electrospray source (Polarity: positive or negative ions, MS2 Scan, Capillary: 4.00 kV, Fragmentor: 100 V, Desolvation Temperature: 350°C, Gas Flow: 11 L/min, Nebulizer Gas: 45 psi, Mass range: 110 to 1000 Da) and a 1200 Series HPLC from Agilent: quaternary pump, heated column compartment and VWD detector. Column: KINETEX EVO C18, 2.6 µm, 50 x 4.6 mm, Temp: 40 °C, Detector VWD Wavelength: 254 nm, Solvent Gradient: A = water + 5% Acetonitrile + 0.1 % HCOOH, B= Acetonitrile + 0.1 % HCOOH: gradient: 0 min 10% B, 90%A; 0.9-1.8 min 100% B; 1.8-2.2 min 100-10% B; 2.2-2.5 min 10%B; Flow (mL/min) 1.8.

### Method 5:

SFC: Waters Acquity UPC²/QDa; PDA Detector Waters Acquity UPC²; Column: Daicel SFC CHIRALPAK^{®} OZ, 3µm, 0.46cm x 10cm, 40°C; Mobile phase: A: CO₂ B: MeOH isocratic: 20% B in 4.8 min; ABPR: 1800 psi; Flow rate: 2.0 ml/min; Detection: 290 nm; Sample concentration: 1 mg/mL in ACN; Injection: 2 µL.

QDa: [MS+]: Mass Spectrometer from Waters (QDa) (Polarity: positive and negative ions), Detector Gain 1, Temperature Sample: 500°C, Cone Voltage: 10V, ESI Capillary Positive Voltage 0.8 - Negative Voltage 0.8, Sampling Frequency 5Hz, Mass range: 100 to 850Da.

### Example P1: Preparation of 8-chloro-1-methyl-4-[[(1S)-1-[2-(1-methyl-6-oxo-pyridazin-3-yl)-1,2,4-triazol-3-yl]ethyl]amino]-6-(trifluoromethyl)quinazolin-2-one (compound P1)

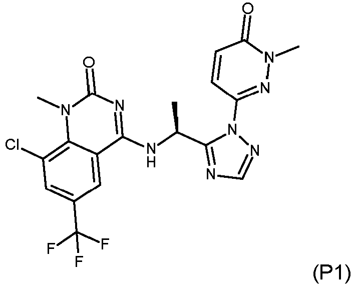

### Step A: Preparation of 8-chloro-1-methyl-6-(trifluoromethyl)quinazoline-2,4-dione (I-1)

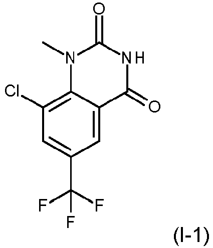

To a mixture of 3-chloro-2-(methylamino)-5-(trifluoromethyl)benzonitrile (CAS 2673195-89-4) (4.26 mmol, 1 g) in dichloromethane (10 mL) was added N-(oxomethylene)sulfamoyl chloride (0.904, 6.39 mmol) at room temperature. The reaction mass was stirred at room temperature for 24 h. The reaction was monitored by thin layer chromatography (TLC) and the conversion was confirmed. The solvent was evaporated on rotavapor and the residue was heated in 150 ml of 2N HCl in water at 100°C for 6 h. The resulting solid was filtered and dried to afford 8-chloro-4-hydroxy-1-methyl-6-(trifluoromethyl)quinazolin-2-one (1.1 g, 83%) as a white solid.

¹H NMR (400 MHz, DMSO-*d6*) δ: 12.03 (s, 1H), 8.22 (d, 1H), 8.16 (d, 1H), 3.65 (m, 3H).

LCMS (method 2): retention time 1.06 min, m/z 276.9 [M-H]⁻.

### Step B: Preparation of 8-chloro-1-methyl-4-thioxo-6-(trifluoromethyl)quinazolin-2-one (I-2)

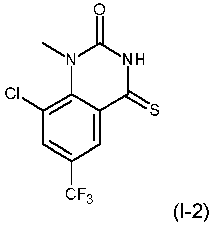

In a 25 ml round bottom flask (RBF), 8-chloro-1-methyl-6-(trifluoromethyl)quinazoline-2,4-dione (I-1) (0.2 g, 0.717 mmol) was taken in toluene (4 mL). Then Lawesson reagent (2,4-bis(4-methoxyphenyl)-2,4-dithioxo-1,3,2λ⁵,4λ⁵-dithiadiphosphetane) (0.299 g, 0.717 mmol) was added at room temperature. The reaction mixture was heated at 90°C for 20 h. The reaction was monitored by LCMS and the conversion was confirmed. The reaction mass was quenched with 2N NaOH and was extracted with EtOAc (50 ml x 2). The organic layer was washed with brine, dried over Na2SO4 and concentrated. The crude was adsorbed on silica gel and purified by combi flash using silica gel column and eluted with gradient system of EtOAc in cyclohexane and the desired fractions were concentrated to get 8-chloro-1-methyl-4-thioxo-6-(trifluoromethyl)quinazolin-2-one (0.25 g, 60 mass%, 70.913%) as a yellow solid. The product was taken for the next reaction as such, without further purification.

LCMS (method 2): retention time 1.13 min, m/z 293.0 [M-H]⁻.

### Step C: Preparation of 8-chloro-1-methyl-4-thioxo-6-(trifluoromethyl)quinazolin-2-one (I-3)

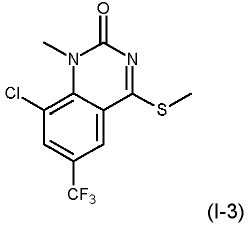

In a 50 mL RBF, 8-chloro-1-methyl-4-thioxo-6-(trifluoromethyl)quinazolin-2-one (I-2) (0.62 g, 1.262 mmol, 60 mass%) was taken in acetonitrile (12.4 mL), then were added iodomethane (0.900 g, 6.3119 mmol) and dicesium carbonate (1.23 g, 3.787 mmol). The reaction mass was stirred at room temperature for 20 h. The reaction was monitored by TLC and the conversion was confirmed. The reaction mass was filtered through a celite bed, washed with EtOAc and the filtrate was concentrated to get the crude. The crude was adsorbed on silica gel and purified by combiflash using silica gel column and eluted with gradient system of EtOAc in cyclohexane. The desired fractions were concentrated to afford 8-chloro-1-methyl-4-methylsulfanyl-6-(trifluoromethyl)quinazolin-2-one (0.2 g, 0.648 mmol, yield 51.3%) as a white solid.

¹H NMR (400 MHz, CDCl₃) δ: 8.16 (s, 1H), 7.94 (d, 1H), 4.01 (s, 3H), 2.72 (s, 3H).

LCMS (method 2): retention time 0.96 min, m/z 309.1 [M+H]⁺.

### Step D: Preparation of 8-chloro-1-methyl-4-[[(1S)-1-[2-(1-methyl-6-oxo-pyridazin-3-yl)-1,2,4-triazol-3-yl]ethyl]amino]-6-(trifluoromethyl)quinazolin-2-one (compound P1)

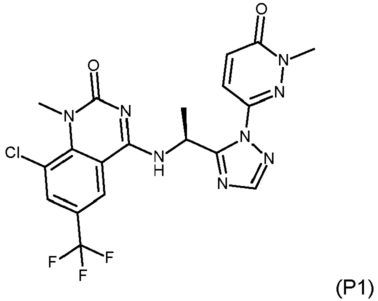

In a 10mL microwave vial were added 8-chloro-1-methyl-4-methylsulfanyl-6-(trifluoromethyl)-quinazolin-2-one (I-3) (0.085 g, 0.275 mmol), [(1S)-1-[2-(1-methyl-6-oxo-pyridazin-3-yl)-1,2,4-triazol-3-yl]ethyl]ammoniu;chloride (compound XX^{a1} prepared as described below) (0.0848g, 0.330 mmol) and acetic acid (0.8908 g, 0.85 mL, 14.8 mmol, 53.6 equiv., 99.5 mass%). The vial was sealed and heated in a microwave at 120°C for 60 min. The reaction was monitored by LCMS and the conversion was confirmed. The reaction mass was concentrated on rotavapor. The crude was adsorbed on celite and purified by reverse phase column chromatography on 40g C18 (40-60µm) using H₂O:ACN. The product fractions were freeze dried to afford 8-chloro-1-methyl-4-[[(1S)-1-[2-(1-methyl-6-oxo-pyridazin-3-yl)-1,2,4-triazol-3-yl]ethyl]amino]-6-(trifluoromethyl)-quinazolin-2-one (0.065 g, 49.1% yield) as white solid.

¹H NMR (400 MHz, acetonitrile-d3) δ: 8.16 (d, 1H), 7.97-8.04 (m, 2H), 7.78-7.84 (m, 1H), 7.02 (d, 1H), 5.94 (q, 1H), 3.76 (s, 3H), 3.65 (s, 3H), 1.76 (d, 3H).

LCMS (method 2): retention time 0.85 min, m/z 481.2 [M+H]⁺.

### Example P2: Preparation of 6-[5-[(1S)-1-[[6-chloro-8-(trifluoromethyl)quinazolin-4-yl]amino]ethyl]-1,2,4-triazol-1-yl]-2-methyl-pyridazin-3-one (compound P2)

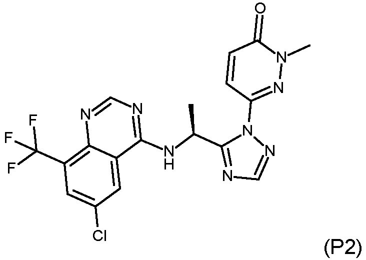

To a mixture of [(1S)-1-[2-(1-methyl-6-oxo-pyridazin-3-yl)-1,2,4-triazol-3-yl]ethyl]ammonium;chloride (compound XX^{a1} prepared as described below) (0.2 g, 0.623 mmol, 80 mass%) and 4,6-dichloro-8-(trifluoromethyl)quinazoline (CAS 1565368-05-9) (0.208 g, 0.77915 mmol) in acetonitrile (4 mL) was added dicesium carbonate Cs2CO₃ (0.305 g, 0.934 mmol) at room temperature. The reaction mixture was heated at 80°C for 60 min. The reaction was monitored by LCMS and the conversion was confirmed. The dicesium carbonate was separated by filtration and the solid was washed with can. The filtrate was then concentrated. The crude was adsorbed on silica gel and purified by combiflash using a silica gel column, and then eluted with gradient system of EtOAc in cyclohexane. The desired fractions were concentrated to afford 6-[5-[(1S)-1-[[6-chloro-8-(trifluoromethyl)quinazolin-4-yl]amino]ethyl]-1,2,4-triazol-1-yl]-2-methyl-pyridazin-3-one (0.2 g, yield 64%) as a white solid.

¹H NMR (400 MHz, DMSO-d6) δ: 9.04 (d, 1H), 8.82 (d, 1H), 8.45 (s, 1H), 8.21 (d, 1H), 8.19 (s, 1H), 7.80 (d, 1H), 7.12 (d, 1H), 5.91 (quin, 1H), 3.48 (s, 3H), 1.75 (d, 3H).

LCMS (method 2): retention time 1.02 min, m/z 451.2 [M+H]⁺.

### Example P3: Preparation of 6-[5-[(1S)-1-[[6,8-bis(trifluoromethyl)quinazolin-4-yl]amino]ethyl]-1,2,4-triazol-1-yl]-2-methyl-pyridazin-3-one (compound P3)

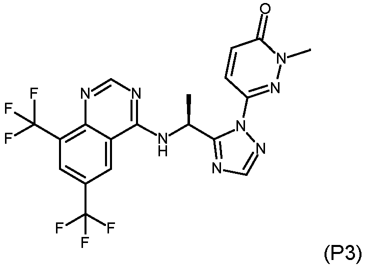

To a mixture of 4-chloro-6,8-bis(trifluoromethyl)quinazoline (CAS 2641011-24-5) (0.234 g, 0.779 mmol) and [(1S)-1-[2-(1-methyl-6-oxo-pyridazin-3-yl)-1,2,4-triazol-3-yl]ethyl]ammonium;chloride (compound XX^{a1} prepared as described below) (0.2 g, 0.623 mmol) in acetonitrile (4 mL) was added dicesium carbonate (0.304 g, 0.934 mmol) at room temperature. The reaction mixture was heated at 80°C for 60 min. The reaction was monitored by LCMS and the conversion was confirmed. Cs2CO₃ was separated by filtration and solid was washed with can. The filtrate was concentrated. The crude was adsorbed on silica gel and purified by combiflash using silica gel column and then eluted with a gradient system of EtOAc in cyclohexane. The desired fractions were concentrated to afford 6-[5-[(1S)-1-[[6,8-bis(trifluoromethyl)quinazolin-4-yl]amino]ethyl]-1,2,4-triazol-1-yl]-2-methyl-pyridazin-3-one (0.15 g, yield 49.6%) as white solid.

¹H NMR (400 MHz, acetonitrile-d3) δ: 8.53 (s, 1H), 8.46 (s, 1H), 8.37 (brd, 1H), 8.19 (s, 1H), 8.12 (s, 1H), 7.81 (d, 1H), 7.09 (d, 1H), 6.04 (quin, 1H), 3.67 (s, 3H), 1.86 (d, 3H).

LCMS (method 2): retention time 0.91 min, m/z 485.2 [M+H]⁺.

### Example P4: Preparation of 6-[5-[(1S)-1-[[2,8-dichloro-6-(trifluoromethyl)quinazolin-4-yl]amino]ethyl]-1,2,4-triazol-1-yl]-2-methyl-pyridazin-3-one (compound P4)

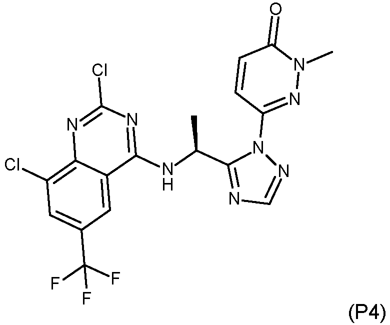

To a mixture of 2,4,8-trichloro-6-(trifluoromethyl)quinazoline (CAS 2673195-86-1) (0.258 g, 0.857 mmol) and [(1S)-1-[2-(1-methyl-6-oxo-pyridazin-3-yl)-1,2,4-triazol-3-yl]ethyl]ammonium;chloride (compound XX^{a1} prepared as described below) (0.2 g, 0.779 mmol) in acetonitrile (4 mL) was added dicesium carbonate (0.38 g, 1.1687 mmol). The reaction mixture was heated at 80°C for 15 min. The reaction was monitored by LCMS and the conversion was confirmed. Cs2CO₃ was separated by filtration and solid was washed with ACN, and filtrate was concentrated. The crude was adsorbed on silica gel and purified by combiflash using a silica gel column and then eluted with a gradient system of EtOAc in cyclohexane. The desired fractions were concentrated to afford 6-[5-[(1S)-1-[[2,8-dichloro-6-(trifluoromethyl)quinazolin-4-yl]amino]ethyl]-1,2,4-triazol-1-yl]-2-methyl-pyridazin-3-one (0.22 g, yield 54.7%) as a white solid.

¹H NMR (400 MHz, DMSO-d6) δ: 9.75 (br d, 1H), 8.88 (s, 1H), 8.34 (s, 1H), 8.24 (s, 1H), 8.13 (s, 1H), 7.81 (d, 1H), 7.15 (d, 1H), 5.93-6.00 (m, 1H), 3.54 (s, 3H), 1.76 (d, 3H).

LCMS (method 2): retention time 1.10 min, m/z 485.2 [M+H]⁺.

### Example P5: Preparation of 8-chloro-4-[[(1S)-1-[2-(1-methyl-6-oxo-pyridazin-3-yl)-1,2,4-triazol-3-yl]ethyl]amino]-6-(trifluoromethyl)-1H-quinazolin-2-one (compound P5)

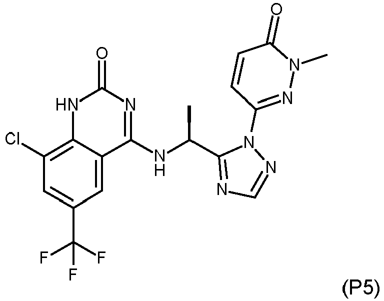

In a 25 ml RBF were charged 6-[5-[(1S)-1-[[2,8-dichloro-6-(trifluoromethyl)quinazolin-4-yl]amino]ethyl]-1,2,4-triazol-1-yl]-2-methyl-pyridazin-3-one (compound P4) (0.2 g, 0.412 mmol) and acetic acid (2 mL, 99.5 mass%). Then the reaction mass was heated at 80°C for 45 minutes. The reaction was monitored by LCMS and confirmed the conversion. The reaction mass was concentrated on rotavapor. Crude was adsorbed on celite and purified by reverse phase column chromatography on 40g C18 (40-60µm) and eluted using H2O and ACN (0.01 % TFA). Product fractions were freeze dried and further purified by preparative HPLC to get 8-chloro-4-[[(1S)-1-[2-(1-methyl-6-oxo-pyridazin-3-yl)-1,2,4-triazol-3-yl]ethyl]amino]-6-(trifluoromethyl)-1H-quinazolin-2-one (0.1 g, 51.9%) as white solid.

¹H NMR (400 MHz, acetonitrile-d3) δ: 8.82 (br s, 1H), 8.15 (s, 1H), 8.04 (s, 1H), 7.96 (s, 1H), 7.82 (d, 1H), 7.73 (br d, 1H), 7.01 (d, 1H), 5.96 (quin, 1H), 3.66 (s, 3H), 1.76 (d, 3H).

LCMS (method 2): retention time 0.81 min, m/z 467.2 [M+H]⁺.

### Example P6: Preparation of 6-[5-[(1S)-1-[(6,8-dibromoquinazolin-4-yl)-methyl-amino]ethyl]-1,2,4-triazol-1-yl]-2-methyl-pyridazin-3-one (compound P14)

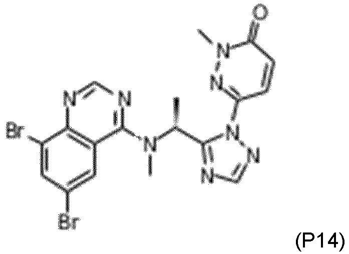

To a mixture of 6-[5-[(1S)-1-[(6,8-dibromoquinazolin-4-yl)amino]ethyl]-1,2,4-triazol-1-yl]-2-methyl-pyridazin-3-one (compound P18) (0.1 g, 0.198 mmol) and cesium carbonate (0.193 g, 0.592 mmol) in acetonitrile (1.5 mL) was added iodomethane (0.141 g, 0.98 mmol) at room temperature. The reaction mixture was stirred at room temperature for 4 hours, then diluted with water and the product extracted with ethyl acetate. The combined organic layers were washed with brine, dried over sodium sulfate, filtered and concentrated. The residue was purified by combiflash (ethyl acetate/cyclohexane) to afford 6-[5-[(1S)-1-[(6,8-dibromoquinazolin-4-yl)-methyl-amino]ethyl]-1,2,4-triazol-1-yl]-2-methyl-pyridazin-3-one as pale yellow solid. LCMS (method 3): retention time 1.09 min, m/z 519/521/523 [M+H]⁺.

¹H NMR (400 MHz, CDCl₃) δ: 8.62 (s, 1H), 8.16 (d, 1H), 8.01 (s, 1H), 7.93 (d, 1H), 7.81 (d, 1H), 7.09 (d, 1H), 6.47 (q, 1H), 3.37 (s, 3H), 3.26 (s, 3H), 1.88 (d, 3H).

### Example P7: Preparation of 6-[3-[1-[[8-chloro-6-(trifluoromethyl)quinazolin-4-yl]amino]ethyl]pyrazin-2-yl]-2-methyl-pyridazin-3-one (compound P13)

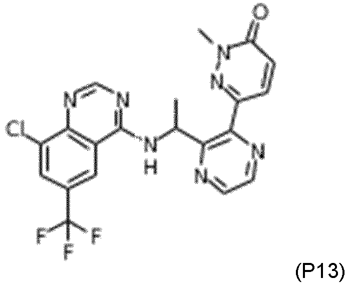

To a solution of 4,8-dichloro-6-(trifluoromethyl)quinazoline (prepared in analogy to descriptions found in WO 2021/083936) (0.162 g, 0.605 mmol) and 6-[3-(1-aminoethyl)pyrazin-2-yl]-2-methyl-pyridazin-3-one (compound XX^{c1} prepared as described below) (0.140 g, 0.605 mmol) in tetrahydrofuran (2 mL) was added triethylamine (0.308 g, 3.027 mmol) at room temperature. The reaction mixture was heated in the microwave at 100 °C for 1 hour, then concentrated *in vacuo*.The residue was purified by combiflash (ethyl acetate/cyclohexane) to afford 6-[3-[1-[[8-chloro-6-(trifluoromethyl)quinazolin-4-yl]amino]ethyl]pyrazin-2-yl]-2-methyl-pyridazin-3-one as a white solid.

LCMS (method 2): retention time 0.88 min, m/z 462/464 [M+H]⁺.

¹H NMR (400 MHz, CDCl₃) δ: 8.73 (s, 1H), 8.60-8.67 (m, 2H), 8.17 (d, 1H), 8.04 (d, 2H), 7.53 (br d, 1H), 7.13 (d, 1H), 6.47 (quin, 1H), 4.00 (s, 3H), 1.75 (d, 3H).

### Example P8: Preparation of 6-[3-[1-[[8-chloro-6-(trifluoromethyl)quinazolin-4-yl]-methyl-amino]ethyl]pyrazin-2-yl]-2-methyl-pyridazin-3-one (compound P12)

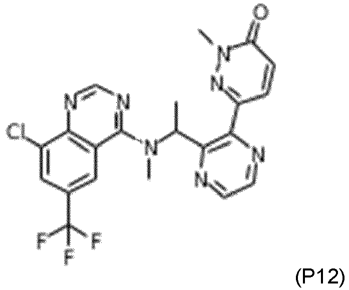

To mixture of 6-[3-[1-[[8-chloro-6-(trifluoromethyl)quinazolin-4-yl]amino]ethyl]pyrazin-2-yl]-2-methyl-pyridazin-3-one (compound P13) (0.075 g, 0.162 mmol) and cesium carbonate (0.159 g, 0.487 mmol) in acetonitrile (1.5 mL) was added iodomethane (0.116 g, 0.812 mmol) at room temperature. The reaction mixture was stirred at room temperature for 4 hours, then diluted with water and the product extracted with ethyl acetate. The combined organic layers were washed with brine, dried over sodium sulfate, filtered and concentrated. The residue was purified by combiflash (ethyl acetate/cyclohexane) to afford 6-[3-[1-[[8-chloro-6-(trifluoromethyl)quinazolin-4-yl]-methyl-amino]ethyl]pyrazin-2-yl]-2-methyl-pyridazin-3-one as a pale yellow solid.

LCMS (method 3): retention time 1.14 min, m/z 474/476 [M-H]⁻.

¹H NMR (400 MHz, CDCl₃) δ: 8.51-8.59 (m, 3H), 8.10 (s, 1H), 7.99 (s, 1H), 7.98 (d, 1H), 7.04 (d, 1H), 6.55 (q, 1H), 3.55 (s, 3H), 3.54 (s, 3H), 1.90 (d, 3H).

### Example P9: Preparation of 6-[5-[(1S)-1-[[8-chloro-6-(trifluoromethyl)quinazolin-4-yl]amino]ethyl]-1,2,4-triazol-1-yl]-2-ethyl-pyridazin-3-one (compound P17)

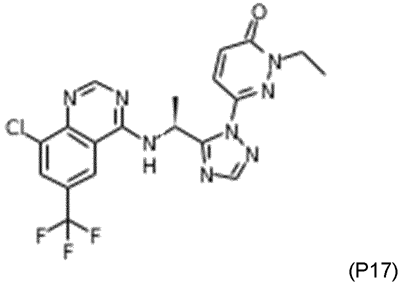

To a solution of 4,8-dichloro-6-(trifluoromethyl)quinazoline (prepared in analogy to descriptions found in WO 2021/083936) (0.271 g. 1.016 mmol) and [(1S)-1-[2-(1-ethyl-6-oxo-pyridazin-3-yl)-1,2,4-triazol-3-yl]ethyl]ammonium;chloride (compound XX^{a2} prepared as described below) (0.250 g, 0.924 mmol) in tetrahydrofuran (5 mL) was added triethylamine (0.470 g, 4.617mmol) at room temperature. The reaction mixture was heated in the microwave at 100 °C for 1 hour, then concentrated *in* vacuo. The residue was purified by combiflash (ethyl acetate/cyclohexane) to afford 6-[5-[(1S)-1-[[8-chloro-6-(trifluoromethyl)quinazolin-4-yl]amino]ethyl]-1,2,4-triazol-1-yl]-2-ethyl-pyridazin-3-one as a white solid.

LCMS (method 3): retention time 1.10 min, m/z 463/465 [M-H]⁻.

¹H NMR (400 MHz, CDCl₃) δ: 8.72 (br d, 1H), 8.48 (s, 1H), 8.11 (s, 1H), 7.96 (d, 1H), 7.83 (s, 1H), 7.81 (s, 1H), 7.24 (d, 1H), 6.14 (quin, 1H), 4.31 (q, 2H), 1.92 (d, 3H), 1.49 (t, 3H).

### Example P10: Preparation of 6-[5-[(1S)-1-[[6,8-bis(trifluoromethyl)quinazolin-4-yl]amino]ethyl]-1,2,4-triazol-1-yl]-2-methyl-4,5-dihydropyridazin-3-one (compound P7)

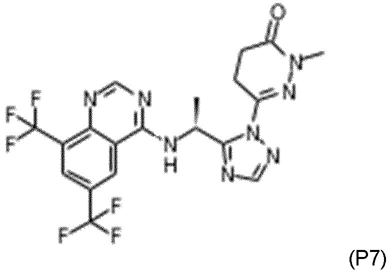

To a degassed solution of 6-[5-[(1S)-1-[[6,8-bis(trifluoromethyl)quinazolin-4-yl]amino]ethyl]-1,2,4-triazol-1-yl]-2-methyl-pyridazin-3-one (compound P3) (0.06 g, 0.124 mmol) in methanol (3 mL) was was added palladium on carbon (0.132 g). The reaction mixture was stirred under a hydrogen balloon atmosphere at room temperature for 20 hours, then filtered through a celite bed. The celite bed was washed with MeOH and the combined filtrate concentrated under reduced pressure. The residue was purified by combiflash (ethyl acetate/cyclohexane) to afford 6-[5-[(1S)-1-[[6,8-bis(trifluoromethyl)-quinazolin-4-yl]amino]ethyl]-1,2,4-triazol-1-yl]-2-methyl-4,5-dihydropyridazin-3-one as a white solid.

LCMS (method 3): retention time 1.11 min, m/z 487 [M+H]⁺.

¹H NMR (400 MHz, acetonitrile-*d₃*) δ: 8.65 (s, 1H), 8.57 (s, 1H), 8.25 (s, 1H), 8.18 (br d, 1H), 8.01 (s, 1H), 6.19 (quin, 1H), 3.20-3.41 (m, 5H), 2.71 (t, 2H), 1.81 (d, 3H).

### Example P11: Preparation of 6-[5-[(1S)-1-[(6-chloro-8-iodo-quinazolin-4-yl)amino]ethyl]-1,2,4-triazol-1-yl]-2-methyl-4,5-dihydropyridazin-3-one (compound P25)

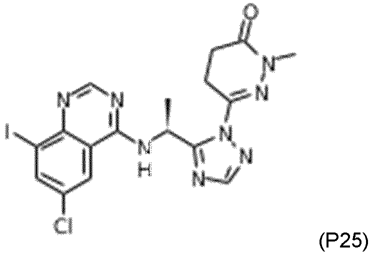

To a solution of 4,6-dichloro-8-iodo-quinazoline (CAS 100949-33-5) (0.165 g, 0.508 mmol) and [(1S)-1-[2-(1-methyl-6-oxo-4,5-dihydropyridazin-3-yl)-1,2,4-triazol-3-yl]ethyl]ammonium;chloride (compound XX^{b1} prepared as described below) (0.144 g, 0.559 mmol) in tetrahydrofuran (4 mL) was added triethylamine (0.258 g, 2.53 mmol) at room temperature. The reaction mixture was heated in the microwave at 100 °C for 1 hour, then concentrated *in vacuo.* The residue was purified by combiflash (ethyl acetate in cyclohexane) to afford 6-[5-[(1S)-1-[(6-chloro-8-iodo-quinazolin-4-yl)amino]ethyl]-1,2,4-triazol-1-yl]-2-methyl-4,5-dihydropyridazin-3-one as a white solid.

LCMS (method 3): retention time 1.06 min, m/z 511/513 [M+H]⁺.

¹H NMR (400 MHz, CDCl₃) δ: 8.52 (s, 1H), 8.24 (s, 1H), 8.03 (s, 2H), 7.61 (s, 1H), 6.05-6.11 (m, 1H), 3.27-3.56 (m, 4H), 3.29-3.46 (m, 1H), 2.78-2.94 (m, 2H), 1.82 (d, 3H).

### Example P12: Preparation of 6-[5-[(1S)-1-[(6-chloro-8-iodo-quinazolin-4-yl)-methyl-amino]ethyl]-1,2,4-triazol-1-yl]-2-methyl-4,5-dihydropyridazin-3-one (compound P26)

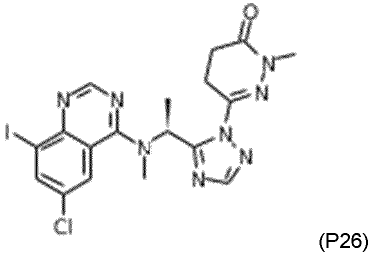

To mixture of 6-[5-[(1S)-1-[(6-chloro-8-iodo-quinazolin-4-yl)amino]ethyl]-1,2,4-triazol-1-yl]-2-methyl-4,5-dihydropyridazin-3-one (compound P25) (0.140 g, 0.274 mmol) and cesium carbonate (0.268 g, 0.822 mmol) in acetonitrile (2 mL) was added iodomethane (0.196 g, 1.371 mmol) at room temperature. The reaction mixture was stirred at room temperature for 4 hours, then diluted with water and the product extracted with ethyl acetate. The combined organic layers were washed with brine, dried over sodium sulfate, filtered and concentrated *in vacuo.* The residue was purified by combiflash (ethyl acetate in cyclohexane) to afford 6-[5-[(1S)-1-[(6-chloro-8-iodo-quinazolin-4-yl)-methyl-amino]ethyl]-1,2,4-triazol-1-yl]-2-methyl-4,5-dihydropyridazin-3-one as a white solid.

LCMS (method 3): retention time 1.10 min, m/z 525/527 [M+H]⁺.

¹H NMR (400 MHz, CDCl₃) δ: 8.66 (s, 1H), 8.29 (d, 1H), 7.94 (s, 1H), 7.91 (d, 1H), 6.48 (q, 1H), 3.39 (s, 3H), 3.23 - 3.37 (m, 2H), 2.82 (s, 3H) 2.60-2.75 (m, 2H), 1.84 (d, 3H).

### Example P13: Preparation of 6-[3-[(1S)-1-[[6-chloro-8-(trifluoromethyl)quinazolin-4-yl]-methyl-amino]ethyl]pyrazin-2-yl]-2-methyl-pyridazin-3-one (compound P27)

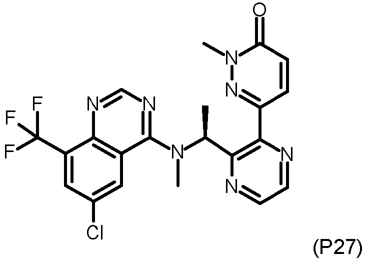

The racemic 6-[3-[1-[[6-chloro-8-(trifluoromethyl)quinazolin-4-yl]-methyl-amino]ethyl]pyrazin-2-yl]-2-methyl-pyridazin-3-one (compound P21) mixture (65 mg) was submitted to chiral resolution by preparative SFC using the conditions outlined hereafter.

### Preparative method:

Sepiatec Prep SFC 100; Column: Daicel CHIRALPAK^{®} OZ, 5µm, 2.0 cm x 25cm; Mobile phase: A: CO₂ B: MeOH isocratic: 20 % B; Backpressure: 150 bar; GLS: -; Flow rate: 60 ml/min; Detection: UV 290 nm; Sample concentration: 65mg in 2ml MeOH/DCM (1/1); Injection: 350µl.

### Results (chiral analytical method 5):

| First eluting enantiomer | Second eluting enantiomer (compound P27) |
|---|---|
| Retention time (min) ~ 2.54 | Retention time (min) ~ 4.03 |
| Chemical purity (area% at 290 nm) 98 | Chemical purity (area% at 290 nm) >99 |
| Enantiomeric excess (%) > 99 | Enantiomeric excess (%) > 99 |
| [M+H]⁺ measured: 476/478 | [M+H]⁺ measured: 476/478 |
| Amount: 26.5 mg | Amount: 32.2 mg |

**Table P: Physical data of compounds of formula (I) (* [M+H]⁺ measured; ** Method used)**

| Entry | IUPAC name | STRUCTURE | RT (min) | [M+H]⁺ * | Meth. ** | MP (°C) |
|---|---|---|---|---|---|---|
| P1 | 8-chloro-1-methyl-4-[[(1S)-1-[2-(1-methyl-6-oxo-pyridazin-3-yl)-1,2,4-triazol-3-yl]ethyl]amino]-6-(trifluoromethyl)quinazolin-2-one | 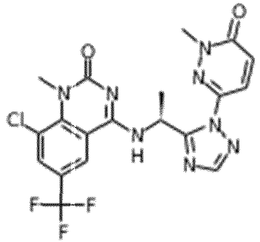 | 0.85 | 481.2 | 2 | 112-116 |
| P2 | 6-[5-[(1S)-1-[[6-chloro-8-(trifluoromethyl)quinazolin-4-yl]amino]ethyl]-1,2,4-triazol-1-yl]-2-methyl-pyridazin-3-one | 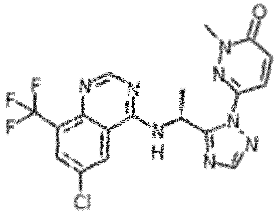 | 1.02 | 451.2 | 2 | 147-151 |
| P3 | 6-[5-[(1S)-1-[[6,8-bis(trifluoromethyl)quinazolin-4-yl]amino]ethyl]-1,2,4-triazol-1-yl]-2-methyl-pyridazin-3-one | 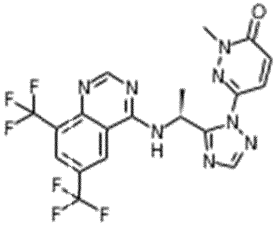 | 0.91 | 485.2 | 2 | 134-138 |
| P4 | 6-[5-[(1S)-1-[[2,8-dichloro-6-(trifluoromethyl)quinazolin-4-yl]amino]ethyl]-1,2,4-triazol-1-yl]-2-methyl-pyridazin-3-one | 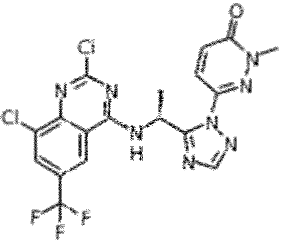 | 1.10 | 485.2 | 2 | 163-167 |
| P5 | 8-chloro-4-[[(1S)-1-[2-(1-mothyl-6-oxo-pyridazin-3-yl)-1,2,4-triazol-3-yl]ethyl]amino]-6-(trifluoromethyl)-1H-quinazolin-2-one | 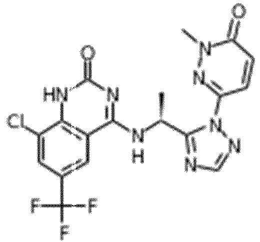 | 0.81 | 467.2 | 2 | - |
| P6 | 6-[5-[(1S)-1-[[6-chloro-8-(trifluoromethyl)quinazolin-4-yl]-methyl-amino]ethyl]-1,2,4-triazol-1-yl]-2-methyl-pyridazin-3-one | 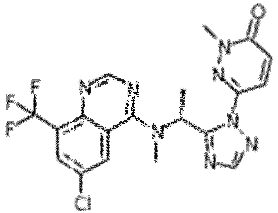 | 1.13 | 465/467 | 3 | 93 - 95 |
| P7 | 6-[5-[(1S)-1-[[6,8-bis(trifluoromethyl)quinazolin-4-yl]amino]ethyl]-1,2,4-triazol-1-yl]-2-methyl-4,5-dihydropyridazin-3-one | 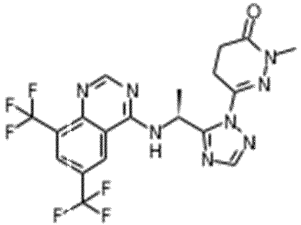 | 1.11 | 487 | 3 | 133 - 137 |
| P8 | 6-[3-[1-[[6,8-bis(trifluoromethyl)quinazolin-4-yl]amino]ethyl]pyrazin-2-yl]-2-methyl-pyridazin-3-one | 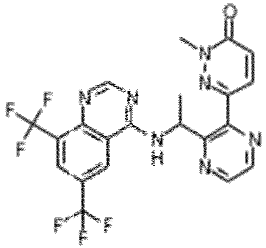 | 0.97 | 496 | 3 | 215 - 217 |
| P9 | 6-[5-[(1S)-1-[[6-chloro-8-(trifluoromethyl)quinazolin-4-yl]amino]ethyl]-1,2,4-triazol-1-yl]-2-methyl-4,5-dihydropyridazin-3-one | 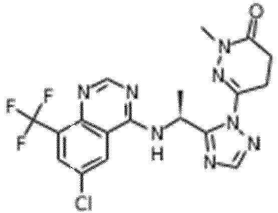 | 1.06 | 453/455 | 3 | 209 - 211 |
| P10 | 6-[3-[1-[[6-chloro-8-(trifluoromethyl)quinazolin-4-yl]amino]ethyl]pyrazin-2-yl]-2-methyl-pyridazin-3-one | 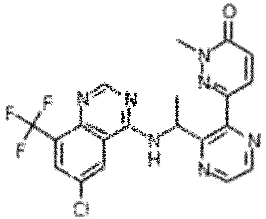 | 1.45 | 462/464 | 4 | 218 - 220 |
| P11 | 6-[5-[(1S)-1-[(6-chloro-8-iodo-quinazolin-4-yl)amino]ethyl]-1,2,4-triazol-1-yl]-2-methyl-pyridazin-3-one | 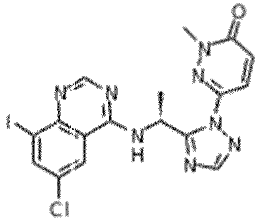 | 1.02 | 509/511 | 3 | 203 - 205 |
| P12 | 6-[3-[1-[[8-chloro-6-(trifluoromethyl)quinazolin-4-yl]-methyl-amino]ethyl]pyrazin-2-yl]-2-methyl-pyridazin-3-one | 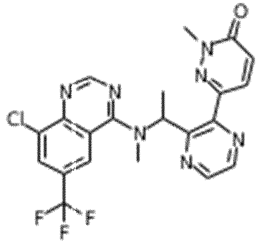 | 1.14 | 474/476 [M-H]⁻ | 3 | 157 - 159 |
| P13 | 6-[3-[1-[[8-chloro-6-(trifluoromethyl)quinazolin-4-yl]amino]ethyl]pyrazin-2-yl]-2-methyl-pyridazin-3-one | 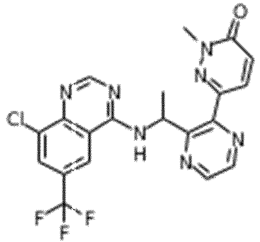 | 0.88 | 462/464 | 3 | 240 - 242 |
| P14 | 6-[5-[(1S)-1-[(6,8-dibromoquinazolin-4-yl)-methyl-amino]ethyl]-1,2,4-triazol-1-yl]-2-methyl-pyridazin-3-one | 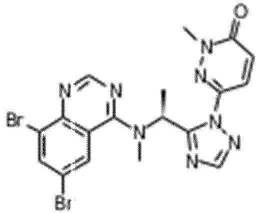 | 1.09 | 519/521 /523 | 3 | 74 - 76 |
| P15 | 6-[5-[(1S)-1-[[6-chloro-8-(trifluoromethyl)quinazolin-4-yl]-methyl-amino]ethyl]-1,2,4-triazol-1-yl]-2-ethyl-pyridazin-3-one | 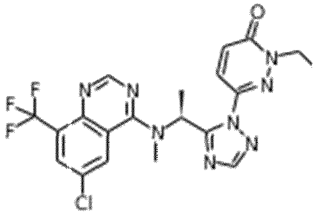 | 1.18 | 479/481 | 3 | 94 - 96 |
| P16 | 6-[5-[(1S)-1-[[8-chloro-6-(trifluoromethyl)quinazolin-4-yl]amino]ethyl]-1,2,4-triazol-1-yl]-2-methyl-pyridazin-3-one | 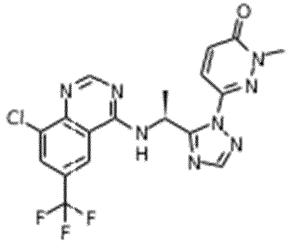 | 1.05 | 451/453 | 3 | - |
| P17 | 6-[5-[(1S)-1-[[8-chloro-6-(trifluoromethyl)quinazolin-4-yl]amino]ethyl]-1,2,4-triazol-1-yl]-2-ethyl-pyridazin-3-one | 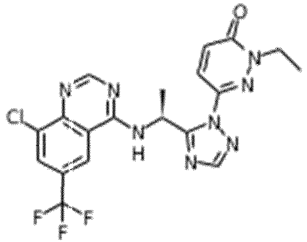 | 1.10 | 463/465 [M-H]⁻ | 3 | 116 - 118 |
| P18 | 6-[5-[(1S)-1-[(6,8-dibromoquinazolin-4-yl)amino]ethyl]-1,2,4-triazol-1-yl]-2-methyl-pyridazin-3-one | 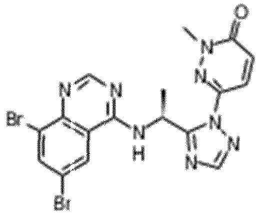 | 1.04 | 503/505 /507 [M-H]⁻ | 3 | 259 - 261 |
| P19 | 6-[5-[(1S)-1-[[6-chloro-8-(trifluoromethyl)quinazolin-4-yl]amino]ethyl]-1,2,4-triazol-1-yl]-2-ethyl-pyridazin-3-one | 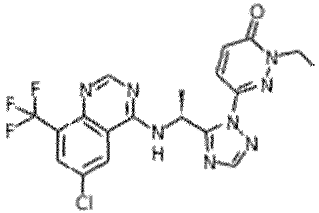 | 1.11 | 463/465 [M-H]⁻ | 3 | 116 - 118 |
| P20 | 6-[5-[(1S)-1-[(8-chloro-6-iodo-quinazolin-4-yl)amino]ethyl]-1,2,4-triazol-1-yl]-2-methyl-pyridazin-3-one | 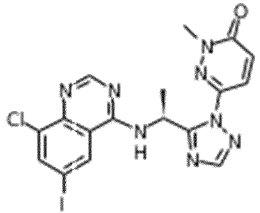 | 1.05 | 507/509 [M-H]⁻ | 3 | - |
| P21 | 6-[3-[1-[[6-chloro-8-(trifluoromethyl)quinazolin-4-yl]-methyl-amino]ethyl]pyrazin-2-yl]-2-methyl-pyridazin-3-one | 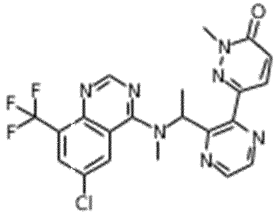 | 1.17 | 474/476 [M-H]⁻ | 3 | 144 - 146 |
| P22 | 6-[5-[(1S)-1-[[8-chloro-6-(trifluoromethyl)quinazolin-4-yl]-methyl-amino]ethyl]-1,2,4-triazol-1-yl]-2-ethyl-pyridazin-3-one | 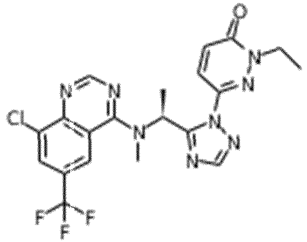 | 1.14 | 477/479 [M-H]⁻ | 3 | 64 - 66 |
| P23 | 6-[5-[(1S)-1-[[8-chloro-6-(trifluoromethyl)quinazolin-4-yl]-methyl-amino]ethyl]-1,2,4-triazol-1-yl]-2-methyl-pyridazin-3-one | 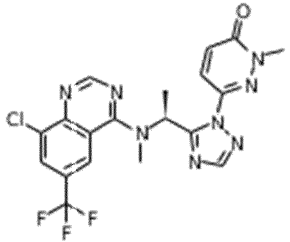 | 1.11 | 463/465 [M-H]⁻ | 3 | 107 - 109 |
| P24 | 6-[5-[(1S)-1-[[6-chloro-8-(trifluoromethyl)quinazolin-4-yl]-methyl-amino]ethyl]-1,2,4-triazol-1-yl]-2-methyl-4,5-dihydropyridazin-3-one | 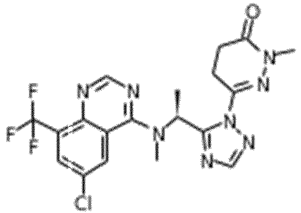 | 1.11 | 467/469 | 3 | 70 - 72 |
| P25 | 6-[5-[(1S)-1-[(6-chloro-8-iodo-quinazolin-4-yl)amino]ethyl]-1,2,4-triazol-1-yl]-2-methyl-4,5-dihydropyridazin-3-one | 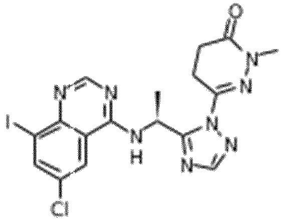 | 1.06 | 511/513 | 3 | 236 - 238 |
| P26 | 6-[5-[(1S)-1-[(6-chloro-8-iodo-quinazolin-4-yl)-methyl-amino]ethyl]-1,2,4-triazol-1-yl]-2-methyl-4,5-dihydropyridazin-3-one | 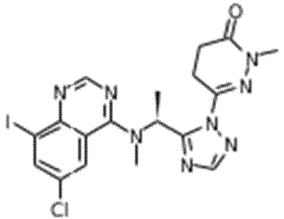 | 1.10 | 525/527 | 3 | 92 - 94 |
| P27 | 6-[3-[(1S)-1-[[6-chloro-8-(trifluoromethyl)quinazolin-4-yl]-methyl-amino]ethyl]pyrazin-2-yl]-2-methyl-pyridazin-3-one | 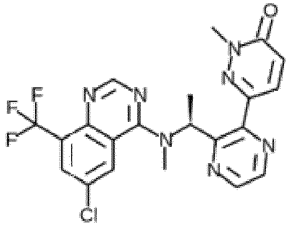 | 4.03 | 476/478 [M+H]⁺ | 5 | - |

### Preparation of intermediates

### Example PI-1: Preparation of [(1S)-1-[2-(1-methyl-6-oxo-pyridazin-3-yl)-1,2,4-triazol-3-yl]ethyl]ammonium;chloride (compound XX^{a1})

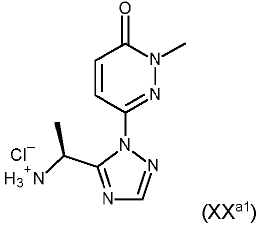

### Step A: Preparation of 6-chloro-2-methyl-pyridazin-3-one (I-4)

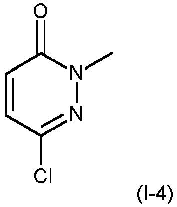

To a solution of 6-chloropyridazin-3-ol (CAS 19064-67-6) (1.00g, 7.661 mmol, 1.00 equiv.) in acetonitrile (10 mL) were added potassium carbonate (3.21 g, 22.98 mmol, 3.00 equiv.) followed by iodomethane (0.584 mL, 9.193 mmol, 1.20 equiv.). The reaction was stirred at room temperature for 48 hours. Then, water was added, and the aqueous layer was extracted with ethyl acetate. Then, the organic layer was washed with brine, dried over sodium sulfate, filtered and concentrated under reduced pressure to afford the desired compound, 6-chloro-2-methyl-pyridazin-3-one, as a brown solid. LCMS (method 1): retention time 0.39 min, m/z 145/147 [M+H]⁺.

¹H NMR (400 MHz, CDCl₃) δ ppm 7.20 (d, J=9.66 Hz, 1 H), 6.92 (d, J=9.66 Hz, 1 H), 3.75 (s, 3 H).

### Step B: Preparation of 6-hydrazino-2-methyl-pyridazin-3-one (I-5)

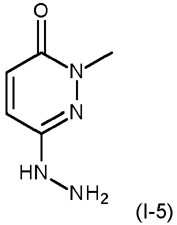

To a 25ml RBF charged with 6-chloro-2-methyl-pyridazin-3-one (I-4) (4.5 g, 31.129 mmol) were added methoxy cyclopentane (CPME) (45 mL) and then hydrazine hydrate (7.79 g, 155.64 mmol) at room temperature. The reaction mixture was heated at 100 °C for 1 hour (biphasic reaction mixture). The reaction mixture was cooled to room temperature. The CPME layer was separated, and the residue layer was washed with TBME using a separating funnel. The residue layer was concentrated at 50 °C, and the obtained crude was adsorbed on celite and then purified by combiflash (silica gel column, elution with EtOAc/MeOH). The product was eluted in 95:5 EtOAc/MeOH to get 6-hydrazino-2-methyl-pyridazin-3-one (2.5 g, 14 mmol, 46%) as a white solid.

LCMS (method 2): retention time 0.14 min, m/z 141.1 [M+H]⁺.

### Alternative preparation of 6-hydrazino-2-methyl-pyridazin-3-one (I-5)

Under nitrogen atmosphere, to a solution of 6-chloro-2-methyl-pyridazin-3-one (I-4) (0.500 g, 3.459 mmol, 1.00 equiv.) in ethanol (10.38 mL) was added hydrazine hydrate (0.845 mL, 17.294 mmol, 5.00 equiv.). The reaction mixture was heated at 100°C for 5 hours. Then, the solution was evaporated under reduced pressure and the residue was purified by flash chromatography to afford the desired compound, 6-hydrazino-2-methyl-pyridazin-3-one. LCMS (method 1): retention time 0.29 min.

¹H NMR (400 MHz, DMSO-d6) δ ppm 7.35 - 7.59 (m, 2 H), 7.02 (d, J=9.66 Hz, 1 H), 6.74 (d, J=9.78 Hz, 1 H), 5.92 (s, 1 H), 3.46 (s, 3 H).

### Step C: Preparation of tert-butyl N-[(1S)-1-[2-(1-methyl-6-oxo-pyridazin-3-yl)-1,2,4-triazol-3-yl]ethyl]carbamate (I-6)

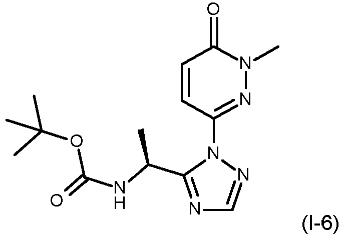

To a 250 mL RBF charged with 6-hydrazino-2-methyl-pyridazin-3-one (I-5) (2.5 g, 14.271 mmol) were added tert-butyl N-[(1S)-2-[(E)-dimethylaminomethyleneamino]-1-methyl-2-oxo-ethyl]carbamate (CAS 2641011-39-2, prepared as described for example in WO21/083936) (4.16 g, 17.126 mmol), 1,4-dioxane (25 mL), molecular sieves 4 Å (5 g) and acetic acid (25 mL). After addition, the reaction mass was heated at 80 °C for 5 h. Progress of the reaction mass was monitored by LCMS. The reaction mixture was filtered through a celite bed, the celite bed was washed with EtOAc and the filtrate was concentrated. The residue was diluted with saturated aqueous NaHCO₃ and the product extracted three times with EtOAc (100 mL x 3). The combined organic layers were washed with brine, dried over sodium sulfate, filtered and concentrated. The crude was adsorbed on silica and purified by combiflash. The product was eluted in 80:20 EtOAc/cyclohexane to afford 2.8 g product, which was purified further by reverse phase column chromatography on 40g C18 (40-60µm) and eluted using H₂O/ACN. Product fractions were freeze dried to get tert-butyl N-[(1S)-1-[2-(1-methyl-6-oxo-pyridazin-3-yl)-1,2,4-triazol-3-yl] ethyl]carbamate (1.2 g, 3.0 mmol, 21%) as a white solid.

¹H NMR (400 MHz, CDCl₃) δ: 7.95 (s, 1H), 7.86 (d, 1H), 7.13 (d, J=9.9 Hz, 1H), 5.46-5.55 (m, 1H), 5.42 (br, 1H), 3.84 (s, 3H), 1.54-1.64 (d, 3H), 1.42 (s, 9H).

### Step D: Preparation of [(1S)-1-[2-(1-methyl-6-oxo-pyridazin-3-yl)-1,2,4-triazol-3-yl]ethyl]ammonium;chloride (compound XX^{a1})

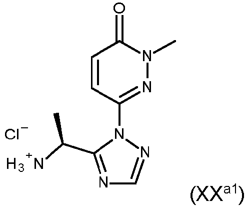

In a 50 mL reaction flask, tert-butyl N-[(1S)-1-[2-(1-methyl-6-oxo-pyridazin-3-yl)-1,2,4-triazol-3-yl]ethyl]carbamate (I-6) (1.5 g, 3.745 mmol) was dissolved in CPME (15 mL) and then hydrochloric acid (4M in dioxane) (14.04 mL, 56.18 mmol) was added into the reaction mixture at room temperature. The reaction mixture was stirred at room temperature for 18 h, during which a white solid precipitated. Progress of the reaction was monitored by LCMS. The reaction mass was concentrated *in vacuo* and the resulting white solid was stirred in ACN (20 mL). The solid was separated by filtration and dried under reduced pressure to get [(1S)-1-[2-(1-methyl-6-oxo-pyridazin-3-yl)-1,2,4-triazol-3-yl]ethyl]ammonium;chloride (1.2 g, 3.90 mmol) as a white solid.

¹H NMR (400 MHz, DMSO-d6) δ ppm 8.39 (s, 1 H), 7.93 (d, 1 H), 7.24 (d, 1 H), 5.07 (br, 1 H), 3.72 (s, 3 H), 1.63 (d, 3 H).

### Example PI-2: Preparation of [(1S)-1-[2-(1-ethyl-6-oxo-pyridazin-3-yl)-1,2,4-triazol-3-yl]ethyl]ammonium;chloride (compound XX^{a2})

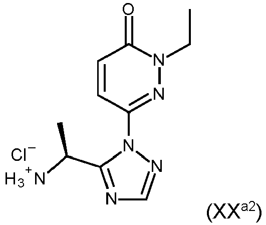

### Step A: Preparation of tert-butyl N-[(1S)-1-[2-(6-chloropyridazin-3-yl)-1,2,4-triazol-3-yl]ethyl]carbamate (I-7)

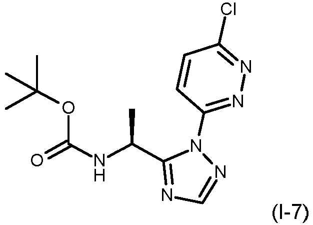

To a stirred solution of tert-butyl N-[(1S)-2-[(E)-dimethylaminomethyleneamino]-1-methyl-2-oxoethyl]carbamate (CAS 2641011-39-2, prepared as described for example in WO21/083936) (5 g, 20.55 mmol) in 1,4-dioxane (50 mL) was added 3-chloro-6-hydrazinopyridazine (CAS 17284-97-8) (3.27 g, 22.61 mmol), followed by acetic acid (50 mL). The reaction mixture was heated to 80 °C for 2 hours, then cooled to RT and concentrated *in vacuo.* The residue was diluted with water and the product extracted with EtOAc. The combined organic layers were washed with a saturated aqueous sodium bicarbonate solution, water and brine, dried over sodium sulfate and concentrated under reduced pressure. The crude was purified by combiflash (gradient ethyl acetate in cyclohexane) to afford tert-butyl N-[(1S)-1-[2-(6-chloropyridazin-3-yl)-1,2,4-triazol-3-yl]ethyl]carbamate (I-7).

LCMS (method 2): retention time 0.98 min, m/z 269/271 [M+H-tBu]⁺, 225/227 [M+H-Boc]⁺.

¹H NMR (400 MHz, CDCl₃) δ ppm 8.13 (d, 1 H), 8.01 (s, 1 H), 7.72 (d, 1 H), 5.78 (quin, 1 H), 5.51 (br s, 1 H), 1.65 (d, 3 H), 1.39 (s, 9 H).

### Step B: Preparation of tert-butyl N-[(1S)-1-[2-(6-oxo-1H-pyridazin-3-yl)-1,2,4-triazol-3-yl]ethyl]carbamate (I-8)

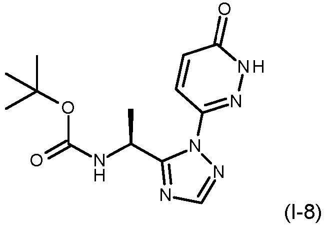

To a solution of tert-butyl N-[(1S)-1-[2-(6-chloropyridazin-3-yl)-1,2,4-triazol-3-yl]ethyl]carbamate (I-7) (4.0 g, 12.32 mmol) and (E)-benzaldehyde oxime (1.99 g, 16.01 mmol) in N,N-dimethylformamide (40 mL) was added cesium carbonate (10.03 g, 30.79 mmol). The reaction mixture was stirred at 100 °C for 2 h, cooled to room temperature and diluted with water. The product was extracted thoroughly with 60% ACN in EtOAc, the combined organic layers washed with brine, dried over sodium sulfate, filtered and concentrated *in vacuo.* The residue was purified by combiflash (EtOAc) to afford tert-butyl N-[(1S)-1-[2-(6-oxo-1H-pyridazin-3-yl)-1,2,4-triazol-3-yl]ethyl]carbamate (I-8) as a gum.

LCMS (method 2): retention time 0.84 min, m/z 305 [M-H]⁻.

¹H NMR (400 MHz, DMSO-*d*₆) δ ppm 13.29 (s, 1 H), 8.14 (s, 1 H), 7.73 (d, 1 H), 7.49 (d, 1 H), 7.13 (d, 1 H), 5.19 (quin, 1 H), 1.42 (d, 3 H), 1.30 (s, 9 H).

### Step C: Preparation of tert-butyl N-[(1S)-1-[2-(1-ethyl-6-oxo-pyridazin-3-yl)-1,2,4-triazol-3-yl]ethyl]carbamate (I-9)

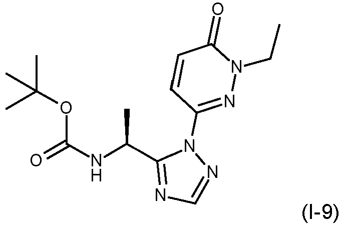

A solution of tert-butyl N-[(1S)-1-[2-(6-oxo-1H-pyridazin-3-yl)-1,2,4-triazol-3-yl]ethyl]carbamate (I-8) (7.15 g, 19.8 mmol), cesium carbonate (6.47 g, 19.8 mmol) and iodoethane (1.74 mL, 21.8 mmol) in acetonitrile (71.5 mL) was stirred at room temperature for 30 hours. The reaction mixture was filtered through a pad of celite and the residue washed with ACN. The filtrate was dried over sodium sulfate and concentrated under reduced pressure. The crude product was purified by combiflash (40-50% gradient EtOAc in cyclohexane) to afford tert-butyl N-[(1S)-1-[2-(1-ethyl-6-oxo-pyridazin-3-yl)-1,2,4-triazol-3-yl]ethyl]carbamate as a solid.

¹H NMR (400 MHz, CDCl₃) δ ppm 7.96 (s, 1 H), 7.85 (d, 1 H), 7.12 (d, 1 H), 5.52 (m, 1 H), 5.44 (m, 1 H), 4.22-4.33 (m, 2 H), 1.61 (d, 3 H), 1.47 (t, 3 H), 1.43 (s, 9 H).

### Step D: Preparation of [(1S)-1-[2-(1-ethyl-6-oxo-pyridazin-3-yl)-1,2,4-triazol-3-yl]ethyl]ammonium;chloride (compound XX^{a2})

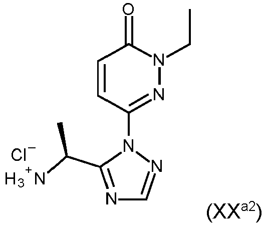

To a solution of tert-butyl N-[(1S)-1-[2-(1-ethyl-6-oxo-pyridazin-3-yl)-1 ,2,4-triazol-3-yl]ethyl]carbamate (I-9) (1.90 g, 5.40 mmol) in CPME (19 mL) was added hydrochloric acid (4M in 1,4-dioxane)(40.0 mL, 160 mmol) and the reaction mixture was stirred at room temperature for 12 hours. Additional hydrochloric acid (4M in 1,4-dioxane, 20 equiv.) was added and stirring continued for 12 hours at room temperature. The mixture was concentrated under reduced pressure to afford [(1S)-1-[2-(1-ethyl-6-oxo-pyridazin-3-yl)-1,2,4-triazol-3-yl]ethyl]ammonium;chloride as a white solid.

¹H NMR (400 MHz, DMSO-*d*₆) δ ppm 8.76 (br s, 3 H), 8.40 (s, 1 H), 7.93 (d, 1 H), 7.24 (d, 1 H), 5.09 (m, 1 H), 4.15 (q, 2 H) 1.64 (d, 3 H), 1.33 (t, 3 H).

### Example PI-3: Preparation of [(1S)-1-[2-(6-oxo-1H-pyridazin-3-yl)-1,2,4-triazol-3-yl]ethyl]ammonium;chloride (compound I-10)

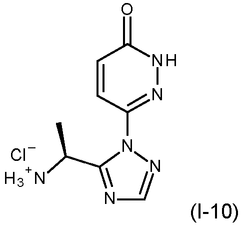

To a solution of tert-butyl N-[(1S)-1-[2-(6-oxo-1H-pyridazin-3-yl)-1 ,2,4-triazol-3-yl]ethyl]carbamate (I-8) (2 g, 5.88 mmol, 90 mass%) in CPME (20 mL) was added hydrochloric acid (4M in dioxane) (2.7 mL) at room temperature and the reaction mixture was stirred for 12 h. After further addition of hydrochloric acid (4M in dioxane) (3.6 mL) stirring was continued for another 12 h. The mixture was concentrated *in vacuo* and dried to afford [(1S)-1-[2-(6-oxo-1H-pyridazin-3-yl)-1,2,4-triazol-3-l]ethyl]ammonium;chloride (I-10) as a white solid.

¹H NMR (400 MHz, DMSO-*d*₆) δ ppm 13.33 (br s, 1 H), 8.78 (br s, 3 H), 8.37 (s, 1 H), 7.88 (d, 1 H), 7.17 (d, 1 H), 4.99 (quin, 1 H), 1.59 (d, 3 H).

### Example PI-4: Preparation of [(1S)-1-[2-(1-methyl-6-oxo-4,5-dihydropyridazin-3-yl)-1,2,4-triazol-3-yl]ethyl]ammonium;chloride (compound XX^{b1})

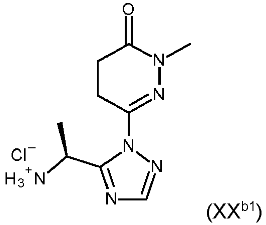

### Step A: Preparation of tert-butyl N-[(1S)-1-[2-(1-methyl-6-oxo-4,5-dihydropyridazin-3-yl)-1,2,4-triazol-3-yl]ethyl]carbamate (I-11)

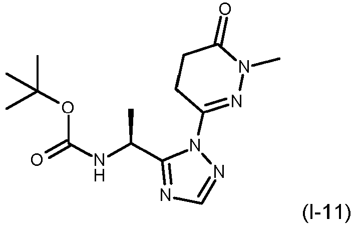

A solution of tert-butyl N-[(1S)-1-[2-(1-methyl-6-oxo-pyridazin-3-yl)-1 ,2,4-triazol-3-yl]ethyl]carbamate (compound I-6, prepared as described above)(300 mg, 0.936 mmol) in methanol (15 mL) was flushed with nitrogen. Palladium on carbon (10%, 199.3 mg) was added and the reaction mixture was stirred under hydrogen atmosphere at room temperature for 36 hours. The mixture was filtered through a pad of celite, the residue washed with methanol and the filtrate concentrated *in vacuo.* The crude product was purified by combiflash (gradient EtOAc in cyclohexane) to afford tert-butyl N-[(1S)-1-[2-(1-methyl-6-oxo-4,5-dihydropyridazin-3-yl)-1,2,4-triazol-3-yl]ethyl]carbamate as a gum. LCMS (method 2): retention time 1.01 min, m/z 323 [M+H]⁺.

¹H NMR (400 MHz, CDCl₃) δ ppm 7.88 (s, 1 H), 5.51 (m, 1 H), 5.43 (m, 1 H), 3.41 (s, 3 H), 3.22 (m, 2 H), 2.74 (m, 2 H), 1.54 (d, 3 H), 1.42 (s, 9 H).

### Step B: Preparation of [(1S)-1-[2-(1-methyl-6-oxo-4,5-dihydropyridazin-3-yl)-1,2,4-triazol-3-yl]ethyl]ammonium;chloride (compound XX^{b1})

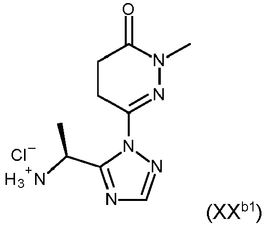

To a solution of tert-butyl N-[(1S)-1-[2-(1-methyl-6-oxo-4,5-dihydropyridazin-3-yl)-1,2,4-triazol-3-yl]ethyl]carbamate (I-11) (2.3 g, 7.1 mmol) in 1,4-dioxane (19 mL) was added hydrochloric acid (4M in 1,4-dioxane)(19 mL, 76 mmol) and the mixture was stirred for 5 hours at room temperature. The mixture was concentrated under reduced pressure and the residue triturated with TBME. The precipitate was filtered and dried to afford [(1S)-1-[2-(1-methyl-6-oxo-4,5-dihydropyridazin-3-yl)-1,2,4-triazol-3-yl]ethyl]ammonium;chloride as a white solid.

LCMS (method 2): retention time 0.23 min, m/z 223 [M+H]⁺ for the free base.

¹H NMR (400 MHz, DMSO-*d*₆) δ ppm 8.79 (br s, 3 H), 8.30 (s, 1 H), 5.06 (m, 1H ), 3.29 (s, 3 H), 3.18 - 3.28 (m, 2 H), 2.69 (m, 2 H), 1.61 (d, 3 H).

### Example PI-5: Preparation of 6-[3-(1-aminoethyl)pyrazin-2-yl]-2-methyl-pyridazin-3-one (compound XX^{c1})

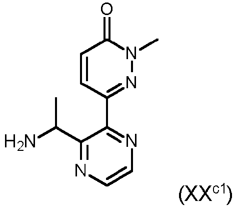

### Step A: Preparation of 6-(3-acetylpyrazin-2-yl)-2-methyl-pyridazin-3-one (I-12)

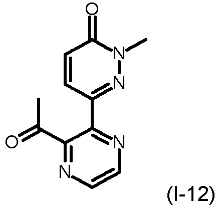

To a stirred solution of 1-(3-chloropyrazin-2-yl)ethanone (CAS 121246-90-0) (3.75 g, 24.0 mmol) in 1,4-dioxane (56.3 mL) was added 2-methyl-6-(4,4,5,5-tetramethyl-1,3,2-dioxaborolan-2-yl)pyridazin-3-one (compound I-16, prepared as described below) (6.79 g, 28.7 mmol), followed by cesium carbonate (23.4 g, 71.9 mmol) and 1,1'-bis(diphenylphosphino)ferrocene-palladium(II)dichloride dichloromethane complex (1.96 g, 2.40 mmol). The reaction mixture was flushed with nitrogen and heated at 120 °C for 1 hour. After cooling to room temperature, the mixture was filtered through a pad of celite and the filtrate diluted with ethyl acetate. The organic layer was washed with water and brine, dried over sodium sulfate, filtered and concentrated under reduced pressure. The residue was purified by combiflash (gradient EtOAc in cyclohexane) to afford 6-(3-acetylpyrazin-2-yl)-2-methyl-pyridazin-3-one as a brown solid. ¹H NMR (400 MHz, CDCl₃) δ ppm 8.67 (d, 1H), 8.58 (d, 1H), 8.03 (d, 1H), 7.06 (d, 1H), 3.80 (s, 3H), 2.72 (s, 3H).

Alternative preparation of 6-(3-acetylpyrazin-2-yl)-2-methyl-pyridazin-3-one (I-12):
To a solution of 6-[3-(1-hydroxyethyl)pyrazin-2-yl]-2-methyl-pyridazin-3-one (compound I-14, prepared as described below) (100 mg, 0.431 mmol) in acetonitrile (8.6 mL) under argon at 0 °C was added Dess-Martin periodinane (CAS 87413-09-0) (188.3 mg, 0.431 mmol). The reaction mixture was stirred at 0°C for 80 minutes, then at room temperature overnight. Additional Dess-Martin periodinane (56.5 mg, 0.129 mmol) was added and stirring continued for another 2 hours at room temperature. The mixture was quenched by addition of sodium thiosulfate, then diluted with ethyl acetate and stirred for 15 minutes at room temperature. The formed white precipitate was filtered off and the organic layer washed with water, aqueous NaHCO₃ and brine, dried over sodium sulfate, filtered and concentrated under reduced pressure. The crude product was purified by combiflash (gradient ethyl acetate in cyclohexane) to afford 6-(3-acetylpyrazin-2-yl)-2-methyl-pyridazin-3-one as a solid. LCMS (method 3): retention time 0.53 min, m/z 231 [M+H]⁺.

### Step B: Preparation of 6-[3-(1-aminoethyl)pyrazin-2-yl]-2-methyl-pyridazin-3-one (compound XX^{c1})

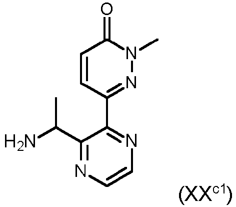

To a stirred solution of 6-(3-acetylpyrazin-2-yl)-2-methyl-pyridazin-3-one (I-12) (1.0 g, 4.34 mmol) in ammonia (7M in methanol, 3.1 mL, 21.7 mmol) at room temperature was added titanium(IV) isopropoxide (2.48 mL, 8.69 mmol) dropwise and the reaction mixture stirred for 16 hours at room temperature. Sodium borohydride (259 mg, 6.52 mmol) was added slowly in portions and stirring continued at room temperature overnight. The mixture was quenched with water and concentrated under reduced pressure. The residue was treated with methanol (10 mL) and stirred for 5 minutes, then filtered through a pad of celite and the filtrate was concentrated *in vacuo.* 2N HCl was added to the residue, the mixture concentrated, the residue was dissolved in methanol and solid sodium carbonate was added. The mixture was stirred for 30 minutes, the methanol layer carefully decanted and concentrated under reduced pressure. The crude product was purified by combiflash (gradient methanol in ethyl acetate) to afford 6-[3-(1-aminoethyl)pyrazin-2-yl]-2-methyl-pyridazin-3-one as a solid. LCMS (method 2): retention time 0.15 min, m/z 232 [M+H]⁺.

¹H NMR (400 MHz, CDCl₃) δ ppm 8.61 (d, 1H), 8.59 (d, 1H), 8.15 (d, 1H), 7.08 (d, 1H), 5.26 (m, 1 H), 3.90 (s, 3H), 1.74 (d, 3H).

### Example PI-6: Alternative preparation of 6-[3-(1-aminoethyl)pyrazin-2-yl]-2-methyl-pyridazin-3-one (compound XX^{c1})

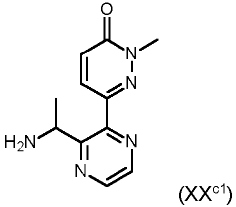

### Step A: Preparation of 1-(3-iodopyrazin-2-yl)ethanol (Int-A)

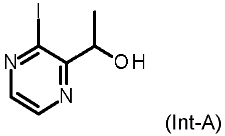

Under an argon atmosphere THF (35 mL) was cooled to 0°C. Then 2,2,6,6-tetramethylpiperidine (5.4 mL, 30.9 mmol, 1.34 equiv.) was added at 0°C followed by a dropwise addition of 2.5M n-BuLi (12 mL, 29.98 mmol, 1.3 equiv.). The reaction mixture was cooled to -78°C, then a solution of 2-iodopyrazine (5.0 g, 23.06 mmol, 1.0 equiv.) in THF (5 mL) was added dropwise. After stirring for 1 hour, acetaldehyde (12 mL, 210 mmol, 9.2 equiv.) was added dropwise at -78°C. After addition, the reaction mixture was allowed to warm up to room temperature before it was quenched with saturated aqueous ammonium chloride solution. The reaction mixture was diluted with water and a mixture of TBME and ethyl acetate. The aqueous layer was acidified with 1M HCl to pH 1-2. The phases were separated and the organic layer was washed with brine, dried over sodium sulfate, filtered and concentrated *in vacuo.* The crude extract was purified by flash chromatography (0-10% ethyl acetate in cyclohexane) to afford 1-(3-iodopyrazin-2-yl)ethanol.

LCMS (method 3): retention time 0.54 min, m/z 251 [M+H]⁺. ¹H NMR (400 MHz, CDCl₃) δ ppm 8.47 (d, 1H), 8.31 (d, 1H), 5.10 (dd, 1H), 3.66-3.73 (m, 1H), 1.52 (d, 3H).

### Step B: Preparation of tert-butyl-[1-(3-iodopyrazin-2-yl)ethoxy]-dimethyl-silane (Int-B)

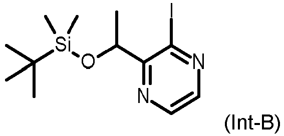

To a solution of 1-(3-iodopyrazin-2-yl)ethanol (Int-A) (1.20 g, 4.80 mmol, 1.0 equiv.) in THF (10 mL) was added imidazole (660 mg, 9.60 mmol, 2.0 equiv.) followed by tert-butyldimethylchlorosilane (1.1 mL, 5.76 mmol, 1.2 equiv.). The resulting reaction mixture was heated to 50°C and was stirred at this temperature for 2 hours before it was allowed to cool down to room temperature. The reaction mixture was filtered. The filtration cake was washed with TBME and the filtrate way concentrated *in vacuo.* The crude extract was purified by flash chromatography (0-3% ethyl acetate in cyclohexane) to afford tert-butyl-[1-(3-iodopyrazin-2-yl)ethoxy]-dimethyl-silane.

LCMS (method 3): retention time 1.30 min, m/z 365 [M+H]⁺. ¹H NMR (400 MHz, CDCl₃) δ ppm 8.52 (d, 1H), 8.24 (d, 1H), 5.31 (q, 1H), 1.51 (d, 3H), 0.88 (s, 9H), 0.07 (s, 3H), 0.05 (s, 3H).

### Step C: Preparation of 6-[3-[1-[tert-butyl(dimethyl)silyl]oxyethyl]pyrazin-2-yl]-2-methyl-pyridazin-3-one (I-13)

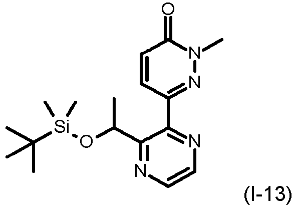

Tert-butyl-[1-(3-iodopyrazin-2-yl)ethoxy]-dimethyl-silane (Int-B, prepared as described above) (1.20 g, 3.29 mmol) was dissolved in dry THF (16.5 mL) in a heat-gun dried RBF under argon. The solution was cooled to -78 °C, then turbo Grignard (1.3 M in THF) (3.3 mL, 4.28 mmol) was added dropwise. The reaction mixture was stirred for 1.5 hours at -78 °C, then zinc chloride was added (1.9 M in 2-methyltetrahydrofuran) (2.3 mL, 4.28 mmol) and stirring continued for 3 hours (resulting solution A). A solution of 6-bromo-2-methyl-pyridazin-3-one (CAS 1123169-25-4) (762 mg, 3.95 mmol), tri(2-furyl)phosphine (96.6 mg, 0.395 mmol) and tris(dibenzylideneacetone) dipalladium(0) (187 mg, 0.198 mmol) in dry THF (16.5 mL) under argon was prepared in a separate vessel and added to solution A at 0 °C. The reaction mixture was heated at 60 °C for 1 hour. After cooling to room temperature, the mixture was diluted with an aqueous saturated solution of ammonium chloride and ethyl acetate. The phases were separated, the aqueous layer extracted with ethyl acetate (3x) and the combined organic layers washed with brine, dried over sodium sulfate, filtered and concentrated under reduced pressure. The residue was purified by combiflash (ethyl acetate in cyclohexane) to afford 6-[3-[1-[tert-butyl(dimethyl)silyl]oxyethyl]pyrazin-2-yl]-2-methyl-pyridazin-3-one as a gum.

LCMS (method 3): retention time 1.05 min, m/z 347 [M+H]⁺.

¹H NMR (400 MHz, MeOD) δ ppm 8.69 (d, 1H), 8.66 (d, 1H), 8.11 (d, 1H), 7.16 (d, 1H), 5.70 (q, 1H), 3.88 (s, 3H), 1.67 (d, 3H), 0.78 (s, 9H), -0.04 (s, 3H), -0.10 (s, 3H).

### Step D: Preparation of 6-[3-(1-hydroxyethyl)pyrazin-2-yl]-2-methyl-pyridazin-3-one (I-14)

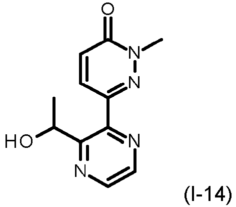

To a solution of 6-[3-[1-[tert-butyl(dimethyl)silyl]oxyethyl]pyrazin-2-yl]-2-methyl-pyridazin-3-one (I-13) (330 mg, 0.629 mmol) in THF (6.29 mL) was added tetrabutylammonium fluoride (1M in THF) (0.94 mL, 0.94 mmol) at 0 °C under argon. The reaction mixture was stirred at room temperature for 1.5 hours, then diluted with brine and ethyl acetate. The phases were separated and the aqueous layer extracted twice with ethyl acetate. The combined organic layers were dried over sodium sulfate, filtered and concentrated under reduced pressure. The residue was purified by combiflash (ethyl acetate in cyclohexane) to afford 6-[3-(1-hydroxyethyl)pyrazin-2-yl]-2-methyl-pyridazin-3-one as an oil. LCMS (method 3): retention time 0.47 min, m/z 233 [M+H]⁺.

¹H NMR (400 MHz, CDCl₃) δ ppm 8.54-8.68 (m, 2H), 8.18 (d, 1H), 7.10 (d, 1H), 5.54 (q, 1H), 3.93 (s, 3H), 1.59 (d, 3H).

### Step E: Preparation of 2-[1-[3-(1-methyl-6-oxo-pyridazin-3-yl)pyrazin-2-yl]ethyl]isoindoline-1,3-dione (I-15)

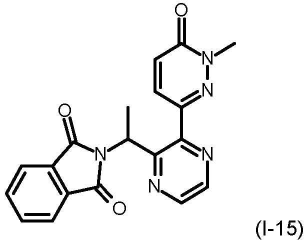

To a solution of 6-[3-(1-hydroxyethyl)pyrazin-2-yl]-2-methyl-pyridazin-3-one (I-14) (150 mg, 0.646 mmol), phthalimide (105.6 mg, 0.71 mmol) and triphenylphosphine (205.3 mg, 0.78 mmol) in THF (1.94 mL) under argon at 0°C was added diisopropyl azodicarboxylate (0.162 mL, 0.7751 mmol). The reaction mixture was allowed to warm to room temperature over 1 hour under stirring, then diluted with water and ethyl acetate. The phases were separated and the aqueous layer extracted with ethyl acetate. The combined organic layers were dried over sodium sulfate, filtered and concentrated under reduced pressure. The residue was purified by combiflash (ethyl acetate in cyclohexane) to afford 2-[1-[3-(1-methyl-6-oxo-pyridazin-3-yl)pyrazin-2-yl]ethyl]isoindoline-1,3-dione as a white foam. LCMS (method 3): retention time 0.81 min, m/z 362 [M+H]⁺.

¹H NMR (400 MHz, CDCl₃) δ ppm 8.61 (d, 1H), 8.55 (d, 1H), 7.84 (d, 1H), 7.79-7.73 (m, 2H), 7.72-7.67 (m, 2H), 6.89 (d, 1H), 6.35 (q, 1H), 3.85 (s, 3H), 1.94 (d, 3H).

### Step F: Preparation of 6-[3-(1-aminoethyl)pyrazin-2-yl]-2-methyl-pyridazin-3-one (compound XX^{c1})

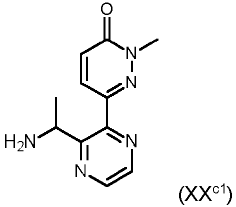

To a suspension of 2-[1-[3-(1-methyl-6-oxo-pyridazin-3-yl)pyrazin-2-yl]ethyl]isoindoline-1,3-dione (I-15) (220 mg, 0.61 mmol) in ethanol (6.09 mL) was added hydrazine hydrate (0.0354 mL, 0.73 mmol) at room temperature. The reaction mixture was stirred overnight at 80 °C, then cooled to room temperature. Few mL of TBME were added and stirring continued for 20 minutes. The white suspension was filtered, and the filtrate evaporated under reduced pressure and dried to afford crude 6-[3-(1-aminoethyl)pyrazin-2-yl]-2-methyl-pyridazin-3-one.

LCMS (method 3): retention time 0.15 min, m/z 232 [M+H]⁺.

### Example PI-7: Preparation of 2-methyl-6-(4,4,5,5-tetramethyl-1,3,2-dioxaborolan-2-yl)pyridazin-3-one (I-16)

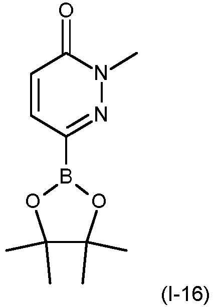

To a stirred solution of 6-chloro-2-methyl-pyridazin-3-one (I-4) (500 mg, 3.4588 mmol) in 1,4-dioxane (15 mL/g) was added 4,4,5,5-tetramethyl-2-(4,4,5,5-tetramethyl-1,3,2-dioxaborolan-2-yl)-1,3,2-dioxaborolane (1.2 equiv., 4.15 mmol), potassium acetate (10.37 mmol), XPhos (0.55 mmol), and palladium(II)acetate (0.38 mmol). The reaction mixture was degassed with nitrogen and then it was heated at 100°C for 3h. The reaction mixture was cooled to room temperature. Progress of the reaction was monitored by LCMS. The reaction mixture was then filtered through a celite bed. Water was added to the filtrate, followed by extraction with EtOAc. The organic layer was washed with brine, dried on sodium sulfate, filtered and concentrated to get the desired compound 2-methyl-6-(4,4,5,5-tetramethyl-1,3,2-dioxaborolan-2-yl)pyridazin-3-one (400 mg) as a brown thick oil which was taken for next step.

¹H NMR (400 MHz, CDCl₃) δ ppm 0.04 (s, 1 H), 1.15 - 1.28 (m, 22 H), 1.33 (s, 12 H), 2.01 (s, 1 H), 3.67 (s, 10 H), 3.85 (s, 3 H), 6.87 (d, J=9.51 Hz, 1 H), 7.27 (s, 1 H), 7.50 (d, J=9.38 Hz, 1 H).

Abbreviations used in synthesis schemes and preparatory examples
- ACN: acetonitrile
- CPME: cyclopentyl methyl ether (or methoxy cyclopentane)
- Boc: t-butoxycarbonyl
- DBU: 1,8-diazabicyclo[5.4.0]undec-7-ene
- DCM: dichloromethane
- DDQ: 2,3-dichloro-5,6-dicyano-1,4-benzoquinone
- DMF: dimethylformamide
- DMSO: dimethyl sulfoxide
- DMSO-*d*₆: deuterated dimethylsulfoxide
- DPEN: diphenylethylenediamine
- Et₃N: triethylamine
- EtOAc: ethyl acetate
- EtOH: ethanol

- HCl: hydrochloric acid
- MeCN: acetonitrile
- MeOH: methanol
- Ms: methanesulfonyl (mesyl)
- n-Bu: n-butyl
- n-BuLi: n-butyllithium
- NaHCO₃: sodium hydrogen carbonate
- NHC: N-heterocyclic carbene
- NPhth: phthalimide-1-yl
- OMs: mesylate group
- OTf: triflate group
- OTs: tosylate group
- PdCl2dppf: 1,1'-bis(diphenylphosphino)ferrocene]palladium(II) dichloride
- TBME: tert-butyl methyl ether
- TEA: triethylamine
- TEMPO: (2,2,6,6-tetramethylpiperidin-1-yl)oxidanyl
- Tf: trifluoromethanesulfonyl (triflyl)
- TFA: trifluoroacetic acid
- THF: tetrahydrofuran
- Ts: p-toluenesulfonyl (tosyl)
- XPhos: 2-dicyclohexylphosphino-2',4',6'-triisopropylbiphenyl
- aq.: aqueous
- °C: degrees Celsius
- equiv.: equivalent
- h: hour(s)
- LC/MS or LCMS: liquid chromatography mass spectrometry
- M: molar
- MHz: megahertz
- min: minutes
- mp or M.P.: melting point
- NMR: nuclear magnetic resonance
- ppm: parts per million
- RT: room temperature
- Rt: retention time
- RBF: round-bottom flask
- TLC: thin layer chromatography

### Biological examples

The Examples which follow serve to illustrate the invention. Certain compounds of the invention can be distinguished from known compounds by virtue of greater efficacy at low application rates, which can be verified by the person skilled in the art using the experimental procedures outlined in the Examples, using lower application rates if necessary, for example 50 ppm, 24 ppm, 12.5 ppm, 6 ppm, 3 ppm, 1.5 ppm, 0.8 ppm or 0.2 ppm.

### Example B1: Activity against Chilo suppressalis (Striped rice stemborer)

24-well microtiter plates with artificial diet were treated with aqueous test solutions prepared from 10'000 ppm DMSO stock solutions by pipetting. After drying, the plates were infested with L2 larvae (6-8 per well). The samples were assessed for mortality, anti-feeding effect, and growth inhibition in comparison to untreated samples 6 days after infestation. Control of *Chilo suppressalis* by a test sample is given when at least one of the categories mortality, anti-feedant effect, and growth inhibition is higher than the untreated sample.

The following compounds resulted in at least 80% control in at least one of the three categories (mortality, anti-feedant effect, or growth inhibition) at an application rate of 200 ppm: P1, P2, P3, P4, P6, P7, P8, P9, P10, P11, P12, P13, P15, P16, P17, P19, P21, P22, P23, P24, P25, P26, P27.

### Example B2: Activity against Diabrotica balteata (Corn root worm)

Maize sprouts placed onto an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 80% control in at least one of the two categories (mortality or growth inhibition) at an application rate of 200 ppm: P2, P3, P4, P6, P7, P8, P9, P10, P12, P13, P14, P15, P16, P17, P19, P21, P22, P23, P24, P25, P26, P27.

### Example B3: Activity against Euschistus heros (Neotropical Brown Stink Buq)

Soybean leaves on agar in 24-well microtiter plates were sprayed with aqueous test solutions prepared from 10'000 ppm DMSO stock solutions. After drying the leaves were infested with N2 nymphs. The samples were assessed for mortality and growth inhibition in comparison to untreated samples 5 days after infestation.

The following compounds gave an effect of at least 80% in at least one of the two categories (mortality or growth inhibition) at an application rate of 200 ppm: P1, P2, P3, P5, P6, P7, P8, P9, P10, P11, P12, P13, P14, P15, P16, P17, P19, P21, P22, P23, P24, P25, P26, P27.

### Example B4: Activity against Frankliniella occidentalis (Western flower thrips). Feeding/contact activity

Sunflower leaf discs were placed on agar in 24-well microtiter plates and sprayed with aqueous test solutions prepared from 10'000 DMSO stock solutions. After drying the leaf discs were infested with a Frankliniella population of mixed ages. The samples were assessed for mortality 7 days after infestation.

The following compounds resulted in at least 80% mortality at an application rate of 200 ppm: P5.

### Example B5: Activity against Myzus persicae (Green peach aphid). Feeding/Contact activity

Sunflower leaf discs were placed onto agar in a 24-well microtiter plate and sprayed with aqueous test solutions prepared from 10'000 ppm DMSO stock solutions. After drying, the leaf discs were infested with an aphid population of mixed ages. The samples were assessed for mortality 6 days after infestation.

The following compounds resulted in at least 80% mortality at an application rate of 200 ppm: P2, P3, P4, P6, P7, P8, P9, P10, P12, P13, P14, P15, P17, P19, P20, P21, P22, P24, P27.

### Example B6: Activity against Myzus persicae (Green peach aphid). Intrinsic activity

Test compounds prepared from 10'000 ppm DMSO stock solutions were applied by pipette into 24-well microtiter plates and mixed with sucrose solution. The plates were closed with a stretched Parafilm. A plastic stencil with 24 holes was placed onto the plate and infested pea seedlings were placed directly on the Parafilm. The infested plate was closed with a gel blotting paper and another plastic stencil and then turned upside down. The samples were assessed for mortality 5 days after infestation.

The following compounds resulted in at least 80% mortality at a test rate of 12 ppm: P3, P6, P7, P8, P9, P10, P11, P12, P13, P14, P15, P16, P17, P19, P21, P22, P23, P24, P25, P26, P27.

### Example B7: Activity against Plutella xylostella (Diamond back moth)

24-well microtiter plates with artificial diet were treated with aqueous test solutions prepared from 10'000 ppm DMSO stock solutions by pipetting. After drying, *Plutella* eggs were pipetted through a plastic stencil onto a gel blotting paper and the plate was closed with it. The samples were assessed for mortality and growth inhibition in comparison to untreated samples 8 days after infestation.

The following compounds gave an effect of at least 80% control in at least one of the two categories (mortality or growth inhibition) at an application rate of 200 ppm: P1, P2, P3, P4, P6, P7, P8, P9, P10, P11, P12, P13, P14, P15, P16, P17, P18, P19, P20, P21, P22, P23, P24, P25, P26, P27.

### Example B8: Activity against Spodoptera littoralis (Eqyptian cotton leaf worm)

Cotton leaf discs were placed onto agar in 24-well microtiter plates and sprayed with aqueous test solutions prepared from 10'000 ppm DMSO stock solutions. After drying the leaf discs were infested with five L1 larvae. The samples were assessed for mortality, anti-feeding effect, and growth inhibition in comparison to untreated samples 3 days after infestation. Control of *Spodoptera littoralis* by a test sample is given when at least one of the categories mortality, anti-feedant effect, and growth inhibition is higher than the untreated sample.

The following compounds resulted in at least 80% control in at least one of the three categories (mortality, anti-feedant effect, or growth inhibition) at an application rate of 200 ppm: P2, P3, P6, P7, P8, P9, P10, P11, P12, P13, P14, P15, P16, P17, P19, P21, P22, P23, P24, P25, P26, P27.

### Example B9: Activity against Tetranychus urticae (Two-spotted spider mite). Feeding/contact activity

Bean leaf discs on agar in 24-well microtiter plates were sprayed with aqueous test solutions prepared from 10'000 ppm DMSO stock solutions. After drying the leaf discs were infested with a mite population of mixed ages. The samples were assessed for mortality on mixed population (mobile stages) 8 days after infestation.

The following compounds resulted in at least 80% mortality at an application rate of 200 ppm: P6, P27.

## Claims

1. A compound of the formula (I) 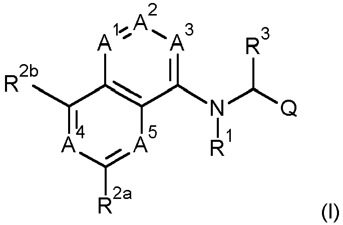 wherein:
A¹, A² and A³ are, independently from each other, N or CR^{Y}; or
A¹=A²-A³, taken together, are NR-C(=X⁰)-N; wherein X° is O or S;
A⁴ and A⁵ are, independently from each other, N or CR^{Y};
Q is 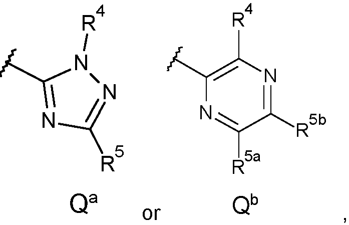 where the staggered line represents the connection of Q to the rest of compound of the formula (I);
R is hydrogen, C₁-C₃alkyl, C₁-C₃haloalkyl, C₁-C₃alkoxy, or C₁-C₃haloalkoxy;
R¹ is hydrogen, C₁-C₆alkyl, C₁-C₆cyanoalkyl, aminocarbonylC₁-C₆alkyl, hydroxycarbonylC₁-C₆alkyl, C₁-C₆nitroalkyl, trimethylsilaneC₁-C₆alkyl, C₁-C₃alkoxy-C₁-C₆alkyl, C₁-C₆haloalkyl, C₂-C₆alkenyl, C₂-C₆haloalkenyl, C₂-C₆alkynyl, C₂-C₆haloalkynyl, C₃-C₄cycloalkylC₁-C₂alkyl-, C₃-C₄cycloalkylC₁-C₂alkyl- wherein the C₃-C₄cycloalkyl group is substituted with 1 or 2 halogen atoms, oxetan-3-yl-CH₂-, C₁-C₆alkylcarbonyl, C₁-C₆alkoxycarbonyl, phenyloxycarbonyl, benzyloxycarbonyl, benzyl or benzyl substituted with 1 to 3 substituents independently selected from halogen, C₁-C₆alkoxy and C₁-C₆haloalkyl;
R^{2a} and R^{2b} are each independently selected from hydrogen, C₁-C₃alkyl, C₁-C₃haloalkyl, C₁-C₃haloalkylsulfanyl, C₁-C₃alkoxy, C₁-C₃haloalkoxy, halogen, NO₂, SF₅, CN, C(O)NH₂, C(O)OH, C(S)NH₂, C₃-C₆cycloalkyl, C₃-C₆cycloalkyl substituted with one to three substituents independently selected from R^{x}, C₃-C₆cycloalkylcarbonyl, phenyl, phenyl substituted with one to three substituents independently selected from R^{x}, heteroaryl, heteroaryl substituted with one to three substituents independently selected from R^{x}, OR⁶, piperidin-2-one-1-yl, piperidin-2-one-1-yl substituted with one to two substituents independently selected from R^{x}, pyridin-2-one-1-yl, pyridin-2-one-1-yl substituted with one to two substituents independently selected from R^{x}, azetidin-1-yl, azetidin-1-yl substituted with one to two substituents independently selected from R^{x}, pyrrolidin-1-yl, pyrrolidin-1-yl substituted with one to two substituents independently selected from R^{x}, C₃-C₆cycloalkylC₁-C₄alkyl, C₃-C₆cycloalkylC₁-C₄alkyl substituted with one to two substituents independently selected from R^{z}, C₃-C₆cycloalkylC₁-C₃alkoxy, C₃-C₆cycloalkylC₁-C₃alkoxy substituted with one to two substituents independently selected from R^{x}, C₁-C₅cyanoalkyl, C₁-C₅cyanoalkoxy, C₁-C₄alkylsulfanyl, C₁-C₄alkylsulfanyl substituted with one to three substituents independently selected from R^{x}, C₁-C₄alkylsulfonyl, C₁-C₄alkylsulfonyl substituted with one to three substituents independently selected from R^{x}, C₁-C₄alkylsulfinyl, and C₁-C₄alkylsulfinyl substituted with one to three substituents independently selected from R^{x};
R³ is C₁-C₃alkyl or C₁-C₃haloalkyl;
R⁴ is 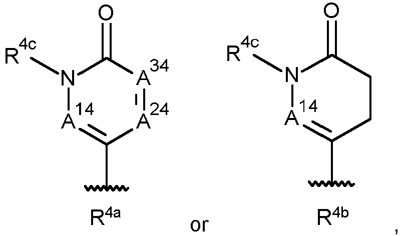 where the staggered line represents the connection of R⁴ to Q^{a} or Q^{b};
A¹⁴, A²⁴, and A³⁴ are, independently of each other, N or CH;
R^{4c} is C₁-C₃alkyl, C₁-C₃haloalkyl, allyl, propargyl, or C₃-C₆cycloalkylC₁-C₄alkyl;
R⁵ is hydrogen, C₁-C₃alkyl, C₁-C₃haloalkyl, C₃-C₄cycloalkyl, C₁-C₃alkoxy, C₃-C₄alkoxyC(O)-, (C₁-C₃alkoxy)₂CH-, halogen, CN, NH₂C(O), amino (i.e NH₂), (C₁-C₃alkyl)amino, di(C₁-C₃alkyl)amino, hydroxy, C₃-C₄halocycloalkyl, C₃-C₄cyanocycloalkyl, C₂-C₆alkenyl, C₂-C₆haloalkenyl, C₂-C₆alkynyl, C₂-C₆haloalkynyl, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl, C₁-C₄alkylsulfonyl, C₁-C₃alkoxy-C₁-C₃alkyl, C₁-C₃alkoxy-C₁-C₃alkoxy-C₁-C₃alkyl, (C₁-C₃alkyl)sulfonylamino, (C₁-C₃alkyl)sulfonyl(C₁-C₃alkyl)amino, (C₁-C₃alkyl)NHC(O), (C₁-C₃alkyl)₂NC(O), (C₁-C₃cycloalkyl)NHC(O), (C₁-C₃cycloalkyl)(C₁-C₃alkyl)NC(O), (C₁-C₃alkyl)C(O)(C₁-C₃alkyl)N, (C₁-C₃alkyl)C(O)NH, (C₁-C₃alkyl)C(O), (C₁-C₃alkoxy)C(O), HC(O), diphenylmethanimine, C₁-C₃haloalkoxy, phenyl, or a 5-membered heteroaromatic ring; or
R⁵ is phenyl substituted with one to three substituents selected from C₁-C₃alkyl, C₁-C₃haloalkyl, C₁-C₃alkoxy, C₃-C₄cycloalkyl, halogen, CN and hydroxyl; or
R⁵ is a 5-membered heteroaromatic ring substituted with one to three substituents selected from C₁-C₃alkyl, C₁-C₃haloalkyl, C₁-C₃alkoxy, C₃-C₄cycloalkyl, halogen, CN and hydroxyl;
R^{5a} and R^{5b} are, independently of each other, selected from hydrogen, halogen, CN, C₁-C₃alkyl, C₁-C₃haloalkyl, C₃-C₄cycloalkyl, C₁-C₃alkoxy, and C₁-C₃haloalkoxy;
R⁶ is phenyl, benzyl, heteroaryl, or C₃-C₆ cycloalkyl; or
R⁶ is phenyl, benzyl, heteroaryl, or C₃-C₆ cycloalkyl, each of which, independent of each other, is substituted with one to three substituents independently selected from R^{x},
R^{x} is independently selected from halogen, C₁-C₃alkyl, C₁-C₃haloalkyl, C₁-C₃alkoxy, C₁-C₃haloalkoxy, NO₂, SF₅, CN, C(O)NH₂, C(S)NH₂, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl and C₁-C₄alkylsulfonyl;
R^{Y} is selected from hydrogen, C₁-C₃ alkyl, C₁-C₃ haloalkyl, hydroxy, C₁-C₃ alkoxy, C₁-C₃ haloalkoxy, halogen, CN and cyclopropyl; and
R^{Z} is selected from oxo, halogen, C₁-C₃ alkyl, C₁-C₃haloalkyl, C₁-C₃alkoxy, C₁-C₃haloalkoxy and CN;
or an agrochemically acceptable salt, stereoisomer, enantiomer, tautomer and N-oxide of the compound of formula I.

2. The compound according to claim 1, wherein A¹ and A³ are N, and A² is CR^{Y}; and R^{Y} is hydrogen, chlorine, methyl, trifluoromethyl, or methoxy; or
wherein A¹=A²-A³, taken together, are NR-C(=O)-N; and R is hydrogen, methyl, ethyl, 2,2-difluoroethyl, or 2,2,2,-trifluoroethyl.

3. The compound according to claim 1 or claim 2, wherein A⁴ and A⁵ are, independently from each other, N or CH; or
wherein A⁴ and A⁵ are both CH.

4. The compound according to any one of claims 1 to 3, wherein
- when R⁴ is R^{4a}: A¹⁴ is N, A²⁴ is N or CH, and A³⁴ is N or CH; or A¹⁴ is N, A²⁴ is N or CH, and A³⁴ is CH; or A¹⁴ is N, A²⁴ is CH, and A³⁴ is N or CH; or A¹⁴ is N, A²⁴ is CH, and A³⁴ is CH; and
- when R⁴ is R^{4b}: A¹⁴ is N; or A¹⁴ is CH;
and wherein R^{4c} is methyl, ethyl, propyl, difluoromethyl, trifluoromethyl, 2,2-difluoroethyl, 2,2,2-trifluoroethyl, allyl, propargyl, cyclopropylmethyl, cyclobutylmethyl, 2-cyclpropylethyl, or 3-cyclopropylpropyl.

5. The compound according to any one of claims 1 to 4, wherein:
- A¹⁴ is N, and, when R⁴ is R^{4a}, A²⁴ and A³⁴ are CH; and
- R^{4c} is methyl, or ethyl.

6. The compound according to any one of claims 1 to 5, wherein X⁰ is O.

7. The compound according to any one of claims 1 to 6, wherein R¹ is hydrogen, methyl, ethyl, cyanomethyl, methoxymethyl, cyclopropyl-methyl, allyl, propargyl, benzyloxycarbonyl, or benzyl.

8. The compound according to any one of claims 1 to 7, wherein R^{2a} is halogen, C₁-C₃haloalkyl, C₁-C₃haloalkylsulfanyl, C₁-C₃haloalkoxy, C₃-C₆cycloalkyl optionally substituted with one or two substituents independently selected from C₁-C₃haloalkyl, cyano and halogen, C₃-C₆cycloalkylC₁-C₄alkyl optionally substituted with one to three substituents independently selected from C₁-C₃haloalkyl, cyano and halogen, C₁-C₅cyanoalkyl, C₁-C₄alkylsulfonyl, C₁-C₄haloalkylsulfonyl, C₁-C₄alkylsulfinyl, C₁-C₄haloalkylsulfinyl, C₃-C₆cycloalkylsulfanyl, C₃-C₆cycloalkylsulfinyl, or C₃-C₆cycloalkylsulfonyl; and
wherein R^{2b} is halogen, C₁-C₃haloalkyl, C₁-C₃haloalkylsulfanyl, C₁-C₃haloalkylsulfonyl, C₁-C₃alkoxy, C₁-C₃haloalkoxy, or CN.

9. The compound according to any one of claims 1 to 8, wherein R^{2a} and R^{2b} are independently selected from chloro, bromo, iodo, and trifluoromethyl.

10. The compound according to any one of claims 1 to 9, wherein R³ is methyl.

11. The compound according to any one of claims 1 to 10, wherein :
- when Q is Q^{a}: R⁵ is hydrogen, methyl, trifluoromethoxy, methoxy, cyclopropyl, 2,2-difluoroethoxy, 2,2,2-trifluoroethoxy, difluoromethoxy, 2,2,2-trifluoroethyl, chloro, bromo, methoxyethoxy, methylcarbonyl or methoxycarbonyl; and
- when Q is Q^{b}: R^{5a} is hydrogen, halogen, CN, C₁-C₃alkyl, C₁-C₃haloalkyl, C₃-C₄cycloalkyl, C₁-C₃alkoxy or C₁-C₃haloalkoxy; and R^{5b} is hydrogen, halogen, CN, C₁-C₃haloalkyl, C₃-C₄cycloalkyl, C₁-C₃alkoxy or C₁-C₃haloalkoxy.

12. A composition comprising a compound as defined in any one of claims 1 to 11, one or more auxiliaries and diluent, and optionally one or more other active ingredient.

13. A method
(i) of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound as defined in any one of claims 1 to 11, or a composition as defined in claim 12; excluding methods for the treatment of the human or animal body by surgery or therapy; or
(ii) for the protection of plant propagation material from the attack by insects, acarines, nematodes or molluscs, which comprises treating the propagation material or the site, where the propagation material is planted, with an effective amount of a compound as defined in any one of claims 1 to 11, or a composition as defined in claim 12; or
(iii) of controlling parasites in or on an animal in need thereof comprising administering an effective amount of a compound as defined in any one of claims 1 to 11, or a composition as defined in claim 12; excluding methods for the treatment of the human or animal body by surgery or therapy.

14. A plant propagation material, such as a seed, comprising, or adhered thereto, a compound as defined in any one of claims 1 to 11, or a composition as defined in claim 12.

## Patentansprüche

1. Verbindung der Formel (I) 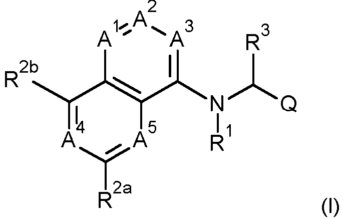 wobei:
A¹, A² und A³ unabhängig voneinander für N oder CR^{Y} stehen;
oder
A¹=A²-A³ zusammengenommen für NR-C(=X⁰)-N stehen; wobei X⁰ für O oder S steht;
A⁴ und A⁵ unabhängig voneinander für N oder CR^{Y} stehen;
Q für 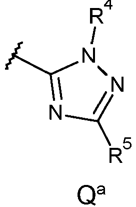 oder 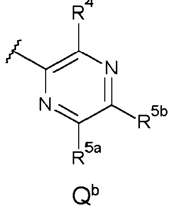 steht, wobei die Wellenlinie die Verbindung von Q mit dem Rest der Verbindung der Formel (I) darstellt;
R für Wasserstoff, C₁-C₃-Alkyl, C₁-C₃-Halogenalkyl, C₁-C₃-Alkoxy oder C₁-C₃-Halogenalkoxy steht;
R¹ für Wasserstoff, C₁-C₆-Alkyl, C₁-C₆-Cyanoalkyl, Aminocarbonyl-C₁-C₆-alkyl, Hydroxycarbonyl-C₁-C₆-alkyl, C₁-C₆-Nitroalkyl, Trimethylsilan-C₁-C₆-alkyl, C₁-C₃-Alkoxy-C₁-C₆alkyl, C₁-C₆-Halogenalkyl, C₂-C₆-Alkenyl, C₂-C₆-Halogenalkyl, C₂-C₆-Alkinyl, C₂-C₆-Halogenalkinyl, C₃-C₄-Cycloalkyl-C₁-C₂-alkyl-, C₃-C₄-Cycloalkyl-C₁-C₂-alkyl-, wobei die C₃-C₄-Cycloalkylgruppe durch 1 oder 2 Halogenatome substituiert ist, Oxetan-3-yl-CH₂-, C₁-C₆-Alkylcarbonyl, C₁-C₆-Alkoxycarbonyl, Phenyloxycarbonyl, Benzyloxycarbonyl, Benzyl oder Benzyl, das durch 1 bis 3 Substituenten, die unabhängig aus Halogen, C₁-C₆-Alkoxy und C₁-C₆-Halogenalkyl ausgewählt sind, substituiert ist, steht;
R^{2a} und R^{2b} jeweils unabhängig aus Wasserstoff, C₁-C₃-Alkyl, C₁-C₃-Halogenalkyl, C₁-C₃-Halogenalkylsulfanyl, C₁-C₃-Alkoxy, C₁-C₃-Halogenalkoxy, Halogen, NO₂, SF₅, CN, C(O)NH₂, C(O)OH, C(S)NH₂, C₃-C₆-Cycloalkyl, C₃-C₆-Cycloalkyl, das durch einen bis drei Substituenten, die unabhängig aus R^{x} ausgewählt sind, substituiert ist, C₃-C₆-Cycloalkylcarbonyl, Phenyl, Phenyl, das durch einen bis drei Substituenten, die unabhängig aus R^{x} ausgewählt sind, substituiert ist, Heteroaryl, Heteroaryl, das durch einen bis drei Substituenten, die unabhängig aus R^{x} ausgewählt sind, substituiert ist, OR⁶, Piperidin-2-on-1-yl, Piperidin-2-on-1-yl, das durch einen bis zwei Substituenten, die unabhängig aus R^{x} ausgewählt sind, substituiert ist, Pyridin-2-on-1-yl, Pyridin-2-on-1-yl, das durch einen bis zwei Substituenten, die unabhängig aus R^{x} ausgewählt sind, substituiert ist, Azetidin-1-yl, Azetidin-1-yl, das durch einen bis zwei Substituenten, die unabhängig aus R^{x} ausgewählt sind, substituiert ist, Pyrrolidin-1-yl, Pyrrolidin-1-yl, das durch einen bis zwei Substituenten, die unabhängig aus R^{x} ausgewählt sind, substituiert ist, C₃-C₆-Cycloalkyl-C₁-C₄-alkyl, C₃-C₆-Cycloalkyl-C₁-C₄-alkyl, das durch einen bis zwei Substituenten, die unabhängig aus R^{z} ausgewählt sind, substituiert ist, C₃-C₆-Cycloalkyl-C₁-C₃-alkoxy, C₃-C₆-Cycloalkyl-C₁-C₃-alkoxy, das durch einen bis zwei Substituenten, die unabhängig aus R^{x} ausgewählt sind, substituiert ist, C₁-C₅-Cyanoalkyl, C₁-C₃-Cyanoalkoxy, C₁-C₄-Alkylsulfanyl, C₁-C₄-Alkylsulfanyl, das durch einen bis drei Substituenten, die unabhängig aus R^{x} ausgewählt sind, substituiert ist, C₁-C₄-Alkylsulfonyl, C₁-C₄-Alkylsulfonyl, das durch einen bis drei Substituenten, die unabhängig aus R^{x} ausgewählt sind, substituiert ist, C₁-C₄-Alkylsulfinyl und C₁-C₄-Alkylsulfinyl, das durch einen bis drei Substituenten, die unabhängig aus R^{x} ausgewählt sind, substituiert ist, ausgewählt sind;
R³ für C₁-C₃-Alkyl oder C₁-C₃-Halogenalkyl steht;
R⁴ für 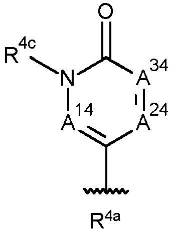 oder 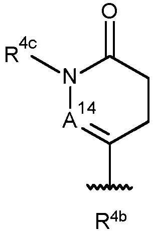 steht, wobei die Wellenlinie die Verbindung von R⁴ mit Q^{a} bzw. Q^{b} darstellt;
A¹⁴, A²⁴ und A³⁴ unabhängig voneinander für N oder CH stehen;
R^{4c} für C₁-C₃-Alkyl, C₁-C₃-Halogenalkyl, Allyl, Propargyl oder C₃-C₆-Cycloalkyl-C₁-C₄-alkyl steht;
R⁵ für Wasserstoff, C₁-C₃-Alkyl, C₁-C₃-Halogenalkyl, C₃-C₄-Cycloalkyl, C₁-C₃-Alkoxy, C₃-C₄-Alkoxy-C(O)-, (C₁-C₃-Alkoxy)₂CH-, Halogen, CN, NH₂C(O), Amino (d. h. NH₂), (C₁-C₃-Alkyl) amino, Di (C₁-C₃-alkyl) amino, Hydroxy, C₃-C₄-Halogencycloalkyl, C₃-C₄-Cyanocycloalkyl, C₂-C₆-Alkenyl, C₂-C₆-Halogenalkenyl, C₂-C₆-Alkinyl, C₂-C₆-Halogenalkinyl, C₁-C₄-Halogenalkylsulfanyl, C₁-C₄-Halogenalkylsulfinyl, C₁-C₄-Halogenalkylsulfonyl, C₁-C₄-Alkylsulfanyl, C₁-C₄-Alkylsulfinyl, C₁-C₄-Alkylsulfonyl, C₁-C₃-Alkoxy-C₁-C₃-alkyl, C₁-C₃-Alkoxy-C₁-C₃-alkoxy-C₁-C₃-alkyl, (C₁-C₃-Alkyl) sulfonylamino, (C₁-C₃-Alkyl) sulfonyl (C₁-C₃-alkyl) amino, (C₁-C₃-Alkyl)NHC(O), (C₁-C₃-Alkyl)₂NC(O), (C₁-C₃-Cycloalkyl)NHC(O), (C₁-C₃-Cycloalkyl)(C₁-C₃-alkyl)NC(O), (C₁-C₃-Alkyl)C(O)(C₁-C₃-alkyl)N, (C₁-C₃-Alkyl)C(O)NH, (C₁-C₃-Alkyl)C(O), (C₁-C₃-Alkoxy)C(O), HC(O), Diphenylmethanimin, C₁-C₃-Halogenalkoxy, Phenyl oder einen 5-gliedrigen heteroaromatischen Ring steht; oder
R⁵ für Phenyl steht, das durch einen bis drei Substituenten, die aus C₁-C₃-Alkyl, C₁-C₃-Halogenalkyl, C₁-C₃-Alkoxy, C₃-C₄-Cycloalkyl, Halogen, CN und Hydroxyl ausgewählt sind, substituiert ist; oder
R⁵ für einen 5-gliedrigen heteroaromatischen Ring steht, der durch einen bis drei Substituenten, die aus C₁-C₃-Alkyl, C₁-C₃-Halogenalkyl, C₁-C₃-Alkoxy, C₃-C₄-Cycloalkyl, Halogen, CN und Hydroxyl ausgewählt sind, substituiert ist;
R^{5a} und R^{5b} unabhängig voneinander aus Wasserstoff, Halogen, CN, C₁-C₃-Alkyl, C₁-C₃-Halogenalkyl, C₃-C₄-Cycloalkyl, C₁-C₃-Alkoxy und C₁-C₃-Halogenalkoxy ausgewählt sind;
R⁶ für Phenyl, Benzyl, Heteroaryl oder C₃-C₆-Cycloalkyl steht; oder
R⁶ für Phenyl, Benzyl, Heteroaryl oder C₃-C₆-Cycloalkyl steht, wobei jede dieser Gruppen unabhängig voneinander durch einen bis drei Substituenten, die unabhängig aus R^{x} ausgewählt sind, substituiert ist;
R^{x} unabhängig aus Halogen, C₁-C₃-Alkyl, C₁-C₃-Halogenalkyl, C₁-C₃-Alkoxy, C₁-C₃-Halogenalkoxy, NO₂, SF₅, CN, C(O)NH₂, C(S)NH₂, C₁-C₄-Halogenalkylsulfanyl, C₁-C₄-Halogenalkylsulfinyl, C₁-C₄-Halogenalkylsulfonyl, C₁-C₄-Alkylsulfanyl, C₁-C₄-Alkylsulfinyl und C₁-C₄-Alkylsulfonyl ausgewählt ist;
R^{Y} aus Wasserstoff, C₁-C₃-Alkyl, C₁-C₃-Halogenalkyl, Hydroxy, C₁-C₃-Alkoxy, C₁-C₃-Halogenalkoxy, Halogen, CN und Cyclopropyl ausgewählt ist; und
R^{z} aus Oxo, Halogen, C₁-C₃-Alkyl, C₁-C₃-Halogenalkyl, C₁-C₃-Alkoxy, C₁-C₃-Halogenalkoxy und CN ausgewählt ist;
oder ein agrochemisch unbedenkliches Salz, Stereoisomer, Enantiomer, Tautomer und N-Oxid der Verbindung der Formel I.

2. Verbindung nach Anspruch 1, wobei A¹ und A³ für N stehen und A² für CR^{Y} steht; und R^{Y} für Wasserstoff, Chlor, Methyl, Trifluormethyl oder Methoxy steht; oder
wobei A¹=A²-A³ zusammengenommen für NR-C(=O)-N stehen und R für Wasserstoff, Methyl, Ethyl, 2,2-Difluorethyl oder 2,2,2-Trifluorethyl steht.

3. Verbindung nach Anspruch 1 oder Anspruch 2, wobei A⁴ und A⁵ unabhängig voneinander für N oder CH stehen; oder wobei A⁴ und A⁵ beide für CH stehen.

4. Verbindung nach einem der Ansprüche 1 bis 3, wobei
- dann, wenn R⁴ für R^{4a} steht: A¹⁴ für N steht, A²⁴ für N oder CH steht und A³⁴ für N oder CH steht; oder A¹⁴ für N steht, A²⁴ für N oder CH steht und A³⁴ für CH steht; oder A¹⁴ für N steht, A²⁴ für CH steht und A³⁴ für N oder CH steht; oder A¹⁴ für N steht, A²⁴ für CH steht und A³⁴ für CH steht; und
- dann, wenn R⁴ für R^{4b} steht: A¹⁴ für N steht; oder A¹⁴ für CH steht;
und wobei R^{4c} für Methyl, Ethyl, Propyl, Difluormethyl, Trifluormethyl, 2,2-Difluorethyl, 2,2,2-Trifluorethyl, Allyl, Propargyl, Cyclopropylmethyl, Cyclobutylmethyl, 2-Cyclpropylethyl oder 3-Cyclopropylpropyl steht.

5. Verbindung nach einem der Ansprüche 1 bis 4, wobei:
- A¹⁴ für N steht und dann, wenn R⁴ für R^{4a} steht, A²⁴ und A³⁴ für CH stehen; und
- R^{4c} für Methyl oder Ethyl steht.

6. Verbindung nach einem der Ansprüche 1 bis 5, wobei X⁰ für O steht.

7. Verbindung nach einem der Ansprüche 1 bis 6, wobei R¹ für Wasserstoff, Methyl, Ethyl, Cyanomethyl, Methoxymethyl, Cyclopropylmethyl, Allyl, Propargyl, Benzyloxycarbonyl oder Benzyl steht.

8. Verbindung nach einem der Ansprüche 1 bis 7, wobei R^{2a} für Halogen, C₁-C₃-Halogenalkyl, C₁-C₃-Halogenalkylsulfanyl, C₁-C₃-Halogenalkoxy, C₃-C₆-Cycloalkyl, das gegebenenfalls durch einen oder zwei Substituenten, die unabhängig aus C₁-C₃-Halogenalkyl, Cyano und Halogen ausgewählt sind, substituiert ist, C₃-C₆-Cycloalkyl-C₁-C₄-alkyl, das gegebenenfalls durch einen bis drei Substituenten, die unabhängig aus C₁-C₃-Halogenalkyl, Cyano und Halogen ausgewählt sind, substituiert ist, C₁-C₅-Cyanoalkyl, C₁-C₄-Alkylsulfonyl, C₁-C₄-Halogenalkylsulfonyl, C₁-C₄-Alkylsulfinyl, C₁-C₄-Halogenalkylsulfinyl, C₃-C₆-Cycloalkylsulfanyl, C₃-C₆-Cycloalkylsulfinyl oder C₃-C₆Cycloalkylsulfonyl steht; und
wobei R^{2b} für Halogen, C₁-C₃-Halogenalkyl, C₁-C₃-Halogenalkylsulfanyl, C₁-C₃-Halogenalkylsulfonyl, C₁-C₃-Alkoxy, C₁-C₃-Halogenalkoxy oder CN steht.

9. Verbindung nach einem der Ansprüche 1 bis 8, wobei R^{2a} und R^{2b} unabhängig aus Chlor, Brom, Iod und Trifluormethyl ausgewählt sind.

10. Verbindung nach einem der Ansprüche 1 bis 9, wobei R³ für Methyl steht.

11. Verbindung nach einem der Ansprüche 1 bis 10, wobei:
- dann, wenn Q für Q^{a} steht: R⁵ für Wasserstoff, Methyl, Trifluormethoxy, Methoxy, Cyclopropyl, 2,2-Difluorethoxy, 2,2,2-Trifluorethoxy, Difluormethoxy, 2,2,2-Trifluorethyl, Chlor, Brom, Methoxyethoxy, Methylcarbonyl oder Methoxycarbonyl steht; und
- dann, wenn Q für Q^{b} steht: R^{5a} für Wasserstoff, Halogen, CN, C₁-C₃-Alkyl, C₁-C₃-Halogenalkyl, C₃-C₄-Cycloalkyl, C₁-C₃-Alkoxy oder C₁-C₃-Halogenalkoxy steht; und R^{5b} für Wasserstoff, Halogen, CN, C₁-C₃-Halogenalkyl, C₃-C₄-Cycloalkyl, C₁-C₃-Alkoxy oder C₁-C₃-Halogenalkoxy steht.

12. Zusammensetzung, umfassend eine wie in einem der Ansprüche 1 bis 11 definierte Verbindung, einen oder mehrere Hilfsstoffe und Verdünnungsmittel und gegebenenfalls einen oder mehrere andere Wirkstoffe.

13. Verfahren
(i) zur Bekämpfung und Kontrolle von Insekten, Akarina, Nematoden oder Mollusken, bei dem man auf einen Schädling, auf einen Standort eines Schädlings oder auf eine Pflanze, die gegenüber Schädlingsbefall anfällig ist, eine insektizid, akarizid, nematizid bzw. molluskizid wirksame Menge einer wie in einem der Ansprüche 1 bis 11 definierten Verbindung oder einer wie in Anspruch 12 definierten Zusammensetzung ausbringt, wobei Verfahren zur chirurgischen oder therapeutischen Behandlung des menschlichen oder tierischen Körpers ausgeschlossen sind; oder
(ii) zum Schutz von Pflanzenfortpflanzungsmaterial gegen Befall durch Insekten, Akarina, Nematoden oder Mollusken, bei dem man das Fortpflanzungsmaterial oder die Stelle, an der das Fortpflanzungsmaterial gepflanzt ist, mit einer wirksamen Menge einer wie in einem der Ansprüche 1 bis 11 definierten Verbindung oder einer wie in Anspruch 12 definierten Zusammensetzung behandelt; oder
(iii) zur Kontrolle von Parasiten in oder auf einem Tier, bei dem diesbezüglicher Bedarf besteht, bei dem man eine wirksame Menge einer wie in einem der Ansprüche 1 bis 11 definierten Verbindung oder einer wie in Anspruch 12 definierten Zusammensetzung verabreicht, wobei Verfahren zur chirurgischen oder therapeutischen Behandlung des menschlichen oder tierischen Körpers ausgeschlossen sind.

14. Pflanzenfortpflanzungsmaterial, wie ein Samen, das eine wie in einem der Ansprüche 1 bis 11 definierte Verbindung oder eine wie in Anspruch 12 definierte Zusammensetzung umfasst oder an dem eine wie in einem der Ansprüche 1 bis 11 definierte Verbindung oder eine wie in Anspruch 12 definierte Zusammensetzung haftet.

## Revendications

1. Composé de formule (I) 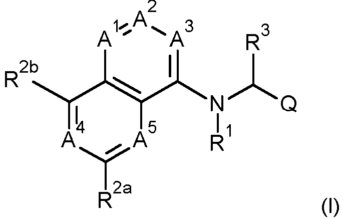
A¹, A² et A³ étant, indépendamment les uns des autres, N ou CR^{Y} ; ou
A¹=A²-A³, pris ensemble, étant NR-C(=X⁰)-N ; X⁰ étant O ou S ;
A⁴ et A⁵ étant, indépendamment l'un de l'autre, N ou CR^{Y} ;
Q étant 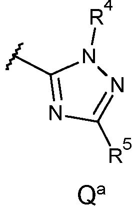 ou 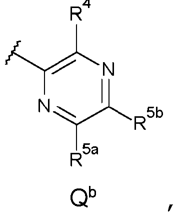 où la ligne décalée représente la connexion de Q au reste du composé de la formule (I) ;
R étant hydrogène, C₁-C₃alkyle, C₁-C₃halogénoalkyle, C₁-C₃alcoxy ou C₁-C₃halogénoalcoxy ;
R¹ étant hydrogène, C₁-C₆alkyle, C₁-C₆cyanoalkyle, aminocarbonylC₁-C₆alkyle, hydroxycarbonylC₁-C₆alkyle, C₁-C₆nitroalkyle, triméthylsilaneC₁-C₆alkyle, C₁-C₃alcoxy-C₁-C₆alkyle, C₁-C₆halogénoalkyle, C₂-C₆alcényle, C₂-C₆halogénoalcényle, C₂-C₆alcynyle, C₂-C₆halogénoalcynyle, C₃-C₄cycloalkylC₁-C₂alkyle-, C₃-C₄cycloalkylC₁-C₂alkyle-, le groupe C₃-C₄cycloalkyle étant substitué par 1 ou 2 atomes d'halogène, oxétan-3-yl-CH₂-, C₁-C₆alkylcarbonyle, C₁-C₆alcoxycarbonyle, phényloxycarbonyle, benzyloxycarbonyle, benzyle ou benzyle substitué par 1 à 3 substituants indépendamment choisis parmi halogène, C₁-C₆alcoxy et C₁-C₆halogénoalkyle ;
R^{2a} et R^{2b} étant chacun indépendamment choisis parmi hydrogène, C₁-C₃alkyle, C₁-C₃halogénoalkyle, C₁-C₃halogénoalkylsulfanyle, C₁-C₃alcoxy, C₁-C₃halogénoalcoxy, halogène, NO₂, SF₅, CN, C(O)NH₂, C(O)OH, C(S)NH₂, C₃-C₆cycloalkyle, C₃-C₆cycloalkyle substitué par un à trois substituants indépendamment choisis parmi R^{x}, C₃-C₆cycloalkylcarbonyle, phényle, phényle substitué par un à trois substituants indépendamment choisis parmi R^{x}, hétéroaryle, hétéroaryle substitué par un à trois substituants indépendamment choisis parmi R^{x}, OR⁶, pipéridin-2-one-1-yle, pipéridin-2-one-1-yle substitué par un à deux substituants indépendamment choisis parmi R^{x}, pyridin-2-one-1-yle, pyridin-2-one-1-yle substitué par un à deux substituants indépendamment choisis parmi R^{x}, azétidin-1-yle, azétidin-1-yle substitué par un à deux substituants indépendamment choisis parmi R^{x}, pyrrolidin-1-yle, pyrrolidin-1-yle substitué par un à deux substituants indépendamment choisis parmi R^{x}, C₃-C₆cycloalkylC₁-C₄alkyle, C₃-C₆cycloalkylC₁-C₄alkyle substitué par un à deux substituants indépendamment choisis parmi R^{z}, C₃-C₆cycloalkylC₁-C₃alcoxy, C₃-C₆cycloalkylC₁-C₃alcoxy substitué par un à deux substituants indépendamment choisis parmi R^{x}, C₁-C₅cyanoalkyle, C₁-C₃cyanoalcoxy, C₁-C₄alkylsulfanyle, C₁-C₄alkylsulfanyle substitué par un à trois substituants indépendamment choisis parmi R^{x}, C₁-C₄alkylsulfonyle, C₁-C₄alkylsulfonyle substitué par un à trois substituants indépendamment choisis parmi R^{x}, C₁-C₄alkylsulfinyle, et C₁-C₄alkylsulfinyle substitué par un à trois substituants indépendamment choisis parmi R^{x} ;
R³ étant C₁-C₃alkyle ou C₁-C₃halogénoalkyle ;
R⁴ étant 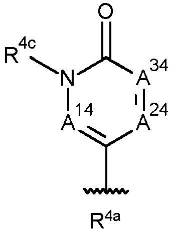 ou 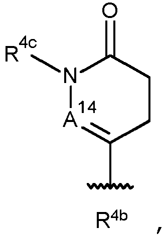 où la ligne décalée représente la connexion de R⁴ à Q^{a} ou Q^{b} ;
A¹⁴, A²⁴, et A³⁴ étant, indépendamment les uns des autres, N ou CH ;
R^{4c} étant C₁-C₃alkyle, C₁-C₃halogénoalkyle, allyle, propargyle, ou C₃-C₆cycloalkylC₁-C₄alkyle ;
R⁵ étant hydrogène, C₁-C₃alkyle, C₁-C₃halogénoalkyle, C₃-C₄cycloalkyle, C₁-C₃alcoxy, C₃-C₄alcoxyC(O)-, (C₁-C₃alcoxy)₂CH-, halogène, CN, NH₂C(O), amino (c'est-à-dire NH₂), (C₁-C₃alkyl) amino, di(C₁-C₃alkyl)amino, hydroxy, C₃-C₄halogénocycloalkyle, C₃-C₄cyanocycloalkyle, C₂-C₆alcényle, C₂-C₆halogénoalcényle, C₂-C₆alcynyle, C₂-C₆halogénoalcynyle, C₁-C₄halogénoalkylsulfanyle, C₁-C₄halogénoalkylsulfinyle, C₁-C₄halogénoalkylsulfonyle, C₁-C₄alkylsulfanyle, C₁-C₄alkylsulfinyle, C₁-C₄alkylsulfonyle, C₁-C₃alcoxy-C₁-C₃alkyle, C₁-C₃alcoxy-C₁-C₃alcoxy-C₁-C₃alkyle, (C₁-C₃alkyl) sulfonylamino, (C₁-C₃alkyl)sulfonyl(C₁-C₃alkyl)amino, (C₁-C₃alkyl)NHC(O), (C₁-C₃alkyl)₂NC(O), (C₁-C₃cycloalkyl)NHC(O), (C₁-C₃cycloalkyl)(C₁-C₃alkyl)NC(O), (C₁-C₃alkyl) C (O)(C₁-C₃alkyl)N, (C₁-C₃alkyl)C(O)NH, (C₁-C₃alkyl)C(O), (C₁-C₃alcoxy)C(O), HC(O), diphénylméthanimine, C₁-C₃halogénoalcoxy, phényle, ou un cycle hétéroaromatique à 5 chaînons ; ou
R⁵ étant phényle substitué par un à trois substituants choisis parmi C₁-C₃alkyle, C₁-C₃halogénoalkyle, C₁-C₃alcoxy, C₃-C₄cycloalkyle, halogène, CN et hydroxyle ; ou
R⁵ étant un cycle hétéroaromatique à 5 chaînons substitué par un à trois substituants choisis parmi C₁-C₃alkyle, C₁-C₃halogénoalkyle, C₁-C₃alcoxy, C₃-C₄cycloalkyle, halogène, CN et hydroxyle ;
R^{5a} et R^{5b} étant, indépendamment les uns des autres, choisis parmi hydrogène, halogène, CN, C₁-C₃alkyle, C₁-C₃halogénoalkyle, C₃-C₄cycloalkyle, C₁-C₃alcoxy, et C₁-C₃halogénoalcoxy ;
R⁶ étant phényle, benzyle, hétéroaryle, ou C₃-C₆ cycloalkyle ; ou
R⁶ étant phényle, benzyle, hétéroaryle, ou C₃-C₆ cycloalkyle, chacun desquels, indépendamment les uns des autres, étant substitué par un à trois substituants indépendamment choisis parmi R^{x} ;
R^{x} étant indépendamment choisi parmi halogène, C₁-C₃alkyle, C₁-C₃halogénoalkyle, C₁-C₃alcoxy, C₁-C₃halogénoalcoxy, NO₂, SF₅, CN, C(O)NH₂, C(S)NH₂, C₁-C₄halogénoalkylsulfanyle, C₁-C₄halogénoalkylsulfinyle, C₁-C₄halogénoalkylsulfonyle, C₁-C₄alkylsulfanyle, C₁-C₄alkylsulfinyle et C₁-C₄alkylsulfonyle ;
R^{Y} étant choisi parmi hydrogène, C₁-C₃ alkyle, C₁-C₃ halogénoalkyle, hydroxy, C₁-C₃ alcoxy, C₁-C₃ halogénoalcoxy, halogène, CN et cyclopropyle ; et
R^{z} étant choisi parmi oxo, halogène, C₁-C₃ alkyle, C₁-C₃halogénoalkyle, C₁-C₃alcoxy, C₁-C₃halogénoalcoxy et CN ; ou sel, stéréoisomère, énantiomère, forme tautomère et N-oxyde acceptable sur le plan agrochimique du composé de formule I.

2. Composé selon la revendication 1, A¹ et A³ étant N, et A² étant CR^{Y} ; et R^{Y} étant hydrogène, chlore, méthyle, trifluorométhyle, ou méthoxy ; ou
A¹=A²-A³, pris ensemble, étant NR-C(=O)-N ; et R étant hydrogène, méthyle, éthyle, 2,2-difluoroéthyle, ou 2,2,2,-trifluoroéthyle.

3. Composé selon la revendication 1 ou la revendication 2, A⁴ et A⁵ étant, indépendamment l'un de l'autre, N ou CH ; ou
A⁴ et A⁵ étant tous deux CH.

4. Composé selon l'une quelconque des revendications 1 à 3,
- lorsque R⁴ est R^{4a}: A¹⁴ est N, A²⁴ est N ou CH, et A³⁴ est N ou CH ; ou A¹⁴ est N, A²⁴ est N ou CH, et A³⁴ est CH ; ou A¹⁴ est N, A²⁴ est CH, et A³⁴ est N ou CH ; ou A¹⁴ est N, A²⁴ est CH, et A³⁴ est CH ; et
- lorsque R⁴ est R^{4b}: A¹⁴ est N ; ou A¹⁴ est CH ;
et R^{4c} étant méthyle, éthyle, propyle, difluorométhyle, trifluorométhyle, 2,2-difluoroéthyle, 2,2,2-trifluoroéthyle, allyle, propargyle, cyclopropylméthyle, cyclobutylméthyle, 2-cyclpropyléthyle, ou 3-cyclopropylpropyle.

5. Composé selon l'une quelconque des revendications 1 à 4,
- A¹⁴ étant N, et, lorsque R⁴ est R^{4a}, A²⁴ et A³⁴ sont CH ; et
- R^{4c} étant méthyle, ou éthyle.

6. Composé selon l'une quelconque des revendications 1 à 5, X⁰ étant O.

7. Composé selon l'une quelconque des revendications 1 à 6, R¹ étant hydrogène, méthyle, éthyle, cyanométhyle, méthoxyméthyle, cyclopropyl-méthyle, allyle, propargyle, benzyloxycarbonyle, ou benzyle.

8. Composé selon l'une quelconque des revendications 1 à 7, R^{2a} étant halogène, C₁-C₃halogénoalkyle, C₁-C₃halogénoalkylsulfanyle, C₁-C₃halogénoalcoxy, C₃-C₆cycloalkyle éventuellement substitué par un ou deux substituants indépendamment choisis parmi C₁-C₃halogénoalkyle, cyano et halogène, C₃-C₆cycloalkylC₁-C₄alkyle éventuellement substitué par un à trois substituants indépendamment choisis parmi C₁-C₃halogénoalkyle, cyano et halogène, C₁-C₃cyanoalkyle, C₁-C₄alkylsulfonyle, C₁-C₄halogénoalkylsulfonyle, C₁-C₄alkylsulfinyle, C₁-C₄halogénoalkylsulfinyle, C₃-C₆cycloalkylsulfanyle, C₃-C₆cycloalkylsulfinyle, ou C₃-C₆cycloalkylsulfonyle ; et
R^{2b} étant halogène, C₁-C₃halogénoalkyle, C₁-C₃halogénoalkylsulfanyle, C₁-C₃halogénoalkylsulfonyle, C₁-C₃alcoxy, C₁-C₃halogénoalcoxy, ou CN.

9. Composé selon l'une quelconque des revendications 1 à 8, R^{2a} et R^{2b} étant indépendamment choisis parmi chloro, bromo, iodo, et trifluorométhyle.

10. Composé selon l'une quelconque des revendications 1 à 9, R³ étant méthyle.

11. Composé selon l'une quelconque des revendications 1 à 10,
- lorsque Q est Q^{a}: R⁵ est hydrogène, méthyle, trifluorométhoxy, méthoxy, cyclopropyle, 2,2-difluoroéthoxy, 2,2,2-trifluoroéthoxy, difluorométhoxy, 2,2,2-trifluoroéthyle, chloro, bromo, méthoxyéthoxy, méthylcarbonyle ou méthoxycarbonyle ; et
- lorsque Q est Q^{b}: R^{5a} est hydrogène, halogène, CN, C₁-C₃alkyle, C₁-C₃halogénoalkyle, C₃-C₄cycloalkyle, C₁-C₃alcoxy ou C₁-C₃halogénoalcoxy ; et R^{5b} est hydrogène, halogène, CN, C₁-C₃halogénoalkyle, C₃-C₄cycloalkyle, C₁-C₃alcoxy ou C₁-C₃halogénoalcoxy.

12. Composition comprenant un composé tel que défini dans l'une quelconque revendication 1 à 11, un ou plusieurs adjuvants et diluants, et facultativement un ou plusieurs autres principes actifs.

13. Procédé
(i) de lutte contre et de régulation d'insectes, d'acariens, de nématodes ou de mollusques qui comprend une application sur un organisme nuisible, sur un site d'un organisme nuisible ou sur un végétal susceptible d'être attaqué par un organisme nuisible, d'une quantité efficace sur le plan insecticide, sur le plan acaricide, sur le plan nématicide ou sur le plan molluscicide d'un composé tel que défini dans l'une quelconque des revendications 1 à 11 ou d'une composition telle que définie dans la revendication 12, à l'exclusion de procédés pour le traitement du corps humain ou animal par chirurgie ou thérapie ; ou
(ii) pour la protection d'un matériel de propagation végétale contre l'attaque par des insectes, des acariens, des nématodes ou des mollusques, qui comprend le traitement du matériel de propagation ou le site, où le matériel de propagation est planté, avec une quantité efficace d'un composé tel que défini dans l'une quelconque des revendications 1 à 11 ou d'une composition telle que définie dans la revendication 12 ; ou
(iii) de lutte contre des parasites sur un animal qui en a besoin comprenant une administration d'une quantité efficace d'un composé tel que défini dans l'une quelconque des revendications 1 à 11 ou d'une composition telle que définie dans la revendication 12 ; à l'exclusion de procédés pour le traitement du corps humain ou animal par chirurgie ou thérapie.

14. Matériel de propagation végétale, tel qu'une graine, comprenant, ou collé(e) sur celle-ci, un composé tel que défini dans l'une quelconque des revendications 1 à 11 ou une composition telle que définie dans la revendication 12.
